# EUROPEAN PATENT APPLICATION

(11) **EP 2 385 134 A1**
(43) Date of publication of application: **09.11.2011**
(21) Application number: 10004898.2
(22) Date of filing: 07.05.2010
(51) Int. Cl.: C12Q 1/68

(54) **Risk prognosis method for chronic lymphocytic leukemia**

(71) Applicant: Ludwig-Maximilians-Universität München, 80359 München (DE)
(72) Inventor: Herold, Tobias, 82346 Andechs (DE); Jurinovic, Vindi, 85521 Ottobrunn (DE); Buske, Christian, 89075 Ulm (DE); Bohlander, Stefan, 82131 Gauting (DE)
(74) Representative: Vossius & Partner

(57) **Abstract**

The present invention relates to a kit consisting of or comprising nucleic acid molecules specific for the expression product of at least two genes,selected from the group consisting of (i) NRIP1; (ii) SFTPB; (iii) PLEKHA1; (iv) PDE8A; (v) TCF7; (vi) MGC29506; (vii) MSI2; and (viii) one gene selected from the group consisting of MGAT4A, NELL2, BCL11B, CD247, SORL1, KLRB1, IL7R, RUNX2, MAL, RASGRF2, TIAM1, PRKCH, FNIP2, GIMAP7, PAG1, ITK, IL2RB, SATB1, PRKCQ, TRAT1, CLEC4A, wherein each of the nucleic acid molecules has a length of at least 8 nucleotides. The present invention further relates to a kit consisting of or comprising nucleic acid molecules specific for the expression product of at least one reference gene and nucleic acid molecules specific for the expression product of at least two genes selected from the group consisting of (i) NRIP1; (ii) SFTPB; (iii) PLEKHA1; (iv) PDE8A; (v) TCF7; (vi) MGC29506; (vii) MSI2; and (viii) one gene selected from the group consisting of MGAT4A, NELL2, BCL11B, CD247, SORL1, KLRB1, IL7R, RUNX2, MAL, RASGRF2, TIAM1, PRKCH, FNIP2, GIMAP7, PAG1, ITK, IL2RB, SATB1, PRKCQ, TRAT1, CLEC4A, wherein each of the nucleic acid molecules has a length of at least 8 nucleotides. The present invention further relates to a method of diagnosing a patient suffering from chronic lymphocytic leukemia as being a poor prognosis or good prognosis patient.

SEQUENCE LISTING

<110>  Ludwig-Maximilians-Universität München

<120>  Risk prognosis method for chronic lymphocytic leukemia

<130>  R3183 EP

<160>  57

<170>  PatentIn version 3.3

<210>  1
<211>  7556
<212>  DNA
<213>  Homo sapiens

<400>  1
gcaggcgcct tcgcggaccg agcctgacgg agccggaggc tgggagccgc ggcggcctgg      60

ggaagtgttt ggattgtgag ctatttcaga actgttctca ggactcatta ttttaacatt     120

tgggagaaac acagccagaa gatgcacact tgactgaagg aggacaggga atctgaagac     180

tccggatgac atcagagcta cttttcaaca gccttctcaa ttttctttct cagaaagcag     240

aggctcagag cttggagaca gacgaacact gatatttgca tttaatgggg aacaaaagat     300

gaagaaggaa aaggaatata ttcactaagg attctatctg cttactgcta cagacctatg     360

tgttaaggaa ttcttctcct cctccttgcg tagaagttga tcagcactgt ggtcagactg     420

catttatctt gtcattgcca gaagaaatct tggacagaat gtaacagtac gtctctctct     480

gattgcgatg gaaggtgata aactgatact cctttattaa agttacatcg cactcaccac     540

agaaaaccat tctttaaagt gaatagaaac caagcccttg tgaacacttc tattgaacat     600

gactcatgga gaagagcttg gctctgatgt gcaccaggat tctattgttt taacttacct     660

agaaggatta ctaatgcatc aggcagcagg gggatcaggt actgccgttg acaaaaagtc     720

tgctgggcat aatgaagagg atcagaactt taacatttct ggcagtgcat ttcccacctg     780

tcaaagtaat ggtccagttc tcaatacaca tacatatcag gggtctggca tgctgcacct     840

caaaaaagcc agactgttgc agtcttctga ggactggaat gcagcaaagc ggaagaggct     900

gtctgattct atcatgaatt taaacgtaaa gaaggaagct ttgctagctg gcatggttga     960

cagtgtgcct aaaggcaaac aggatagcac attactggcc tctttgcttc agtcattcag    1020

ctctaggctg cagactgttg ctctgtcaca acaaatcagg cagagcctca aggagcaagg    1080

atatgccctc agtcatgatt ctttaaaagt ggagaaggat ttaaggtgct atggtgttgc    1140

atcaagtcac ttaaaaactt tgttgaagaa aagtaaagtt aaagatcaaa agcctgatac    1200

gaatcttcct gatgtgacta aaaacctcat cagagatagg tttgcagagt ctcctcatca    1260

tgttggacaa agtggaacaa aggtcatgag tgaaccgttg tcatgtgctg caagattaca    1320

ggctgttgca agcatggtgg aaaaaagggc tagtcctgcc acctcaccta aacctagtgt    1380

tgcttgtagc cagttagcat tacttctgtc aagcgaagcc catttgcagc agtattctcg    1440

agaacacgct ttaaaaacgc aaaatgcaaa tcaagcagca agtgaaagac ttgctgctat    1500

ggccagattg caagaaaatg gccagaagga tgttggcagt taccagctcc caaaaggaat 1560
gtcaagccat cttaatggtc aggcaagaac atcatcaagc aaactgatgg ctagcaaaag 1620
tagtgctaca gtgtttcaaa atccaatggg tatcattcct tcttccccta aaaatgcagg 1680
ttataagaac tcactggaaa gaaacaatat aaaacaagct gctaacaata gtttgctttt 1740
acatcttctt aaaagccaga ctatacctaa gccaatgaat ggacacagtc acagtgagag 1800
aggaagcatt tttgaggaaa gtagtacacc tacaactatt gatgaatatt cagataacaa 1860
tcctagtttt acagatgaca gcagtggtga tgaaagttct tattccaact gtgttcccat 1920
agacttgtct tgcaaacacc gaactgaaaa atcagaatct gaccaacctg tttccctgga 1980
taacttcact caatccttgc taaacacttg ggatccaaaa gtcccagatg tagatatcaa 2040
agaagatcaa gatacctcaa agaattctaa gctaaactca caccagaaag taacacttct 2100
tcaattgcta cttggccata agaatgaaga aaatgtagaa aaaaacacca gccctcaggg 2160
agtacacaat gatgtgagca agttcaatac acaaaattat gcaaggactt ctgtgataga 2220
aagccccagt acaaatcgga ctactccagt gagcactcca cctttactta catcaagcaa 2280
agcagggtct cccatcaatc tctctcaaca ctctctggtc atcaaatgga attccccacc 2340
atatgtctgc agtactcagt ctgaaaagct aacaaatact gcatctaacc actcaatgga 2400
ccttacaaaa agcaaagacc caccaggaga gaaaccagcc caaaatgaag gtgcacagaa 2460
ctctgcaacg tttagtgcca gtaagctgtt acaaaattta gcacaatgtg gaatgcagtc 2520
atccatgtca gtggaagagc agagacccag caaacagctg ttaactggaa acacagataa 2580
accgataggt atgattgata gattaaatag ccctttgctc tcaaataaaa caaatgcagt 2640
tgaagaaaat aaagcattta gtagtcaacc aacaggtcct gaaccagggc tttctggttc 2700
tgaaatagaa aatctgcttg aaagacgtac tgtcctccag ttgctcctgg ggaaccccaa 2760
caaagggaag agtgaaaaaa aagagaaaac tcccttaaga gatgaaagta ctcaggaaca 2820
ctcagagaga gctttaagtg aacaaatact gatggtgaaa ataaaatctg agccttgtga 2880
tgacttacaa attcctaaca caaatgtgca cttgagccat gatgctaaga gtgccccatt 2940
cttgggtatg gctcctgctg tgcagagaag cgcacctgcc ttaccagtgt ccgaagactt 3000
taaatcggag cctgtttcac ctcaggattt ttctttctcc aagaatggtc tgctaagtcg 3060
attgctaaga caaaatcaag atagttacct ggcagatgat tcagacagga gtcacagaaa 3120
taatgaaatg gcacttctag aatcaaagaa tctttgcatg gtccctaaga aaaggaagct 3180
ttatactgag ccattagaaa atccatttaa aaagatgaaa aacaacattg ttgatgctgc 3240
aaacaatcac agtgccccag aagtactgta tgggtccttg cttaaccagg aagagctgaa 3300
atttagcaga aatgatcttg aatttaaata tcctgctggt catggctcag ccagcgaaag 3360
tgaacacagg agttgggcca gagagagcaa aagctttaat gttctgaaac agctgcttct 3420
ctcagaaaac tgtgtgcgag atttgtcccc gcacagaagt aactctgtgg ctgacagtaa 3480
aaagaaagga cacaaaaata atgtgaccaa cagcaaacct gaatttagca tttcttcttt 3540

aaatggactg atgtacagtt ccactcagcc cagcagttgc atggataaca ggacattttc 3600
atacccaggt gtagtaaaaa ctcctgtgag tcctactttc cctgagcact tgggctgtgc 3660
agggtctaga ccagaatctg ggcttttgaa tgggtgttcc atgcccagtg agaaaggacc 3720
cattaagtgg gttatcactg atgcggagaa gaatgagtat gaaaaagact ctccaagatt 3780
gaccaaaacc aacccaatac tatattacat gcttcaaaaa ggaggcaatt ctgttaccag 3840
tcgagaaaca caagacaagg acatttggag ggaggcttca tctgctgaaa gtgtctcaca 3900
ggtcacagcc aaagaagagt tacttcctac tgcagaaacg aaagcttctt tctttaattt 3960
aagaagccct tacaatagcc atatgggaaa taatgcttct cgcccacaca gcgcaaatgg 4020
agaagtttat ggacttctgg gaagcgtgct aacgataaag aaagaatcag aataaaatgt 4080
acctgccatc cagttttgga tctttttaaa actaatgagt atgaacttga gatctgtata 4140
aataagagca tgatttgaaa aaaagcatgg tataattgaa acttttttca ttttgaaaag 4200
tattggttac tggtgatgtt gaaatatgca tactaatttt tgcttaacat tagatgtcat 4260
gaggaaacta ctgaactagc aattggttgt ttaacacttc tgtatgcatc agataacaac 4320
tgtgagtagc ctatgaatga aattctttta taaatattag gcataaatta aaatgtaaaa 4380
ctccattcat agtggattaa tgcattttgc tgcctttatt agggtacttt attttgcttt 4440
tcagaagtca gcctacataa cacattttta aagtctaaac tgttaaacaa ctctttaaag 4500
gataattatc caataaaaaa aaacctagtg ctgattcaca gcttattatc caattcaaaa 4560
ataaattaga aaaatatatg cttacatttt tcacttttgc taaaaagaaa aaaaaaaggt 4620
gtttattttt aactcttgga agaggttttg tggttcccaa tgtgtctgtc ccaccctgat 4680
ccttttcaat atatatttct ttaaaccttg tgctacttag taaaaattga ttacaattga 4740
gggaagtttg atagatcctt taaaaaaaag gcagatttcc attttttgta ttttaactac 4800
tttactaaat taatactcct ccttttacag aattagaaaa gttaacattt atctttaggt 4860
ggtttcctga aaagttgaat atttaagaaa ttgtttttaa cagaagcaaa atggcttttc 4920
tttggacagt tttcaccatc tcttgtaaaa gttaattctc accattcctg tggtacctgc 4980
gagtgttatg accaggattc cttaaacctg aactcagacc acttgcatta gaaccatctg 5040
gagcacttgt tttaaaatgc agattcatag gcagcatctc agatctacag aacaagaatc 5100
tctgctaagt ggacctggaa tcttccatct gcatcttaac atgctctcta ggtgtttctt 5160
gtgtttgaga accatgactt atgactttcc tcagaacatg agactgtaaa acaaaaacaa 5220
aaaactatgt gatgcctcta ttttccccaa tacagtcaca catcagctca aaatttgcaa 5280
tattgtagtt catatattac cgttatgtct ttggaaatcg ggttcagaac actttttatg 5340
acaaaaattg ggtggagggg ataactttca tatctggctc aacatctcag gaaaatctgt 5400
gattatttgt gtgttctaat gagtaacatc tacttagtta gccttaggga tggaaaaaca 5460
gggccactta ccaaactcag gtgattccag gatggtttgg aaacttctcc tgaatgcatc 5520
cttaaccttt attaaaacca ttgtcctaag aacaatgcca acaaagctta caacatttag 5580

tttaaaccca agaagggcac taaactcaga ttgactaaat aaaaagtaca aagggcacat 5640
atacgtgaca gaattgtaca caatcactcc attggatctt ttactttaaa gtagtgatga 5700
aaagtacatg ttgatactgt cttagaagaa attaatatat tagtgaagcc acatggggtt 5760
tcagttgcga aacaggtctg tttttatgtt cagtttgtac aatccacaat tcattcacca 5820
gatattttgt tcttaattgt gaaccaggtt agcaaatgac ctatcaaaaa ttattctata 5880
atcactacta gttaggatat tgatttaaaa ttgttctact tgaagtggtt tctaagattt 5940
ttatattaaa aataggtgtg atttcctaat atgatctaaa accctaaatg gttatttttc 6000
ctcagaatga tttgtaaata gctactggaa atattataca gtaataggag tgggtattat 6060
gcaacatcat ggagaagtga aggcataggc ttattctgac ataaaattcc actggccagt 6120
tgaatatatt ctattccatg tccatactat gacaatctta ttgtcaacac tatataaata 6180
agcttttaaa caagtcattt ttcttgatcg ttgtggaagg tttggagcct tagaggtatg 6240
tcagaaaaaa tatgttggta ttctcccttg ggtaggggga aatgaccttt ttacaagaga 6300
gtgaaattta ggtcagggaa aagaccaagg gccagcattg ctacttttgt gtgtgtgtgt 6360
gtgggttttg ttttgttttt ttggttggct ggttgttttc gttgttgtta acaaaggaat 6420
gagaatatgt aatacttaaa taaacatgac cacgaagaat gctgttctga tttactagag 6480
aatgttccca atttgaattt agggtgattt taaagaacag tgagaaaggg catacatcca 6540
cagattcact ttgtttatgc atatgtagat acaaggatgc acatatacac attttcaagg 6600
actattttag atatctagac aatttcttct aataaagtca tttgtgaaag ggtactacag 6660
cttattgaca tcagtaaggt agcattcatt acctgtttat tctctgctgc atcttacaga 6720
agagtaaact ggtgagagta tatattttat atatatatat atatatatat atataatatg 6780
tatatatata tatattgact tgttacatga agatgttaaa atcggttttt aaaggtgatg 6840
taaatagtga tttccttaat gaaaaataca tattttgtat tgttctaatg caacagaaaa 6900
gccttttaat ctctttggtt cctgtatatt ccatgtataa gtgtaaatat aatcagacag 6960
gtttaaaagt tgtgcatgta tgtatacagt tgcaagtctg gacaaatgta tagaataaac 7020
cttttattta agttgtgatt acctgctgca tgaaaagtgc atgggggacc ctgtgcatct 7080
gtgcatttgg caaaatgtct taacaaatca gatcagatgt tcatcctaac atgacagtat 7140
tccatttctg gacatgacgt ctgtggttta agctttgtga aagaatgtgc tttgattcga 7200
agggtcttaa agaatttttt taatcgtcaa ccacttttaa acataaagaa ttcacacaac 7260
tactttcatg aattttttaa tcccattgca aacattattc caagagtatc ccagtattag 7320
caatactgga atataggcac attaccattc atagtaagaa ttctggtgtt tacacaacca 7380
aatttgatgc gatctgctca gtaatataat ttgccatttt tattagaaat ttaatttctt 7440
catgtgatgt catgaaactg tacatactgc agtgtgaatt tttttgtttt gtttttaat 7500
cttttagtgt ttacttcctg cagtgaattt gaataaatga gaaaaaatgc attgtc 7556

<210> 2

<211>  2724
<212>  DNA
<213>  Homo sapiens

<400>  2
ggtgccatgg ctgagtcaca cctgctgcag tggctgctgc tgctgctgcc cacgctctgt      60

ggcccaggca ctgctgcctg gaccacctca tccttggcct gtgcccaggg ccctgagttc     120

tggtgccaaa gcctggagca agcattgcag tgcagagccc tagggcattg cctacaggaa     180

gtctggggac atgtgggagc cgatgaccta tgccaagagt gtgaggacat cgtccacatc     240

cttaacaaga tggccaagga ggccattttc caggacacga tgaggaagtt cctggagcag     300

gagtgcaacg tcctcccctt gaagctgctc atgccccagt gcaaccaagt gcttgacgac     360

tacttccccc tggtcatcga ctacttccag aaccagactg actcaaacgg catctgtatg     420

cacctgggcc tgtgcaaatc ccggcagcca gagccagagc aggagccagg gatgtcagac     480

cccctgccca aacctctgcg ggaccctctg ccagaccctc tgctggacaa gctcgtcctc     540

cctgtgctgc ccggggccct ccaggcgagg cctgggcctc acacacagga tctctccgag     600

cagcaattcc ccattcctct cccctattgc tggctctgca gggctctgat caagcggatc     660

caagccatga ttcccaaggg tgcgctagct gtggcagtgg cccaggtgtg ccgcgtggta     720

cctctggtgg cgggcggcat ctgccagtgc ctggctgagc gctactccgt catcctgctc     780

gacacgctgc tgggccgcat gctgccccag ctggtctgcc gcctcgtcct ccggtgctcc     840

atggatgaca gcgctggccc aaggtcgccg acaggagaat ggctgccgcg agactctgag     900

tgccacctct gcatgtccgt gaccacccag gccgggaaca gcagcgagca ggccatacca     960

caggcaatgc tccaggcctg tgttggctcc tggctggaca gggaaaagtg caagcaattt    1020

gtggagcagc acacgcccca gctgctgacc ctggtgccca ggggctggga tgcccacacc    1080

acctgccagg ccctcggggt gtgtgggacc atgtccagcc ctctccagtg tatccacagc    1140

cccgaccttt gatgagaact cagctgtcca gaaaaagaca ccgtccttta aagtgctgca    1200

gtatggccag acgtggtggc tcacacctgc aatcccagca ccttaggagg ccgaggcagg    1260

aggatccttg aggtcaggag ttcgagacca gcctcgccaa catggtgaaa ccccatttct    1320

actaaaaata caaaaaatta gccaagtgtg gtggcatatg cctgtaatcc caactactca    1380

gaaggccgag gcaggagaat tacttgaacg caggagaatc actgcagccc aggaggcaga    1440

ggttgcagtg agccgagatt gcaccactgc actccagcct gggtgacaga gcaagactcc    1500

atctcagtaa ataaataaat aaataaaaag cgctgcagta gctgtggcct caccctgaag    1560

tcagcgggcc caggcctacc tcactctctc ccttggcaga gaagcagacg tccatagctc    1620

ctctccctca caagcgctcc cagcctgccc tccagctgct gctctcccct cccagtctct    1680

actcactggg atgaggttag gtcatgagga caccaaaaac ctaaaaataa acaaaaagcc    1740

aaacaagcct tagcttttct taaagactga aatgcctgga agtgtccctt tatttataaa    1800

ataacttttg tcatatttct tatacatgtt tcttgtaaga aattcagaaa ctacagacaa    1860

agagagtgga aattacccac tgtcaggcct ctgagcccaa gctaagccat catatcccct    1920

gtgccctgca cgtatacacc cagatggcct gaagcaactg aagatccaca aaagaagtga 1980

aaatagccag ttcctgcctt aactgatgac attccaccat tgtgatttgt tcctgcccca 2040

ccctaactga tcaattgacc ttgtgacaat acaccttccc cacccttgag aaggtgcttt 2100

gtaatattct ccccacccac cccacgcccg cacccccgca cccttaagaa ggtattttgt 2160

aatattctct ccgccattga gaatgtgctt tgtaagatcc accccctgcc cacaaaaaat 2220

tgctcctaac tccaccgcct atcccaaacc tacaagaact aatgataatc ccaccaccct 2280

ttgctgactc tttttggact cagcccacct gcacccaggt gattaaaaag ctttattgtt 2340

cacacaaagc ctgtttggta gtctcttcac agggaagcat gtgacaccca caatcccacc 2400

tagcccagga gagagctacg gcagggtgtg tgttttgaca ctgagcttgg ggctttttcc 2460

atcttctccc cacagcctct ggctccacac ctccaccgtt caagcgccag aaagagctgt 2520

ctatgcagcc tgctcttggg cctggggatg agacacacaa ttcattggct cctggatttt 2580

aagtagacat ttgtaaatct atagctaact actgtcctta aagccattgt ttccattaca 2640

aaatccaact ctctgagaga aaagggtgtt ttaaatttaa aaaaataaaa acaaaaaagt 2700

ttgattgaga aaaaaaaaaa aaaa 2724

<210> 3
<211> 2026
<212> DNA
<213> Homo sapiens

<400> 3
gccatggctg agtcacacct gctgcagtgg ctgctgctgc tgctgcccac gctctgtggc 60

ccaggcactg ctgcctggac cacctcatcc ttggcctgtg cccagggccc tgagttctgg 120

tgccaaagcc tggagcaagc attgcagtgc agagccctag ggcattgcct acaggaagtc 180

tggggacatg tgggagccga tgacctatgc caagagtgtg aggacatcgt ccacatcctt 240

aacaagatgg ccaaggaggc cattttccag gacacgatga ggaagttcct ggagcaggag 300

tgcaacgtcc tccccttgaa gctgctcatg ccccagtgca accaagtgct tgacgactac 360

ttccccctgg tcatcgacta cttccagaac cagactgact caaacggcat ctgtatgcac 420

ctgggcctgt gcaaatcccg gcagccagag ccagagcagg agccagggat gtcagacccc 480

ctgcccaaac ctctgcggga ccctctgcca gaccctctgc tggacaagct cgtcctccct 540

gtgctgcccg gggccctcca ggcgaggcct gggcctcaca cacaggatct ctccgagcag 600

caattcccca ttcctctccc ctattgctgg ctctgcaggg ctctgatcaa gcggatccaa 660

gccatgattc ccaagggtgc gctagctgtg gcagtggccc aggtgtgccg cgtggtacct 720

ctggtggcgg gcggcatctg ccagtgcctg gctgagcgct actccgtcat cctgctcgac 780

acgctgctgg gccgcatgct gccccagctg gtctgccgcc tcgtcctccg gtgctccatg 840

gatgacagcg ctggcccaag gtcgccgaca ggagaatggc tgccgcgaga ctctgagtgc 900

cacctctgca tgtccgtgac cacccaggcc gggaacagca gcgagcaggc cataccacag 960

gcaatgctcc aggcctgtgt tggctcctgg ctggacaggg aaaagtgcaa gcaatttgtg 1020

gagcagcaca cgccccagct gctgaccctg gtgcccaggg gctgggatgc ccacaccacc 1080

tgccaggccc tcggggtgtg tgggaccatg tccagccctc tccagtgtat ccacagcccc 1140

gacctttgat gagaactcag ctgtccagct gcaaaggaaa agccaagtga gacgggctct 1200

gggaccatgg tgaccaggct cttcccctgc tccctggccc tcgccagctg ccaggctgaa 1260

aagaagcctc agctcccaca ccgccctcct caccgccctt cctcggcagt cacttccact 1320

ggtggaccac gggcccccag ccctgtgtcg gccttgtctg tctcagctca accacagtct 1380

gacaccagag cccacttcca tcctctctgg tgtgaggcac agcgagggca gcatctggag 1440

gagctctgca gcctccacac ctaccacgac ctcccagggc tgggctcagg aaaaaccagc 1500

cactgcttta caggacaggg ggttgaagct gagccccgcc tcacacccac ccccatgcac 1560

tcaaagattg gatttacag ctacttgcaa ttcaaaattc agaagaataa aaaatgggaa 1620

catacagaac tctaaaagat agacatcaga aattgttaag ttaagctttt tcaaaaaatc 1680

agcaattccc cagcgtagtc aagggtggac actgcacgct ctggcatgat gggatggcga 1740

ccgggcaagc tttcttcctc gagatgctct gctgcttgag agctattgct ttgttaagat 1800

ataaaaaggg gtttcttttt gtctttctgt aaggtggact tccagctttt gattgaaagt 1860

cctagggtga ttctatttct gctgtgattt atctgctgaa agctcagctg gggttgtgca 1920

agctagggac ccattcctgt gtaatacaat gtctgcacca atgctaataa agtcctattc 1980

tcttttatga aaaaaaaaaa aaaaaaaaaa aaaaaaaaaa aaaaaa 2026

<210> 4
<211> 3750
<212> DNA
<213> Homo sapiens

<400> 4
gtgcgcagtg cgcgggctgg ccgtcgcgga cgccgctccg ggcagccgag cctctgtggg 60

agccggggcc gcggcggcgc gggtgctccg ggccgaggcc gcgtgtaatg ttcaagctca 120

gaaatgcctt atgtggatcg tcagaatcgc atttgtggtt ttctagacat tgaagaaaat 180

gaaaacagtg ggaaatttct tcgaaggtac ttcatactgg ataccagaga agatagtttc 240

gtgtggtaca tggataatcc acagaaccta ccttctggat catcacgtgt tggagccatt 300

aagcttacct acatttcaaa ggttagcgat gctactaagc taaggccaaa ggcggagttc 360

tgttttgtta tgaatgcagg aatgaggaag tacttcctac aagccaatga tcagcaggac 420

ctagtggaat gggtaaatgt gttaaacaaa gctataaaaa ttacagtacc aaagcagtca 480

gactcacagc ctaattctga taacctaagt cgccatggtg aatgtgggaa aaagcaagtg 540

tcttacagaa ctgatattgt tggtggcgta cccatcatta ctcccactca gaaagaagaa 600

gtaaatgaat gtggtgaaag tattgacaga aataatctga aacggtcaca aagccatctt 660

ccttacttta ctcctaaacc acctcaagat agtgcggtta tcaaagctgg atattgtgta 720

aaacaaggag cagtgatgaa aaactggaag agaagatatt ttcaattgga tgaaaacaca 780

ataggctact tcaaatctga actggaaaag gaacctcttc gtgtaatacc acttaaagag 840
gttcataaag tccaggaatg taagcaaagc gacataatga tgagggacaa cctctttgaa 900
attgtaacaa cgtctcgaac tttctatgtg caggctgata gccctgaaga gatgcacagt 960
tggattaaag cagtctctgg cgccattgta gcacagcggg gtcccggcag atctgcgtct 1020
tctgagcatc cccccggtcc ttcagaatcc aaacacgctt tccgtcctac caacgcagcc 1080
accgccacct cacattccac agcctctcgc agcaactctt tggtctcaac ctttaccatg 1140
gagaagcgag gattttacga gtctcttgcc aaggtcaagc cagggaactt caaggtccag 1200
actgtctctc caagagaacc agcttccaaa gtgactgaac aagctctgtt aagacctcaa 1260
agtaaaaatg gccctcagga aaaagattgt gacctagtag acttggacga tgcgagcctt 1320
ccggtcagtg acgtgtgagg cagaagcgca cggagcctgc ctgcctctgc cgtcctcagt 1380
ttcctttcat gaggcttcta gccaaagatg ataaaggggg aaatggtttt tagtgcgtat 1440
attatactgc ctcttaggtg tactctttat aagctggtaa accaagaatc tagggagtgg 1500
ccaaactaaa tataatttct ttaaaaaaga aagaaaaagg aaaaatccaa aatatctcag 1560
tatcatctgt ctgaagcatt gtgtgttctc attcgggtgg taacaatgta tgtgtaatat 1620
tttttttcta gtgattttga cagtttaaat gtttaacaac ttaaagcatt aaaaatgctt 1680
attaataact ttggtcattt aaaaaatgct acaatgactt cctattaaag gttcaatatt 1740
tacacattct tattggttga tattaccaca tgaaatattg ccagaccaag atacctaaac 1800
tcatgatgtc tgtggtgctg taaaatttat actaaaatgt ggactatttt gaaattataa 1860
ccatttttga tgccctgaat cttgaggtga cttattttgc atagaggata tggatagacc 1920
aacagtaagg gttgtgggtt ataccggcat agagaaaaga agaaaactag aattgaaaca 1980
actgtgtctt agacatttgt ttgaaaaagc ttcatcaggc cttggagcag tcactgcttt 2040
attccgcaaa aattatttgg taggaaattt ttgggagatc tgattttctt atccaagttt 2100
tgtaaatagt tgttatttct atatttggat tggtgaaaga cctcaagttt atatgtaaag 2160
acataactgc ccttagtcat gaaattgttg tgacctctac tttttgtcac taatagccta 2220
cattcaagtg cctgtgtttt ttcatcttgt ttaggaatgt tttgagatta atgtgcttaa 2280
aagtcctacg tgactgattt taaacattgt gataaaatta attttcagta gaatagttga 2340
atgtgttaag ataggatttt atgttagaga taccagaatg ctggtattct actacttgtg 2400
caatatatat gttttaaaaa acaaatttga gaatacattt aatcataggg atatagatat 2460
aagcacctct ctaaagaatc tttagtttcc gacgttgaat tatagaccag tttgagtaag 2520
tactgctttt tttttttgga gacgggctct cgctctgttg ctcaggctgg agtgcagtgg 2580
cgtgatctca gctcactaca gcctccacct cgtgggttca aacagttgtg ctgcctcagc 2640
ttcccgagta gctaggatta caggtgcatg ccaccacacc tggctaattt ttgtatttta 2700
gtagagatgg ggtttcacca tgttggccag gctcgtctcg aactcctgac ttcaggtgat 2760
ccacccatct cagcctccca aagtgctggg attacaggcg tgagccacca tgcccggccc 2820

ataagtaccg tttttgaggt tcagtcttaa acatttgctt taagaaaaca gtcttgaatt 2880

tcacatgctg ctatttttat attttgccat tttacagtac tgttttgttt tgaattcatg 2940

catatcattg aaaatttctc gttttcattt tcttagatga cttcttgtct gagacagaaa 3000

aatttcctac tacagcagtg cagtccagag gttaagatgt attagaatta tacaatatca 3060

gtttaaaaat ctgtatgcat aaagaatgca ccactcaact tttttattca taagctaata 3120

tttttttaaa gttacattaa gattttttct cttttgcagc tacatttgaa agtgatagaa 3180

taaagagatt ttaatgagtt atcacttttt cagctgatat attcatttta atggcttttt 3240

tgaaagttcc tttttcatga acacacccga gaaatcttaa atagacactt tgcaatattt 3300

aagaacctaa tgctgtttaa ttttggtaca gcttccacat tgcatgttca ctttagtatt 3360

tgcaatttga tatatttcat ggtggcaaaa tattagctct gttttgggac attttaaaat 3420

agaactatcc ttgttcgata gcataggaaa atgttctggt gattgtcagg gtctcctaat 3480

atttatctca attcttttat aagtctatgg aaattattta attattttaa aacgtacaca 3540

cttttcttgt aaatatgtca catctgagtt caaaaaaatt actttgaata ccttaatatt 3600

tgctgcattt ttttccgtat atataacatg tcttctttca gaatgggaat atatgtgtgc 3660

ctcccaacat ttactgttaa agtgtgttat ctttatatgt caaactggtt gaacactgta 3720

atgagaataa actgcacaga gtttattctg 3750

<210> 5
<211> 3693
<212> DNA
<213> Homo sapiens

<400> 5
tttggtgcag tttagcatgt tcctctgtgt tctgcatctc ctgtagtgta atgttcaagc 60

tcagaaatgc cttatgtgga tcgtcagaat cgcatttgtg gttttctaga cattgaagaa 120

aatgaaaaca gtgggaaatt tcttcgaagg tacttcatac tggataccag agaagatagt 180

ttcgtgtggt acatggataa tccacagaac ctaccttctg gatcatcacg tgttggagcc 240

attaagctta cctacatttc aaaggttagc gatgctacta agctaaggcc aaaggcggag 300

ttctgttttg ttatgaatgc aggaatgagg aagtacttcc tacaagccaa tgatcagcag 360

gacctagtgg aatgggtaaa tgtgttaaac aaagctataa aaattacagt accaaagcag 420

tcagactcac agcctaattc tgataaccta agtcgccatg gtgaatgtgg gaaaaagcaa 480

gtgtcttaca gaactgatat tgttggtggc gtacccatca ttactcccac tcagaaagaa 540

gaagtaaatg aatgtggtga aagtattgac agaaataatc tgaaacggtc acaaagccat 600

cttccttact ttactcctaa accacctcaa gatagtgcgg ttatcaaagc tggatattgt 660

gtaaaacaag gagcagtgat gaaaaactgg aagagaagat attttcaatt ggatgaaaac 720

acaataggct acttcaaatc tgaactggaa aaggaacctc ttcgtgtaat accacttaaa 780

gaggttcata aagtccagga atgtaagcaa agcgacataa tgatgaggga caacctcttt 840

gaaattgtaa caacgtctcg aactttctat gtgcaggctg atagccctga agagatgcac 900
agttggatta aagcagtctc tggcgccatt gtagcacagc ggggtcccgg cagatctgcg 960
tcttctgagc atccccccgg tccttcagaa tccaaacacg ctttccgtcc taccaacgca 1020
gccaccgcca cctcacattc cacagcctct cgcagcaact ctttggtctc aacctttacc 1080
atggagaagc gaggatttta cgagtctctt gccaaggtca agccagggaa cttcaaggtc 1140
cagactgtct ctccaagaga accagcttcc aaagtgactg aacaagctct gttaagacct 1200
caaagtaaaa atggccctca ggaaaaagat tgtgacctag tagacttgga cgatgcgagc 1260
cttccggtca gtgacgtgtg aggcagaagc gcacggagcc tgcctgcctc tgccgtcctc 1320
agtttccttt catgaggctt ctagccaaag atgataaagg gggaaatggt ttttagtgcg 1380
tatattatac tgcctcttag gtgtactctt tataagctgg taaaccaaga atctagggag 1440
tggccaaact aaatataatt tctttaaaaa agaaagaaaa aggaaaaatc caaaatatct 1500
cagtatcatc tgtctgaagc attgtgtgtt ctcattcggg tggtaacaat gtatgtgtaa 1560
tatttttttc ttagtgattt tgacagttta aatgtttaac aacttaaagc attaaaaatg 1620
cttattaata actttggtca tttaaaaaat gctacaatga cttcctatta aaggttcaat 1680
atttacacat tcttattggt tgatattacc acatgaaata ttgccagacc aagataccta 1740
aactcatgat gtctgtggtg ctgtaaaatt tatactaaaa tgtggactat tttgaaatta 1800
taaccatttt tgatgccctg aatcttgagg tgacttattt tgcatagagg atatggatag 1860
accaacagta agggttgtgg gttataccgg catagagaaa agaagaaaac tagaattgaa 1920
acaactgtgt cttagacatt tgtttgaaaa agcttcatca ggccttggag cagtcactgc 1980
tttattccgc aaaaattatt tggtaggaaa tttttgggag atctgatttt cttatccaag 2040
ttttgtaaat agttgttatt tctatatttg gattggtgaa agacctcaag tttatatgta 2100
aagacataac tgcccttagt catgaaattg ttgtgacctc tactttttgt cactaatagc 2160
ctacattcaa gtgcctgtgt tttttcatct tgtttaggaa tgttttgaga ttaatgtgct 2220
taaaagtcct acgtgactga ttttaaacat tgtgataaaa ttaattttca gtagaatagt 2280
tgaatgtgtt aagataggat tttatgttag agataccaga atgctggtat tctactactt 2340
gtgcaatata tatgttttaa aaaacaaatt tgagaataca tttaatcata gggatataga 2400
tataagcacc tctctaaaga atctttagtt tccgacgttg aattatagac cagtttgagt 2460
aagtactgct tttttttttt ggagacgggc tctcgctctg ttgctcaggc tggagtgcag 2520
tggcgtgatc tcagctcact acagcctcca cctcgtgggt tcaaacagtt gtgctgcctc 2580
agcttcccga gtagctagga ttacaggtgc atgccaccac acctggctaa tttttgtatt 2640
ttagtagaga tggggtttca ccatgttggc caggctcgtc tcgaactcct gacttcaggt 2700
gatccaccca tctcagcctc ccaaagtgct gggattacag gcgtgagcca ccatgcccgg 2760
cccataagta ccgtttttga ggttcagtct taaacatttg ctttaagaaa acagtcttga 2820
atttcacatg ctgctatttt tatattttgc cattttacag tactgttttg ttttgaattc 2880

atgcatatca ttgaaaattt ctcgttttca ttttcttaga tgacttcttg tctgagacag 2940

aaaaatttcc tactacagca gtgcagtcca gaggttaaga tgtattagaa ttatacaata 3000

tcagtttaaa aatctgtatg cataaagaat gcaccactca acttttttat tcataagcta 3060

atattttttt aaagttacat taagattttt tctcttttgc agctacattt gaaagtgata 3120

gaataaagag attttaatga gttatcactt tttcagctga tatattcatt ttaatggctt 3180

ttttgaaagt tcctttttca tgaacacacc cgagaaatct taaatagaca ctttgcaata 3240

tttaagaacc taatgctgtt taattttggt acagcttcca cattgcatgt tcactttagt 3300

atttgcaatt tgatatattt catggtggca aaatattagc tctgttttgg gacattttaa 3360

aatagaacta tccttgttcg atagcatagg aaaatgttct ggtgattgtc agggtctcct 3420

aatatttatc tcaattcttt tataagtcta tggaaattat ttaattattt taaaacgtac 3480

acacttttct tgtaaatatg tcacatctga gttcaaaaaa attactttga ataccttaat 3540

atttgctgca tttttttccg tatatataac atgtcttctt tcagaatggg aatatatgtg 3600

tgcctcccaa catttactgt taaagtgtgt tatctttata tgtcaaactg gttgaacact 3660

gtaatgagaa taaactgcac agagtttatt ctg 3693

<210> 6
<211> 3931
<212> DNA
<213> Homo sapiens

<400> 6
ccgccgccgc cgcagcgccc gcaccgcgat aaaaggggcg gccgcgtttc ctgacgcgag 60

atccgcgctc gccgccgccc gcccaggcgg cgatgacacg gcgcccgcgg cggcccggag 120

gcgccgggtg ggccgtttgc tgaccggatc gcggctaccc gccagcgtgt ccgcggcgcc 180

gccgccagca tgggctgtgc cccgagcatc cacatttccg agcgcctggt ggccgaggac 240

gcgcctagcc ccgcggcacc gccgctgtcg tccggcgggc cgcgcctccc gcagggccag 300

aagacggccg ccttgccccg gacccgcggc gccggcctct tggagtcgga gcttcgcgac 360

ggcagcggca agaaggtagc agtagctgat gtgcagtttg gccccatgag atttcatcaa 420

gatcaacttc aggtactttt agtgtttacc aaagaagata accaatgtaa tggattctgc 480

agggcatgtg aaaaagcagg gtttaagtgt acagttacca aggaggctca ggctgtcctt 540

gcctgtttcc tggacaaaca tcatgacatt atcatcatag accacagaaa tcctcgacag 600

ctggatgcag aggcactgtg caggtctatc agatcatcaa aactctcaga aaacacagtt 660

attgttggtg tagtacgcag ggtggataga gaagagttgt ccgtaatgcc tttcatttct 720

gctggattta caaggaggta tgtagaaaac cccaacatca tggcctgcta caatgaactg 780

ctccagctgg agtttggaga ggtgcgatca caactgaaac tcagggcttg taactcagta 840

ttcactgcat tagaaaacag tgaagatgca attgaaatta caagcgaaga ccgttttata 900

cagtatgcaa atcctgcatt tgaaacaaca atgggctatc agtcaggtga attaataggg 960

aaggagttag gagaagtgcc tataaatgaa aaaaaggctg acttgctcga tactataaat 1020

tcatgcatca ggataggcaa ggagtggcaa ggaatttact atgccaaaaa gaaaaacgga 1080
gataatatac aacaaaatgt gaagataata cctgtcattg gacagggagg aaaaattaga 1140
cactatgtgt ccattatcag agtgtgcaat ggcaacaata aggctgagaa aatatccgaa 1200
tgtgttcagt ctgacactca tacagataat cagacaggca aacataaaga caggagaaaa 1260
ggctcactag acgtcaaagc tgttgcctcc cgtgcaactg aagtttccag ccagagacga 1320
cactcttcca tggcccggat acattccatg acaattgagg cgcccatcac caaggtaatc 1380
aatattatca atgctgccca ggaaagtagt cccatgcctg tgacagaagc cctagaccgt 1440
gtgctggaaa ttctaagaac cactgagtta tattcaccac agtttggtgc taaagatgat 1500
gatccccatg ccaatgacct tgttgggggc ttaatgtctg atggtttgcg aagactatca 1560
gggaatgaat atgttctttc aacaaaaaac actcaaatgg tttcaagcaa tataatcact 1620
cccatctccc ttgatgatgt cccaccacgg atagctcggg ccatggaaaa tgaggaatac 1680
tgggactttg atatttttga actggaggct gccacccaca ataggccttt gatttatctt 1740
ggtctcaaaa tgtttgctcg ctttggaatc tgtgaattct tacactgctc cgagtcaacg 1800
ctaagatcat ggttacaaat tatcgaagcc aattatcatt cctccaatcc ctaccacaat 1860
tctacacatt ctgctgatgt gcttcatgcc actgcctatt ttctctccaa ggagaggata 1920
aaggaaactt tagatccaat tgatgaggtc gctgcactca tcgcagccac cattcatgat 1980
gtggatcacc ctgggagaac caactccttc ctgtgtaatg ctggaagtga gctggccatt 2040
ttgtacaatg acactgctgt gctggagagc caccatgcgg ccttggcctt ccagctgacc 2100
actggagatg ataaatgcaa tatatttaaa aacatggaga ggaatgatta tcggacactg 2160
cgccagggga ttatcgacat ggtcttagcc acagaaatga caaagcactt tgagcatgtc 2220
aacaaatttg tcaacagcat caacaaaccc ttggcaacac tagaagaaaa tggggaaact 2280
gataaaaacc aggaagtgat aaacactatg cttaggactc cagagaaccg gaccctaatc 2340
aaacgaatgc tgattaaatg tgctgatgtg tccaatccct gccgacccct gcagtactgc 2400
atcgagtggg ctgcacgcat ttcggaagaa tatttttctc agactgatga agagaagcag 2460
cagggcttac ctgtggtgat gccagtgttt gacagaaata cctgcagcat ccccaaatcc 2520
caaatctctt tcattgatta cttcatcaca gacatgtttg atgcttggga tgcctttgta 2580
gacctgcctg atttaatgca gcatcttgac aacaacttta aatactggaa aggactggac 2640
gaaatgaagc tgcggaacct ccgaccacct cctgaatagt gggagacacc acccagagcc 2700
ctgaagcttt gttccttcgg tcatttggaa ttcctgaggg cagccagagc tccttggtcc 2760
tttcagtact aggcagaaca gcccccgatc tgcatagcct gtgaaagccc acggggacat 2820
cagtaacctt ctgcagccac catccaatgc cattactgtc aagtgagact tggccactgt 2880
agcctgggcc tgctgcagga gctcttcaga aaggcacatg aggaccacgg tttgcctcag 2940
tttctggtaa aacacaaggt ctggagtgcc cctgcaaagg gtattgatgg acttcctgcc 3000
agtgacagag catgtctatt gcaaacaatt ctctcagtta cgttcagcac ttaagaacgg 3060

ctaatggcaa taggatcttt agcaactttt tcacatcata gaaggtgcaa tcgctcactt 3120
gggaacacta ctgagagtga cttctctttt aaaattgagt agcagatgaa aaattaaaat 3180
ttgaacttga ttattaatat caattaaaat gttttattta ttttattaaa agctcaatat 3240
tttctatgaa ttcaaaaata cttcagagcc aaagccaact tcaaataccg tgaccaaatt 3300
tacatgattc atattcatta tgcattactt ggtatacaga cttattttca taatgcaaat 3360
taataaaatg acacttttac tgcactatag aaatattcat gtatgttaaa cttttctgat 3420
tgaggctaac tggaaaaagc tggggtcgta ttctaagtgc taaagaaggc tgcttctact 3480
gtatagaacc cagggctctg aaacagctct agccgcctaa tgcacttcac aggtaactcc 3540
ccaaggtaaa actagactct cttgttggtt cgcaaagaaa agttaggact taacactttt 3600
ttctaaaatt ttataattca atttccaaaa gtctactcta ttttatactg tttctacaaa 3660
atattcctta taaaaacaaa gaacaaaaat tgaatattta atgaattgac attttataac 3720
caacctgttt ttatctacgg tgggaatctt tgatgccaga aatttataaa gaggttctgt 3780
atcttcacac cttgaataag cataatacca taaaaaatga cacttgacat gtcaatgtat 3840
ttgtcatttc attttaaact cgtatttgtg gtttttttcc cagataaaaa tgaaattaaa 3900
ccatttcttt ttaagaaaaa aaaaaaaaaa a 3931

<210> 7
<211> 3793
<212> DNA
<213> Homo sapiens

<400> 7
ccgccgccgc cgcagcgccc gcaccgcgat aaaaggggcg gccgcgtttc ctgacgcgag 60
atccgcgctc gccgccgccc gcccaggcgg cgatgacacg gcgcccgcgg cggcccggag 120
gcgccgggtg ggccgtttgc tgaccggatc gcggctaccc gccagcgtgt ccgcggcgcc 180
gccgccagca tgggctgtgc cccgagcatc cacatttccg agcgcctggt ggccgaggac 240
gcgcctagcc ccgcggcacc gccgctgtcg tccggcgggc cgcgcctccc gcagggccag 300
aagacggccg ccttgccccg gacccgcggc gccggcctct tggagtcgga gcttcgcgac 360
ggcagcggca agaaggtagc agtagctgat gtgcagtttg gccccatgag atttcatcaa 420
gatcaacttc aggtactttt agtgtttacc aaagaagata accaatgtaa tggattctgc 480
agggcatgtg aaaaagcagg gtttaagtgt acagttacca aggaggctca ggctgtcctt 540
gcctgtttcc tggacaaaca tcatgacatt atcatcatag accacagaaa tcctcgacag 600
ctggatgcag aggcactgtg caggtctatc agatcatcaa aactctcaga aaacacagtt 660
attgttggtg tagtacgcag ggtggataga gaagagttgt ccgtaatgcc tttcatttct 720
gctggattta caaggaggta tgtagaaaac cccaacatca tggcctgcta caatgaactg 780
ctccagctgg agtttggaga ggtgcgatca caactgaaac tcagggcttg taactcagta 840
ttcactgcat tagaaaacag tgaagatgca attgaaatta caagcgaaga ccgttttata 900

caggagtggc aaggaattta ctatgccaaa aagaaaaacg gagataatat acaacaaaat 960
gtgaagataa tacctgtcat tggacaggga ggaaaaatta gacactatgt gtccattatc 1020
agagtgtgca atggcaacaa taaggctgag aaaatatccg aatgtgttca gtctgacact 1080
catacagata atcagacagg caaacataaa gacaggagaa aaggctcact agacgtcaaa 1140
gctgttgcct cccgtgcaac tgaagtttcc agccagagac gacactcttc catggcccgg 1200
atacattcca tgacaattga ggcgcccatc accaaggtaa tcaatattat caatgctgcc 1260
caggaaagta gtcccatgcc tgtgacagaa gccctagacc gtgtgctgga aattctaaga 1320
accactgagt tatattcacc acagtttggt gctaaagatg atgatcccca tgccaatgac 1380
cttgttgggg gcttaatgtc tgatggtttg cgaagactat cagggaatga atatgttctt 1440
tcaacaaaaa acactcaaat ggtttcaagc aatataatca ctcccatctc ccttgatgat 1500
gtcccaccac ggatagctcg ggccatggaa aatgaggaat actgggactt tgatattttt 1560
gaactggagg ctgccaccca caataggcct ttgatttatc ttggtctcaa aatgtttgct 1620
cgctttggaa tctgtgaatt cttacactgc tccgagtcaa cgctaagatc atggttacaa 1680
attatcgaag ccaattatca ttcctccaat ccctaccaca attctacaca ttctgctgat 1740
gtgcttcatg ccactgccta ttttctctcc aaggagagga taaaggaaac tttagatcca 1800
attgatgagg tcgctgcact catcgcagcc accattcatg atgtggatca ccctgggaga 1860
accaactcct tcctgtgtaa tgctggaagt gagctggcca ttttgtacaa tgacactgct 1920
gtgctggaga gccaccatgc ggccttggcc ttccagctga ccactggaga tgataaatgc 1980
aatatattta aaaacatgga gaggaatgat tatcggacac tgcgccaggg gattatcgac 2040
atggtcttag ccacagaaat gacaaagcac tttgagcatg tcaacaaatt tgtcaacagc 2100
atcaacaaac ccttggcaac actagaagaa aatggggaaa ctgataaaaa ccaggaagtg 2160
ataaacacta tgcttaggac tccagagaac cggaccctaa tcaaacgaat gctgattaaa 2220
tgtgctgatg tgtccaatcc ctgccgaccc ctgcagtact gcatcgagtg ggctgcacgc 2280
atttcggaag aatatttttc tcagactgat gaagagaagc agcagggctt acctgtggtg 2340
atgccagtgt ttgacagaaa tacctgcagc atccccaaat cccaaatctc tttcattgat 2400
tacttcatca cagacatgtt tgatgcttgg gatgcctttg tagacctgcc tgatttaatg 2460
cagcatcttg acaacaactt taaatactgg aaaggactgg acgaaatgaa gctgcggaac 2520
ctccgaccac ctcctgaata gtgggagaca ccacccagag ccctgaagct ttgttccttc 2580
ggtcatttgg aattcctgag ggcagccaga gctccttggt cctttcagta ctaggcagaa 2640
cagcccccga tctgcatagc ctgtgaaagc ccacggggac atcagtaacc ttctgcagcc 2700
accatccaat gccattactg tcaagtgaga cttggccact gtagcctggg cctgctgcag 2760
gagctcttca gaaaggcaca tgaggaccac ggtttgcctc agtttctggt aaaacacaag 2820
gtctggagtg cccctgcaaa gggtattgat ggacttcctg ccagtgacag agcatgtcta 2880
ttgcaaacaa ttctctcagt tacgttcagc acttaagaac ggctaatggc aataggatct 2940

ttagcaactt tttcacatca tagaaggtgc aatcgctcac ttgggaacac tactgagagt 3000
gacttctctt ttaaaattga gtagcagatg aaaaattaaa atttgaactt gattattaat 3060
atcaattaaa atgttttatt tattttatta aaagctcaat attttctatg aattcaaaaa 3120
tacttcagag ccaaagccaa cttcaaatac cgtgaccaaa tttacatgat tcatattcat 3180
tatgcattac ttggtataca gacttatttt cataatgcaa attaataaaa tgacactttt 3240
actgcactat agaaatattc atgtatgtta aacttttctg attgaggcta actggaaaaa 3300
gctggggtcg tattctaagt gctaaagaag gctgcttcta ctgtatagaa cccagggctc 3360
tgaaacagct ctagccgcct aatgcacttc acaggtaact ccccaaggta aaactagact 3420
ctcttgttgg ttcgcaaaga aaagttagga cttaacactt ttttctaaaa ttttataatt 3480
caatttccaa aagtctactc tattttatac tgtttctaca aaatattcct tataaaaaca 3540
aagaacaaaa attgaatatt taatgaattg acattttata accaacctgt ttttatctac 3600
ggtgggaatc tttgatgcca gaaatttata aagaggttct gtatcttcac accttgaata 3660
agcataatac cataaaaaat gacacttgac atgtcaatgt atttgtcatt tcattttaaa 3720
ctcgtatttg tggttttttt cccagataaa aatgaaatta aaccatttct ttttaagaaa 3780
aaaaaaaaaa aaa 3793

<210> 8
<211> 3277
<212> DNA
<213> Homo sapiens

<400> 8
cccgccagct cgcggagccg ctctgccccg cgccctagcc cgcgcctgca gcccgcccag 60
gcggagtcag cccgcgctcc gcccgccgcg atccgagctc ggaggttcgg actccgggct 120
cgccgccccc cgggccggct ccgcgccccg cactcccggc gcccagcgcc ccgcgccccg 180
gcgggcggag cgcaccatgc cgcagctgga ctccggcggg ggcggcgcgg gcggcggcga 240
cgacctcggc gcgccggacg agctgctggc cttccaggat gaaggcgagg agcaggacga 300
caagagccgc gacagcgccg ccggtcccga gcgcgacctg gccgagctca agtcgtcgct 360
cgtgaacgag tccgagggcg cggccggcgg cgcagggatc ccgggggtcc cgggggccgg 420
cgccggggcc cgcggcgagg ccgaggctct cgggcgggaa cacgctgcgc agagactctt 480
cccggacaaa cttccagagc ccctggagga cggcctgaag gccccggagt gcaccagcgg 540
catgtacaaa gagaccgtct actccgcctt caatctgctc atgcattacc cacccccctc 600
gggagcaggg cagcaccccc agccgcagcc cccgctgcac aaggccaatc agccccccca 660
cggtgtcccc caactctctc tctacgaaca tttcaacagc ccacatccca cccctgcacc 720
tgcggacatc agccagaagc aagttcacag gcctctgcag acccctgacc tctctggctt 780
ctactccctg acctcaggca gcatggggca gctcccccac actgtgagct ggttcaccca 840
cccatccttg atgctaggtt ctggtgtacc tggtcaccca gcagccatcc cccacccggc 900
cattgtgccc ccctcaggga agcaggagct gcagcccttc gaccgcaacc tgaagacaca 960

agcagagtcc aaggcagaga aggaggccaa gaagccaacc atcaagaagc ccctcaatgc 1020
cttcatgctg tacatgaagg agatgagagc caaggtcatt gcagagtgca cacttaagga 1080
gagcgctgcc atcaaccaga tcctgggccg caggtggcac gcgctgtcgc gagaagagca 1140
ggccaagtac tatgagctgg cccgcaagga gaggcagctg cacatgcagc tatacccagg 1200
ctggtcagcg cgggacaact acgggaagaa gaagaggcgg tcgagggaaa agcaccaaga 1260
atccaccaca ggaggaaaaa gaaatgcatt cggtacttac ccggagaagg ccgctgcccc 1320
agccccgttc cttccgatga cagtgctcta ggctgccccg ggtccccagc tccccaggac 1380
tcaccctcat accatctgct gccccgcttc cccacagaac tgcttactag ccctgcggag 1440
ccggcaccta catccccagg tctctccact gctctcagcc tcccaacccc agggccccca 1500
caggcccccc gcagcaccct gcagagcaca caggtacagc aacaggaatc tcagagacag 1560
gtggcctagc aggcacagga cacctggccg cctccaggag cctaccccct gaaagtgaca 1620
gagacccaga tctcatggaa actggccagg ggtcctgtta acgtcatctc agggtccaga 1680
ccctgaagat ttcagaggct gcaggacttc tgcctgaacc tggggtcatc gattcaaact 1740
gctccaagtg gtgggaatca gatctgtctt gatgtgtcat ctaattaagg gaatcccttg 1800
tacctatggc tgcctgcatc tattctttgt accatctgtc ttgccagcca gaagcctctg 1860
cctccctagc ttttctgcta taggtcagag atgggctgaa ctgagcctag ctaccttctc 1920
tacccatctc ccccatcccc cactgccaca ccctccccat tcagacactt catggaccaa 1980
gaatgagctg gtttgtcaaa caacatgtga gcatggtcac aagcacaaag ctcaagatga 2040
cagctcttct aaggaaatgg agaagctctg tttataaaaa caaaaacaaa accagctgct 2100
actcataagt tggaccagag gaagcccctt actatgatct caggagcttg caagaagcag 2160
gaaggggaat ggaataggtt aagtttaggc ctatcaacct aagcaacaga aataatctga 2220
cactacctta tcaggcaaat tggggagggg agggtgtatc tagctctagt tcaaattatt 2280
tggaagtgtt ccctgagaaa cccaccagcc taagaagctc tggccccagg cttgtcacta 2340
gcagctgcag tcaacagttc aaagaagtca tggcccaaat ccagtgtgca cccctcccca 2400
ttcacagagc ctttttcaca attccatttc cagttcatct atggcagtcc agccagctcc 2460
tgggcagctt gagagggcaa acccaaaacc tcatgacagc cagagcctgt ctttcagcat 2520
tcagtccgcc tggccggctc cagtttcccc atggggctgc gggacagagg accattacaa 2580
ctagatcaag gagcccagaa aacctccagt agtggacaac aggttttcac catagcctac 2640
gttaacccat ttttgagcca agcttcaacc ctcagccttg aaaaacaagt ctttaattta 2700
atttttgttt tttgcctaaa tccaaagaaa aagggctgcc gggccaggcg cggtggctca 2760
cgcctgtaat cccagcactt tggcaggccg aggcaggtgg atcacctgac gtcagtagtt 2820
tgagaccagc ctggccaaca tggtgaaacc ctgtatctac taaaaataca aaaattagcc 2880
ggacgtggtg gtgcgcgcat gtaatcccag ctactcggga ggctgaggcg gaagaatcac 2940
ttgaacccgg gaggcggagg ttccagtgag ccgagatggc gctattgcac tccagtctgg 3000

gtaacaggga gactgcatct caaaaaaaaa aaaaaaaaaa aaaaagggct gtccatgtat 3060
tcacacaccc ctcaaaaaaa gcctttagtt cctactttag ccactggttt ctcagaatcc 3120
aaagatcaca tattctagtg taacactgca agaagtcttg agaaaaagat tattgtagtg 3180
ttcaaaatat ttttgtattg ttaatgcatc atcatagaaa aacttttaaa catgagaata 3240
aagatacttt ttactgggtt tgtttttcaa agcctga 3277

<210> 9
<211> 2911
<212> DNA
<213> Homo sapiens

<400> 9
gcccaggtga ctgactaatc cgccgccttc aggagacaga attggccaag gcctgaaggc 60
cccggagtgc accagcggca tgtacaaaga gaccgtctac tccgccttca atctgctcat 120
gcattaccca cccccctcgg gagcagggca gcacccccag ccgcagcccc cgctgcacaa 180
ggccaatcag cccccccacg gtgtccccca actctctctc tacgaacatt tcaacagccc 240
acatcccacc cctgcacctg cggacatcag ccagaagcaa gttcacaggc ctctgcagac 300
ccctgacctc tctggcttct actccctgac ctcaggcagc atggggcagc tcccccacac 360
tgtgagctgg ttcacccacc catccttgat gctaggttct ggtgtacctg gtcacccagc 420
agccatcccc cacccggcca ttgtgccccc ctcagggaag caggagctgc agcccttcga 480
ccgcaacctg aagacacaag cagagtccaa ggcagagaag gaggccaaga agccaaccat 540
caagaagccc ctcaatgcct tcatgctgta catgaaggag atgagagcca aggtcattgc 600
agagtgcaca cttaaggaga gcgctgccat caaccagatc ctgggccgca ggtggcacgc 660
gctgtcgcga gaagagcagg ccaagtacta tgagctggcc cgcaaggaga ggcagctgca 720
catgcagcta tacccaggct ggtcagcgcg ggacaactac gggaagaaga agaggcggtc 780
gagggaaaag caccaagaat ccaccacaga ccctggctcg cctaagaaat gccgtgctcg 840
ctttggcctc aaccagcaga cggattggtg tggtccgtgc agataactct cttcactatt 900
cctaggagga aaaagaaatg cattcggtac ttacccggag aaggccgctg ccccagcccc 960
gttccttccg atgacagtgc tctaggctgc cccgggtccc cagctcccca ggactcaccc 1020
tcataccatc tgctgccccg cttccccaca gaactgctta ctagccctgc ggagccggca 1080
cctacatccc caggtctctc cactgctctc agcctcccaa ccccagggcc cccacaggcc 1140
ccccgcagca ccctgcagag cacacaggta cagcaacagg aatctcagag acaggtggcc 1200
tagcaggcac aggacacctg gccgcctcca ggagcctacc ccctgaaagt gacagagacc 1260
cagatctcat ggaaactggc caggggtcct gttaacgtca tctcagggtc cagaccctga 1320
agatttcaga ggctgcagga cttctgcctg aacctggggt catcgattca aactgctcca 1380
agtggtggga atcagatctg tcttgatgtg tcatctaatt aagggaatcc cttgtaccta 1440
tggctgcctg catctattct ttgtaccatc tgtcttgcca gccagaagcc tctgcctccc 1500

tagcttttct gctataggtc agagatgggc tgaactgagc ctagctacct tctctaccca 1560
tctcccccat cccccactgc cacaccctcc ccattcagac acttcatgga ccaagaatga 1620
gctggtttgt caaacaacat gtgagcatgg tcacaagcac aaagctcaag atgacagctc 1680
ttctaaggaa atggagaagc tctgtttata aaaacaaaaa caaaaccagc tgctactcat 1740
aagttggacc agaggaagcc ccttactatg atctcaggag cttgcaagaa gcaggaaggg 1800
gaatggaata ggttaagttt aggcctatca acctaagcaa cagaaataat ctgacactac 1860
cttatcaggc aaattgggga ggggagggtg tatctagctc tagttcaaat tatttggaag 1920
tgttccctga gaaacccacc agcctaagaa gctctggccc caggcttgtc actagcagct 1980
gcagtcaaca gttcaaagaa gtcatggccc aaatccagtg tgcacccctc cccattcaca 2040
gagccttttt cacaattcca tttccagttc atctatggca gtccagccag ctcctgggca 2100
gcttgagagg gcaaacccaa aacctcatga cagccagagc ctgtctttca gcattcagtc 2160
cgcctggccg gctccagttt ccccatgggg ctgcgggaca gaggaccatt acaactagat 2220
caaggagccc agaaaacctc cagtagtgga caacaggttt tcaccatagc ctacgttaac 2280
ccatttttga gccaagcttc aaccctcagc cttgaaaaac aagtctttaa tttaattttt 2340
gttttttgcc taaatccaaa gaaaaagggc tgccgggcca ggcgcggtgg ctcacgcctg 2400
taatcccagc actttggcag gccgaggcag gtggatcacc tgacgtcagt agtttgagac 2460
cagcctggcc aacatggtga aaccctgtat ctactaaaaa tacaaaaatt agccggacgt 2520
ggtggtgcgc gcatgtaatc ccagctactc gggaggctga ggcggaagaa tcacttgaac 2580
ccgggaggcg gaggttccag tgagccgaga tggcgctatt gcactccagt ctgggtaaca 2640
gggagactgc atctcaaaaa aaaaaaaaaa aaaaaaaaag ggctgtccat gtattcacac 2700
acccctcaaa aaaagccttt agttcctact ttagccactg gtttctcaga atccaaagat 2760
cacatattct agtgtaacac tgcaagaagt cttgagaaaa agattattgt agtgttcaaa 2820
atatttttgt attgttaatg catcatcata gaaaaacttt taaacatgag aataaagata 2880
ctttttactg ggtttgtttt tcaaagcctg a 2911

<210> 10
<211> 2815
<212> DNA
<213> Homo sapiens

<400> 10
gcccaggtga ctgactaatc cgccgccttc aggagacaga attggccaag gcctgaaggc 60
cccggagtgc accagcggca tgtacaaaga gaccgtctac tccgccttca atctgctcat 120
gcattaccca cccccctcgg gagcagggca gcacccccag ccgcagcccc cgctgcacaa 180
ggccaatcag cccccccacg gtgtccccca actctctctc tacgaacatt tcaacagccc 240
acatcccacc cctgcacctg cggacatcag ccagaagcaa gttcacaggc ctctgcagac 300
ccctgacctc tctggcttct actccctgac ctcaggcagc atggggcagc tcccccacac 360
tgtgagctgg ttcacccacc catccttgat gctaggttct ggtgtacctg gtcacccagc 420

agccatcccc cacccggcca ttgtgccccc ctcagggaag caggagctgc agcccttcga 480
ccgcaacctg aagacacaag cagagtccaa ggcagagaag gaggccaaga agccaaccat 540
caagaagccc ctcaatgcct tcatgctgta catgaaggag atgagagcca aggtcattgc 600
agagtgcaca cttaaggaga gcgctgccat caaccagatc ctgggccgca ggtggcacgc 660
gctgtcgcga gaagagcagg ccaagtacta tgagctggcc cgcaaggaga ggcagctgca 720
catgcagcta tacccaggct ggtcagcgcg ggacaactac gggaagaaga agaggcggtc 780
gagggaaaag caccaagaat ccaccacagg aggaaaaaga aatgcattcg gtacttaccc 840
ggagaaggcc gctgccccag ccccgttcct tccgatgaca gtgctctagg ctgccccggg 900
tccccagctc cccaggactc accctcatac catctgctgc ccgcttcccc cacagaactg 960
cttactagcc ctgcggagcc ggcacctaca tccccaggtc tctccactgc tctcagcctc 1020
ccaaccccag ggcccccaca ggccccccgc agcaccctgc agagcacaca ggtacagcaa 1080
caggaatctc agagacaggt ggcctagcag gcacaggaca cctggccgcc tccaggagcc 1140
taccccctga aagtgacaga gacccagatc tcatggaaac tggccagggg tcctgttaac 1200
gtcatctcag ggtccagacc ctgaagattt cagaggctgc aggacttctg cctgaacctg 1260
gggtcatcga ttcaaactgc tccaagtggt gggaatcaga tctgtcttga tgtgtcatct 1320
aattaaggga atcccttgta cctatggctg cctgcatcta ttctttgtac catctgtctt 1380
gccagccaga agcctctgcc tccctagctt ttctgctata ggtcagagat gggctgaact 1440
gagcctagct accttctcta cccatctccc ccatccccca ctgccacacc ctccccattc 1500
agacacttca tggaccaaga atgagctggt ttgtcaaaca acatgtgagc atggtcacaa 1560
gcacaaagct caagatgaca gctcttctaa ggaaatggag aagctctgtt tataaaaaca 1620
aaaacaaaac cagctgctac tcataagttg gaccagagga agccccttac tatgatctca 1680
ggagcttgca agaagcagga aggggaatgg aataggttaa gtttaggcct atcaacctaa 1740
gcaacagaaa taatctgaca ctaccttatc aggcaaattg gggaggggag ggtgtatcta 1800
gctctagttc aaattatttg gaagtgttcc ctgagaaacc caccagccta agaagctctg 1860
gccccaggct tgtcactagc agctgcagtc aacagttcaa agaagtcatg gcccaaatcc 1920
agtgtgcacc cctccccatt cacagagcct ttttcacaat tccatttcca gttcatctat 1980
ggcagtccag ccagctcctg ggcagcttga gagggcaaac ccaaaacctc atgacagcca 2040
gagcctgtct ttcagcattc agtccgcctg gccggctcca gtttccccat ggggctgcgg 2100
gacagaggac cattacaact agatcaagga gcccagaaaa cctccagtag tggacaacag 2160
gttttcacca tagcctacgt taacccattt ttgagccaag cttcaaccct cagccttgaa 2220
aaacaagtct ttaatttaat ttttgttttt tgcctaaatc caaagaaaaa gggctgccgg 2280
gccaggcgcg gtggctcacg cctgtaatcc cagcactttg gcaggccgag gcaggtggat 2340
cacctgacgt cagtagtttg agaccagcct ggccaacatg gtgaaaccct gtatctacta 2400
aaaatacaaa aattagccgg acgtggtggt gcgcgcatgt aatcccagct actcgggagg 2460

ctgaggcgga agaatcactt gaacccggga ggcggaggtt ccagtgagcc gagatggcgc 2520

tattgcactc cagtctgggt aacagggaga ctgcatctca aaaaaaaaaa aaaaaaaaaa 2580

aaagggctgt ccatgtattc acacacccct caaaaaaagc ctttagttcc tactttagcc 2640

actggtttct cagaatccaa agatcacata ttctagtgta acactgcaag aagtcttgag 2700

aaaaagatta ttgtagtgtt caaaatattt ttgtattgtt aatgcatcat catagaaaaa 2760

cttttaaaca tgagaataaa gatacttttt actgggtttg tttttcaaag cctga 2815

<210> 11
<211> 1254
<212> DNA
<213> Homo sapiens

<400> 11
gcccaggtga ctgactaatc cgccgccttc aggagacaga attggccaag gcctgaaggc 60

cccggagtgc accagcggca tgtacaaaga gaccgtctac tccgccttca atctgctcat 120

gcattaccca cccccctcgg gagcagggca gcacccccag ccgcagcccc cgctgcacaa 180

ggccaatcag cccccccacg gtgtccccca actctctctc tacgaacatt tcaacagccc 240

acatcccacc cctgcacctg cggacatcag ccagaagcaa gttcacaggc ctctgcagac 300

ccctgacctc tctggcttct actccctgac ctcaggcagc atggggcagc tcccccacac 360

tgtgagctgg ttcacccacc catccttgat gctaggttct ggtgtacctg gtcacccagc 420

agccatcccc cacccggcca ttgtgccccc ctcagggaag caggagctgc agcccttcga 480

ccgcaacctg aagacacaag cagagtccaa ggcagagaag gaggccaaga agccaaccat 540

caagaagccc ctcaatgcct tcatgctgta catgaaggag atgagagcca aggtcattgc 600

agagtgcaca cttaaggaga gcgctgccat caaccagatc ctgggccgca ggtggcacgc 660

gctgtcgcga gaagagcagg ccaagtacta tgagctggcc cgcaaggaga ggcagctgca 720

catgcagcta tacccaggct ggtcagcgcg ggacaactac gggaagaaga agaggcggtc 780

gagggaaaag caccaagaat ccaccacaga gacaaactgg cccagagaac tcaaggatgg 840

taatggacaa gagtcactgt ccatgtcttc ttcctctagc ccagcttgag gactgggatg 900

gctgggcaag gaagccatag gcattgcggc cccttgcctt ggtgcagatg tgagtcccac 960

aaacacatct ggagaagctc aaaggccggg actgggagat gactcccttg gaagacagga 1020

gagatgactc ccttggaaga cagatgacag cccataggcc tagtgacaaa aggccccttt 1080

gccaccttgt ggctgttctg ggaactgcac ctgtcctagg tctgggccag accaagcaga 1140

atggcagtct gaggacactg acttaccacc caagtcccag gaagagagga caaggaatca 1200

gccaggcctg tgcaaaggca gcattttttg gttgtggtgt atgactatga attc 1254

<210> 12
<211> 2938
<212> DNA
<213> Homo sapiens

<400> 12
cctccaggtc cttcccctaa aacttggcac tgccgatact cccagcccgt tccttcccaa 60
gtcaggaact tgcaggggac cccttggcaa ttctttttct ctcaagagca gacagccttc 120
agtcccagcc gctgccaggg ctggtgtgtc tgacccagct gtggtttttc caggcctgaa 180
ggccccggag tgcaccagcg gcatgtacaa agagaccgtc tactccgcct tcaatctgct 240
catgcattac ccacccccct cgggagcagg gcagcacccc cagccgcagc ccccgctgca 300
caaggccaat cagccccccc acggtgtccc ccaactctct ctctacgaac atttcaacag 360
cccacatccc acccctgcac ctgcggacat cagccagaag caagttcaca ggcctctgca 420
gacccctgac ctctctggct tctactccct gacctcaggc agcatggggc agctccccca 480
cactgtgagc tggttcaccc acccatcctt gatgctaggt tctggtgtac ctggtcaccc 540
agcagccatc ccccacccgg ccattgtgcc cccctcaggg aagcaggagc tgcagccctt 600
cgaccgcaac ctgaagacac aagcagagtc caaggcagag aaggaggcca agaagccaac 660
catcaagaag cccctcaatg ccttcatgct gtacatgaag gagatgagag ccaaggtcat 720
tgcagagtgc acacttaagg agagcgctgc catcaaccag atcctgggcc gcaggtggca 780
cgcgctgtcg cgagaagagc aggccaagta ctatgagctg gcccgcaagg agaggcagct 840
gcacatgcag ctatacccag gctggtcagc gcgggacaac tacgggaaga agaagaggcg 900
gtcgagggaa aagcaccaag aatccaccac aggaggaaaa agaaatgcat tcggtactta 960
cccggagaag gccgctgccc cagccccgtt ccttccgatg acagtgctct aggctgcccc 1020
gggtccccag ctccccagga ctcaccctca taccatctgc tgccccgctt ccccacagaa 1080
ctgcttacta gccctgcgga gccggcacct acatccccag gtctctccac tgctctcagc 1140
ctcccaaccc cagggccccc acaggccccc cgcagcaccc tgcagagcac acaggtacag 1200
caacaggaat ctcagagaca ggtggcctag caggcacagg acacctggcc gcctccagga 1260
gcctaccccc tgaaagtgac agagacccag atctcatgga aactggccag gggtcctgtt 1320
aacgtcatct cagggtccag accctgaaga tttcagaggc tgcaggactt ctgcctgaac 1380
ctggggtcat cgattcaaac tgctccaagt ggtgggaatc agatctgtct tgatgtgtca 1440
tctaattaag ggaatccctt gtacctatgg ctgcctgcat ctattctttg taccatctgt 1500
cttgccagcc agaagcctct gcctccctag cttttctgct ataggtcaga gatgggctga 1560
actgagccta gctaccttct ctacccatct cccccatccc ccactgccac accctcccca 1620
ttcagacact tcatggacca agaatgagct ggtttgtcaa acaacatgtg agcatggtca 1680
caagcacaaa gctcaagatg acagctcttc taaggaaatg gagaagctct gtttataaaa 1740
acaaaaacaa aaccagctgc tactcataag ttggaccaga ggaagcccct tactatgatc 1800
tcaggagctt gcaagaagca ggaaggggaa tggaataggt taagtttagg cctatcaacc 1860
taagcaacag aaataatctg acactacctt atcaggcaaa ttggggaggg gagggtgtat 1920
ctagctctag ttcaaattat ttggaagtgt tccctgagaa acccaccagc ctaagaagct 1980
ctggccccag gcttgtcact agcagctgca gtcaacagtt caaagaagtc atggcccaaa 2040

tccagtgtgc acccctcccc attcacagag cctttttcac aattccattt ccagttcatc 2100
tatggcagtc cagccagctc ctgggcagct tgagagggca aacccaaaac ctcatgacag 2160
ccagagcctg tctttcagca ttcagtccgc ctggccggct ccagtttccc catggggctg 2220
cgggacagag gaccattaca actagatcaa ggagcccaga aaacctccag tagtggacaa 2280
caggttttca ccatagccta cgttaaccca tttttgagcc aagcttcaac cctcagcctt 2340
gaaaaacaag tctttaattt aatttttgtt ttttgcctaa atccaaagaa aaagggctgc 2400
cgggccaggc gcggtggctc acgcctgtaa tcccagcact ttggcaggcc gaggcaggtg 2460
gatcacctga cgtcagtagt ttgagaccag cctggccaac atggtgaaac cctgtatcta 2520
ctaaaaatac aaaaattagc cggacgtggt ggtgcgcgca tgtaatccca gctactcggg 2580
aggctgaggc ggaagaatca cttgaacccg ggaggcggag gttccagtga gccgagatgg 2640
cgctattgca ctccagtctg ggtaacaggg agactgcatc tcaaaaaaaa aaaaaaaaaa 2700
aaaaaagggc tgtccatgta ttcacacacc cctcaaaaaa agcctttagt tcctacttta 2760
gccactggtt tctcagaatc caaagatcac atattctagt gtaacactgc aagaagtctt 2820
gagaaaaaga ttattgtagt gttcaaaata tttttgtatt gttaatgcat catcatagaa 2880
aaacttttaa acatgagaat aaagatactt tttactgggt ttgtttttca aagcctga 2938

<210> 13
<211> 2838
<212> DNA
<213> Homo sapiens

<400> 13
   gcctgaaggc cccggagtgc accagcggca tgtacaaaga gaccgtctac tccgccttca 60
   atctgctcat gcattaccca cccccctcgg gagcagggca gcacccccag ccgcagcccc 120
   cgctgcacaa ggccaatcag cccccccacg gtgtccccca actctctctc tacgaacatt 180
   tcaacagccc acatcccacc cctgcacctg cggacatcag ccagaagcaa gttcacaggc 240
   ctctgcagac ccctgacctc tctggcttct actccctgac ctcaggcagc atggggcagc 300
   tcccccacac tgtgagctgg ttcacccacc catccttgat gctaggttct ggtgtacctg 360
   gtcacccagc agccatcccc cacccggcca ttgtgccccc ctcagggaag caggagctgc 420
   agcccttcga ccgcaacctg aagacacaag cagagtccaa ggcagagaag gaggccaaga 480
   agccaaccat caagaagccc ctcaatgcct tcatgctgta catgaaggag atgagagcca 540
   aggtcattgc agagtgcaca cttaaggaga gcgctgccat caaccagatc ctgggccgca 600
   ggtggcacgc gctgtcgcga gaagagcagg ccaagtacta tgagctggcc cgcaaggaga 660
   ggcagctgca catgcagcta tacccaggct ggtcagcgcg ggacaactac gggaagaaga 720
   agaggcggtc gagggaaaag caccaagaat ccaccacaga ccctggctcg cctaagaaat 780
   gccgtgctcg ctttggcctc aaccagcaga cggattggtg tggtccgtgc aggaggaaaa 840
   agaaatgcat tcggtactta cccggagaag gccgctgccc cagccccgtt ccttccgatg 900
   acagtgctct aggctgcccc gggtccccag ctccccagga ctcaccctca taccatctgc 960
   tgccccgctt ccccacagaa ctgcttacta gccctgcgga gccggcacct acatccccag 1020
   gtctctccac tgctctcagc ctcccaaccc cagggccccc acaggccccc cgcagcaccc 1080
   tgcagagcac acaggtacag caacaggaat ctcagagaca ggtggcctag caggcacagg 1140
   acacctggcc gcctccagga gcctaccccc tgaaagtgac agagacccag atctcatgga 1200
   aactggccag gggtcctgtt aacgtcatct cagggtccag accctgaaga tttcagaggc 1260
   tgcaggactt ctgcctgaac ctggggtcat cgattcaaac tgctccaagt ggtgggaatc 1320
   agatctgtct tgatgtgtca tctaattaag ggaatccctt gtacctatgg ctgcctgcat 1380
   ctattctttg taccatctgt cttgccagcc agaagcctct gcctccctag cttttctgct 1440
   ataggtcaga gatgggctga actgagccta gctaccttct ctacccatct cccccatccc 1500
   ccactgccac accctcccca ttcagacact tcatggacca agaatgagct ggtttgtcaa 1560
   acaacatgtg agcatggtca caagcacaaa gctcaagatg acagctcttc taaggaaatg 1620
   gagaagctct gtttataaaa acaaaaacaa aaccagctgc tactcataag ttggaccaga 1680
   ggaagcccct tactatgatc tcaggagctt gcaagaagca ggaaggggaa tggaataggt 1740
   taagtttagg cctatcaacc taagcaacag aaataatctg acactacctt atcaggcaaa 1800
   ttggggaggg gagggtgtat ctagctctag ttcaaattat ttggaagtgt tccctgagaa 1860
   acccaccagc ctaagaagct ctggccccag gcttgtcact agcagctgca gtcaacagtt 1920
   caaagaagtc atggcccaaa tccagtgtgc acccctcccc attcacagag cctttttcac 1980
   aattccattt ccagttcatc tatggcagtc cagccagctc ctgggcagct tgagagggca 2040
   aacccaaaac ctcatgacag ccagagcctg tctttcagca ttcagtccgc ctggccggct 2100
   ccagtttccc catggggctg cgggacagag gaccattaca actagatcaa ggagcccaga 2160
   aaacctccag tagtggacaa caggttttca ccatagccta cgttaaccca tttttgagcc 2220
   aagcttcaac cctcagcctt gaaaaacaag tctttaattt aatttttgtt ttttgcctaa 2280
   atccaaagaa aaagggctgc cgggccaggc gcggtggctc acgcctgtaa tcccagcact 2340
   ttggcaggcc gaggcaggtg gatcacctga cgtcagtagt ttgagaccag cctggccaac 2400
   atggtgaaac cctgtatcta ctaaaaatac aaaaattagc cggacgtggt ggtgcgcgca 2460
   tgtaatccca gctactcggg aggctgaggc ggaagaatca cttgaacccg ggaggcggag 2520
   gttccagtga gccgagatgg cgctattgca ctccagtctg ggtaacaggg agactgcatc 2580
   tcaaaaaaaa aaaaaaaaaa aaaaaagggc tgtccatgta ttcacacacc cctcaaaaaa 2640
   agcctttagt tcctacttta gccactggtt tctcagaatc caaagatcac atattctagt 2700
   gtaacactgc aagaagtctt gagaaaaaga ttattgtagt gttcaaaata tttttgtatt 2760
   gttaatgcat catcatagaa aaacttttaa acatgagaat aaagatactt tttactgggt 2820
   ttgtttttca aagcctga 2838

<210> 14

<211>  2788
<212>  DNA
<213>  Homo sapiens

<400>  14
gcctgaaggc cccggagtgc accagcggca tgtacaaaga gaccgtctac tccgccttca      60

atctgctcat gcattaccca cccccctcgg gagcagggca gcacccccag ccgcagcccc     120

cgctgcacaa ggccaatcag cccccccacg gtgtccccca actctctctc tacgaacatt     180

tcaacagccc acatcccacc cctgcacctg cggacatcag ccagaagcaa gttcacaggc     240

ctctgcagac ccctgacctc tctggcttct actccctgac ctcaggcagc atggggcagc     300

tcccccacac tgtgagctgg ttcacccacc catccttgat gctaggttct ggtgtacctg     360

gtcacccagc agccatcccc cacccggcca ttgtgccccc ctcagggaag caggagctgc     420

agcccttcga ccgcaacctg aagacacaag cagagtccaa ggcagagaag gaggccaaga     480

agccaaccat caagaagccc ctcaatgcct tcatgctgta catgaaggag atgagagcca     540

aggtcattgc agagtgcaca cttaaggaga gcgctgccat caaccagatc ctgggccgca     600

ggtggcacgc gctgtcgcga gaagagcagg ccaagtacta tgagctggcc cgcaaggaga     660

ggcagctgca catgcagcta tacccaggct ggtcagcgcg ggacaactac gggaagaaga     720

agaggcggtc gagggaaaag caccaagaat ccaccacaga taactctctt cactattcct     780

aggaggaaaa agaaatgcat tcggtactta cccggagaag gccgctgccc cagccccgtt     840

ccttccgatg acagtgctct aggctgcccc gggtccccag ctccccagga ctcaccctca     900

taccatctgc tgccccgctt ccccacagaa ctgcttacta gccctgcgga gccggcacct     960

acatccccag gtctctccac tgctctcagc ctcccaaccc cagggccccc acaggccccc    1020

cgcagcaccc tgcagagcac acaggtacag caacaggaat ctcagagaca ggtggcctag    1080

caggcacagg acacctggcc gcctccagga gcctaccccc tgaaagtgac agagacccag    1140

atctcatgga aactggccag gggtcctgtt aacgtcatct cagggtccag accctgaaga    1200

tttcagaggc tgcaggactt ctgcctgaac ctggggtcat cgattcaaac tgctccaagt    1260

ggtgggaatc agatctgtct tgatgtgtca tctaattaag ggaatccctt gtacctatgg    1320

ctgcctgcat ctattctttg taccatctgt cttgccagcc agaagcctct gcctccctag    1380

cttttctgct ataggtcaga gatgggctga actgagccta gctaccttct ctacccatct    1440

cccccatccc ccactgccac accctcccca ttcagacact tcatggacca agaatgagct    1500

ggtttgtcaa acaacatgtg agcatggtca caagcacaaa gctcaagatg acagctcttc    1560

taaggaaatg gagaagctct gtttataaaa acaaaaacaa aaccagctgc tactcataag    1620

ttggaccaga ggaagcccct tactatgatc tcaggagctt gcaagaagca ggaaggggaa    1680

tggaataggt taagtttagg cctatcaacc taagcaacag aaataatctg acactacctt    1740

atcaggcaaa ttggggaggg gagggtgtat ctagctctag ttcaaattat ttggaagtgt    1800

tccctgagaa acccaccagc ctaagaagct ctggccccag gcttgtcact agcagctgca    1860

gtcaacagtt caaagaagtc atggcccaaa tccagtgtgc acccctcccc attcacagag    1920

cctttttcac aattccattt ccagttcatc tatggcagtc cagccagctc ctgggcagct 1980

tgagagggca aacccaaaac ctcatgacag ccagagcctg tctttcagca ttcagtccgc 2040

ctggccggct ccagtttccc catggggctg cgggacagag gaccattaca actagatcaa 2100

ggagcccaga aaacctccag tagtggacaa caggttttca ccatagccta cgttaaccca 2160

tttttgagcc aagcttcaac cctcagcctt gaaaaacaag tctttaattt aatttttgtt 2220

ttttgcctaa atccaaagaa aaagggctgc cgggccaggc gcggtggctc acgcctgtaa 2280

tcccagcact ttggcaggcc gaggcaggtg gatcacctga cgtcagtagt ttgagaccag 2340

cctggccaac atggtgaaac cctgtatcta ctaaaaatac aaaaattagc cggacgtggt 2400

ggtgcgcgca tgtaatccca gctactcggg aggctgaggc ggaagaatca cttgaacccg 2460

ggaggcggag gttccagtga gccgagatgg cgctattgca ctccagtctg ggtaacaggg 2520

agactgcatc tcaaaaaaaa aaaaaaaaaa aaaaaagggc tgtccatgta ttcacacacc 2580

cctcaaaaaa agcctttagt tcctacttta gccactggtt tctcagaatc caaagatcac 2640

atattctagt gtaacactgc aagaagtctt gagaaaaaga ttattgtagt gttcaaaata 2700

tttttgtatt gttaatgcat catcatagaa aaacttttaa acatgagaat aaagatactt 2760

tttactgggt ttgtttttca aagcctga 2788

<210> 15
<211> 845
<212> DNA
<213> Homo sapiens

<400> 15
agcacacaca catctgcacc tcaaccacag actacacttg ctgaactggc tcctggggcc 60

atgaggctgt cactgccact gctgctgctg ctgctgggag cctgggccat cccagggggc 120

ctcggggaca gggcgccact cacagccaca gccccacaac tggatgatga ggagatgtac 180

tcagcccaca tgcccgctca cctgcgctgt gatgcctgca gagctgtggc ttaccagatg 240

tggcaaaatc tggcaaaggc agagaccaaa cttcatacct caaactctgg ggggcggcgg 300

gagctgagcg agttggtcta cacggatgtc ctggaccgga gctgctcccg gaactggcag 360

gactacggag ttcgagaagt ggaccaagtg aaacgtctca caggcccagg acttagcgag 420

gggccagagc caagcatcag cgtgatggtc acagggggcc cctggcctac caggctctcc 480

aggacatgtt tgcactactt gggggagttt ggagaagacc agatctatga agcccaccaa 540

caaggccgag gggctctgga ggcattgcta tgtgggggac cccagggggc ctgctcagag 600

aaggtgtcag ccacaagaga agagctctag tcctggactc taccctcctc tgaaagaagc 660

tggggcttgc tctgacggtc tccactcccg tctgcaggca gccaggaggg caggaagccc 720

ttgctctgtg ctgccatcct gcctccctcc tccagcctca gggcactcgg gcctgggtgg 780

gagtcaacgc cttcccctct ggactcaaat aaaacccagt gacctcaaaa aaaaaaaaaa 840

aaaaa 845

<210>  16
<211>  1581
<212>  DNA
<213>  Homo sapiens

<400>  16
ctcgccgctg ccccggctcc gccgctcgca gagagattcg gaggagcccg ggcggggggg      60

aggaggaggg ggaggaggga gcggagatct cggggctcgg agccggccgc cgctccgctc     120

cgatcgctgt ggggcttggt tttttggggg tggggggcgg ggggggctca gatatggagg     180

caaatgggag ccaaggcacc tcgggcagcg ccaacgactc ccagcacgac cccggtaaaa     240

tgtttatcgg tggactgagc tggcagacct caccagatag ccttagagac tattttagca     300

aatttggaga aattagagaa tgtatggtca tgagagatcc cactacgaaa cgctccagag     360

gcttcggttt cgtcacgttc gcagacccag caagtgtaga taaagtatta ggtcagcccc     420

accatgagtt agattccaag acgattgacc ccaaagttgc atttcctcgt cgagcgcaac     480

ccaagatggt cacaagaaca aagaaaatat ttgtaggcgg gttatctgcg aacacagtag     540

tggaagatgt aaagcaatat ttcgagcagt ttggcaaggt ggaagatgca atgctgatgt     600

ttgataaaac taccaacagg cacagagggt ttggctttgt cacttttgag aatgaagatg     660

ttgtggagaa agtctgtgag attcatttcc atgaaatcaa taataaaatg gtagaatgta     720

agaaagctca gccgaaagaa gtcatgttcc cacctgggac aagaggccgg gcccggggac     780

tgccttacac catggacgcg ttcatgcttg gcatggggat gctgggatat cccaacttcg     840

tggcgaccta tggccgtggc taccccggat ttgctccaag ctatggctat cagttcccag     900

gcttcccagc agcggcttat ggaccagtgg cagcagcggc ggtggcggca gcaagaggat     960

caggctccaa cccggcgcgg cccggaggct tcccgggggc caacagccca ggacctgtcg    1020

ccgatctcta cggccctgcc agccaggact ccggagtggg gaattacata agtgcggcca    1080

gcccacagcc gggctcgggc ttcggccacg gcatagctgg acctttgatt gcaacggcct    1140

ttacaaatgg ataccattga gcaggtgctt tcgttgccat ctcactctga gagcatacct    1200

ggatgtccag gcaagactgg gcgaagtttc tgagtggccc tttgtttagg tgatgtcctc    1260

agacctggac ccccaccagc ctcactcccc atcccaacca gagatggctc acttcggatc    1320

gagggttgac tacatctcat catctcacga atctgctgta atataagaca acagctttta    1380

aatgtgtata taacccatga tttcggtttt gttttgtttt gtttttcttg atggtttccc    1440

tctccctccc tctcttccca ttctcctttt aaatctcttt gaatcacatt tggtagtgat    1500

tttgacttag tccagtagtc acatagcttt aatatctagt tcaaagctaa ccatagtata    1560

attgttatat taaggagtta t                                              1581

<210>  17
<211>  2151
<212>  DNA
<213>  Homo sapiens

<400>  17

gctttccttt tagcttttgt aagttacacg tcaaaatggc cgatctgaca tcggtgctca 60
cttctgttat gttttctccc tctagtaaaa tgtttatcgg tggactgagc tggcagacct 120
caccagatag ccttagagac tattttagca aatttggaga aattagagaa tgtatggtca 180
tgagagatcc cactacgaaa cgctccagag gcttcggttt cgtcacgttc gcagacccag 240
caagtgtaga taaagtatta ggtcagcccc accatgagtt agattccaag acgattgacc 300
ccaaagttgc atttcctcgt cgagcgcaac ccaagatggt cacaagaaca aagaaaatat 360
ttgtaggcgg gttatctgcg aacacagtag tggaagatgt aaagcaatat ttcgagcagt 420
ttggcaaggt ggaagatgca atgctgatgt ttgataaaac taccaacagg cacagagggt 480
ttggctttgt cacttttgag aatgaagatg ttgtggagaa agtctgtgag attcatttcc 540
atgaaatcaa taataaaatg gtagaatgta agaaagctca gccgaaagaa gtcatgttcc 600
cacctgggac aagaggccgg gcccggggac tgccttacac catggacgcg ttcatgcttg 660
gcatggggat gctgggatat cccaacttcg tggcgaccta tggccgtggc taccccggat 720
ttgctccaag ctatggctat cagttcccag actatttgcc ggtttcacaa gacataattt 780
ttatcaacta gctcttaaga gaggcatagc aaagtggggg ttgctaccat ttctagagag 840
agaggacaca gtcccggcct gggctgcccc cgctccagtc aatgctcact gaaagtctgt 900
cttagctgcc tgtttgaatg actgttcttt ttctcatttt taattcttgg actcatgtcc 960
tcattgcttc actcaattaa aaaaaaatta ttctccagtc ccctcccact ttgcttcttg 1020
tatgcattgt gaccgacccc acttcctcag aatgtaacgg ggccagaggg aaacttctca 1080
caaacttcgt agagcctcct caggggaagc taggaagaag acatcaaatg tttttaagtc 1140
atgaccaaac aggcttgttg gggacatatc atggggtgag ctttgaagtg ctggtggtcc 1200
agaggggttg cagatacgtg acttggagca cccgtgtctt acgatggaca gtgataaagg 1260
tgaacacaca gagacagact attctctaag aatgtgagaa acctgatctg gaagaggagc 1320
tatataaaca ctatctgact atctttgtct tttggggcca gtggctgttg gcataatcac 1380
aagcctgtct gtcttcgaga agggacagtg gagtcatcca ggtgctgcca catgacaggc 1440
acggtgggca ccgatccaca gtgggccccg ccttccccag ctcgcctccc tgcctgtgct 1500
ggcctggcct tgcctgctgg caccattgga gtaggagggg gtggaacaca gggggcccat 1560
cctgatcagg ccccatctca aggttggcac tcctgcccat cacccttaga aggatctttt 1620
cccatggctt gacttccttc atttccctaa ctgaaataca cccactctct tggaataatg 1680
acgtaccact cagttggacc ctcaagagtc actgctttgt ctgtgctggt agtttgtgag 1740
aagtgacccg cacgcttcca tttgatgcat ttgatgtgag tgaatccata catttgaatg 1800
tcattgtcct tgagacccta catgtgcagt ttggctcatc tcattaaaga tgcttgatgt 1860
aataattggt tagtttcctt ttattttcct gcaggctttt ccatgagtat tatttttttc 1920
aaagaacaaa tctgtatggc ttttccccat ctccatattt tgttttgcta tgaattgctt 1980
tgctttggtg aacttgtcct agtatgcttg cctcacaaac gttttagcca ttgtgaattt 2040

tcttcatctc tgtaaatagt tcatctgtgc ttctccctga tgacgtttta ttttttttcc 2100

cctgtaagca accgaggtag aaaaataaat tgtttaccat ggaaaaaaaa a 2151

<210> 18
<211> 8382
<212> DNA
<213> Homo sapiens

<400> 18
cgcagcggcg gcggcagcgg cggcggcggc tacagcagct ccgccggcag ccgcgggagc 60

acggcgacgc cagcggcggg aagggaaaag gccgaggcat cagcgtgtga agaccgcaaa 120

gacgatcccg agtacagttg tgaacagcat tgctgctagg ctcctcctgc agatcatctg 180

aaatgaacct ctcttattga tttttattgg cctagagcca ggagtactgc attcagttga 240

ctttcagggt aaaaagaaaa cagtcctggt tgttgtcatc ataaacatat ggaccagtgt 300

gatggtgaaa tgagatgagg ctccgcaatg gaactgtagc cactgcttta gcatttatca 360

cttccttcct tactttgtct tggtatacta catggcaaaa tgggaaagaa aaactgattg 420

cttatcaacg agaattcctt gctttgaaag aacgtcttcg aatagctgaa cacagaatct 480

cacagcgctc ttctgaatta aatacgattg tgcaacagtt caagcgtgta ggagcagaaa 540

caaatggaag taaggatgcg ttgaataagt tttcagataa taccctaaag ctgttaaagg 600

agttaacaag caaaaaatct cttcaagtgc caagtattta ttatcatttg cctcatttat 660

tgaaaaatga aggaagtctt caacctgctg tacagattgg caacggaaga acaggagttt 720

caatagtcat gggcattccc acagtgaaga gagaagttaa atcttacctc atagaaactc 780

ttcattccct tattgataac ctgtatcctg aagagaagtt ggactgtgtt atagtagtct 840

tcataggaga gacagatatt gattatgtac atggtgttgt agccaacctg gagaaagaat 900

tttctaaaga aatcagttct ggcttggtgg aagtcatatc accccctgaa agctattatc 960

ctgacttgac aaacctaaag gagacatttg gagactccaa agaaagagta agatggagaa 1020

caaagcaaaa cctagattac tgttttctaa tgatgtatgc tcaagaaaag ggcatatatt 1080

acattcagct tgaagatgat attattgtca aacaaaatta ttttaatacc ataaaaaatt 1140

ttgcacttca actttcttct gaggaatgga tgattctaga gttttcccag ctgggcttca 1200

ttggtaaaat gtttcaagcg ccggatctta ctctgattgt agaattcata ttcatgtttt 1260

acaaggagaa acccattgat tggctcctgg accatattct ctgggtgaaa gtctgcaacc 1320

ctgaaaaaga tgcaaaacat tgtgatagac agaaagcaaa tctgcgaatt cgcttcagac 1380

cttccctttt ccaacatgtt ggtctgcact catcactatc aggaaaaatc caaaaactca 1440

cggataaaga ttatatgaaa ccattacttc ttaaaatcca tgtaaaccca cctgcggagg 1500

tatctacttc cttgaaggtc taccaagggc atacgctgga gaaaacttac atgggagagg 1560

atttcttctg ggctatcaca ccgatagctg gagactacat cttgtttaaa tttgataaac 1620

cagtcaatgt agaaagttat ttgttccata gcggcaacca agaacatcct ggagatattc 1680

tgctaaacac aactgtggaa gttttgcctt ttaagagtga aggtttggaa ataagcaaag 1740

aaaccaaaga caaacgatta gaagatggct atttcagaat aggaaaattt gagaatggtg 1800
ttgcagaagg aatggtggat ccaagtctca atcccatttc agcctttcga ctttcagtta 1860
ttcagaattc tgctgtttgg gccattctta atgagattca tattaaaaaa gccaccaact 1920
gatcatctga gaaaccaaca cattttttcc tgtgaatttg ttaattaaag atagttaagc 1980
atgtatcttt tttttatttc tacttgaaca ctacctcttg tgaagtctac tgtagataag 2040
acgattgtca tttccacttg gaaagtgaat ctcccataat aattgtattt gtttgaaact 2100
aagctgtcct cagattttaa cttgactcaa acatttttca attatgacag cctgttaata 2160
tgacttgtac tattttggta ttatactaat acataagagt tgtacatatt gttacattct 2220
ttaaatttga gaaaaactaa tgttacatac attttatgaa gggggtactt ttgaggttca 2280
cttattttac tattatagac cctcttttat agattatcag ggattatata tataaatata 2340
taaatataca taaaaatgtt atggaattaa tttattagaa acacttaaga agtacatatt 2400
tttgtgcagt agatttttga atcgatactt tttttgaaaa ccagtttcct tgctttttta 2460
agttctttct atatttttct ttggaaatgc aaacattaca aaccaatgcc atttttcaaa 2520
tgcactgcca tttaagattg attatagatg gatttcttaa ttgaagtact tttataatca 2580
cagtgactga acaaaatatt ttcaaagaca tttgtcattc cttaaagcca agattttaaa 2640
gactaatgtc cttcctgagg gttactttac tatactgtgt atggtgtata gccacagaaa 2700
gtcagtctga taaattttca atgtgtaagt gtgatgcatt caacccagat tattgtagac 2760
ttaacttatt atatttcctc tgatatgtaa aatgacaaac tcttctttcc tttttaatct 2820
ttgtctttaa tccatgaatt aggtgcatta tctgctgttg attcttttat atctgcactc 2880
attttatgac gtcagacaaa atgttttcta ggtgttgcta ttgtatgagt cataattggt 2940
ataattttaa agaaaattgt aatattgttt tttggtgaaa tatattatta gtagtatatt 3000
ttccaaaatg attttattgt agtatgaact gataagtgag tcccgtgtcc aagagactgt 3060
atttattact cttaaacacg ctattccaaa tgatattact actataaaca aagaatatta 3120
ccggtctcag caatcagtga tctttataaa agtactttta aaaaatgatg aattatcacc 3180
aggcgctgtg gctcacgcct gtaatcccag cactttggga ggccgaggcg ggcggatcac 3240
gaggtcagga gatcaagacc atcctggcta acacggtgaa accctgtctc tactaaaaat 3300
acaaaaaatt agccaggcgt ggtggcaggt gcctgtaatc ccagctactt gggaggctga 3360
ggcaggagaa tggcgtgaac ccaggaggca gagctggcag tgagctgaga tcgcaccact 3420
gcactccagc ctgggcgaca cagctagact ccgtctcaaa aaaaaaaaaa gatgaagtat 3480
caacatcagt tcccagccag tctgcttgga cacactgggg tgccacaagt gggttgcagg 3540
ttggttgcag acgggcagag atggattctg ctgagctttc agagtgacca ctggctgggg 3600
cctagcgctg gggcaaggtt ccagctggct gcctctagcc tggaggagct tcatctagtt 3660
acccaatgtg ctgaaccaac agcatgaaat agcatcattt cctgtgctgt gttacaaagt 3720
gcaaacgttt ggaaacactg atacagtaca attatttcca tcactccatg ttgttataat 3780

ttttagttac ttttcttgat tgaaaagtca atcacattgc caaaaatgat ttttatgtca 3840
gttaaaggcc agttgaaatt tttttattag gatgttttaa atttactttt cactttcttg 3900
aatttttttc ttctcaaatt tccttggccc agtgcccatg agcaaatttc attcaaaaac 3960
aggaataacc ttttgtcata aaattttggc accagcttgg tatactaaat tactgcaaag 4020
ataactgagt accagaagta aaaatctctg caggtgggct taggagtact cttgggtccc 4080
tggttcctca gttgccaact ctggtttcat gaagaaagac catgctctga aaatctaggg 4140
aggagaaagt tattgcagca cctggtgtga tttgttacaa cttctttaaa gacagcctgc 4200
ctggctagct ttttcctgtt ttcatgcagt ttgccctccc agcattggtc cacctgttgt 4260
tcccaagaaa ctcattcttg ttcccaagac tccttttagc caaatctttc ctatcattcc 4320
aggacttttt tgtgcttctc tccccatgaa tctacccgtg cctagggtgt gtgccaggca 4380
cgtggcaggt ggccagtcag catctggatg tgagaccaat tattccagcc tgccctcctc 4440
tttctctttt tatcatttgg tattattttt gagacagggt ctcgctttgt tgcccaggct 4500
ggagtgcagt ggtgctctca tagctcactg cagcctcgat ctctggcctc aagcaatcct 4560
cctgcctcag cctcccaaag tgctgggatt ataggtgtgt gccaccatgt ccagccccct 4620
ttctctctct taactccctc tccctgctgt cgttgccata ctcttgcata cacaatcggt 4680
tgtctagttt ttgattgttt gtgtgtatgg tgtgtgctgt ctctctgacc agatttcagg 4740
ttcctgaggc gagcctgcag ctcatactgc tcatctgtcc tctcctatgg tgggtgctca 4800
gggcctctca ctgttagtta ctccctcctt tctgcccagt tctgcactca actagtagaa 4860
gcagccatcc tttccccaag caggaaattg tagtggtcgc ccttaagagc agtgtgaggg 4920
cagaagatta agggagggga agagtccctg gaactggaag aaggtaaata ctttgccttg 4980
agagggcgcc gaatcatttt accaaaatag taaatggaaa aagtgtcaaa ggttgggact 5040
agttttaaaa acacatatag ccagtagaca catgggggct gtttaaataa aaacattttt 5100
ttacccggtt ctgccatcac acacactagt gacattccaa gtactcagtg gccatgtgga 5160
gctagtggcc accacacttg acaccacgga tgcagaactt ttccatcact gcagaaagct 5220
ctgttgaaca gtactgtgtc cagagtgcta attatgaaca gcttagaaac cacaattaat 5280
acattctcat gaggttaaaa gcagacatgg gaatagaaca taatgcaaat gatgatgaat 5340
attttgggtg agaagagaaa gctgaggacc catttctagg attctaagat gtaagatttc 5400
ttaagttctt tatcttagtc tcatgcattc tccacatcac gcgctgtacc atactgtgta 5460
gtcagaacag acagtgtgat tgaaaagctt tggaaaaagt taacacaaag gattatttag 5520
cacataggct gtagatacgt atgtgtgtat ttgttcaaca attggagatg gttgaatacc 5580
cttgaacaaa gtgtgtatct tctcaaatca gtggttgcac tagtcaataa ttagaaggtg 5640
ttgttatttt taaaactata agcaaaatta tgaaggcctt taaaaaatct atcataataa 5700
tgaaaaagag gttgtctccc aacagtgctg tccctcaaag aaaagactgg ttatgtggaa 5760
acagcacgtt tggagagatt attctagtga ataacagtgt atatttgtgg taggcaaatt 5820

tctaagaatg acccctactg acacccccac caatgacctt gtataatatc ctcccacttt 5880
gagcatgggt ggaacctgtt tgatccatga ttatatcatg ttatgtagca aaaggaagac 5940
tgtctggggg tggctaatca aatcttgtga ctcctttaaa agcagagagt tttctcgggc 6000
tggtggcaga agtcaagaga caaagcagaa ggacctcagg caccattgct gttttaaggt 6060
agagggggct gcatgagaag gaatgcacat ggctttgagg agctgagaga ggccttggct 6120
aacagcaaag gagctgggaa ctgcagtcct acagccacgg gaactgaatt cagcccataa 6180
gctcattcat aggcttggaa aggcattcat ctccagtctc cagataagac ccagcctggc 6240
caacactttg aatccagcct tgtgagaccc taagccgaga actcacttaa gcctgcttgg 6300
acttctgacc tacagaacta tgagataata aatcagtggt gttttaagct gctaaatttg 6360
tggcaatttg ttatgtagga ataggaaatg aatacagtat tttaaaagag gtaaagagga 6420
aatcttcata cttgattccc tgtagtatgc tggatatttc ttggtaaatg catgaataaa 6480
gtctttagta gcaagtcatg tacctggacc ttgttgggga tgttctcgcc agagatctca 6540
attagtcttc attggacgtt tgttggcaaa gttgcagcat cttactgagt agcgtttggt 6600
aaaggaactg ctagaatgag actataggaa taggactgaa acagtgtgta agggatcttt 6660
tttatttcta gaagaggtgt tactttatac tgatttagac tgctctgagc ctgtcagaag 6720
taaattcaag aatgattaac agtggtattt tagatacatt aagagcaaaa ataacctttg 6780
ccttgtagag atcagataat ctgtgtatcg agggatttaa ttttttttaa ccaaaactta 6840
gtttctcttt ttatcatcta gcacagatca aatcctgtat gggccagcca aatgtttgta 6900
gtttgagaat gttttgcaaa tacctctctg cttccaagcc attaatagct aatgaagttt 6960
tattttggtt tgtccattaa tttttctgct tatttttact gtattgccat gtccttgata 7020
cgagacaagt agaactaaaa gagagaaaat gagaggttaa gactgtcaga agatgagaaa 7080
tgtgagtcat aaccatacct tagtgtttta agggaacttt atccagtcca atccctttat 7140
agaaaaggaa cttttccaag ttatgttgca gtatttatag tgtgtaaaat tttgtttgac 7200
tttggtagtt gtgcatttta aattatgaat cttatatgct acgataacta gtatgcttac 7260
tttctttaat gcatgtgatc atacaatgct aaaaaggtat tttttaaaga cccaaccaaa 7320
ctgaggattt tgttatacat atctgtatca acacctatat ataaactctt cttcttacct 7380
gaaacactcc ttcacccggt ttgggcacta actagatcat tttcaaagga acaaacagga 7440
aggagcaagc tggcactcca gggtgcatct gagctgagaa tttagaagac ctcattcaag 7500
aaagtaaact gagcattgat gggaaagcac tttgcgcgaa gtaacattcc cactgttaat 7560
gttgcagtca aaattgtgga aaaaaaaaag tggtgctaaa attgttctga aatgaagatt 7620
gtttgttgtg gggaatattt taatactctt ggactttagg gaggctattt tatgtgtcac 7680
ttgctcctag ttgtttcatt gtattagtaa ttagcaaact gtcctggaac caagggtttg 7740
gtacttttgc ctgattagca ttccattatt tatttgaaat gctcagtgtt tgctcaaatt 7800
tggtgaaaat atcttttgaa gacatattct ctgagcctta aaaatggctg ctatcgacag 7860

tactgtaaga tgcactgcca gtagatttga tacttttcat ttcagtgtag gcagtaatgt 7920

tttctaagaa agatttagaa atcagcaaag tgtaacaatt gtgtttcatt tatggaatgg 7980

aaaaactgaa gtgtcccatt cagaacactt tggaaaggtt gtctgcatta ggggatgttt 8040

ctggaagcat cccttagaga agctgcctga ggaatctcct tgccaggttg taccagattt 8100

ttttttttct taaatgtatt gtatatattt tccttaaaat gtcttcattg gctgaatgat 8160

tcctatccgt agtaatattt taggtacttg ttggattttt aaaggaagat tatttatttt 8220

cagatttgct gctataattg aaaacctagt aactggtcct gttgattgtg ccttcatcac 8280

tgtttctctg tgccacgacc gttcatgtgt atttgaaata aagaagttta aaaagccatg 8340

ttgaattcaa gattccttct aataaaaata ggtgaaactt aa 8382

<210> 19
<211> 3271
<212> DNA
<213> Homo sapiens

<400> 19
agaaggatcc atctaacatt tactgaaata aggaagatgc tttattcaat ctaaaagtga 60

aaaaagaacc ttttagacat tattattata aaatgtctat aagaagactt ctcattttaa 120

tcctcaaaat agggagacga tggactgagc tgatccgcac catggagtct cgggtcttac 180

tgagaacatt ctgtttgatc ttcggtctcg gagcagtttg ggggcttggt gtggaccctt 240

ccctacagat tgacgtctta acagagttag aacttgggga gtccacgacc ggagtgcgtc 300

aggtcccggg gctgcataat gggacgaaag cctttctctt tcaagatact cccagaagca 360

taaaagcatc cactgctaca gctgaacagt tttttcagaa gctgagaaat aaacatgaat 420

ttactatttt ggtgacccta aaacagaccc acttaaattc aggagttatt ctctcaattc 480

accacttgga tcacaggtac ctggaactgg aaagtagtgg ccatcggaat gaagtcagac 540

tgcattaccg ctcaggcagt caccgccctc acacagaagt gtttccttac attttggctg 600

atgacaagtg gcacaagctc tccttagcca tcagtgcttc ccatttgatt ttacacattg 660

actgcaataa aatttatgaa agggtagtag aaaagccctc cacagacttg cctctaggca 720

caacattttg gctaggacag agaaataatg cgcatggata ttttaagggt ataatgcaag 780

atgtccaatt acttgtcatg ccccagggat ttattgctca gtgcccagat cttaatcgca 840

cctgtccaac ttgcaatgac ttccatggac ttgtgcagaa aatcatggag ctacaggata 900

ttttagccaa aacatcagcc aagctgtctc gagctgaaca gcgaatgaat agattggatc 960

agtgctattg tgaaaggact tgcaccatga agggaaccac ctaccgagaa tttgagtcct 1020

ggatagacgg ctgtaagaac tgcacatgcc tgaatggaac catccagtgt gaaactctaa 1080

tctgcccaaa tcctgactgc ccacttaagt cggctcttgc gtatgtggat ggcaaatgct 1140

gtaaggaatg caaatcgata tgccaatttc aaggacgaac ctactttgaa ggagaaagaa 1200

atacagtcta ttcctcttct ggagtatgtg ttctctatga gtgcaaggac cagaccatga 1260

aacttgttga gagttcaggc tgtccagctt tggattgtcc agagtctcat cagataacct 1320
tgtctcacag ctgttgcaaa gtttgtaaag gttatgactt ttgttctgaa aggcataact 1380
gcatggagaa ttccatctgc agaaatctga atgacagggc tgtttgtagc tgtcgagatg 1440
gttttagggc tcttcgagag gataatgcct actgtgaaga catcgatgag tgtgctgaag 1500
ggcgccatta ctgtcgtgaa aatacaatgt gtgtcaacac cccgggttct tttatgtgca 1560
tctgcaaaac tggatacatc agaattgatg attattcatg tacagaacat gatgagtgta 1620
tcacaaatca gcacaactgt gatgaaaatg ctttatgctt caacactgtt ggaggacaca 1680
actgtgtttg caagccgggc tatacaggga atggaacgac atgcaaagca ttttgcaaag 1740
atggctgtag gaatggagga gcctgtattg ccgctaatgt gtgtgcctgc ccacaaggct 1800
tcactggacc cagctgtgaa acggacattg atgaatgctc tgatggtttt gttcaatgtg 1860
acagtcgtgc taattgcatt aacctgcctg gatggtacca ctgtgagtgc agagatggct 1920
accatgacaa tgggatgttt tcaccaagtg gagaatcgtg tgaagatatt gatgagtgtg 1980
ggaccgggag gcacagctgt gccaatgata ccatttgctt caatttggat ggcggatatg 2040
attgtcgatg tcctcatgga aagaattgca caggggactg catccatgat ggaaaagtta 2100
agcacaatgg tcagatttgg gtgttggaaa atgacaggtg ctctgtgtgc tcatgtcaga 2160
atggattcgt tatgtgtcga cggatggtct gtgactgtga gaatcccaca gttgatcttt 2220
tttgctgccc tgaatgtgac ccaaggctta gtagtcagtg cctccatcaa aatggggaaa 2280
ctttgtataa cagtggtgac acctgggtcc agaattgtca acagtgccgc tgcttgcaag 2340
gggaagttga ttgttggccc ctgccttgcc cagatgtgga gtgtgaattc agcattctcc 2400
cagagaatga gtgctgcccg cgctgtgtca cagacccttg ccaggctgac accatccgca 2460
atgacatcac caagacttgc ctggacgaaa tgaatgtggt tcgcttcacc gggtcctctt 2520
ggatcaaaca tggcactgag tgtactctct gccagtgcaa gaatggccac atctgttgct 2580
cagtggatcc acagtgcctt caggaactgt gaagttaact gtctcatggg agatttctgt 2640
taaaagaatg ttctttcatt aaaagaccaa aaagaagtta aaacttaaat tgggtgattt 2700
gtgggcagct aaatgcagct ttgttaatag ctgagtgaac tttcaattat gaaatttgtg 2760
gagcttgaca aaatcacaaa aggaaaatta ctggggcaaa attagacctc aagtctgcct 2820
ctactgtgtc tcacatcacc atgtagaaga atgggcgtac agtatatacc gtgacatcct 2880
gaaccctgga tagaaagcct gagcccattg gatctgtgaa agcctctagc ttcactggtg 2940
cagaaaattt tcctctagat cagaatcttc aagaatcagt taggttcctc actgcaagaa 3000
ataaaatgtc aggcagtgaa tgaattatat tttcagaagt aaagcaaaga agctataaca 3060
tgttatgtac agtacactct gaaaagaaat ctgaaacaag ttattgtaat gataaaaata 3120
atgcacaggc atggttactt aatattttct aacaggaaaa gtcatcccta tttccttgtt 3180
ttactgcact taatattatt tggttgaatt tgttcagtat aagctcgttc ttgtgcaaaa 3240
ttaaataaat atttctctta ccttataaca c 3271

<210> 20
<211> 3385
<212> DNA
<213> Homo sapiens

<400> 20
ctctacctac tttgcccagc tccacctcgg cagtgcagcg tgttttggtg gccttcctcc 60

gcacgccctg gagggggagt gccctgcacc ccggggctgc tccggagccc agtgcacgag 120

tgcacatggg cttccctcct ttgcttaaag ggcaggcgag cgctactcgc tccagccttg 180

cctcctgcag ctgggtggtc ttttttctct cctgtctttc aagacacgcg cccgaaatcg 240

agggagggag acgatggact gagctgatcc gcaccatgga gtctcgggtc ttactgagaa 300

cattctgttt gatcttcggt ctcggagcag tttgggggct tggtgtggac ccttccctac 360

agattgacgt cttaacagag ttagaacttg gggagtccac gaccggagtg cgtcaggtcc 420

cggggctgca taatgggacg aaagcctttc tctttcaaga tactcccaga agcataaaag 480

catccactgc tacagctgaa cagttttttc agaagctgag aaataaacat gaatttacta 540

ttttggtgac cctaaaacag acccacttaa attcaggagt tattctctca attcaccact 600

tggatcacag gtacctggaa ctggaaagta gtggccatcg gaatgaagtc agactgcatt 660

accgctcagg cagtcaccgc cctcacacag aagtgtttcc ttacattttg gctgatgaca 720

agtggcacaa gctctcctta gccatcagtg cttcccattt gattttacac attgactgca 780

ataaaattta tgaaagggta gtagaaaagc cctccacaga cttgcctcta ggcacaacat 840

tttggctagg acagagaaat aatgcgcatg gatattttaa gggtataatg caagatgtcc 900

aattacttgt catgccccag ggatttattg ctcagtgccc agatcttaat cgcacctgtc 960

caacttgcaa tgacttccat ggacttgtgc agaaaatcat ggagctacag gatattttag 1020

ccaaaacatc agccaagctg tctcgagctg aacagcgaat gaatagattg gatcagtgct 1080

attgtgaaag gacttgcacc atgaagggaa ccacctaccg agaatttgag tcctggatag 1140

acggctgtaa gaactgcaca tgcctgaatg gaaccatcca gtgtgaaact ctaatctgcc 1200

caaatcctga ctgcccactt aagtcggctc ttgcgtatgt ggatggcaaa tgctgtaagg 1260

aatgcaaatc gatatgccaa tttcaaggac gaacctactt tgaaggagaa agaaatacag 1320

tctattcctc ttctggagta tgtgttctct atgagtgcaa ggaccagacc atgaaacttg 1380

ttgagagttc aggctgtcca gctttggatt gtccagagtc tcatcagata accttgtctc 1440

acagctgttg caaagtttgt aaaggttatg acttttgttc tgaaaggcat aactgcatgg 1500

agaattccat ctgcagaaat ctgaatgaca gggctgtttg tagctgtcga gatggtttta 1560

gggctcttcg agaggataat gcctactgtg aagacatcga tgagtgtgct gaagggcgcc 1620

attactgtcg tgaaaataca atgtgtgtca acaccccggg ttcttttatg tgcatctgca 1680

aaactggata catcagaatt gatgattatt catgtacaga acatgatgag tgtatcacaa 1740

atcagcacaa ctgtgatgaa aatgctttat gcttcaacac tgttggagga cacaactgtg 1800

tttgcaagcc gggctataca gggaatggaa cgacatgcaa agcattttgc aaagatggct 1860

gtaggaatgg aggagcctgt attgccgcta atgtgtgtgc ctgcccacaa ggcttcactg 1920
gacccagctg tgaaacggac attgatgaat gctctgatgg ttttgttcaa tgtgacagtc 1980
gtgctaattg cattaacctg cctggatggt accactgtga gtgcagagat ggctaccatg 2040
acaatgggat gttttcacca agtggagaat cgtgtgaaga tattgatgag tgtgggaccg 2100
ggaggcacag ctgtgccaat gataccattt gcttcaattt ggatggcgga tatgattgtc 2160
gatgtcctca tggaaagaat tgcacagggg actgcatcca tgatggaaaa gttaagcaca 2220
atggtcagat ttgggtgttg gaaaatgaca ggtgctctgt gtgctcatgt cagaatggat 2280
tcgttatgtg tcgacggatg gtctgtgact gtgagaatcc cacagttgat cttttttgct 2340
gccctgaatg tgacccaagg cttagtagtc agtgcctcca tcaaaatggg gaaactttgt 2400
ataacagtgg tgacacctgg gtccagaatt gtcaacagtg ccgctgcttg caaggggaag 2460
ttgattgttg gcccctgcct tgcccagatg tggagtgtga attcagcatt ctcccagaga 2520
atgagtgctg cccgcgctgt gtcacagacc cttgccaggc tgacaccatc cgcaatgaca 2580
tcaccaagac ttgcctggac gaaatgaatg tggttcgctt caccgggtcc tcttggatca 2640
aacatggcac tgagtgtact ctctgccagt gcaagaatgg ccacatctgt tgctcagtgg 2700
atccacagtg ccttcaggaa ctgtgaagtt aactgtctca tgggagattt ctgttaaaag 2760
aatgttcttt cattaaaaga ccaaaaagaa gttaaaactt aaattgggtg atttgtgggc 2820
agctaaatgc agctttgtta atagctgagt gaactttcaa ttatgaaatt tgtggagctt 2880
gacaaaatca caaaaggaaa attactgggg caaaattaga cctcaagtct gcctctactg 2940
tgtctcacat caccatgtag aagaatgggc gtacagtata taccgtgaca tcctgaaccc 3000
tggatagaaa gcctgagccc attggatctg tgaaagcctc tagcttcact ggtgcagaaa 3060
attttcctct agatcagaat cttcaagaat cagttaggtt cctcactgca agaaataaaa 3120
tgtcaggcag tgaatgaatt atattttcag aagtaaagca aagaagctat aacatgttat 3180
gtacagtaca ctctgaaaag aaatctgaaa caagttattg taatgataaa aataatgcac 3240
aggcatggtt acttaatatt ttctaacagg aaaagtcatc cctatttcct tgttttactg 3300
cacttaatat tatttggttg aatttgttca gtataagctc gttcttgtgc aaaattaaat 3360
aaatatttct cttaccttat aacac 3385

<210> 21
<211> 3705
<212> DNA
<213> Homo sapiens

<400> 21
tttggcatcc aggagaccgt gcgggagacc gacgctgagc gccggcctcg ccctcggcgg 60
cccctccctc ctccttaccc cgcctccccc ggcccgcccc tccctccccg gaggagcagt 120
ctctccgcgc gtctcccgga gctttctcca ttgtctctgc ctttacaaca ggttcgggcg 180
gcggggaaga cgggggggtg cggggccgcc ccagcccggg ctttcttggg gccgcccccc 240

ttctaccggg tgtgcgagtc tttggctgct tttattcggc tcgggagcta attccccgac 300
ggagccgcgc cggggcgagt ccgacccctc cctccgggcc ccctccgggc cgcgctgccg 360
cctcggccct gcgtgtggga atgatgtgcg cattggaggg tctaagttct tcacgcgcct 420
ggggaggcct ccctttcttt tcttaggcaa ccaaagcgta ttaatcctac tgatcagtaa 480
atccgaggca gcagcaggag agacaaacgt tattttcccg cttgattcca agaacctctt 540
cgatttttat ttttattttt aaagagggag acgatggact gagctgatcc gcaccatgga 600
gtctcgggtc ttactgagaa cattctgttt gatcttcggt ctcggagcag tttgggggct 660
tggtgtggac ccttccctac agattgacgt cttaacagag ttagaacttg gggagtccac 720
gaccggagtg cgtcaggtcc cggggctgca taatgggacg aaagcctttc tctttcaaga 780
tactcccaga agcataaaag catccactgc tacagctgaa cagttttttc agaagctgag 840
aaataaacat gaatttacta ttttggtgac cctaaaacag acccacttaa attcaggagt 900
tattctctca attcaccact tggatcacag gtacctggaa ctggaaagta gtggccatcg 960
gaatgaagtc agactgcatt accgctcagg cagtcaccgc cctcacacag aagtgtttcc 1020
ttacattttg gctgatgaca agtggcacaa gctctcctta gccatcagtg cttcccattt 1080
gattttacac attgactgca ataaaattta tgaaagggta gtagaaaagc cctccacaga 1140
cttgcctcta ggcacaacat tttggctagg acagagaaat aatgcgcatg gatattttaa 1200
gggtataatg caagatgtcc aattacttgt catgccccag ggatttattg ctcagtgccc 1260
agatcttaat cgcacctgtc caacttgcaa tgacttccat ggacttgtgc agaaaatcat 1320
ggagctacag gatattttag ccaaaacatc agccaagctg tctcgagctg aacagcgaat 1380
gaatagattg gatcagtgct attgtgaaag gacttgcacc atgaagggaa ccacctaccg 1440
agaatttgag tcctggatag acggctgtaa gaactgcaca tgcctgaatg gaaccatcca 1500
gtgtgaaact ctaatctgcc caaatcctga ctgcccactt aagtcggctc ttgcgtatgt 1560
ggatggcaaa tgctgtaagg aatgcaaatc gatatgccaa tttcaaggac gaacctactt 1620
tgaaggagaa agaaatacag tctattcctc ttctggagta tgtgttctct atgagtgcaa 1680
ggaccagacc atgaaacttg ttgagagttc aggctgtcca gctttggatt gtccagagtc 1740
tcatcagata accttgtctc acagctgttg caaagtttgt aaaggttatg acttttgttc 1800
tgaaaggcat aactgcatgg agaattccat ctgcagaaat ctgaatgaca gggctgtttg 1860
tagctgtcga gatggtttta gggctcttcg agaggataat gcctactgtg aagacatcga 1920
tgagtgtgct gaagggcgcc attactgtcg tgaaaataca atgtgtgtca acaccccggg 1980
ttcttttatg tgcatctgca aaactggata catcagaatt gatgattatt catgtacaga 2040
acatgatgag tgtatcacaa atcagcacaa ctgtgatgaa aatgctttat gcttcaacac 2100
tgttggagga cacaactgtg tttgcaagcc gggctataca gggaatggaa cgacatgcaa 2160
agcattttgc aaagatggct gtaggaatgg aggagcctgt attgccgcta atgtgtgtgc 2220
ctgcccacaa ggcttcactg gacccagctg tgaaacggac attgatgaat gctctgatgg 2280

ttttgttcaa tgtgacagtc gtgctaattg cattaacctg cctggatggt accactgtga 2340

gtgcagagat ggctaccatg acaatgggat gttttcacca agtggagaat cgtgtgaaga 2400

tattgatgag tgtgggaccg ggaggcacag ctgtgccaat gataccattt gcttcaattt 2460

ggatggcgga tatgattgtc gatgtcctca tggaaagaat tgcacagggg actgcatcca 2520

tgatggaaaa gttaagcaca atggtcagat ttgggtgttg gaaaatgaca ggtgctctgt 2580

gtgctcatgt cagaatggat tcgttatgtg tcgacggatg gtctgtgact gtgagaatcc 2640

cacagttgat cttttttgct gccctgaatg tgacccaagg cttagtagtc agtgcctcca 2700

tcaaaatggg gaaactttgt ataacagtgg tgacacctgg gtccagaatt gtcaacagtg 2760

ccgctgcttg caaggggaag ttgattgttg gcccctgcct tgcccagatg tggagtgtga 2820

attcagcatt ctcccagaga atgagtgctg cccgcgctgt gtcacagacc cttgccaggc 2880

tgacaccatc cgcaatgaca tcaccaagac ttgcctggac gaaatgaatg tggttcgctt 2940

caccgggtcc tcttggatca aacatggcac tgagtgtact ctctgccagt gcaagaatgg 3000

ccacatctgt tgctcagtgg atccacagtg ccttcaggaa ctgtgaagtt aactgtctca 3060

tgggagattt ctgttaaaag aatgttcttt cattaaaaga ccaaaaagaa gttaaaactt 3120

aaattgggtg atttgtgggc agctaaatgc agctttgtta atagctgagt gaactttcaa 3180

ttatgaaatt tgtggagctt gacaaaatca caaaaggaaa attactgggg caaaattaga 3240

cctcaagtct gcctctactg tgtctcacat caccatgtag aagaatgggc gtacagtata 3300

taccgtgaca tcctgaaccc tggatagaaa gcctgagccc attggatctg tgaaagcctc 3360

tagcttcact ggtgcagaaa attttcctct agatcagaat cttcaagaat cagttaggtt 3420

cctcactgca agaaataaaa tgtcaggcag tgaatgaatt atattttcag aagtaaagca 3480

aagaagctat aacatgttat gtacagtaca ctctgaaaag aaatctgaaa caagttattg 3540

taatgataaa aataatgcac aggcatggtt acttaatatt ttctaacagg aaaagtcatc 3600

cctatttcct tgttttactg cacttaatat tatttggttg aatttgttca gtataagctc 3660

gttcttgtgc aaaattaaat aaatatttct cttaccttat aacac 3705

<210> 22
<211> 3312
<212> DNA
<213> Homo sapiens

<400> 22
tttggcatcc aggagaccgt gcgggagacc gacgctgagc gccggcctcg ccctcggcgg 60

cccctccctc ctccttaccc cgcctccccc ggcccgcccc tccctccccg gaggagcagt 120

ctctccgcgc gtctcccgga gctttctcca ttgtctctgc ctttacaaca gagggagacg 180

atggactgag ctgatccgca ccatggagtc tcgggtctta ctgagaacat tctgtttgat 240

cttcggtctc ggagcagttt gggggcttgg tgtggaccct tccctacaga ttgacgtctt 300

aacagagtta gaacttgggg agtccacgac cggagtgcgt caggtcccgg ggctgcataa 360

tgggacgaaa gcctttctct ttcaagatac tcccagaagc ataaaagcat ccactgctac 420

agctgaacag ttttttcaga agctgagaaa taaacatgaa tttactattt tggtgaccct 480
aaaacagacc cacttaaatt caggagttat tctctcaatt caccacttgg atcacaggta 540
cctggaactg gaaagtagtg gccatcggaa tgaagtcaga ctgcattacc gctcaggcag 600
tcaccgccct cacacagaag tgtttcctta cattttggct gatgacaagt ggcacaagct 660
ctccttagcc atcagtgctt cccatttgat tttacacatt gactgcaata aaatttatga 720
aagggtagta gaaaagccct ccacagactt gcctctaggc acaacatttt ggctaggaca 780
gagaaataat gcgcatggat attttaaggg tataatgcaa gatgtccaat tacttgtcat 840
gccccaggga tttattgctc agtgcccaga tcttaatcgc acctgtccaa cttgcaatga 900
cttccatgga cttgtgcaga aaatcatgga gctacaggat attttagcca aaacatcagc 960
caagctgtct cgagctgaac agcgaatgaa tagattggat cagtgctatt gtgaaaggac 1020
ttgcaccatg aagggaacca cctaccgaga atttgagtcc tggatagacg gctgtaagaa 1080
ctgcacatgc ctgaatggaa ccatccagtg tgaaactcta atctgcccaa atcctgactg 1140
cccacttaag tcggctcttg cgtatgtgga tggcaaatgc tgtaaggaat gcaaatcgat 1200
atgccaattt caaggacgaa cctactttga aggagaaaga aatacagtct attcctcttc 1260
tggagtatgt gttctctatg agtgcaagga ccagaccatg aaacttgttg agagttcagg 1320
ctgtccagct ttggattgtc cagagtctca tcagataacc ttgtctcaca gctgttgcaa 1380
agtttgtaaa ggttatgact tttgttctga aaggcataac tgcatggaga attccatctg 1440
cagaaatctg aatgacaggg ctgtttgtag ctgtcgagat ggttttaggg ctcttcgaga 1500
ggataatgcc tactgtgaag acatcgatga gtgtgctgaa gggcgccatt actgtcgtga 1560
aaatacaatg tgtgtcaaca ccccgggttc ttttatgtgc atctgcaaaa ctggatacat 1620
cagaattgat gattattcat gtacagaaca tgatgagtgt atcacaaatc agcacaactg 1680
tgatgaaaat gctttatgct tcaacactgt tggaggacac aactgtgttt gcaagccggg 1740
ctatacaggg aatggaacga catgcaaagc attttgcaaa gatggctgta ggaatggagg 1800
agcctgtatt gccgctaatg tgtgtgcctg cccacaaggc ttcactggac ccagctgtga 1860
aacggacatt gatgaatgct ctgatggttt tgttcaatgt gacagtcgtg ctaattgcat 1920
taacctgcct ggatggtacc actgtgagtg cagagatggc taccatgaca atgggatgtt 1980
ttcaccaagt ggagaatcgt gtgaagatat tgatgagtgt gggaccggga ggcacagctg 2040
tgccaatgat accatttgct tcaatttgga tggcggatat gattgtcgat gtcctcatgg 2100
aaagaattgc acaggggact gcatccatga tggaaaagtt aagcacaatg gtcagatttg 2160
ggtgttggaa aatgacaggt gctctgtgtg ctcatgtcag aatggattcg ttatgtgtcg 2220
acggatggtc tgtgactgtg agaatcccac agttgatctt ttttgctgcc ctgaatgtga 2280
cccaaggctt agtagtcagt gcctccatca aaatggggaa actttgtata acagtggtga 2340
cacctgggtc cagaattgtc aacagtgccg ctgcttgcaa ggggaagttg attgttggcc 2400
cctgccttgc ccagatgtgg agtgtgaatt cagcattctc ccagagaatg agtgctgccc 2460

gcgctgtgtc acagaccctt gccaggctga caccatccgc aatgacatca ccaagacttg 2520

cctggacgaa atgaatgtgg ttcgcttcac cgggtcctct tggatcaaac atggcactga 2580

gtgtactctc tgccagtgca agaatggcca catctgttgc tcagtggatc cacagtgcct 2640

tcaggaactg tgaagttaac tgtctcatgg gagatttctg ttaaaagaat gttctttcat 2700

taaaagacca aaaagaagtt aaaacttaaa ttgggtgatt tgtgggcagc taaatgcagc 2760

tttgttaata gctgagtgaa ctttcaatta tgaaatttgt ggagcttgac aaaatcacaa 2820

aaggaaaatt actggggcaa aattagacct caagtctgcc tctactgtgt ctcacatcac 2880

catgtagaag aatgggcgta cagtatatac cgtgacatcc tgaaccctgg atagaaagcc 2940

tgagcccatt ggatctgtga aagcctctag cttcactggt gcagaaaatt ttcctctaga 3000

tcagaatctt caagaatcag ttaggttcct cactgcaaga aataaaatgt caggcagtga 3060

atgaattata ttttcagaag taaagcaaag aagctataac atgttatgta cagtacactc 3120

tgaaaagaaa tctgaaacaa gttattgtaa tgataaaaat aatgcacagg catggttact 3180

taatattttc taacaggaaa agtcatccct atttccttgt tttactgcac ttaatattat 3240

ttggttgaat ttgttcagta taagctcgtt cttgtgcaaa attaaataaa tatttctctt 3300

accttataac ac 3312

<210> 23
<211> 3210
<212> DNA
<213> Homo sapiens

<400> 23
tttttctgtg cttgccttgg agaccgcgag gcggatcacg cccctagtcc agggtcggag 60

ggctgagggg gaactttgca gagagaaggg tttaaaggag gtcatggaaa cgggactggg 120

tgctccgcta tttaaagctt ggctgctgat ctcagtttgg gggcttggtg tggacccttc 180

cctacagatt gacgtcttaa cagagttaga acttggggag tccacgaccg gagtgcgtca 240

ggtcccgggg ctgcataatg ggacgaaagc ctttctcttt caagatactc ccagaagcat 300

aaaagcatcc actgctacag ctgaacagtt ttttcagaag ctgagaaata aacatgaatt 360

tactattttg gtgaccctaa aacagaccca cttaaattca ggagttattc tctcaattca 420

ccacttggat cacaggtacc tggaactgga aagtagtggc catcggaatg aagtcagact 480

gcattaccgc tcaggcagtc accgccctca cacagaagtg tttccttaca ttttggctga 540

tgacaagtgg cacaagctct ccttagccat cagtgcttcc catttgattt tacacattga 600

ctgcaataaa atttatgaaa gggtagtaga aaagccctcc acagacttgc ctctaggcac 660

aacattttgg ctaggacaga gaaataatgc gcatggatat tttaagggta taatgcaaga 720

tgtccaatta cttgtcatgc cccagggatt tattgctcag tgcccagatc ttaatcgcac 780

ctgtccaact tgcaatgact tccatggact tgtgcagaaa atcatggagc tacaggatat 840

tttagccaaa acatcagcca agctgtctcg agctgaacag cgaatgaata gattggatca 900

gtgctattgt gaaaggactt gcaccatgaa gggaaccacc taccgagaat ttgagtcctg 960
gatagacggc tgtaagaact gcacatgcct gaatggaacc atccagtgtg aaactctaat 1020
ctgcccaaat cctgactgcc cacttaagtc ggctcttgcg tatgtggatg gcaaatgctg 1080
taaggaatgc aaatcgatat gccaatttca aggacgaacc tactttgaag gagaaagaaa 1140
tacagtctat tcctcttctg gagtatgtgt tctctatgag tgcaaggacc agaccatgaa 1200
acttgttgag agttcaggct gtccagcttt ggattgtcca gagtctcatc agataacctt 1260
gtctcacagc tgttgcaaag tttgtaaagg ttatgacttt tgttctgaaa ggcataactg 1320
catggagaat tccatctgca gaaatctgaa tgacagggct gtttgtagct gtcgagatgg 1380
ttttagggct cttcgagagg ataatgccta ctgtgaagac atcgatgagt gtgctgaagg 1440
gcgccattac tgtcgtgaaa atacaatgtg tgtcaacacc ccgggttctt ttatgtgcat 1500
ctgcaaaact ggatacatca gaattgatga ttattcatgt acagaacatg atgagtgtat 1560
cacaaatcag cacaactgtg atgaaaatgc tttatgcttc aacactgttg gaggacacaa 1620
ctgtgtttgc aagccgggct atacagggaa tggaacgaca tgcaaagcat tttgcaaaga 1680
tggctgtagg aatggaggag cctgtattgc cgctaatgtg tgtgcctgcc cacaaggctt 1740
cactggaccc agctgtgaaa cggacattga tgaatgctct gatggttttg ttcaatgtga 1800
cagtcgtgct aattgcatta acctgcctgg atggtaccac tgtgagtgca gagatggcta 1860
ccatgacaat gggatgtttt caccaagtgg agaatcgtgt gaagatattg atgagtgtgg 1920
gaccgggagg cacagctgtg ccaatgatac catttgcttc aatttggatg gcggatatga 1980
ttgtcgatgt cctcatggaa agaattgcac aggggactgc atccatgatg gaaaagttaa 2040
gcacaatggt cagatttggg tgttggaaaa tgacaggtgc tctgtgtgct catgtcagaa 2100
tggattcgtt atgtgtcgac ggatggtctg tgactgtgag aatcccacag ttgatctttt 2160
ttgctgccct gaatgtgacc caaggcttag tagtcagtgc ctccatcaaa atggggaaac 2220
tttgtataac agtggtgaca cctgggtcca gaattgtcaa cagtgccgct gcttgcaagg 2280
ggaagttgat tgttggcccc tgccttgccc agatgtggag tgtgaattca gcattctccc 2340
agagaatgag tgctgcccgc gctgtgtcac agacccttgc caggctgaca ccatccgcaa 2400
tgacatcacc aagacttgcc tggacgaaat gaatgtggtt cgcttcaccg ggtcctcttg 2460
gatcaaacat ggcactgagt gtactctctg ccagtgcaag aatggccaca tctgttgctc 2520
agtggatcca cagtgccttc aggaactgtg aagttaactg tctcatggga gatttctgtt 2580
aaaagaatgt tctttcatta aaagaccaaa aagaagttaa aacttaaatt gggtgatttg 2640
tgggcagcta aatgcagctt tgttaatagc tgagtgaact ttcaattatg aaatttgtgg 2700
agcttgacaa aatcacaaaa ggaaaattac tggggcaaaa ttagacctca agtctgcctc 2760
tactgtgtct cacatcacca tgtagaagaa tgggcgtaca gtatataccg tgacatcctg 2820
aaccctggat agaaagcctg agcccattgg atctgtgaaa gcctctagct tcactggtgc 2880
agaaaatttt cctctagatc agaatcttca agaatcagtt aggttcctca ctgcaagaaa 2940

taaaatgtca ggcagtgaat gaattatatt ttcagaagta aagcaaagaa gctataacat 3000

gttatgtaca gtacactctg aaaagaaatc tgaaacaagt tattgtaatg ataaaaataa 3060

tgcacaggca tggttactta atattttcta acaggaaaag tcatccctat ttccttgttt 3120

tactgcactt aatattattt ggttgaattt gttcagtata agctcgttct tgtgcaaaat 3180

taaataaata tttctcttac cttataacac 3210

<210> 24
<211> 7603
<212> DNA
<213> Homo sapiens

<400> 24
ttttcttgct tttcttccct ttttttcttt tttgcaaaca aaacaaaaaa cagcatagaa 60

gaaagagcaa aataaagaag aagaagagga ggaagagagg gaaagagagg aagggaaaaa 120

aaacaccaac ccgggcagag gaggaggtgc ggcggcggcg gcggcggcgg cagcggcggc 180

agcggcgcgg cggcggctcg gaccccctcc cccggctccc cccatcagtg cagctctccg 240

ggcgatgcca gaatagatgc cggggcaatg tcccgccgca aacagggcaa cccgcagcac 300

ttgtcccaga gggagctcat caccccagag gctgaccatg tggaggccgc catcctcgaa 360

gaagacgagg gtctggagat agaggagcca agtggcctgg ggctgatggt gggtggcccc 420

gaccctgacc tgctcacctg tggccagtgt caaatgaact tccccttggg ggacatcctg 480

gtttttatag agcacaaaag gaagcagtgt ggcggcagct tgggtgcctg ctatgacaag 540

gccctggaca aggacagccc gccaccctcc tcacgctccg agctcaggaa agtgtccgag 600

ccggtggaga tcgggatcca agtcaccccc gacgaagatg accacctgct ctcacccacg 660

aaaggcatct gtcccaagca ggagaacatt gcaggtaaag atgagccttc cagctacatt 720

tgcacaacat gcaagcagcc cttcaacagc gcgtggttcc tgctgcagca cgcgcagaac 780

acgcacggct tccgcatcta cctggagccc gggccggcca gcagctcgct cacgccgcgg 840

ctcaccatcc cgccgccgct cgggccggag gccgtggcgc agtccccgct catgaatttc 900

ctgggcgaca gcaacccctt caacctgctg cgcatgacgg gccccatcct gcgggaccac 960

ccgggcttcg gcgagggccg cctgccgggc acgccgcctc tcttcagtcc cccgccgcgc 1020

caccacctgg acccgcaccg cctcagtgcc gaggagatgg ggctcgtcgc ccagcacccc 1080

agtgccttcg accgagtcat gcgcctgaac cccatggcca tcgactcgcc cgccatggac 1140

ttctcgcggc ggctccgcga gctggcgggc aacagctcca cgccgccgcc cgtgtccccg 1200

ggccgcggca accctatgca ccggctcctg aaccccttcc agcccagccc caagtccccg 1260

ttcctgagca cgccgccgct gccgcccatg ccccctggcg gcacgccgcc cccgcagccg 1320

ccagccaaga gcaagtcgtg cgagttctgc ggcaagacct tcaagttcca gagcaatctc 1380

atcgtgcacc ggcgcagtca cacgggcgag aagccctaca agtgccagct gtgcgaccac 1440

gcgtgctcgc aggccagcaa gctcaagcgc cacatgaaga cgcacatgca caaggccggc 1500

tcgctggccg gccgctccga cgacgggctc tcggccgcca gctcccccga gcccggcacc 1560

agcgagctgg cgggcgaggg cctcaaggcg gccgacggtg acttccgcca ccacgagagc 1620
gacccgtcgc tgggccacga gccggaggag gaggacgagg aggaggagga ggaggaggag 1680
gagctgctac tggagaacga gagccggccc gagtcgagct tcagcatgga ctcggagctg 1740
agccgcaacc gcgagaacgg cggtggtggg gtgcccgggg tcccgggcgc ggggggcggc 1800
gcggccaagg cgctggctga cgagaaggcg ctggtgctgg gcaaggtcat ggagaacgtg 1860
ggcctaggcg cactgccgca gtacggcgag ctcctggccg acaagcagaa gcgcggcgcc 1920
ttcctgaagc gtgcggcggg cggcggggac gcgggcgacg acgacgacgc gggcggctgc 1980
ggggacgcgg gcgcgggcgg cgcggtcaac gggcgcgggg gcggcttcgc gccaggcacc 2040
gagcccttcc ccgggctctt cccgcgcaag cccgcgccgc tgcccagccc cgggctcaac 2100
agcgccgcca agcgcatcaa ggtggagaag gacctggagc tgccgcccgc cgcgctcatc 2160
ccgtccgaga acgtgtactc gcagtggctg gtgggctacg cggcgtcgcg gcacttcatg 2220
aaggacccct tcctgggctt cacggacgca cgacagtcgc ccttcgccac gtcgtccgag 2280
cactcgtccg agaacggcag cctgcgcttc tccacgccgc ccggggacct gctggacggc 2340
ggcctctcgg gccgcagcgg cacggccagc ggaggcagca ccccgcacct gggcggcccg 2400
ggccccgggc ggcccagctc caaggagggc cgccgcagcg acacgtgcga gtactgcggc 2460
aaggtgttca agaactgcag caacttgacg gtgcaccggc ggagccacac cggcgagcgg 2520
ccttacaagt gcgagctgtg caactacgcg tgcgcgcaga gcagcaagct cacgcgccac 2580
atgaagacgc acgggcagat cggcaaggag gtgtaccgct gcgacatctg ccagatgccc 2640
ttcagcgtct acagcaccct ggagaaacac atgaaaaagt ggcacggcga gcacttgctg 2700
actaacgacg tcaaaatcga gcaggccgag aggagctaag cgcgcgggcc ccggcgcccc 2760
gcacctgtac agtggaaccg ttgccaaccg agagaatgct gacctgactt gcctccgtgt 2820
caccgccacc ccgcaccccg cgtgtccccg gggcccaggg gaggcggcac tccaacctaa 2880
cctgtgtctg cgaagtccta tggaaacccg agggttgatt aaggcagtac aaattgtgga 2940
gccttttaac tgtgcaataa tttctgtatt tattgggttt tgtaattttt ttggcatgtg 3000
caggtacttt ttattattat tttttctgtt tgaattcctt taagagattt tgttgggtat 3060
ccatcccttc tttgtttttt ttttaacccg gtagtagcct gagcaatgac tcgcaagcaa 3120
tgttagaggg gaagcatatc ttttaaatta taatttgggg ggaggggtgg tgctgctttt 3180
ttgaaattta agctaagcat gtgtaatttc ttgtgaagaa gccaacactc aaatgacttt 3240
taaagttgtt tactttttca ttccttcctt ttttttgtcc tgaaataaaa agtggcatgc 3300
agtttttttt ttaattattt tttaattttt tttttggttt ttgtttttgg ggtggggggt 3360
gtggatgtac agcggataac aatctttcaa gtcgtagcac tttgtttcag aactggaatg 3420
gagatgtagc actcatgtcg tcccgagtca agcggccttt tctgtgttga tttcggcttt 3480
catattacat aagggaaacc ttgagtggtg gtgctggggg aggcacccca cagactcagc 3540
gccgccagag atagggtttt tggagggctc ctctgggaaa tggcccgaca gcattctgag 3600

gttgtgcatg accagcagat actatcctgt tggtgtgccc tggggtgcca tggctgctat 3660
tcgctgtaga ttaggctaca taaaatgggc tgagggtacc tttttgggga gatggggtgg 3720
cctgcagtga cacagaaagg aagaaactag cggtgttctt ttaggcgttt tctggcttga 3780
cggcttctct ctttttttaa atcaccccca ccacataaat ctcaaatcct atgttgctac 3840
aaggggtcat ccatcatttc ccaagcagac gaatgcccta attaattgaa gttagtgttc 3900
tctcatttaa tgcacactga tgatattgta gggatgggtg gggtggggat cttgcaaatt 3960
tctattctct tttactgaaa aagcagggga tgagttccat cagaaggtgc ccagcgctac 4020
ttcccaggtt tttatttttt ttttcctatc tcattaggtt ggaaggtact aaatattgaa 4080
ctgttaagat tagacatttg aattctgttg acccgcactt taaagctttt gtttgcattt 4140
aaattaaatg gcttctaaac aagaaattgc agcatattct tctctttggc ccagaggtgg 4200
gttaaactgt aagggacagc tgagattgag tgtcagtatt gctaagcgtg gcattcacaa 4260
tactggcact ataaagaaca aaataaaata ataatttata ggacagtttt tctactgcca 4320
ttcaatttga tgtgagtgcc ttgaaaactg atcttcctat ttgagtctct tgagacaaat 4380
gcaaaacttt ttttttgaaa tgaaaagact ttttaaaaaa gtaaaacaag aaaagtacat 4440
tctttagaaa ctaacaaagc cacatttact ttaagtaaaa aaaaaaaaaa ttctggttga 4500
agatagagga tatgaaatgc cataagaccc aatcaaatga agaaataaac ccagcacaac 4560
cttggacatc cattagctga attatcctca gccccttttg tttttgggac aacgctgctt 4620
agatatggag tggaggtgat ttactgctga attaaaactc aagtgacaca agttacaagt 4680
tgatatcgtt gaatgaaaag caaaacaaaa acaattcagg aacaacggct aattttttct 4740
aaagttaaat ttagtgcact ctgtcttaaa aatacgttta cagtattggg tacatacaag 4800
ggtaaaaaaa aaattgtgtg tatgtgtgtt ggagcgatct ttttttttca aagtttgctt 4860
aataggttat acaaaaatgc cacagtggcc gcgtgtatat tgttttcttt tggtgacggg 4920
gttttagtat atattatata tattaaaatt tcttgattac tgtaaaagtg gaccagtatt 4980
tgtaataatc gagaatgcct gggcatttta caaaacaaga aaaaaaatac ccttttcttt 5040
tccttgaaaa tgttgcagta aaatttaaat ggtgggtcta taaatttgtt cttgttacag 5100
taactgtaaa gtcggagttt tagtaaattt ttttctgcct tgggtgttga atttttattt 5160
caaaaaaaat gtatagaaac ttgtatttgg ggattcaaag gggattgcta caccatgtag 5220
aaaaagtatg tagaaaaaaa gtgcttaata ttgttattgc tttgcagaaa aaaaaaaaat 5280
cacatttctg acctgtactt atttttctct tcccgcctcc ctctggaatg gatatattgg 5340
ttggttcata tgatgtaggc acttgctgta tttttactgg agctcgtaat tttttaactg 5400
taagcttgtc cttttaaagg gatttaatgt acctttttgt tagtgaattt ggaaataaaa 5460
agaaaaaaaa aacaaaaaca aacaggctgc cataatatat ttttttaatt tggcaggata 5520
aaatattgca aaaaaaacac atttgtatgt taagtcctat tgtacaggag aaaaagggtt 5580
gtttgacaac ctttgagaaa aagaaacaaa aggaagtagt taaatgcttt ggttcacaaa 5640

tcatttagtt gtatatattt tttgtcggaa ttggcctaca cagagaaccg ttcgtgttgg 5700
gcttctctct gaacgccccg aaccttgcat caaggctcct tggtgtggcc acagcagacc 5760
agatgggaaa ttatttgtgt tgagtggaaa aaaatcagtt tttgtaaaga tgtcagtaac 5820
attccacatc gtcctccctt tctctaagag gccatctcta agatgtcaga tgtagaggag 5880
agagagcgag agaacatctt ccttctctac catcactcct gtggcggtca ccaccaccac 5940
ctctcccgcc cttaccagca gaaagcaatg caaactgagc tgctttagtc cttgagaaat 6000
tgtgaaacaa acacaaatat cataaaagga gctggtgatt cagctgggtc caggtgaagt 6060
gacctgctgt tgagaccggt acaaattgga tttcaggaag gagactccat cacagccagg 6120
acctttcgtg ccatggagag tgttggcctc ttgtctttct tccctgcttt gctgctttgc 6180
tctctgaaac ctacattccg tcagtttccg aatgcgaggg cctgggatga atttggtgcc 6240
tttccatatc tcgttctctc tccttcccct gcgtttcctc tccatccttc atcctccatt 6300
ggtccttttt ttttctttca ttttttattt aatttctttt cttcctgtct gttcctcccc 6360
taatcctcta ttttattttt attttttgta aagccaagta gctttaagat aaagtggtgg 6420
tcttttggat gagggaataa tgcattttta aataaaatac caatatcagg aagccatttt 6480
ttatttcagg aaatgtaaga aaccattatt tcaggttatg aaagtataac caagcatcct 6540
tttgggcaat tccttaccaa atgcagaagc ttttctgttc gatgcactct ttcctccttg 6600
ccacttacct ttgcaaagtt aaaaaaaagg ggggagggaa tgggagagaa agctgagatt 6660
tcagtttcct actgcagttt cctacctgca gatccagggg ctgctgttgc ctttggatgc 6720
cccactgagg tcctagagtg cctccagggt ggtcttcctg tagtcataac agctagccag 6780
tgctcaccag cttaccagat tgccaggact aagccatccc aaagcacaag cattgtgtgt 6840
ctctgtgact gcagagaaga gagaattttg cttctgtttt gtgtttaaaa aaccaacacg 6900
gaagcagatg atcccgagag agaggcctct agcatgggtg acccagccga cctcaggccg 6960
gtttccgcac tgccacaact ttgttcaaag ttgcccccaa ttggaacctg ccacttggca 7020
ttagagggtc tttcatgggg agagaaggag actgaattac tctaagcaaa atgtgaaaag 7080
taaggaaatc agcctttcat cccggtccta agtaaccgtc agccgaaggt ctcgtggaac 7140
acaggcaaac ccgtgatttt ggtgctcctt gtaactcagc cctgcaaagc aaagtcccat 7200
tgatttaagt tgtttgcatt tgtactggca aggcaaaata tttttattac cttttctatt 7260
acttattgta tgagcttttg ttgtttactt ggaggttttg tcttttacta caagtttgga 7320
actatttatt attgcttggt atttgtgctc tgtttaagaa acaggcactt ttttttatta 7380
tggataaaat gttgagatga caggaggtca tttcaatatg gcttagtaaa atatttattg 7440
ttcctttatt ctctgtacaa gattttgggc ctcttttttt ccttaatgtc acaatgttga 7500
gttcagcatg tgtctgccat ttcatttgta cgcttgttca aaaccaagtt tgttctggtt 7560
tcaagttata aaaataaatt ggacatttaa cttgatctcc aaa 7603

<210> 25
<211> 7816
<212> DNA
<213> Homo sapiens

<400> 25
ttttcttgct tttcttccct tttttttctt tttgcaaaca aaacaaaaaa cagcatagaa 60

gaaagagcaa aataaagaag aagaagagga ggaagagagg gaaagagagg aagggaaaaa 120

aaacaccaac ccgggcagag gaggaggtgc ggcggcggcg gcggcggcgg cagcggcggc 180

agcggcgcgg cggcggctcg gaccccctcc cccggctccc cccatcagtg cagctctccg 240

ggcgatgcca gaatagatgc cggggcaatg tcccgccgca aacagggcaa cccgcagcac 300

ttgtcccaga gggagctcat caccccagag gctgaccatg tggaggccgc catcctcgaa 360

gaagacgagg gtctggagat agaggagcca agtggcctgg ggctgatggt gggtggcccc 420

gaccctgacc tgctcacctg tggccagtgt caaatgaact tccccttggg ggacatcctg 480

gtttttatag agcacaaaag gaagcagtgt ggcggcagct tgggtgcctg ctatgacaag 540

gccctggaca aggacagccc gccaccctcc tcacgctccg agctcaggaa agtgtccgag 600

ccggtggaga tcgggatcca agtcaccccc gacgaagatg accacctgct ctcacccacg 660

aaaggcatct gtcccaagca ggagaacatt gcagggccgt gcaggcctgc ccagctgcca 720

gcggtggccc ccatagctgc ctcctcccac cctcactcat ccgtgatcac ttcacctctg 780

cgtgccctgg gcgctctccc gccctgcctc cccctgccgt gctgcagcgc gcgcccggtc 840

tcgggtgacg ggactcaggg tgagggtcag acggaggctc cctttggatg ccagtgtcag 900

ttgtcaggta aagatgagcc ttccagctac atttgcacaa catgcaagca gcccttcaac 960

agcgcgtggt tcctgctgca gcacgcgcag aacacgcacg gcttccgcat ctacctggag 1020

cccgggccgg ccagcagctc gctcacgccg cggctcacca tcccgccgcc gctcgggccg 1080

gaggccgtgg cgcagtcccc gctcatgaat ttcctgggcg acagcaaccc cttcaacctg 1140

ctgcgcatga cgggccccat cctgcgggac cacccgggct tcggcgaggg ccgcctgccg 1200

ggcacgccgc ctctcttcag tcccccgccg cgccaccacc tggacccgca ccgcctcagt 1260

gccgaggaga tggggctcgt cgcccagcac cccagtgcct tcgaccgagt catgcgcctg 1320

aaccccatgg ccatcgactc gcccgccatg gacttctcgc ggcggctccg cgagctggcg 1380

ggcaacagct ccacgccgcc gcccgtgtcc ccgggccgcg gcaaccctat gcaccggctc 1440

ctgaacccct tccagcccag ccccaagtcc ccgttcctga gcacgccgcc gctgccgccc 1500

atgccccctg gcggcacgcc gcccccgcag ccgccagcca agagcaagtc gtgcgagttc 1560

tgcggcaaga ccttcaagtt ccagagcaat ctcatcgtgc accggcgcag tcacacgggc 1620

gagaagccct acaagtgcca gctgtgcgac cacgcgtgct cgcaggccag caagctcaag 1680

cgccacatga agacgcacat gcacaaggcc ggctcgctgg ccggccgctc cgacgacggg 1740

ctctcggccg ccagctcccc cgagcccggc accagcgagc tggcgggcga gggcctcaag 1800

gcggccgacg gtgacttccg ccaccacgag agcgacccgt cgctgggcca cgagccggag 1860

gaggaggacg aggaggagga ggaggaggag gaggagctgc tactggagaa cgagagccgg 1920
cccgagtcga gcttcagcat ggactcggag ctgagccgca accgcgagaa cggcggtggt 1980
ggggtgcccg gggtcccggg cgcggggggc ggcgcggcca aggcgctggc tgacgagaag 2040
gcgctggtgc tgggcaaggt catggagaac gtgggcctag gcgcactgcc gcagtacggc 2100
gagctcctgg ccgacaagca gaagcgcggc gccttcctga agcgtgcggc gggcggcggg 2160
gacgcgggcg acgacgacga cgcgggcggc tgcggggacg cgggcgcggg cggcgcggtc 2220
aacgggcgcg ggggcggctt cgcgccaggc accgagccct tccccgggct cttcccgcgc 2280
aagcccgcgc cgctgcccag ccccgggctc aacagcgccg ccaagcgcat caaggtggag 2340
aaggacctgg agctgccgcc cgccgcgctc atcccgtccg agaacgtgta ctcgcagtgg 2400
ctggtgggct acgcggcgtc gcggcacttc atgaaggacc ccttcctggg cttcacggac 2460
gcacgacagt cgcccttcgc cacgtcgtcc gagcactcgt ccgagaacgg cagcctgcgc 2520
ttctccacgc cgcccgggga cctgctggac ggcggcctct cgggccgcag cggcacggcc 2580
agcggaggca gcaccccgca cctgggcggc ccgggccccg ggcggcccag ctccaaggag 2640
ggccgccgca gcgacacgtg cgagtactgc ggcaaggtgt tcaagaactg cagcaacttg 2700
acggtgcacc ggcggagcca caccggcgag cggccttaca agtgcgagct gtgcaactac 2760
gcgtgcgcgc agagcagcaa gctcacgcgc cacatgaaga cgcacgggca gatcggcaag 2820
gaggtgtacc gctgcgacat ctgccagatg cccttcagcg tctacagcac cctggagaaa 2880
cacatgaaaa agtggcacgg cgagcacttg ctgactaacg acgtcaaaat cgagcaggcc 2940
gagaggagct aagcgcgcgg gccccggcgc cccgcacctg tacagtggaa ccgttgccaa 3000
ccgagagaat gctgacctga cttgcctccg tgtcaccgcc accccgcacc ccgcgtgtcc 3060
ccggggccca ggggaggcgg cactccaacc taacctgtgt ctgcgaagtc ctatggaaac 3120
ccgagggttg attaaggcag tacaaattgt ggagcctttt aactgtgcaa taatttctgt 3180
atttattggg ttttgtaatt tttttggcat gtgcaggtac tttttattat tatttttct 3240
gtttgaattc ctttaagaga ttttgttggg tatccatccc ttctttgttt tttttttaac 3300
ccggtagtag cctgagcaat gactcgcaag caatgttaga ggggaagcat atcttttaaa 3360
ttataatttg gggggagggg tggtgctgct tttttgaaat ttaagctaag catgtgtaat 3420
ttcttgtgaa gaagccaaca ctcaaatgac ttttaaagtt gtttactttt tcattccttc 3480
cttttttttg tcctgaaata aaaagtggca tgcagttttt tttttaatta ttttttaatt 3540
ttttttttgg tttttgtttt tggggtgggg ggtgtggatg tacagcggat aacaatcttt 3600
caagtcgtag cactttgttt cagaactgga atggagatgt agcactcatg tcgtcccgag 3660
tcaagcggcc ttttctgtgt tgatttcggc tttcatatta cataaggaaa accttgagtg 3720
gtggtgctgg gggaggcacc ccacagactc agcgccgcca gagatagggt ttttggaggg 3780
ctcctctggg aaatggcccg acagcattct gaggttgtgc atgaccagca gatactatcc 3840
tgttggtgtg ccctggggtg ccatggctgc tattcgctgt agattaggct acataaaatg 3900

ggctgagggt acctttttgg ggagatgggg tggcctgcag tgacacagaa aggaagaaac 3960
tagcggtgtt cttttaggcg ttttctggct tgacggcttc tctctttttt taaatcaccc 4020
ccaccacata aatctcaaat cctatgttgc tacaaggggt catccatcat ttcccaagca 4080
gacgaatgcc ctaattaatt gaagttagtg ttctctcatt taatgcacac tgatgatatt 4140
gtagggatgg gtggggtggg gatcttgcaa atttctattc tcttttactg aaaaagcagg 4200
ggatgagttc catcagaagg tgcccagcgc tacttcccag gtttttattt tttttttcct 4260
atctcattag gttggaaggt actaaatatt gaactgttaa gattagacat ttgaattctg 4320
ttgacccgca ctttaaagct tttgtttgca tttaaattaa atggcttcta aacaagaaat 4380
tgcagcatat tcttctcttt ggcccagagg tgggttaaac tgtaagggac agctgagatt 4440
gagtgtcagt attgctaagc gtggcattca caatactggc actataaaga acaaaataaa 4500
ataataattt ataggacagt ttttctactg ccattcaatt tgatgtgagt gccttgaaaa 4560
ctgatcttcc tatttgagtc tcttgagaca aatgcaaaac tttttttttg aaatgaaaag 4620
actttttaaa aaagtaaaac aagaaaagta cattctttag aaactaacaa agccacattt 4680
actttaagta aaaaaaaaaa aaattctggt tgaagataga ggatatgaaa tgccataaga 4740
cccaatcaaa tgaagaaata aacccagcac aaccttggac atccattagc tgaattatcc 4800
tcagcccctt ttgtttttgg gacaacgctg cttagatatg gagtggaggt gatttactgc 4860
tgaattaaaa ctcaagtgac acaagttaca agttgatatc gttgaatgaa aagcaaaaca 4920
aaaacaattc aggaacaacg gctaattttt tctaaagtta aatttagtgc actctgtctt 4980
aaaaatacgt ttacagtatt gggtacatac aagggtaaaa aaaaaattgt gtgtatgtgt 5040
gttggagcga tctttttttt tcaaagtttg cttaataggt tatacaaaaa tgccacagtg 5100
gccgcgtgta tattgttttc ttttggtgac ggggttttag tatatattat atatattaaa 5160
atttcttgat tactgtaaaa gtggaccagt atttgtaata atcgagaatg cctgggcatt 5220
ttacaaaaca agaaaaaaaa tacccttttc ttttccttga aaatgttgca gtaaaattta 5280
aatggtgggt ctataaattt gttcttgtta cagtaactgt aaagtcggag ttttagtaaa 5340
tttttttctg ccttgggtgt tgaattttta tttcaaaaaa aatgtataga aacttgtatt 5400
tggggattca aaggggattg ctacaccatg tagaaaaagt atgtagaaaa aaagtgctta 5460
atattgttat tgctttgcag aaaaaaaaaa aatcacattt ctgacctgta cttatttttc 5520
tcttcccgcc tccctctgga atggatatat tggttggttc atatgatgta ggcacttgct 5580
gtatttttac tggagctcgt aattttttaa ctgtaagctt gtccttttaa agggatttaa 5640
tgtacctttt tgttagtgaa tttggaaata aaaagaaaaa aaaaacaaaa acaaacaggc 5700
tgccataata tattttttta atttggcagg ataaaatatt gcaaaaaaaa cacatttgta 5760
tgttaagtcc tattgtacag gagaaaaagg gttgtttgac aacctttgag aaaaagaaac 5820
aaaaggaagt agttaaatgc tttggttcac aaatcattta gttgtatata ttttttgtcg 5880
gaattggcct acacagagaa ccgttcgtgt tgggcttctc tctgaacgcc ccgaaccttg 5940

catcaaggct ccttggtgtg gccacagcag accagatggg aaattatttg tgttgagtgg 6000
aaaaaaatca gtttttgtaa agatgtcagt aacattccac atcgtcctcc ctttctctaa 6060
gaggccatct ctaagatgtc agatgtagag gagagagagc gagagaacat cttccttctc 6120
taccatcact cctgtggcgg tcaccaccac cacctctccc gcccttacca gcagaaagca 6180
atgcaaactg agctgcttta gtccttgaga aattgtgaaa caaacacaaa tatcataaaa 6240
ggagctggtg attcagctgg gtccaggtga agtgacctgc tgttgagacc ggtacaaatt 6300
ggatttcagg aaggagactc catcacagcc aggacctttc gtgccatgga gagtgttggc 6360
ctcttgtctt tcttccctgc tttgctgctt tgctctctga aacctacatt ccgtcagttt 6420
ccgaatgcga gggcctggga tgaatttggt gcctttccat atctcgttct ctctccttcc 6480
cctgcgtttc ctctccatcc ttcatcctcc attggtcctt tttttttctt tcatttttta 6540
tttaatttct tttcttcctg tctgttcctc ccctaatcct ctattttatt tttatttttt 6600
gtaaagccaa gtagctttaa gataaagtgg tggtcttttg gatgagggaa taatgcattt 6660
ttaaataaaa taccaatatc aggaagccat tttttatttc aggaaatgta agaaaccatt 6720
atttcaggtt atgaaagtat aaccaagcat ccttttgggc aattccttac caaatgcaga 6780
agcttttctg ttcgatgcac tctttcctcc ttgccactta cctttgcaaa gttaaaaaaa 6840
aggggggagg gaatgggaga gaaagctgag atttcagttt cctactgcag tttcctacct 6900
gcagatccag gggctgctgt tgcctttgga tgccccactg aggtcctaga gtgcctccag 6960
ggtggtcttc ctgtagtcat aacagctagc cagtgctcac cagcttacca gattgccagg 7020
actaagccat cccaaagcac aagcattgtg tgtctctgtg actgcagaga agagagaatt 7080
ttgcttctgt tttgtgttta aaaaaccaac acggaagcag atgatcccga gagagaggcc 7140
tctagcatgg gtgacccagc cgacctcagg ccggtttccg cactgccaca actttgttca 7200
aagttgcccc caattggaac ctgccacttg gcattagagg gtctttcatg gggagagaag 7260
gagactgaat tactctaagc aaaatgtgaa aagtaaggaa atcagccttt catcccggtc 7320
ctaagtaacc gtcagccgaa ggtctcgtgg aacacaggca aacccgtgat tttggtgctc 7380
cttgtaactc agccctgcaa agcaaagtcc cattgattta agttgtttgc atttgtactg 7440
gcaaggcaaa atattttat taccttttct attacttatt gtatgagctt ttgttgttta 7500
cttggaggtt ttgtctttta ctacaagttt ggaactattt attattgctt ggtatttgtg 7560
ctctgtttaa gaaacaggca cttttttta ttatggataa aatgttgaga tgacaggagg 7620
tcatttcaat atggcttagt aaaatattta ttgttccttt attctctgta caagatttg 7680
ggcctctttt tttccttaat gtcacaatgt tgagttcagc atgtgtctgc catttcattt 7740
gtacgcttgt tcaaaaccaa gtttgttctg gtttcaagtt ataaaaataa attggacatt 7800
taacttgatc tccaaa 7816

<210> 26
<211> 1690
<212> DNA

<213>  Homo sapiens

<400>  26
tgctttctca aaggccccac agtcctccac ttcctgggga ggtagctgca gaataaaacc      60

agcagagact ccttttctcc taaccgtccc ggccaccgct gcctcagcct ctgcctccca     120

gcctctttct gagggaaagg acaagatgaa gtggaaggcg cttttcaccg cggccatcct     180

gcaggcacag ttgccgatta cagaggcaca gagctttggc ctgctggatc ccaaactctg     240

ctacctgctg gatggaatcc tcttcatcta tggtgtcatt ctcactgcct tgttcctgag     300

agtgaagttc agcaggagcg cagacgcccc cgcgtaccag cagggccaga accagctcta     360

taacgagctc aatctaggac gaagagagga gtacgatgtt ttggacaaga gacgtggccg     420

ggaccctgag atggggggaa agccgcagag aaggaagaac cctcaggaag gcctgtacaa     480

tgaactgcag aaagataaga tggcggaggc ctacagtgag attgggatga aaggcgagcg     540

ccggaggggc aaggggcacg atggccttta ccagggtctc agtacagcca ccaaggacac     600

ctacgacgcc cttcacatgc aggccctgcc ccctcgctaa cagccagggg atttcaccac     660

tcaaaggcca gacctgcaga cgcccagatt atgagacaca ggatgaagca tttacaaccc     720

ggttcactct tctcagccac tgaagtattc ccctttatgt acaggatgct ttggttatat     780

ttagctccaa accttcacac acagactgtt gtccctgcac tctttaaggg agtgtactcc     840

cagggcttac ggccctggcc ttgggccctc tggtttgccg gtggtgcagg tagacctgtc     900

tcctggcggt tcctcgttct ccctgggagg cgggcgcact gcctctcaca gctgagttgt     960

tgagtctgtt ttgtaaagtc cccagagaaa gcgcagatgc tagcacatgc cctaatgtct    1020

gtatcactct gtgtctgagt ggcttcactc ctgctgtaaa tttggcttct gttgtcacct    1080

tcacctcctt tcaaggtaac tgtactgggc catgttgtgc ctccctggtg agagggccgg    1140

gcagaggggc agatggaaag gagcctaggc caggtgcaac cagggagctg caggggcatg    1200

ggaaggtggg cgggcagggg agggtcagcc agggcctgcg agggcagcgg gagcctccct    1260

gcctcaggcc tctgtgccgc accattgaac tgtaccatgt gctacagggg ccagaagatg    1320

aacagactga ccttgatgag ctgtgcacaa agtggcataa aaaacatgtg gttacacagt    1380

gtgaataaag tgctgcggag caagaggagg ccgttgattc acttcacgct ttcagcgaat    1440

gacaaaatca tctttgtgaa ggcctcgcag gaagacccaa cacatgggac ctataactgc    1500

ccagcggaca gtggcaggac aggaaaaacc cgtcaatgta ctaggatact gctgcgtcat    1560

tacagggcac aggccatgga tggaaaacgc tctctgctct gctttttttc tactgtttta    1620

atttatactg gcatgctaaa gccttcctat tttgcataat aaatgcttca gtgaaaatgc    1680

aaaaaaaaaa                                                           1690

<210>  27
<211>  1687
<212>  DNA
<213>  Homo sapiens

<400>  27

tgctttctca aaggccccac agtcctccac ttcctgggga ggtagctgca gaataaaacc 60

agcagagact ccttttctcc taaccgtccc ggccaccgct gcctcagcct ctgcctccca 120

gcctctttct gagggaaagg acaagatgaa gtggaaggcg cttttcaccg cggccatcct 180

gcaggcacag ttgccgatta cagaggcaca gagctttggc ctgctggatc ccaaactctg 240

ctacctgctg gatggaatcc tcttcatcta tggtgtcatt ctcactgcct tgttcctgag 300

agtgaagttc agcaggagcg cagacgcccc cgcgtaccag cagggccaga accagctcta 360

taacgagctc aatctaggac gaagagagga gtacgatgtt ttggacaaga gacgtggccg 420

ggaccctgag atggggggaa agccgagaag gaagaaccct caggaaggcc tgtacaatga 480

actgcagaaa gataagatgg cggaggccta cagtgagatt gggatgaaag gcgagcgccg 540

gaggggcaag gggcacgatg gcctttacca gggtctcagt acagccacca aggacaccta 600

cgacgccctt cacatgcagg ccctgccccc tcgctaacag ccaggggatt tcaccactca 660

aaggccagac ctgcagacgc ccagattatg agacacagga tgaagcattt acaacccggt 720

tcactcttct cagccactga agtattcccc tttatgtaca ggatgctttg gttatattta 780

gctccaaacc ttcacacaca gactgttgtc cctgcactct ttaagggagt gtactcccag 840

ggcttacggc cctggccttg ggccctctgg tttgccggtg gtgcaggtag acctgtctcc 900

tggcggttcc tcgttctccc tgggaggcgg gcgcactgcc tctcacagct gagttgttga 960

gtctgttttg taaagtcccc agagaaagcg cagatgctag cacatgccct aatgtctgta 1020

tcactctgtg tctgagtggc ttcactcctg ctgtaaattt ggcttctgtt gtcaccttca 1080

cctcctttca aggtaactgt actgggccat gttgtgcctc cctggtgaga gggccgggca 1140

gaggggcaga tggaaaggag cctaggccag gtgcaaccag ggagctgcag gggcatggga 1200

aggtgggcgg gcaggggagg gtcagccagg gcctgcgagg gcagcgggag cctccctgcc 1260

tcaggcctct gtgccgcacc attgaactgt accatgtgct acaggggcca gaagatgaac 1320

agactgacct tgatgagctg tgcacaaagt ggcataaaaa acatgtggtt acacagtgtg 1380

aataaagtgc tgcggagcaa gaggaggccg ttgattcact tcacgctttc agcgaatgac 1440

aaaatcatct ttgtgaaggc ctcgcaggaa gacccaacac atgggaccta taactgccca 1500

gcggacagtg gcaggacagg aaaaacccgt caatgtacta ggatactgct gcgtcattac 1560

agggcacagg ccatggatgg aaaacgctct ctgctctgct ttttttctac tgttttaatt 1620

tatactggca tgctaaagcc ttcctatttt gcataataaa tgcttcagtg aaaatgcaaa 1680

aaaaaaa 1687

<210> 28
<211> 10924
<212> DNA
<213> Homo sapiens

<400> 28
   ccggcccagc ggctctcctg gcctcgcgct gcacattctc tcctggcggc ggcgccacct 60
   gcagtagcgt tcgcccgaac atggcgacac ggagcagcag gagggagtcg cgactcccgt 120
   tcctattcac cctggtcgca ctgctgccgc ccggagctct ctgcgaagtc tggacgcaga 180
   ggctgcacgg cggcagcgcg cccttgcccc aggaccgggg cttcctcgtg gtgcagggcg 240
   acccgcgcga gctgcggctg tgggcgcgcg gggatgccag gggggcgagc cgcgcggacg 300
   agaagccgct ccggaggaaa cggagcgctg ccctgcagcc cgagcccatc aaggtgtacg 360
   gacaggttag tctgaatgat tcccacaatc agatggtggt gcactgggct ggagagaaaa 420
   gcaacgtgat cgtggccttg gcccgagata gcctggcatt ggcgaggccc aagagcagtg 480
   atgtgtacgt gtcttacgac tatggaaaat cattcaagaa aatttcagac aagttaaact 540
   ttggcttggg aaataggagt gaagctgtta tcgcccagtt ctaccacagc cctgcggaca 600
   acaagcggta catctttgca gacgcttatg cccagtacct ctggatcacg tttgacttct 660
   gcaacactct tcaaggcttt tccatcccat ttcgggcagc tgatctcctc ctacacagta 720
   aggcctccaa ccttctcttg ggctttgaca ggtcccaccc caacaagcag ctgtggaagt 780
   cagatgactt tggccagacc tggatcatga ttcaggaaca tgtcaagtcc ttttcttggg 840
   gaattgatcc ctatgacaaa ccaaatacca tctacattga acgacatgaa ccctctggct 900
   actccactgt cttccgaagt acagatttct tccagtcccg ggaaaaccag gaagtgatcc 960
   ttgaggaagt gagagatttt cagcttcggg acaagtacat gtttgctaca aaggtggtgc 1020
   atctcttggg cagtgaacag cagtcttctg tccagctctg ggtctccttt ggccggaagc 1080
   ccatgagagc agcccagttt gtcacaagac atcctattaa tgaatattac atcgcagatg 1140
   cctccgagga ccaggtgttt gtgtgtgtca gccacagtaa caaccgcacc aatttataca 1200
   tctcagaggc agaggggctg aagttctccc tgtccttgga gaacgtgctc tattacagcc 1260
   caggaggggc cggcagtgac accttggtga ggtattttgc aaatgaacca tttgctgact 1320
   tccaccgagt ggaaggattg caaggagtct acattgctac tctgattaat ggttctatga 1380
   atgaggagaa catgagatcg gtcatcacct ttgacaaagg gggaacctgg gagtttcttc 1440
   aggctccagc cttcacggga tatggagaga aaatcaattg tgagctttcc cagggctgtt 1500
   cccttcatct ggctcagcgc ctcagtcagc tcctcaacct ccagctccgg agaatgccca 1560
   tcctgtccaa ggagtcggct ccaggcctca tcatcgccac tggctcagtg ggaaagaact 1620
   tggctagcaa gacaaacgtg tacatctcta gcagtgctgg agccaggtgg cgagaggcac 1680
   ttcctggacc tcactactac acatggggag accacggcgg aatcatcacg gccattgccc 1740
   agggcatgga aaccaacgag ctaaaataca gtaccaatga aggggagacc tggaaaacat 1800
   tcatcttctc tgagaagcca gtgtttgtgt atggcctcct cacagaacct ggggagaaga 1860
   gcactgtctt caccatcttt ggctcgaaca aagagaatgt ccacagctgg ctgatcctcc 1920
   aggtcaatgc cacggatgcc ttgggagttc cctgcacaga gaatgactac aagctgtggt 1980
   caccatctga tgagcggggg aatgagtgtt tgctgggaca caagactgtt ttcaaacggc 2040
   ggacccccca tgccacatgc ttcaatggag aggactttga caggccggtg gtcgtgtcca 2100
   actgctcctg cacccgggag gactatgagt gtgacttcgg tttcaagatg agtgaagatt 2160
   tgtcattaga ggtttgtgtt ccagatccgg aattttctgg aaagtcatac tcccctcctg 2220
   tgccttgccc tgtgggttct acttacagga gaacgagagg ctaccggaag atttctgggg 2280
   acacttgtag cggaggagat gttgaagcgc gactggaagg agagctggtc ccctgtcccc 2340
   tggcagaaga gaacgagttc attctgtatg ctgtgaggaa atccatctac cgctatgacc 2400
   tggcctcggg agccaccgag cagttgcctc tcaccgggct acgggcagca gtggccctgg 2460
   actttgacta tgagcacaac tgtttgtatt ggtccgacct ggccttggac gtcatccagc 2520
   gcctctgttt gaatggaagc acagggcaag aggtgatcat caattctggc ctggagacag 2580
   tagaagcttt ggcttttgaa cccctcagcc agctgcttta ctgggtagat gcaggcttca 2640
   aaaagattga ggtagctaat ccagatggcg acttccgact cacaatcgtc aattcctctg 2700
   tgcttgatcg tcccagggct ctggtcctcg tgccccaaga gggggtgatg ttctggacag 2760
   actggggaga cctgaagcct gggatttatc ggagcaatat ggatggttct gctgcctatc 2820
   acctggtgtc tgaggatgtg aagtggccca atggcatctc tgtggacgac cagtggattt 2880
   actggacgga tgcctacctg gagtgcatag agcggatcac gttcagtggc cagcagcgct 2940
   ctgtcattct ggacaacctc ccgcacccct atgccattgc tgtctttaag aatgaaatct 3000
   actgggatga ctggtcacag ctcagcatat tccgagcttc caaatacagt gggtcccaga 3060
   tggagattct ggcaaaccag ctcacggggc tcatggacat gaagattttc tacaagggga 3120
   agaacactgg aagcaatgcc tgtgtgccca ggccatgcag cctgctgtgc ctgcccaagg 3180
   ccaacaacag tagaagctgc aggtgtccag aggatgtgtc cagcagtgtg cttccatcag 3240
   gggacctgat gtgtgactgc cctcagggct atcagctcaa gaacaatacc tgtgtcaaag 3300
   aagagaacac ctgtcttcgc aaccagtatc gctgcagcaa cgggaactgt atcaacagca 3360
   tttggtggtg tgactttgac aacgactgtg gagacatgag cgatgagaga aactgcccta 3420
   ccaccatctg tgacctggac acccagtttc gttgccagga gtctgggact tgtatcccac 3480
   tgtcctataa atgtgacctt gaggatgact gtggagacaa cagtgatgaa agtcattgtg 3540
   aaatgcacca gtgccggagt gacgagtaca actgcagttc cggcatgtgc atccgctcct 3600
   cctgggtatg tgacggggac aacgactgca gggactggtc tgatgaagcc aactgtaccg 3660
   ccatctatca cacctgtgag gcctccaact tccagtgccg aaacgggcac tgcatccccc 3720
   agcggtgggc gtgtgacggg gatacggact gccaggatgg ttccgatgag gatccagtca 3780
   actgtgagaa gaagtgcaat ggattccgct gcccaaacgg cacttgcatc ccatccagca 3840
   aacattgtga tggtctgcgt gattgctctg atggctccga tgaacagcac tgcgagcccc 3900
   tctgtacgca cttcatggac tttgtgtgta agaaccgcca gcagtgcctg ttccactcca 3960
   tggtctgtga cggaatcatc cagtgccgcg acgggtccga tgaggatgcg gcgtttgcag 4020
   gatgctccca agatcctgag ttccacaagg tatgtgatga gttcggtttc cagtgtcaga 4080
   atggagtgtg catcagtttg atttggaagt gcgacgggat ggatgattgc ggcgattatt 4140
   ctgatgaagc caactgcgaa aaccccacag aagccccaaa ctgctcccgc tacttccagt 4200
   ttcggtgtga gaatggccac tgcatcccca acagatggaa atgtgacagg gagaacgact 4260
   gtggggactg gtctgatgag aaggattgtg gagattcaca tattcttccc ttctcgactc 4320
   ctgggccctc cacgtgtctg cccaattact accgctgcag cagtgggacc tgcgtgatgg 4380
   acacctgggt gtgcgacggg taccgagatt gtgcagatgg ctctgacgag gaagcctgcc 4440
   ccttgcttgc aaacgtcact gctgcctcca ctcccaccca acttgggcga tgtgaccgat 4500
   ttgagttcga atgccaccaa ccgaagacgt gtattcccaa ctggaagcgc tgtgacggcc 4560
   accaagattg ccaggatggc cgggacgagg ccaattgccc cacacacagc accttgactt 4620
   gcatgagcag ggagttccag tgcgaggacg gggaggcctg cattgtgctc tcggagcgct 4680
   gcgacggctt cctggactgc tcggacgaga gcgatgaaaa ggcctgcagt gatgagttga 4740
   ctgtgtacaa agtacagaat cttcagtgga cagctgactt ctctggggat gtgactttga 4800
   cctggatgag gcccaaaaaa atgccctctg cttcttgtgt atataatgtc tactacaggg 4860
   tggttggaga gagcatatgg aagactctgg agacccacag caataagaca aacactgtat 4920
   taaaagtctt gaaaccagat accacgtatc aggttaaagt acaggttcag tgtctcagca 4980
   aggcacacaa caccaatgac tttgtgaccc tgaggacccc agagggattg ccagatgccc 5040
   ctcgaaatct ccagctgtca ctccccaggg aagcagaagg tgtgattgta ggccactggg 5100
   ctcctcccat ccacacccat ggcctcatcc gtgagtacat tgtagaatac agcaggagtg 5160
   gttccaagat gtgggcctcc cagagggctg ctagtaactt tacagaaatc aagaacttat 5220
   tggtcaacac tctatacacc gtcagagtgg ctgcggtgac tagtcgtgga ataggaaact 5280
   ggagcgattc taaatccatt accaccataa aaggaaaagt gatcccacca ccagatatcc 5340
   acattgacag ctatggtgaa aattatctaa gcttcaccct gaccatggag agtgatatca 5400
   aggtgaatgg ctatgtggtg aaccttttct gggcatttga cacccacaag caagagagga 5460
   gaactttgaa cttccgagga agcatattgt cacacaaagt tggcaatctg acagctcata 5520
   catcctatga gatttctgcc tgggccaaga ctgacttggg ggatagccct ctggcatttg 5580
   agcatgttat gaccagaggg gttcgcccac ctgcacctag cctcaaggcc aaagccatca 5640
   accagactgc agtggaatgt acctggaccg gcccccggaa tgtggtttat ggtattttct 5700
   atgccacgtc ctttcttgac ctctatcgca acccgaagag cttgactact tcactccaca 5760
   acaagacggt cattgtcagt aaggatgagc agtatttgtt tctggtccgt gtagtggtac 5820
   cctaccaggg gccatcctct gactacgttg tagtgaagat gatcccggac agcaggcttc 5880
   caccccgtca cctgcatgtg gttcatacgg gcaaaacctc cgtggtcatc aagtgggaat 5940
   caccgtatga ctctcctgac caggacttgt tgtatgcaat tgcagtcaaa gatctcataa 6000
   gaaagactga caggagctac aaagtaaaat cccgtaacag cactgtggaa tacaccctta 6060
   acaagttgga gcctggcggg aaataccaca tcattgtcca actggggaac atgagcaaag 6120
   attccagcat aaaaattacc acagtttcat tatcagcacc tgatgcctta aaaatcataa 6180
   cagaaaatga tcatgttctt ctgttttgga aaagcctggc tttaaaggaa aagcatttta 6240
   atgaaagcag gggctatgag atacacatgt ttgatagtgc catgaatatc acagcttacc 6300
   ttgggaatac tactgacaat ttctttaaaa tttccaacct gaagatgggt cataattaca 6360
   cgttcaccgt ccaagcaaga tgcctttttg gcaaccagat ctgtggggag cctgccatcc 6420
   tgctgtacga tgagctgggg tctggtgcag atgcatctgc aacgcaggct gccagatcta 6480
   cggatgttgc tgctgtggtg gtgcccatct tattcctgat actgctgagc ctgggggtgg 6540
   ggtttgccat cctgtacacg aagcaccgga ggctgcagag cagcttcacc gccttcgcca 6600
   acagccacta cagctccagg ctggggtccg caatcttctc ctctggggat gacctggggg 6660
   aagatgatga agatgcccct atgataactg gattttcaga tgacgtcccc atggtgatag 6720
   cctgaaagag ctttcctcac tagaaaccaa atggtgtaaa tattttattt gataaagata 6780
   gttgatggtt tattttaaaa gatgcacttt gagttgcaat atgttatttt tatatgggcc 6840
   aaaaacaaaa aacaaaaaaa aaaaaaagga aagaaaggaa tgaataaact ttgtagtaat 6900
   caactgtgaa cttcaaacca ggttgatttt agtaacccaa ttgctttgat ttgacattaa 6960
   tgtagtctta cagggctgtg cttgctgggc atgcttttac gtctgtgaga taatttcggt 7020
   tcagtaaatt ggccaatctt tttatttttc taagacacag aaatgtattt aataaaaacc 7080
   tcgagagagt gatgggtgga accccttctc cttgaaagtg tgtacagata ttccattttg 7140
   tttggatata gtttatagga aagtgtgtgg atgtattatg gcggaaggtt tctttatgtt 7200
   attttgttaa tttattggga ctctgtgtaa ggccaggctt tagtggtcat tagacaccac 7260
   atgtgttatg agccccttac ccatagggtt gggggtggga agagaagcat atttttttgc 7320
   cattccggaa gcaatccatt tttattcact tgtgtgtcat gtaatggtct ttggcaggag 7380
   agagcactga gtcattgctg gagttcagtt caacagagct gcagcttggg aagccctgta 7440
   agcccacagc ttcctctctt atattaattg atggaatttt actgtatgtg cctctgtaca 7500
   agatgtagct ttgagagcta caaaatgata acactgcttt attacacact ggtttcattg 7560
   tcattgcaaa aacttaccct ggttgtgggg gagagttcta gatctgtgcc atgatccata 7620
   cactggctaa tagagtacat aatttttcca ttttccattt tttgttttta cttactactg 7680
   aaggatctca gatgtaaaat tatgtatttg gtttgagatg gccacttatt gtccttaaaa 7740
   atccatactg atatatgcag tcattttgaa ttggacagtg ccttctcttt ttttttctcc 7800
   tcttcttcca tctccctcac ccatgccccc acccaatcta aagagacagt gctgtacatt 7860
   ctcatagaga tagagaagat ctaaaaagtt gagactactc aatccagtta acaacagcag 7920
   gagcactaga gtttgttcat ttattctctc tgtaaaacaa gctgtgcttt ttttcttctg 7980
   cctttaaaat gccacccgtg tattcaaacc atggccactt gatacttatg tagaatccat 8040
   cgtgggctga tgcaagccct ttatttaggc ttagtgttgt gggcaccaat gtcgagcatc 8100
   gttgtgactt gtgctgtatg attctcactg aagaatttcc tttcagccaa gaagcagtga 8160
   ggtctgggaa tattccaaag tcatgtctct gaatatgtgt ccttgacgtg caagctttgt 8220
   aaaaccccat ccccgcttag gtgcgaggca tcaccttctc acaagtgttt agtttctttt 8280
   aaccacaagt atcattcttg ggtgataata tagtttcatt ctacttaggg attgtttaga 8340
   aaacaaagaa agagccaatt aaatttttta gtttttgaaa tttttattta tatgtatact 8400
   tagatgagta ttttaagctg tcgaccttta gtttgccata cgggtaggac tgtatttcat 8460
   gttaacaact ggtggtaatg ataagccttc ttctagcgta ttttctcttc tttcctgtca 8520
   ctttcctaag tttttttttt taaagactgg aatttttttt ggctttatct tgtcttaccg 8580
   tagagatttg ttcaaaactc taagccctac cacctcccct ttaataagct ctttaaatag 8640
   ttgaatcatt aacaacctgg tgggaggcaa gtcatttaat tgaaccacta ggaagtgtat 8700
   tttcttttct ttttctgcca actttttggt ggcatttgta aaagctgata taaaaggctc 8760
   tgagatgtta ttttcagtta ttccataggc aagccttttt acagagcata tgtctccagt 8820
   tggcagcttg agatatttcc gagcatccgg ttctagctac cagtgcctcc caatgcttag 8880
   tgcacagtac tgtagactgg ccatcacccc tctccttgga aatgccactt gtgctgtttg 8940
   aaaaaaagca gccttttagg gctagagtat tttatataaa cagaagagct aagttcctga 9000
   agactaagct agatagctgc agctatatgt aaattgtata tttttatgaa cttttgaagc 9060
   acacactcct gtttccctct gtgtagcttt gtggggattt catgtatata tgctgtctga 9120
   aagaatccag aggttggagt gccaatagaa aatgaaaaca aatgccttgt actacaggca 9180
   gcctctgaag gtgaccacat aactgtcttc actgtgacca atcggagtcc ctgcttgctt 9240
   gtgaagaagg ggcttttgta ccttgttgga gatgccacct cagaagttca cactgtgcag 9300
   gaaaaaggtt ttattctctc ctggcataca ttagaatgtc agatgcttgc atccatgtgg 9360
   accacgatgg gcctctaaaa attggtgggc agggggtttg cttatgagtt ttctctggaa 9420
   accgatttta ctcctggatg tattgaatgc cccttgagct ttatgagata cgagtccaca 9480
   tggataaaat gttagagagt ggagttctac agaggattcc aggaagaggc catgtctgtg 9540
   cagtcctagt tccagacagg tgagaagctc caggaactac tggctacctt gacaagctgg 9600
   gtaaatagtt atcattctgg gtaactggtt gaaactctga cttttggaca agtaattcct 9660
   ggggttctgt ctttggtagc atcaccaggg atatttgggt gggacagaca gaagacacac 9720
   agctgcctgt tctctcctgc ccatcatgtt tggcccacta gatgaagctg tactcagcaa 9780
   tttagggaat gtaacccttc tcagaactgg ccattttcag gggaagcttg ggagagcaat 9840
   agtatggtga gccccttaga gatgagcgcc tactccttct tggcgaatgc tgccttcaga 9900
   tgcttaccaa gtggtcactg catctagtaa gattatattt ccagtacact tccttagggc 9960
   agaaacacca tcctatcagg tttggtcagt cccttcttca tgaagggagt catggggaat 10020
   tcctgaaaat tttcttcctt ctgcagacag ttggatgagt cccttagaga aggcatccag 10080
   agacataact aaactgaata tcatcccata ttgattttag gaattgactc taaaactctg 10140
   tgcagaatct tgtgttggga ttgtatcttg acattcctgt tgtgttattt ttcttaactg 10200
   gagtgtgtgc tgcctttcag gtacaatttt tgtgtaataa aagccagtgc attaagttta 10260
   tatagactac tttctatgca agactgagat atggaataga taggaagaga tatgtactgc 10320
   tgggtacatg gacagtaagt gtgttttcag atggagtacc agcaccgaaa atgggttgag 10380
   ggaggatggg ttgtatgtat gtttctgccc actaattttg agcagccata ttatgaatta 10440
   aatcgtcaca gccaagtaat aacccaagaa tggtatgagt ttcatgtgta atagctcaaa 10500
   tggaataagc atgaatgctg gagtggacca ttatcctcaa atattctatg tcacttctca 10560
   tttaaagact cttgttatga actattagaa actttaggca aaatcaaaag tatttgcggc 10620
   aaaataaagg cctattctac tcttatttaa agtgaaacac tgtatacttg tttctctcca 10680
   aagcgaaatt aagtatttat aatttcaatt gcctcgataa gtttccaagt cactgaaatc 10740
   tgctgaaggt tttactgtat tgttgcacaa ctttaagata atttttgtct caatgtcaac 10800
   ttttttcact gaataaaaat ttaactgggt caagaaaaca cctctttgaa aatccactgt 10860
   ctctgtgtgt ctcgagctgt tctttagagc gcaataaaga tggctgacgc agtctccaaa 10920
   cccc 10924

<210> 29
<211> 740
<212> DNA
<213> Homo sapiens

<400> 29
gcctcacaga attgagagtt tgttcttaca cacaagttta atgccacctt cctctgtctg 60

ccatggacca acaagcaata tatgctgagt taaacttacc cacagactca ggcccagaaa 120

gttcttcacc ttcatctctt cctcgggatg tctgtcaggg ttcaccttgg catcaatttg 180

ccctgaaact tagctgtgct gggattattc tccttgtctt ggttgttact gggttgagtg 240

tttcagtgac atccttaata cagaaatcat caatagaaaa atgcagtgtg gacattcaac 300

agagcaggaa taaaacaaca gagagaccgg gtctcttaaa ctgcccaata tattggcagc 360

aactccgaga gaaatgcttg ttattttctc acactgtcaa cccttggaat aacagtctag 420

ctgattgttc caccaaagaa tccagcctgc tgcttattcg agataaggat gaattgatac 480

acacacagaa cctgatacgt gacaaagcaa ttctgttttg gattggatta aatttttcat 540

tatcagaaaa gaactggaag tggataaacg gctctttttt aaattctaat gacttagaaa 600

ttagaggtga tgctaaagaa aacagctgta tttccatctc acagacatct gtgtattctg 660

agtactgtag tacagaaatc agatggatct gccaaaaaga actaacacct gtgagaaata 720

aagtgtatcc tgactcttga 740

<210> 30
<211> 1809
<212> DNA
<213> Homo sapiens

<400> 30
gtcttcctcc ctccctccct tcctcttact ctcattcatt tcatacacac tggctcacac 60

atctactctc tctctctatc tctctcagaa tgacaattct aggtacaact tttggcatgg 120

tttttctttt acttcaagtc gtttctggag aaagtggcta tgctcaaaat ggagacttgg 180

aagatgcaga actggatgac tactcattct catgctatag ccagttggaa gtgaatggat 240

cgcagcactc actgacctgt gcttttgagg acccagatgt caacatcacc aatctggaat 300

ttgaaatatg tggggccctc gtggaggtaa agtgcctgaa tttcaggaaa ctacaagaga 360

tatatttcat cgagacaaag aaattcttac tgattggaaa gagcaatata tgtgtgaagg 420

ttggagaaaa gagtctaacc tgcaaaaaaa tagacctaac cactatagtt aaacctgagg 480

ctccttttga cctgagtgtc gtctatcggg aaggagccaa tgactttgtg gtgacattta 540

atacatcaca cttgcaaaag aagtatgtaa aagttttaat gcacgatgta gcttaccgcc 600

aggaaaagga tgaaaacaaa tggacgcatg tgaatttatc cagcacaaag ctgacactcc 660

tgcagagaaa gctccaaccg gcagcaatgt atgagattaa agttcgatcc atccctgatc 720

actattttaa aggcttctgg agtgaatgga gtccaagtta ttacttcaga actccagaga 780

tcaataatag ctcaggggag atggatccta tcttactaac catcagcatt ttgagttttt 840

tctctgtcgc tctgttggtc atcttggcct gtgtgttatg gaaaaaaagg attaagccta 900

tcgtatggcc cagtctcccc gatcataaga agactctgga acatctttgt aagaaaccaa 960

gaaaaaattt aaatgtgagt ttcaatcctg aaagtttcct ggactgccag attcataggg 1020

tggatgacat tcaagctaga gatgaagtgg aaggttttct gcaagatacg tttcctcagc 1080

aactagaaga atctgagaag cagaggcttg gaggggatgt gcagagcccc aactgcccat 1140

ctgaggatgt agtcatcact ccagaaagct ttggaagaga ttcatccctc acatgcctgg 1200

ctgggaatgt cagtgcatgt gacgccccta ttctctcctc ttccaggtcc ctagactgca 1260

gggagagtgg caagaatggg cctcatgtgt accaggacct cctgcttagc cttgggacta 1320

caaacagcac gctgccccct ccattttctc tccaatctgg aatcctgaca ttgaacccag 1380

ttgctcaggg tcagcccatt cttacttccc tgggatcaaa tcaagaagaa gcatatgtca 1440

ccatgtccag cttctaccaa aaccagtgaa gtgtaagaaa cccagactga acttaccgtg 1500

agcgacaaag atgatttaaa agggaagtct agagttccta gtctccctca cagcacagag 1560

aagacaaaat tagcaaaacc ccactacaca gtctgcaaga ttctgaaaca ttgctttgac 1620

cactcttcct gagttcagtg gcactcaaca tgagtcaaga gcatcctgct tctaccatgt 1680

ggatttggtc acaaggttta aggtgaccca atgattcagc tatttaaaaa aaaaagagga 1740

aagaatgaaa gagtaaagga aatgattgag gagtgaggaa ggcaggaaga gagcatgaga 1800

ggaaaaaaa 1809

<210> 31
<211> 5572
<212> DNA
<213> Homo sapiens

<400> 31
gtgtgaatgc ttcattcgcc tcacaaacaa ccacagaacc acaagtgcgg tgcaaacttt 60

ctccaggagg acagcaagaa gtctctggtt tttaaatggt taatctccgc aggtcactac 120

cagccaccga gaccaacaga gtcatttaag gctgcaagca gtatttacaa cagagggtac 180
aagttctatc tgaaaaaaaa aggagggact atggcatcaa acagcctctt cagcacagtg 240
acaccatgtc agcaaaactt cttttgggat ccgagcacca gccggcgctt cagccccccc 300
tccagcagcc tgcagcccgg caaaatgagc gacgtgagcc cggtggtggc tgcgcaacag 360
cagcagcaac agcagcagca gcaacagcag cagcagcagc agcaacagca gcagcagcag 420
caggaggcgg cggcggcggc tgcggcggcg gcggcggctg cggcggcggc agctgcagtg 480
ccccggttgc ggccgcccca cgacaaccgc accatggtgg agatcatcgc cgaccacccg 540
gccgaactcg tccgcaccga cagccccaac ttcctgtgct cggtgctgcc ctcgcactgg 600
cgctgcaaca agaccctgcc cgtggccttc aaggtggtag ccctcggaga ggtaccagat 660
gggactgtgg ttactgtcat ggcgggtaac gatgaaaatt attctgctga gctccggaat 720
gcctctgctg ttatgaaaaa ccaagtagca aggttcaacg atctgagatt tgtgggccgg 780
agtggacgag gcaagagttt caccttgacc ataaccgtct tcacaaatcc tccccaagta 840
gctacctatc acagagcaat taaagttaca gtagatggac ctcgggaacc cagaaggcac 900
agacagaagc ttgatgactc taaacctagt ttgttctctg accgcctcag tgatttaggg 960
cgcattcctc atcccagtat gagagtaggt gtcccgcctc agaacccacg gccctccctg 1020
aactctgcac caagtccttt taatccacaa ggacagagtc agattacaga ccccaggcag 1080
gcacagtctt ccccgccgtg gtcctatgac cagtcttacc cctcctacct gagccagatg 1140
acgtccccgt ccatccactc taccaccccg ctgtcttcca cacggggcac tgggcttcct 1200
gccatcaccg atgtgcctag gcgcatttca gatgatgaca ctgccacctc tgacttctgc 1260
ctctggcctt ccactctcag taagaagagc caggcaggtg cttcagaact gggccctttt 1320
tcagacccca ggcagttccc aagcatttca tccctcactg agagccgctt ctccaaccca 1380
cgaatgcact atccagccac ctttacttac accccgccag tcacctcagg catgtccctc 1440
ggtatgtccg ccaccactca ctaccacacc tacctgccac caccctaccc cggctcttcc 1500
caaagccaga gtggaccctt ccagaccagc agcactccat atctctacta tggcacttcg 1560
tcaggatcct atcagtttcc catggtgccg gggggagacc ggtctccttc cagaatgctt 1620
ccgccatgca ccaccacctc gaatggcagc acgctattaa atccaaattt gcctaaccag 1680
aatgatggtg ttgacgctga tggaagccac agcagttccc caactgtttt gaattctagt 1740
ggcagaatgg atgaatctgt ttggcgacca tattgaaatt cctcagcagt ggcccagtgg 1800
tatctggggg ccacatccca cacgtatcaa tatatacata tatagagaga gtgcatatat 1860
atgtatatcg attagctatc tacaaagtgc ctatttttta gaagattttt cattcactca 1920
ctcagtcatg atcttgcagc cataagaggg tagatattga gaagcagaag gctcaagaga 1980
gacaattgca atcgagcttc agattgttta ctatttaaga tgtactttta caaaggaaca 2040
aagaagggaa aaggtatttt tgtttttgtt gtttggtctg ttatcatcaa taacctgttc 2100
atatgccaat tcagagaggt ggactccagg ttcaggaggg agaagagcaa agccgcttcc 2160

tctctgtgct ttgaaacttc acaccctcac ggtggcagct gtgtatggac cagtgccctc 2220
cgcagacagc tcacaaaacc agttgaggtg cactaaaggg acatgaggta gaatggatgc 2280
ttccatcaca gtaccatcat tcagaataac tcttccaatt tctgctttca gacatgctgc 2340
aggtcctcat ctgaactgtt gggttcgttt tttttttttt ttttcctgct ccaagaaagt 2400
gacttcaaaa ataactgatc aggatagatt attttatttt actttttaac actccttctc 2460
cccttttccc actgaaccaa aaagaaatcc catccctaaa acctgccttc tccttttatg 2520
caaaactgaa aatggcaata cattattata gccataatgg tatagatagt gattgcgttt 2580
ggctatgtgt tgttttcttt ttttttaaat tatgaatatg tgtaaaatct gaggtaactt 2640
gctaacgtga atggtcatat aactttaaag atatatttat aattatttaa tgacatttgg 2700
acccttgaaa catttcttag tgtattgata tgttgacttc ggtctctaaa agtgctcttt 2760
attaaataac aaatttcttc agtggtctag agccatatct gaaatattgc taagcaattt 2820
cagttcatcc aggcacaatg tgattttaaa aaatacttcc atctccaaat attttagata 2880
tagattgttt ttgtgatgta tgaaggaaat gttatgttta gttctttcag atctttgaat 2940
gcctctaaca cagctttgcc ttctaaagcg gtaattaggg atttaaaaaa caacctttag 3000
ccctttatca gcatgaaatg ctggagtgat gtggttttct aatttctttg gggtaattat 3060
gactcttgtc atattaaaaa gacaagcaca agtaaatcat tgaactacag aaaaatgttc 3120
tgtggtttca tagttaagca aaactctaaa tcgccaggct tcatagcaaa gacatagtca 3180
gctaaaagcc gcacatgtgg atagagggtt caattatgag acacctagta caggagagca 3240
aaattgcacc agagattctt aaccaaccag ccttaccaaa caacacaaca ggggaacccc 3300
aatctgcctt acccaaggcc ccactggcag ctttccacag aatttgcatt tagaggagca 3360
gaatgacatc actgtccttt gggagtaggt cctctgaaaa ggcagcaggt tccagcaggt 3420
agctgagctg agaggacata tggcccacgg ggacctacag acagcctttg acatttgtat 3480
ttcttacaat ggagggccaa ggagggcaag gggctgtgga gtttggtgtc tactagtgtg 3540
tatgaatttg agctagagtc cttctgtggc atgcactttg accactcctg gcagtcacat 3600
ggcagatttc caagtgcaaa tccttaatcc aaacaaggat catctaatga caccaccagg 3660
ccaatccctg ctctcctccc cgaaaagtca gggtcccttc attggaatcc tccacccacc 3720
caagcagaat ttagcagaga tttgccttca aaccctaacg gcccccttgt tctctggtcc 3780
ttctcaaacc cacctttgta ggccacccag cattgcagga cagcgtgtgg ggcagctgga 3840
cctgtgcttc ctgcctggga gtctcccttg gaattcatcc tgactccttc taataaaaat 3900
ggatgggaaa gcaaaacact ttgccttcta aaggccgtat accaagtatg cttagataaa 3960
taagccactt ttctattact taagtaagaa ggaagtagta attgatacta tttattgttt 4020
gtgtgtggta gcttgaagca caccactgtc catttatttg taagtgtaaa atatgtgtgt 4080
ttgtttcagc agcacttaaa aaagccagtg tctggttaca catttcaatt ttaattaatt 4140
gacataaaaa tgctaccgcc agtgccagct gcatcctatt taattaaaaa ggtactatat 4200

ttgtacatta ttttttaatg ttaaaagggc ttttttaagt ttacagtaca cataccgagt 4260

gactttaggg atgcttttgt gttgaaatgt tactatagtg gctgcaggca gcaacccaga 4320

aacactttag aagctttttt tccttgggaa aaattcaagc acttcttccc tccaccctca 4380

ctccaaccac cccaatgggg gtaattcaca tttcttagaa caaattctgc ccttttttgg 4440

tctagggatt aaaattttgt ttttctttct ttcttttttt ttttttttca ctgaaccctt 4500

aatttgcact gggtcatgtg tttgatttgt gatttcaaga ccaaagcaaa gtcttactac 4560

tactgtggaa ccatgtacta gttcctggga attaaaatag cgtggttctc tttgtagcac 4620

aaacattgct ggaatttgca gtcttttcaa tgcagccaca tttttatcca tttcagttgt 4680

ctcacaaatt ttaacccata tcagagttcc agaacaggta ccacagcttt ggttttagat 4740

tagtggaata acattcagcc cagaactgag aaactcaaca gattaactat cgtttgctct 4800

ttagacggtc tcactgcctc tcacttgcca gagccctttc aaaatgagca gagaagtcca 4860

caccattagg gaccatctgt gataaattca gaagggagga gatgtgtgta cagctttaag 4920

gattccctca attccgagga aagggactgg cccagaatcc aggttaatac atggaaacac 4980

gaagcattag caaaagtaat aattatacct atggtatttg aaagaacaat aataaaagac 5040

acttcttcca aaccttgaat ttgttgtttt tagaaaacga atgcatttaa aaatattttc 5100

tatgtgagaa ttttttagat gtgtgtttac ttcatgttta caaataactg tttgcttttt 5160

aatgcagtac tttgaaatat atcagccaaa accataactt acaataattt cttaggtatt 5220

ctgaataaaa ttccatttct tttggatatg ctttaccatt cttaggtttc tgtggaacaa 5280

aaatatttgt agcattttgt gtaaatacaa gctttcattt ttattttttc caattgctat 5340

tgcccaagaa ttgctttcca tgcacatatt gtaaaaattc cgctttgtgc cacaggtcat 5400

gattgtggat gagtttactc ttaacttcaa agggactatt tgtattgtat gttgcaactg 5460

taaattgaat tatttggcat ttttctcatg attgtaatat taatttgaag tttgaattta 5520

attttcaata aaatggcttt tttggttttg ttaaaaaaaa aaaaaaaaaa aa 5572

<210> 32
<211> 5506
<212> DNA
<213> Homo sapiens

<400> 32
gtgtgaatgc ttcattcgcc tcacaaacaa ccacagaacc acaagtgcgg tgcaaacttt 60

ctccaggagg acagcaagaa gtctctggtt tttaaatggt taatctccgc aggtcactac 120

cagccaccga gaccaacaga gtcatttaag gctgcaagca gtatttacaa cagagggtac 180

aagttctatc tgaaaaaaaa aggagggact atggcatcaa acagcctctt cagcacagtg 240

acaccatgtc agcaaaactt cttttgggat ccgagcacca gccggcgctt cagccccccc 300

tccagcagcc tgcagcccgg caaaatgagc gacgtgagcc cggtggtggc tgcgcaacag 360

cagcagcaac agcagcagca gcaacagcag cagcagcagc agcaacagca gcagcagcag 420

caggaggcgg cggcggcggc tgcggcggcg gcggcggctg cggcggcggc agctgcagtg 480

ccccggttgc ggccgcccca cgacaaccgc accatggtgg agatcatcgc cgaccacccg 540
gccgaactcg tccgcaccga cagccccaac ttcctgtgct cggtgctgcc ctcgcactgg 600
cgctgcaaca agaccctgcc cgtggccttc aaggtggtag ccctcggaga ggtaccagat 660
gggactgtgg ttactgtcat ggcgggtaac gatgaaaatt attctgctga gctccggaat 720
gcctctgctg ttatgaaaaa ccaagtagca aggttcaacg atctgagatt tgtgggccgg 780
agtggacgag gcaagagttt caccttgacc ataaccgtct tcacaaatcc tccccaagta 840
gctacctatc acagagcaat taaagttaca gtagatggac ctcgggaacc cagaaggcac 900
agacagaagc ttgatgactc taaacctagt ttgttctctg accgcctcag tgatttaggg 960
cgcattcctc atcccagtat gagagtaggt gtcccgcctc agaacccacg gccctccctg 1020
aactctgcac caagtccttt taatccacaa ggacagagtc agattacaga ccccaggcag 1080
gcacagtctt ccccgccgtg gtcctatgac cagtcttacc cctcctacct gagccagatg 1140
acgtccccgt ccatccactc taccaccccg ctgtcttcca cacggggcac tgggcttcct 1200
gccatcaccg atgtgcctag gcgcatttca ggtgcttcag aactgggccc tttttcagac 1260
cccaggcagt tcccaagcat ttcatccctc actgagagcc gcttctccaa cccacgaatg 1320
cactatccag ccacctttac ttacaccccg ccagtcacct caggcatgtc cctcggtatg 1380
tccgccacca ctcactacca cacctacctg ccaccaccct accccggctc ttcccaaagc 1440
cagagtggac ccttccagac cagcagcact ccatatctct actatggcac ttcgtcagga 1500
tcctatcagt ttcccatggt gccgggggga gaccggtctc cttccagaat gcttccgcca 1560
tgcaccacca cctcgaatgg cagcacgcta ttaaatccaa atttgcctaa ccagaatgat 1620
ggtgttgacg ctgatggaag ccacagcagt tccccaactg ttttgaattc tagtggcaga 1680
atggatgaat ctgtttggcg accatattga aattcctcag cagtggccca gtggtatctg 1740
ggggccacat cccacacgta tcaatatata catatataga gagagtgcat atatatgtat 1800
atcgattagc tatctacaaa gtgcctattt tttagaagat ttttcattca ctcactcagt 1860
catgatcttg cagccataag agggtagata ttgagaagca gaaggctcaa gagagacaat 1920
tgcaatcgag cttcagattg tttactattt aagatgtact tttacaaagg aacaaagaag 1980
ggaaaaggta tttttgtttt tgttgtttgg tctgttatca tcaataacct gttcatatgc 2040
caattcagag aggtggactc caggttcagg agggagaaga gcaaagccgc ttcctctctg 2100
tgctttgaaa cttcacaccc tcacggtggc agctgtgtat ggaccagtgc cctccgcaga 2160
cagctcacaa aaccagttga ggtgcactaa agggacatga ggtagaatgg atgcttccat 2220
cacagtacca tcattcagaa taactcttcc aatttctgct ttcagacatg ctgcaggtcc 2280
tcatctgaac tgttgggttc gttttttttt ttttttttcc tgctccaaga aagtgacttc 2340
aaaaataact gatcaggata gattatttta ttttactttt taacactcct tctccccttt 2400
tcccactgaa ccaaaaagaa atcccatccc taaaacctgc cttctccttt tatgcaaaac 2460
tgaaaatggc aatacattat tatagccata atggtataga tagtgattgc gtttggctat 2520

gtgttgtttt cttttttttt aaattatgaa tatgtgtaaa atctgaggta acttgctaac 2580
gtgaatggtc atataacttt aaagatatat ttataattat ttaatgacat ttggaccctt 2640
gaaacatttc ttagtgtatt gatatgttga cttcggtctc taaaagtgct ctttattaaa 2700
taacaaattt cttcagtggt ctagagccat atctgaaata ttgctaagca atttcagttc 2760
atccaggcac aatgtgattt taaaaaatac ttccatctcc aaatatttta gatatagatt 2820
gtttttgtga tgtatgaagg aaatgttatg tttagttctt tcagatcttt gaatgcctct 2880
aacacagctt tgccttctaa agcggtaatt agggatttaa aaaacaacct ttagcccttt 2940
atcagcatga aatgctggag tgatgtggtt ttctaatttc tttggggtaa ttatgactct 3000
tgtcatatta aaaagacaag cacaagtaaa tcattgaact acagaaaaat gttctgtggt 3060
ttcatagtta agcaaaactc taaatcgcca ggcttcatag caaagacata gtcagctaaa 3120
agccgcacat gtggatagag ggttcaatta tgagacacct agtacaggag agcaaaattg 3180
caccagagat tcttaaccaa ccagccttac caaacaacac aacaggggaa ccccaatctg 3240
ccttacccaa ggccccactg gcagctttcc acagaatttg catttagagg agcagaatga 3300
catcactgtc ctttgggagt aggtcctctg aaaaggcagc aggttccagc aggtagctga 3360
gctgagagga catatggccc acggggacct acagacagcc tttgacattt gtatttctta 3420
caatggaggg ccaaggaggg caaggggctg tggagtttgg tgtctactag tgtgtatgaa 3480
tttgagctag agtccttctg tggcatgcac tttgaccact cctggcagtc acatggcaga 3540
tttccaagtg caaatcctta atccaaacaa ggatcatcta atgacaccac caggccaatc 3600
cctgctctcc tccccgaaaa gtcagggtcc cttcattgga atcctccacc cacccaagca 3660
gaatttagca gagatttgcc ttcaaaccct aacggccccc ttgttctctg gtccttctca 3720
aacccacctt tgtaggccac ccagcattgc aggacagcgt gtggggcagc tggacctgtg 3780
cttcctgcct gggagtctcc cttggaattc atcctgactc cttctaataa aaatggatgg 3840
gaaagcaaaa cactttgcct tctaaaggcc gtataccaag tatgcttaga taaataagcc 3900
acttttctat tacttaagta agaaggaagt agtaattgat actatttatt gtttgtgtgt 3960
ggtagcttga agcacaccac tgtccattta tttgtaagtg taaaatatgt gtgtttgttt 4020
cagcagcact taaaaaagcc agtgtctggt tacacatttc aattttaatt aattgacata 4080
aaaatgctac cgccagtgcc agctgcatcc tatttaatta aaaaggtact atatttgtac 4140
attatttttt aatgttaaaa gggctttttt aagtttacag tacacatacc gagtgacttt 4200
agggatgctt ttgtgttgaa atgttactat agtggctgca ggcagcaacc cagaaacact 4260
ttagaagctt tttttccttg ggaaaaattc aagcacttct tccctccacc ctcactccaa 4320
ccaccccaat gggggtaatt cacatttctt agaacaaatt ctgccctttt ttggtctagg 4380
gattaaaatt ttgtttttct ttctttcttt tttttttttt ttcactgaac ccttaatttg 4440
cactgggtca tgtgtttgat ttgtgatttc aagaccaaag caaagtctta ctactactgt 4500
ggaaccatgt actagttcct gggaattaaa atagcgtggt tctctttgta gcacaaacat 4560

tgctggaatt tgcagtcttt tcaatgcagc cacattttta tccatttcag ttgtctcaca 4620
aattttaacc catatcagag ttccagaaca ggtaccacag ctttggtttt agattagtgg 4680
aataacattc agcccagaac tgagaaactc aacagattaa ctatcgtttg ctctttagac 4740
ggtctcactg cctctcactt gccagagccc tttcaaaatg agcagagaag tccacaccat 4800
tagggaccat ctgtgataaa ttcagaaggg aggagatgtg tgtacagctt taaggattcc 4860
ctcaattccg aggaaaggga ctggcccaga atccaggtta atacatggaa acacgaagca 4920
ttagcaaaag taataattat acctatggta tttgaaagaa caataataaa agacacttct 4980
tccaaacctt gaatttgttg tttttagaaa acgaatgcat ttaaaaatat tttctatgtg 5040
agaatttttt agatgtgtgt ttacttcatg tttacaaata actgtttgct ttttaatgca 5100
gtactttgaa atatatcagc caaaaccata acttacaata atttcttagg tattctgaat 5160
aaaattccat ttcttttgga tatgctttac cattcttagg tttctgtgga acaaaaatat 5220
ttgtagcatt ttgtgtaaat acaagctttc atttttattt tttccaattg ctattgccca 5280
agaattgctt tccatgcaca tattgtaaaa attccgcttt gtgccacagg tcatgattgt 5340
ggatgagttt actcttaact tcaaagggac tatttgtatt gtatgttgca actgtaaatt 5400
gaattatttg gcatttttct catgattgta atattaattt gaagtttgaa tttaattttc 5460
aataaaatgg ctttttggt tttgttaaaa aaaaaaaaaa aaaaaa 5506

<210> 33
<211> 5720
<212> DNA
<213> Homo sapiens

<400> 33
   ggactccaac tgggaggggg cgccagcgca cccaggacgc ggtgccccaa gggaccccat 60
   tccaagctgc aaactcaagt cccccgcctc cccaggccaa acacaccccc gcgcccgttc 120
   gcagctgcca ccccgggtgt tccaaagact ccggcaaaga tctgcgcctc ccgggtctcc 180
   ctacccgcca gcccgactgc gccgcatcgc cagactctgt tggcccatca gactccgccc 240
   ggcagtcggc ctcatcaaac ttgatttctc acctcctcag ccccatcacc tccatcctct 300
   ttccccccgt ctcgccttca cccccccaat ttcctccttg cccctcattt ccaccctcct 360
   ccccctcccc cggccacttc gctaacttgt ggctgttgtg atgcgtattc ccgtagatcc 420
   gagcaccagc cggcgcttca gccccccctc cagcagcctg cagcccggca aaatgagcga 480
   cgtgagcccg gtggtggctg cgcaacagca gcagcaacag cagcagcagc aacagcagca 540
   gcagcagcag caacagcagc agcagcagca ggaggcggcg gcggcggctg cggcggcggc 600
   ggcggctgcg gcggcggcag ctgcagtgcc ccggttgcgg ccgccccacg acaaccgcac 660
   catggtggag atcatcgccg accacccggc cgaactcgtc cgcaccgaca gccccaactt 720
   cctgtgctcg gtgctgccct cgcactggcg ctgcaacaag accctgcccg tggccttcaa 780
   ggtggtagcc ctcggagagg taccagatgg gactgtggtt actgtcatgg cgggtaacga 840
   tgaaaattat tctgctgagc tccggaatgc ctctgctgtt atgaaaaacc aagtagcaag 900
   gttcaacgat ctgagatttg tgggccggag tggacgaggc aagagtttca ccttgaccat 960
   aaccgtcttc acaaatcctc cccaagtagc tacctatcac agagcaatta aagttacagt 1020
   agatggacct cgggaaccca gaaggcacag acagaagctt gatgactcta aacctagttt 1080
   gttctctgac cgcctcagtg atttagggcg cattcctcat cccagtatga gagtaggtgt 1140
   cccgcctcag aacccacggc cctccctgaa ctctgcacca agtcctttta atccacaagg 1200
   acagagtcag attacagacc ccaggcaggc acagtcttcc ccgccgtggt cctatgacca 1260
   gtcttacccc tcctacctga gccagatgac gtccccgtcc atccactcta ccaccccgct 1320
   gtcttccaca cggggcactg ggcttcctgc catcaccgat gtgcctaggc gcatttcaga 1380
   tgatgacact gccacctctg acttctgcct ctggccttcc actctcagta agaagagcca 1440
   ggcaggtgct tcagaactgg gccctttttc agaccccagg cagttcccaa gcatttcatc 1500
   cctcactgag agccgcttct ccaacccacg aatgcactat ccagccacct ttacttacac 1560
   cccgccagtc acctcaggca tgtccctcgg tatgtccgcc accactcact accacaccta 1620
   cctgccacca ccctaccccg gctcttccca aagccagagt ggacccttcc agaccagcag 1680
   cactccatat ctctactatg gcacttcgtc aggatcctat cagtttccca tggtgccggg 1740
   gggagaccgg tctccttcca gaatgcttcc gccatgcacc accacctcga atggcagcac 1800
   gctattaaat ccaaatttgc ctaaccagaa tgatggtgtt gacgctgatg gaagccacag 1860
   cagttcccca actgttttga attctagtgg cagaatggat gaatctgttt ggcgaccata 1920
   ttgaaattcc tcagcagtgg cccagtggta tctgggggcc acatcccaca cgtatcaata 1980
   tatacatata tagagagagt gcatatatat gtatatcgat tagctatcta caaagtgcct 2040
   attttttaga agatttttca ttcactcact cagtcatgat cttgcagcca taagagggta 2100
   gatattgaga agcagaaggc tcaagagaga caattgcaat cgagcttcag attgtttact 2160
   atttaagatg tacttttaca aaggaacaaa gaagggaaaa ggtatttttg tttttgttgt 2220
   ttggtctgtt atcatcaata acctgttcat atgccaattc agagaggtgg actccaggtt 2280
   caggagggag aagagcaaag ccgcttcctc tctgtgcttt gaaacttcac accctcacgg 2340
   tggcagctgt gtatggacca gtgccctccg cagacagctc acaaaaccag ttgaggtgca 2400
   ctaaagggac atgaggtaga atggatgctt ccatcacagt accatcattc agaataactc 2460
   ttccaatttc tgctttcaga catgctgcag gtcctcatct gaactgttgg gttcgttttt 2520
   tttttttttt ttcctgctcc aagaaagtga cttcaaaaat aactgatcag gatagattat 2580
   tttattttac tttttaacac tccttctccc cttttcccac tgaaccaaaa agaaatccca 2640
   tccctaaaac ctgccttctc cttttatgca aaactgaaaa tggcaataca ttattatagc 2700
   cataatggta tagatagtga ttgcgtttgg ctatgtgttg ttttcttttt ttttaaatta 2760
   tgaatatgtg taaaatctga ggtaacttgc taacgtgaat ggtcatataa ctttaaagat 2820
   atatttataa ttatttaatg acatttggac ccttgaaaca tttcttagtg tattgatatg 2880
   ttgacttcgg tctctaaaag tgctctttat taaataacaa atttcttcag tggtctagag 2940
   ccatatctga aatattgcta agcaatttca gttcatccag gcacaatgtg attttaaaaa 3000
   atacttccat ctccaaatat tttagatata gattgttttt gtgatgtatg aaggaaatgt 3060
   tatgtttagt tctttcagat ctttgaatgc ctctaacaca gctttgcctt ctaaagcggt 3120
   aattagggat ttaaaaaaca acctttagcc ctttatcagc atgaaatgct ggagtgatgt 3180
   ggttttctaa tttctttggg gtaattatga ctcttgtcat attaaaaaga caagcacaag 3240
   taaatcattg aactacagaa aaatgttctg tggtttcata gttaagcaaa actctaaatc 3300
   gccaggcttc atagcaaaga catagtcagc taaaagccgc acatgtggat agagggttca 3360
   attatgagac acctagtaca ggagagcaaa attgcaccag agattcttaa ccaaccagcc 3420
   ttaccaaaca acacaacagg ggaaccccaa tctgccttac ccaaggcccc actggcagct 3480
   ttccacagaa tttgcattta gaggagcaga atgacatcac tgtcctttgg gagtaggtcc 3540
   tctgaaaagg cagcaggttc cagcaggtag ctgagctgag aggacatatg gcccacgggg 3600
   acctacagac agcctttgac atttgtattt cttacaatgg agggccaagg agggcaaggg 3660
   gctgtggagt ttggtgtcta ctagtgtgta tgaatttgag ctagagtcct tctgtggcat 3720
   gcactttgac cactcctggc agtcacatgg cagatttcca agtgcaaatc cttaatccaa 3780
   acaaggatca tctaatgaca ccaccaggcc aatccctgct ctcctccccg aaaagtcagg 3840
   gtcccttcat tggaatcctc cacccaccca agcagaattt agcagagatt tgccttcaaa 3900
   ccctaacggc ccccttgttc tctggtcctt ctcaaaccca cctttgtagg ccacccagca 3960
   ttgcaggaca gcgtgtgggg cagctggacc tgtgcttcct gcctgggagt ctcccttgga 4020
   attcatcctg actccttcta ataaaaatgg atgggaaagc aaaacacttt gccttctaaa 4080
   ggccgtatac caagtatgct tagataaata agccactttt ctattactta agtaagaagg 4140
   aagtagtaat tgatactatt tattgtttgt gtgtggtagc ttgaagcaca ccactgtcca 4200
   tttatttgta agtgtaaaat atgtgtgttt gtttcagcag cacttaaaaa agccagtgtc 4260
   tggttacaca tttcaatttt aattaattga cataaaaatg ctaccgccag tgccagctgc 4320
   atcctattta attaaaaagg tactatattt gtacattatt ttttaatgtt aaaagggctt 4380
   ttttaagttt acagtacaca taccgagtga ctttagggat gcttttgtgt tgaaatgtta 4440
   ctatagtggc tgcaggcagc aacccagaaa cactttagaa gctttttttc cttgggaaaa 4500
   attcaagcac ttcttccctc caccctcact ccaaccaccc caatgggggt aattcacatt 4560
   tcttagaaca aattctgccc ttttttggtc tagggattaa aattttgttt ttctttcttt 4620
   cttttttttt ttttttcact gaacccttaa tttgcactgg gtcatgtgtt tgatttgtga 4680
   tttcaagacc aaagcaaagt cttactacta ctgtggaacc atgtactagt tcctgggaat 4740
   taaaatagcg tggttctctt tgtagcacaa acattgctgg aatttgcagt cttttcaatg 4800
   cagccacatt tttatccatt tcagttgtct cacaaatttt aacccatatc agagttccag 4860
   aacaggtacc acagctttgg ttttagatta gtggaataac attcagccca gaactgagaa 4920
   actcaacaga ttaactatcg tttgctcttt agacggtctc actgcctctc acttgccaga 4980
   gccctttcaa aatgagcaga gaagtccaca ccattaggga ccatctgtga taaattcaga 5040
   agggaggaga tgtgtgtaca gctttaagga ttccctcaat tccgaggaaa gggactggcc 5100
   cagaatccag gttaatacat ggaaacacga agcattagca aaagtaataa ttatacctat 5160
   ggtatttgaa agaacaataa taaaagacac ttcttccaaa ccttgaattt gttgttttta 5220
   gaaaacgaat gcatttaaaa atattttcta tgtgagaatt ttttagatgt gtgtttactt 5280
   catgtttaca aataactgtt tgctttttaa tgcagtactt tgaaatatat cagccaaaac 5340
   cataacttac aataatttct taggtattct gaataaaatt ccatttcttt tggatatgct 5400
   ttaccattct taggtttctg tggaacaaaa atatttgtag cattttgtgt aaatacaagc 5460
   tttcattttt attttttcca attgctattg cccaagaatt gctttccatg cacatattgt 5520
   aaaaattccg ctttgtgcca caggtcatga ttgtggatga gtttactctt aacttcaaag 5580
   ggactatttg tattgtatgt tgcaactgta aattgaatta tttggcattt ttctcatgat 5640
   tgtaatatta atttgaagtt tgaatttaat tttcaataaa atggcttttt tggttttgtt 5700
   aaaaaaaaaa aaaaaaaaaa 5720

<210> 34
<211> 1056
<212> DNA
<213> Homo sapiens

<400> 34
gtctgcgcgg agtctgagcg gcgctcgtcc cgtcccaagg ccgacgccag cacgccgtca 60

tggcccccgc agcggcgacg gggggcagca ccctgcccag tggcttctcg gtcttcacca 120

ccttgcccga cttgctcttc atctttgagt ttatcttcgg gggcctggtg tggatcctgg 180

tggcctcctc cctggtgccc tggcccctgg tccagggctg ggtgatgttc gtgtctgtgt 240

tctgcttcgt ggccaccacc accttgatca tcctgtacat aattggagcc cacggtggag 300

agacttcctg ggtcaccttg gacgcagcct accactgcac cgctgccctc ttttacctca 360

gcgcctcagt cctggaggcc ctggccacca tcacgatgca agacggcttc acctacaggc 420

actaccatga aaacattgct gccgtggtgt tctcctacat agccactctg ctctacgtgg 480

tccatgcggt gttctcttta atcagatgga agtcttcata aagccgcagt agaacttgag 540

ctgaaaaccc agatggtgtt aactggccgc cccactttcc ggcataactt tttagaaaac 600

agaaatgccc ttgatggtgg aaaaaagaaa acaaccaccc ccccactgcc caaaaaaaaa 660

agccctgccc tgttgctcgt gggtgctgtg tttactctcc cgtgtgcctt cgcgtccggg 720

ttgggagctt gctgtgtcta acctccaact gctgtgctgt ctgctagggt cacctcctgt 780

ttgtgaaagg ggaccttctt gttcgggggt gggaagtggc gaccgtgacc tgagaaggaa 840

agaaagatcc tctgctgacc cctggagcag ctctcgagaa ctacctgttg gtattgtcca 900

caagctctcc cgagcgcccc atcttgtgcc atgttttaag tcttcatgga tgttctgcat 960

gtcatgggga ctaaaactca cccaacagat ctttccagag gtccatggtg gaagacgata 1020

accctgtgaa atactttata aaatgtctta atgttc 1056

<210> 35
<211> 930
<212> DNA
<213> Homo sapiens

<400> 35
gtctgcgcgg agtctgagcg gcgctcgtcc cgtcccaagg ccgacgccag cacgccgtca 60

tggcccccgc agcggcgacg gggggcagca ccctgcccag tggcttctcg gtcttcacca 120

ccttgcccga cttgctcttc atctttgagt ttatcttcgg gggcctggtg tggatcctgg 180

tggcctcctc cctggtgccc tggcccctgg tccagggctg ggtgatgttc gtgtctgtgt 240

tctgcttcgt ggccaccacc accttgatca tcctgtacat aattggagcc cacggtggag 300

agacttcctg ggtcaccttg gtgttctcct acatagccac tctgctctac gtggtccatg 360

cggtgttctc tttaatcaga tggaagtctt cataaagccg cagtagaact tgagctgaaa 420

acccagatgg tgttaactgg ccgccccact ttccggcata actttttaga aaacagaaat 480

gcccttgatg gtggaaaaaa gaaaacaacc acccccccac tgcccaaaaa aaaaagccct 540

gccctgttgc tcgtgggtgc tgtgtttact ctcccgtgtg ccttcgcgtc cgggttggga 600

gcttgctgtg tctaacctcc aactgctgtg ctgtctgcta gggtcacctc ctgtttgtga 660

aaggggacct tcttgttcgg gggtgggaag tggccaccgt gacctgagaa ggaaagaaag 720

atcctctgct gacccctgga gcagctctcg agaactacct gttggtattg tccacaagct 780

ctcccgagcg ccccatcttg tgccatgttt taagtcttca tggatgttct gcatgtcatg 840

cggactaaaa ctcacccaac agatctttcc agaggtccat ggtggaagac gataaccctg 900

tgaaatactt tataaaatgt cttaatgttc 930

<210> 36
<211> 888
<212> DNA
<213> Homo sapiens

<400> 36
gtctgcgcgg agtctgagcg gcgctcgtcc cgtcccaagg ccgacgccag cacgccgtca 60

tggcccccgc agcggcgacg gggggcagca ccctgcccag tggcttctcg gtcttcacca 120

ccttgcccga cttgctcttc atctttgagt ttgacgcagc ctaccactgc accgctgccc 180

tcttttacct cagcgcctca gtcctggagg ccctggccac catcacgatg caagacggct 240

tcacctacag gcactaccat gaaaacattg ctgccgtggt gttctcctac atagccactc 300

tgctctacgt ggtccatgcg gtgttctctt taatcagatg gaagtcttca taaagccgca 360

gtagaacttg agctgaaaac ccagatggtg ttaactggcc gccccacttt ccggcataac 420

tttttagaaa acagaaatgc ccttgatggt ggaaaaaaga aaacaaccac ccccccactg 480

cccaaaaaaa aaagccctgc cctgttgctc gtgggtgctg tgtttactct cccgtgtgcc 540

ttcgcgtccg ggttgggagc ttgctgtgtc taacctccaa ctgctgtgct gtctgctagg 600

gtcacctcct gtttgtgaaa ggggaccttc ttgttcgggg gtgggaagtg gccaccgtga 660

cctgagaagg aaagaaagat cctctgctga cccctggagc agctctcgag aactacctgt 720

tggtattgtc cacaagctct cccgagcgcc ccatcttgtg ccatgtttta agtcttcatg 780

gatgttctgc atgtcatgcg gactaaaact cacccaacag atctttccag aggtccatgg 840

tggaagacga taaccctgtg aaatacttta taaaatgtct taatgttc 888

<210> 37
<211> 762
<212> DNA
<213> Homo sapiens

<400> 37
gtctgcgcgg agtctgagcg gcgctcgtcc cgtcccaagg ccgacgccag cacgccgtca 60

tggcccccgc agcggcgacg gggggcagca ccctgcccag tggcttctcg gtcttcacca 120

ccttgcccga cttgctcttc atctttgagt ttgtgttctc ctacatagcc actctgctct 180

acgtggtcca tgcggtgttc tctttaatca gatggaagtc ttcataaagc cgcagtagaa 240

cttgagctga aaacccagat ggtgttaact ggccgcccca ctttccggca taacttttta 300

gaaaacagaa atgcccttga tggtggaaaa aagaaaacaa ccaccccccc actgcccaaa 360

aaaaaaagcc ctgccctgtt gctcgtgggt gctgtgttta ctctcccgtg tgccttcgcg 420

tccgggttgg gagcttgctg tgtctaacct ccaactgctg tgctgtctgc tagggtcacc 480

tcctgtttgt gaaaggggac cttcttgttc gggggtggga agtggccacc gtgacctgag 540

aaggaaagaa agatcctctg ctgacccctg gagcagctct cgagaactac ctgttggtat 600

tgtccacaag ctctcccgag cgccccatct tgtgccatgt tttaagtctt catggatgtt 660

ctgcatgtca tgcggactaa aactcaccca acagatcttt ccagaggtcc atggtggaag 720

acgataaccc tgtgaaatac tttataaaat gtcttaatgt tc 762

<210> 38
<211> 4078
<212> DNA
<213> Homo sapiens

<400> 38
atgcagaaga gcgtgcgcta caacgagggg cacgccctgt acctggcctt tctggcgcgc 60

aaggagggca ccaagcgcgg cttcctgagt aagaagacgg ccgaggcgag ccgctggcac 120

gagaagtggt tcgccctcta ccagaatgtg ctcttctact tcgagggcga gcagagctgc 180

cgcccggcgg gcatgtacct cctggagggc tgcagctgcg aacgaacgcc cgcgccaccc 240

agggccggcg ccgggcaggg aggcgtccga gacgcgctgg acaagcagta ttactttact 300

gttctttttg gccatgaagg tcagaagcca ctggagctgc gctgtgagga ggagcaggat 360

ggtaaagagt ggatggaggc cattcaccaa gccagttatg cagacatttt gattgagagg 420

gaagtattaa tgcagaagta cattcatcta gttcagatcg tagagacaga aaaaattgca 480

gctaaccaac tccgacatca acttgaagat caagacacag aaatcgaaag gcttaaatca 540

gagattattg ctcttaataa aaccaaagaa cgaatgcgac cttaccaaag caaccaagaa 600
gacgaagatc cagacatcaa gaagattaaa aaggttcaga gcttcatgcg aggatggttg 660
tgcagaagga aatggaagac catcgtgcag gattacattt gttctcctca tgctgaaagt 720
atgaggaaga gaaaccagat tgtgttcacc atggtggagg cagagtcaga gtacgttcac 780
cagctctaca tcctggtcaa tggctttctc cggcccctgc gtatggccgc cagctccaag 840
aagcccccca tcagccacga cgacgtcagc agtatttttc ttaacagtga aacaatcatg 900
tttcttcatg aaatatttca tcaaggacta aaggcaagga tagcaaactg gcccacttta 960
attttagctg atctgtttga tattttgctc cccatgctga acatttatca agaatttgtg 1020
cgtaatcacc agtacagcct gcaagttctc gccaattgta agcaaaacag agattttgac 1080
aaactcttaa aacagtatga agccaatcca gcctgtgagg ggaggatgct ggagacattc 1140
ttgacctatc ccatgtttca gatccccaga tatatcatca cactccatga gctccttgct 1200
cacacacccc atgagcatgt ggaaaggaaa agcctggagt ttgccaaatc aaagctagag 1260
gaactatcca gagtaatgca cgatgaagtc agcgacactg aaaacataag gaaaaacctt 1320
gccatcgaaa gaatgatcgt ggagggctgt gacatcttgc tggacaccag ccaaacgttc 1380
atccgccaag gttctcttat tcaagtacct tccgttgaga gggggaaact tagtaaagtt 1440
cgcctgggtt cgttgtcttt gaaaaaggaa ggagagagac aatgcttctt atttacaaaa 1500
cactttttaa tatgtacaag aagttcagga gggaagcttc atctgctcaa gacaggtggg 1560
gttctgtctc taatagactg cacattgatt gaggagccag atgcaagcga tgatgactct 1620
aaaggttctg ggcaagtgtt tgggcacctg gattttaaaa tagtggtgga gcctcctgac 1680
gctgccgcct tcactgttgt cttgttagca ccctcacgcc aggagaaagc tgcctggatg 1740
agtgacatca gtcagtgtgt ggacaatata cgatgtaatg gtttaatgac tatagtgttt 1800
gaagagaatt ccaaagtcac tgtgccacat atgattaagt ctgatgcccg tcttcataaa 1860
gacgacactg acatttgctt cagtaaaaca ctcaactcct gcaaagtgcc ccagatccgt 1920
tatgccagcg tggagcgcct cttggaacga ctgacagact tgcggtttct tagtattgat 1980
ttcctcaaca cctttctgca cacctatcgt attttcacta ctgccgctgt ggtgctgggg 2040
aaactctccg acatatacaa gaggcctttc acctccatcc ctgtcaggtc attggaattg 2100
ttttttgcta ccagccagaa caacagaggt gaacatttgg tggatggcaa atccccacgt 2160
ctgtgtcgca aattctcttc cccgccacca ctggctgtgt ccagaacatc ttccccagtg 2220
agggccagaa agctgtcttt gacttctccc ttgaactcaa agataggagc attggacctg 2280
acaacttcca gcagtcccac caccaccacc cagagtcccg ctgcgtctcc accaccacac 2340
actggtcaga taccactgga tctcagcaga ggcctctctt ctccagagca aagcccggga 2400
acggtagaag agaatgtcga taacccacgc gtggatctgt gtaacaagct aaaacgaagt 2460
attcaaaaag cagtcctaga gtctgcacca gcggaccgag caggagtgga aagctcccct 2520
gcagcggaca ccacagaact ttcaccttgc agatccccct caactcctcg gcacctccgc 2580

tatcgacagc ctggaggaca gacggcggac aatgcccact gctctgtttc accggcttct 2640

gcttttgcaa tagccacagc tgcagcagga catgggagtc caccaggctt taacaacacc 2700

gagagaacat gtgataaaga gtttattata cggagaacgg ctaccaatcg agttctgaac 2760

gtcctccgtc actgggtctc aaagcacgca caggatttcg aactcaacaa tgaactaaag 2820

atgaatgtcc taaatttgct agaagaagtt ttgcgagacc cagaccttct tccccaagaa 2880

aggaaagccg ccgcgaatat cctcagggcc ctttcacaag atgaccaaga tgacatccac 2940

ctaaaattag aggatataat tcaaatgact gactgcatga aggccgaatg ctttgagtcc 3000

ttgtcggcca tggagctggc agaacagatc accctcctgg accatgtcat tttcagaagc 3060

attccctacg aggagtttct tgggcagggg tggatgaagc tggataaaaa cgaaagaact 3120

ccttacatta tgaaaaccag ccaacacttc aatgacatga gtaacctggt ggcctcccag 3180

ataatgaact atgctgatgt cagctcccgt gccaacgcca tcgagaaatg ggtggcagtg 3240

gcggacatct gccgatgcct gcacaactac aacggcgtgc tggagatcac ctcggcctta 3300

aacagaagtg ccatctacag gctgaagaaa acctgggcca aggtctctaa gcagacaaaa 3360

gctctaatgg acaaacttca aaagactgtt tcctctgaag gaagatttaa aaatcttaga 3420

gaaaccctta aaaattgtaa ccctcctgca gttccttatc ttgggatgta cttgacagac 3480

ctggcattta ttgaagaagg aacaccaaac tttactgagg aaggccttgt caatttctcc 3540

aaaatgagaa tgatatcaca catcatcaga gagatacgcc agttccagca gacttcctac 3600

agaatagatc atcagccaaa ggtcgcacag tacttgcttg acaaagacct tatcatagat 3660

gaagatacgc tatatgagct gtcactaaaa attgaacctc gactccctgc ttgaagatct 3720

ggccttgccc ctgagtccac gggatgttca tggaaagcag gacagacaga attgtgtatg 3780

ccttgcctat cacggtacag cacgaagcca ggctcctttc tccaccaaag aagatggaac 3840

cagactggaa ttctgtctcc agagagaaac ccagctgttt gggtcaaaga cagatgcttc 3900

agacttgggt gggaaggtga aagatggcta tttagaaagc tggtggcacg ttttacataa 3960

gggaatgtca gatgggagat gctagttgcc attttaacaa agcaggtaaa tcggtaaatt 4020

ttaaactctg tccatgttct gttagaactc agggacaagg atccatgaaa aagacctg 4078

<210> 39
<211> 7218
<212> DNA
<213> Homo sapiens

<400> 39
gccccgcatc gtgcccggcc ccgtcgcgga gatcccggac gaccgtcgcg ggttgatggt 60

cgcattccag atgtaaacag cttcagaagc ctgacggtca tatggtagaa tcactgtgga 120

ctgagaccca cctttctaga cctgaagccc aggaggagga agaggaggct ggttggtacc 180

atgggcataa tgctctgaat cctagtctct cacctagtat gtgagcagtc cctgcagatg 240

gcccatttgg agatcttgac aaagcctctt ctgtttccaa tggggttttt ggcgcattct 300

cacagactta gatgaaactg tgatggccac cgcagggggc aggtgctgac atcgtcccca 360
gccctgtggc tgttcatccg gacatcattt ccaacctcaa tatctaaatg ccacagtgct 420
cttggagcaa gttgggctgg ggaccactgt tgccttttaa gaccataaaa ccatgggaaa 480
cgcagaaagt caacatgtag agcacgagtt ttatggagaa aagcatgcca gcctggggcg 540
caagcacact tcccgctccc tgcgcctctc gcacaagacg cggaggacca ggcacgcttc 600
ctcggggaag gtgatccaca ggaactccga agtgagcacc cgatccagca gcacccccag 660
catcccccag tccctggctg aaaatggcct ggagcccttc tcccaagatg gtaccctaga 720
agacttcggg agccccatct gggtggaccg agtggacatg ggcttgagac ctgtgtctta 780
cactgactct tctgtcactc ccagcgtaga cagcagcatc gtcctcacag cagcctctgt 840
gcagagcatg ccagacactg aggagagcag gctttacggg gatgacgcta catatttggc 900
tgagggaggc aggaggcagc attcctatac atccaatggg cccactttca tggagacggc 960
gagctttaag aagaaacgct ccaaatctgc agacatctgg cgggaggaca gcctggaatt 1020
ctcactctct gatctgagcc aagaacattt aacaagcaac gaagaaatct tgggttccgc 1080
cgaagagaag gactgcgagg aggctcgggg gatggaaacg cgggcgagtc cgcggcagct 1140
cagcacctgt cagagagcca attccttggg tgacttgtat gctcagaaaa actctggagt 1200
gacagcaaac ggggggccgg ggagcaaatt tgcaggctac tgtcggaatt tggtgtctga 1260
tattcccaat cttgcaaacc ataagatgcc accagctgct gctgaagaga ctcctccgta 1320
cagtaattat aacacacttc cctgtaggaa atctcactgt ctctctgaag gtgccaccaa 1380
cccacaaatt agccatagca acagcatgca aggcagaaga gctaaaacaa ctcaggatgt 1440
taatgcaggc gagggcagtg agtttgcaga cagtgggatt gaaggggcca ctaccgacac 1500
ggacctcctg tccaggcgat ctaatgccac caactccagc tactcaccca ccacaggccg 1560
ggcctttgtg ggcagcgaca gcggcagcag ctccaccggg gatgcggctc gtcagggggt 1620
gtacgagaac ttccggcggg agctggagat gagcaccacc aacagcgaga gcctggagga 1680
ggccggctcg gcgcacagcg atgagcagag cagcggcacc ctgagctctc cgggccagtc 1740
ggacatcctg ctgaccgccg cacagggcac ggtgcgcaag gccggcgccc tggccgtcaa 1800
gaacttcctg gtgcacaaga agaacaagaa ggtggagtca gccacccgga ggaagtggaa 1860
gcactactgg gtgtccctga aaggatgcac gctatttttc tacgagagcg acggcaggtc 1920
tgggatagac cacaacagca tccccaaaca cgccgtctgg gtggagaaca gcattgtgca 1980
ggcggtgcct gagcacccca agaaggactt tgtcttctgc ctcagcaatt ccctgggtga 2040
tgccttcctt tttcagacca ctagccagac ggagcttgaa aactggatca ccgccatcca 2100
ctctgcctgc gccactgcgg tcgcgaggca ccaccacaag gaagacacgc tccgactcct 2160
gaaatcagag atcaaaaaac tggaacagaa gattgacatg gatgaaaaga tgaagaaaat 2220
gggtgaaatg cagctgtctt cagtcactga ctcaaagaaa aagaaaacaa tattagatca 2280
gatctttgtc tgggagcaaa atctcgagca gttccaaatg gacctgtttc gtttccgctg 2340

ttatttagcc agccttcagg gtggggagct gccaaacccc aaaaggcttc tcgcttttgc 2400
aagtcgacca acgaaagtgg ccatgggccg ccttggaatc ttttcggtat catcgtttca 2460
tgccctggtg gcagcacgca ctggtgaaac tggagtgaga agacgtactc aggccatgtc 2520
cagatccgcg agcaagcgaa ggagcaggtt ttcttctctg tggggtctgg atactacctc 2580
caaaaagaag cagggacggc caagcatcaa tcaggtgttt ggagagggaa ccgaagctgt 2640
aaagaaatct ttagagggaa tatttgatga cattgttcca gatggcaaga gggagaaaga 2700
agtggtctta cctaacgttc accagcacaa ccctgactgc gacatttggg tccacgagta 2760
tttcactcca tcctggttct gtctgcccaa taatcagcct gccctgacgg tcgtccggcc 2820
aggcgacact gcacgggaca ccctggagct gatttgcaag acacatcaac tggatcattc 2880
tgctcattac ctgcgcctga aatttctaat agaaaacaaa atgcagctct atgttccaca 2940
gcccgaggaa gacatctatg agctgctgta caaagaaatt gaaatctgtc caaaagtcac 3000
tcagagcatc cacattgaga agtcagatac agctgctgat acttacgggt tttcactttc 3060
ttctgtggaa gaagatggta ttcgaaggct gtacgtgaat agtgtgaagg aaaccggttt 3120
agcttccaag aaaggcctga aagcaggaga tgagattctt gagatcaata atcgtgctgc 3180
tgacgccctg aactcttcta tgctcaaaga tttcctctca cagccctcgc tgggcctcct 3240
ggtgaggacc taccccgagc tggaggaagg agtggagctg ctggaaagcc cgccccaccg 3300
agtggacggc cctgccgacc ttggcgagag ccccctcgcc tttctcacca gcaacccagg 3360
gcacagcctt tgcagcgagc agggcagcag tgctgagacc gctccagagg agaccgaggg 3420
gccagacttg gaatcctcag atgagactga tcacagcagc aagagtacag aacaggtggc 3480
cgcattttgc cgcagtttgc atgagatgaa cccctctgac cagagcccat ctcctcagga 3540
ctccacgggg cctcagctgg cgaccatgag acaactctcg gatgcagata agctgcgcaa 3600
ggtgatctgc gagctcctgg agacggagcg cacctacgtg aaggatttaa actgtcttat 3660
ggagagatac ctaaagcctc ttcaaaaaga aacttttctc acccaggatg agcttgacgt 3720
gctttttgga aatttaacgg aaatggtaga gtttcaagta gaattcctta aaactctaga 3780
agatggagtg agactggtac ctgatttgga aaagcttgag aaggttgatc aatttaagaa 3840
agtgctgttc tctctggggg gatcattcct gtattatgct gaccgcttca agctctacag 3900
tgccttctgc gccagccaca caaaagttcc caaggtcctg gtgaaagcca agacagacac 3960
ggctttcaag gcattcttgg atgcccagaa cccgaagcag cagcactcat ccacgctgga 4020
gtcgtacctc atcaagccca tccagaggat cctcaagtac ccacttctgc tcagggagct 4080
gttcgccctg accgatgcgg agagcgagga gcactaccac ctggacgtgg ccatcaagac 4140
catgaacaag gttgccagtc acatcaatga gatgcagaaa atccatgaag agtttggggc 4200
tgtgtttgac cagctgattg ctgaacagac tggtgagaaa aaagaggttg cagatctgag 4260
catgggagac ctgcttttgc acactaccgt gatctggctg aacccgccgg cctcgctggg 4320
caagtggaaa aaggaaccag agttggcagc attcgtcttc aaaactgctg tggtccttgt 4380

gtataaagat ggttccaaac agaagaagaa acttgtagga tctcacaggc tttccattta 4440
tgaggactgg gaccccttca gatttcgaca catgatcccc acggaagcgc tgcaggttcg 4500
agctttggcg agtgcagatg cagaggcaaa tgccgtgtgt gaaattgtcc atgtaaaatc 4560
cgagtctgaa gggaggccgg agagggtctt tcacttgtgc tgcagctccc cagagagccg 4620
aaaggatttc ctaaaggctg tgcattcaat cctgcgtgat aagcacagaa gacagctcct 4680
caaaaccgag agccttccct catcccagca atatgtccct tttggaggca aaagattgtg 4740
tgcactgaag ggggccaggc cggccatgag cagggcagtg tctgccccaa gcaagtctct 4800
tgggaggagg aggcggcggc tggctcgaaa caggtttacc attgattctg atgccgtctc 4860
cgcaagcagc ccggagaaag agtcccagca gcccccggt ggtggggaca ctgaccgatg 4920
ggtagaggag cagtttgatc ttgctcagta tgaggagcaa gatgacatca aggagacaga 4980
catcctcagt gacgatgatg agttctgtga gtccgtgaag ggtgcctcag tggacagaga 5040
cctgcaggag cggcttcagg ccacctccat cagtcagcgg gaaagaggcc ggaaaaccct 5100
ggatagtcac gcgtcccgca tggcacagct caagaagcaa gctgccctgt cggggatcaa 5160
tggaggcctg gagagcgcaa gcgaggaagt catttgggtt aggcgtgaag actttgcccc 5220
ctccaggaaa ctgaacactg agatctgact gcgtcacctg ccccgtagag aatgtgtgta 5280
gatacttcct gccctaactc tgcccaccct cctgtaccgt cgacaagaat gtccccttag 5340
gtcgcgctct tgcacacacg gttttggcag ctgacttggt tctgaagcca tgtagccacc 5400
caactttgtc attttcaaca acatcagaaa gaattgatca gaatcccaaa taagcttgag 5460
tcctatcttc tgtatattac taagggcttt tatttattct caataaatca gggcctgaac 5520
aattaaaaga aaaaagattc tatagcactg gaaagcaaat caccccagga gttaacggat 5580
gtacaacaga ttaatttaag ggatagtagc acacacacga tccttctatc tgaaatcagt 5640
ctcctagctg ggaaacctc tttcacacac aaaatgaaat gtgtacagct tgccgtgttc 5700
tgactgtacc cttccctctt ccatgtctga gaatctccgt gtattttaag aatgtgtgag 5760
gagagggtgg cgattcatgt ttcaatgagc ctcttttttt ttttccttcc tgttttggtc 5820
tatggctggt cttactctgt gtccatgttc ggaagctcta gttttgcata gaattataga 5880
gatgccaaac tctttgaaaa gagatccaaa tttatcgctt gagagaaaga aaagaaacac 5940
tatttttgt attttacctg agatacaggg gcacaaatag atgagaattt tacagtgtta 6000
gtgtatgtat ccctgagcct aaaaaatgag gatataacct tttacagaga gagtgaggcg 6060
tggtggtttt atatttatat atgaaaggcc agcaagctca tgcgaaggat atacttttct 6120
tccaaaaagc ggattttttt tttttaatgt ttgaatctat atttgagatg ggagtttggt 6180
tggattaaac atgacacccc ggtgggcggt gtgtgtgtct gttgcacatg gcagggaggg 6240
gagcctcctt ctcatggggt tgccatggtg atcattggtt tttccatcaa aattgcatct 6300
tcatccatag attaccttcc ccttccctga cagtccataa ccaaaccttt aaacagaaca 6360
acctctttaa aaacttctct tgtgtttaac actttcttca tgccaacgaa acagggtaaa 6420

catgctcaaa acattaacag tctaaacaga tatccaaata ctaagaagaa aaacaagtta 6480

tagcactttc aatttttttt ttttttttaa aaaaaggttt atagcttttt cttttcccat 6540

gtcacaatgt ccacttccta agaagggttt aaaatactat gaaaactttc tttttgggga 6600

aaatatctat ttggtgtttg acacatcagt aggtacttta aagacctgaa ttttatagta 6660

gctttaggag ttatatttta taaaaatcag ttatgacttt atatttccag acaatagaga 6720

gttcagtaca tcatgctctt gtgcctctgc ctgcttttcc tgcgttccca ccctgtattc 6780

cccccgcctt tcgggtttcc agggcttcga gcttgatctt ttgaaagttt tattctatta 6840

aatttttgct atatcttctg gttttctgaa aaagctttag aatggtttct ataccctttg 6900

tatcactgca tttttccata tcatctccgg ttcgatcgcg tccagatgga aaacggaagc 6960

agaggcttct aatcgtcgca tttactggct ccagtgcaac acatccatct gaaaacactc 7020

ggaagtctgg tgcttggaga gggtgccatt gtctcttgta cataaggtca tgacgtgtct 7080

atgtcaaaag ttcttatata tttcttttat aagctgaaag aaggtctatt tttatgtttt 7140

taggtctatg aatggaacgt tgtaaatgct tgtcaaacaa taaaaataac gaaaagtgaa 7200

aaaaaaaaaa aaaaaaaa 7218

<210> 40
<211> 3522
<212> DNA
<213> Homo sapiens

<400> 40
aggggcgagt cctgcgcgag tccccgggag gcgccgcgcg cttggaaggg acggtcgggc 60

ttccccggcc cgctgagggc tcggcggcgg gctcccctcc tttccacctc gggagggagg 120

gaaggagggg agggaaaagt cccacggagg aggcagaatg gccagtcgag gggcgcttag 180

gcgctgcctt tccccagggc tgcctcgact cctgcacctg tcccgagggc tggcctgaga 240

cgggactccc ggttctcccg ctgcgaagca gcgcggcccc ccggggccgg ggcagcggcg 300

ccggcatgtc gtctggcacc atgaagttca atggctattt gagggtccgc atcggtgagg 360

cagtggggct gcagcccacc cgctggtccc tgcgccactc gctcttcaag aagggccacc 420

agctgctgga cccctatctg acggtgagcg tggaccaggt gcgcgtgggc cagaccagca 480

ccaagcagaa gaccaacaaa cccacgtaca acgaggagtt ttgcgctaac gtcaccgacg 540

gcggccacct cgagttggcc gtcttccacg agacgcccct gggctacgac cacttcgtgg 600

ccaactgcac cctgcagttc caggagctgc tgcgcacgac cggcgcctcg gacaccttcg 660

agggttgggt ggatctcgag ccagagggga aagtatttgt ggtaataacc cttaccggga 720

gtttcactga agctactctc cagagagacc ggatcttcaa acattttacc aggaagcgcc 780

aaagggctat gcgaaggcga gtccaccaga tcaatggaca caagttcatg gccacgtatc 840

tgaggcagcc cacctactgc tctcactgca gggagtttat ctggggagtg tttgggaaac 900

agggttatca gtgccaagtg tgcacctgtg tcgtccataa acgctgccat catctaattg 960

ttacagcctg tacttgccaa aacaatatta acaaagtgga ttcaaagatt gcagaacaga 1020

ggttcgggat caacatccca cacaagttca gcatccacaa ctacaaagtg ccaacattct 1080
gcgatcactg tggctcactg ctctggggaa taatgcgaca aggacttcag tgtaaaatat 1140
gtaaaatgaa tgtgcatatt cgatgtcaag cgaacgtggc ccctaactgt ggggtaaatg 1200
cggtggaact tgccaagacc ctggcaggga tgggtctcca acccggaaat atttctccaa 1260
cctcgaaact cgtttccaga tcgaccctaa gacgacaggg aaaggagagc agcaaagaag 1320
gaaatgggat tggggttaat tcttccaacc gacttggtat cgacaacttt gagttcatcc 1380
gagtgttggg gaaggggagt tttggaaggg tgatgcttgc aagagtaaaa gaaacaggag 1440
acctctatgc tgtgaaggtg ctgaagaagg acgtgattct gcaggatgat gatgtggaat 1500
gcaccatgac cgagaaaagg atcctgtctc tggcccgcaa tcaccccttc ctcactcagt 1560
tgttctgctg ctttcagacc cccgatcgtc tgttttttgt gatggagttt gtgaatgggg 1620
gtgacttgat gttccacatt cagaagtctc gtcgttttga tgaagcacga gctcgcttct 1680
atgctgcaga aatcatttcg gctctcatgt tcctccatga taaaggaatc atctatagag 1740
atctgaaact ggacaatgtc ctgttggacc acgagggtca ctgtaaactg gcagacttcg 1800
gaatgtgcaa ggaggggatt tgcaatggtg tcaccacggc cacattctgt ggcacgccag 1860
actatatcgc tccagagatc ctccaggaaa tgctgtacgg gcctgcagta gactggtggg 1920
caatgggcgt gttgctctat gagatgctct gtggtcacgc gccttttgag gcagagaacg 1980
aagatgacct ctttgaggcc atactgaatg atgaggtggt ctaccctacc tggctccatg 2040
aagatgccac agggatccta aaatctttca tgaccaagaa ccccaccatg cgcttgggca 2100
gcctgactca gggaggcgag cacgccatct tgagacatcc tttttttaag gaaatcgact 2160
gggcccagct gaaccatcgc caaatagaac cgcctttcag acccagaatc aaatcccgag 2220
aagatgtcag taattttgac cctgacttca taaaggaaga gccagtttta actccaattg 2280
atgagggaca tcttccaatg attaaccagg atgagtttag aaacttttcc tatgtgtctc 2340
cagaattgca accatagcct tatggggagt gagagagagg gcacgagaac ccaaagggaa 2400
tagagattct ccaggaattt cctctatggg accttcccag catcagcctt agaacaagaa 2460
ccttaccttc aaggagcaag tgaagaactc tgtgaaggat ggaactttca gatatcaact 2520
atttagagtc cagagggagc catggcacta gaaatagttg ataatgaaat gagattttat 2580
gaagtatacc gctccaccta tgagcgtctg tctctgtggg cttgggatgt taacaggagc 2640
caaaaggagg gaaagtgtga agaataaagt agatctgaga aattctgagc caatcaggct 2700
tcttaattca agagacaaac caagacgttc tgtcaactgt gctgtgctct tctttaagcc 2760
aatgaacccc aattcctggc agtctacaag aagtctctta atgctaatga agaatttaaa 2820
ggtcttttta aggaaatgaa gggctttcca aatagaatga tttactctga agaaacaaac 2880
aatggtatct ctgaaactca caacctaaag cccaatcttg aaaatatgtt gtgcaccaag 2940
acgactgctt cagcttcttc tcttatcctt actttcttta atagatattt attaaactgt 3000
ccagtgaaaa ggtgccacaa tgcccagtat tgtaaacaac aggtttgcat tcatgaagct 3060

ttcattcatt ctggagtcta ctaatttacc tgaatggtgt ttgcattctg tgaaatgcct 3120

ctccacgttg catatgtcac acttttgtct gcacataact cttttttcac aagaagggtc 3180

actgccacaa cagcacagtc agcgggtgaa ttacaggtgc ctgctgcctg cctacctggg 3240

taatctgatc ttgtctgtat cgccgtgtgc tcatcactga agaattgcag gccactcatg 3300

tcagtgacca gatttgtggc ttataaacat tagcagttta tttatgtttt aagatgcaaa 3360

gatgtgtgtt tgatattcac tttaataatt agaaatggat cttgtaaaca gggcatatat 3420

caaagatgac cttataatat gtacccgaat atacagttca agaattttgt ctgactggaa 3480

ataaatgcat tttgtagcaa aaggaaaaaa aaaaaaaaaa aa 3522

<210> 41
<211> 5801
<212> DNA
<213> Homo sapiens

<400> 41
atatgaccgg cagccggcgg ctccggcgcc gagcagagtt actcagtcgc agcggccccg 60

cgctcccgca aggctgggcg gccgcgccgc cgcgatggcc ccgccaccgc ggccgccgcc 120

cccggctgcg cgctgagccg ccggcccccc gagcgccacg gccggagctg cggcggcggc 180

atcatggccc cgaccctgct ccagaagctc ttcaacaaaa ggggcagcag cggcagctcc 240

gcggcggcgt ctgcccaggg cagggctcct aaggaaggac ccgcctttag ttggtcatgt 300

tcggagtttg acctgaatga gattcgcctg atagtttacc aggactgtga caggagaggc 360

agacaagtct tgtttgactc taaagctgtt caaaagattg aggaggtgac agctcagaaa 420

acagaggatg ttcctattaa aatatcagcc aagtgctgcc agggaagcag cagtgtcagc 480

agcagtagca gcagcagcat ctcttcccac agttcttctg ggggatcttc acatcatgct 540

aaggaacagc ttccaaagta ccagtacaca agaccagctt ccgatgtcaa catgttaggg 600

gaaatgatgt ttggctcagt tgccatgagt tacaaaggct ccaccttaaa gatacactac 660

atacgttctc ctccacaact gatgattagt aaagtcttct ctgctagaat gggcagcttc 720

tgtggaagta caaataactt gcaagacagc tttgagtaca tcaaccaaga tcctaatttg 780

ggaaaactga acacaaatca aaatagtttg ggtccttgtc gtactggaag taacctagca 840

cacagcacac cagttgatat gccaagcaga ggacagaatg aagacaggga cagtggcatt 900

gctcgatcag cctcactaag cagtcttttg atcacacctt tcccatctcc aagctcctct 960

acatcttctt ccagcagtta ccagcgccgc tggcttcgaa gtcagacaac aagtttggaa 1020

aatggcatca tcccaagaag gtcaactgat gagacattca gcttggctga agaaacctgt 1080

agctctaatc cagctatggt taggaggaag aaaattgcca taagcatcat cttttcccta 1140

tgtgagaaag aagaagcaca aaggaatttc caggacttct tcttttctca ttttcccctg 1200

tttgaatctc acatgaacag gctgaagagt gcaattgaaa aggctatgat ctcctgtagg 1260

aaaatagcag aatcaagtct ccgagtccag ttttatgtca gccgtttgat ggaagctctg 1320

ggagaattca gaggaactat ctggaactta tattctgttc caaggatagc tgaacctgta 1380
tggcttacta tgatgtccgg cactttggaa aaaaaccagc tctgccagcg ctttctcaag 1440
gagtttacac ttctgataga acagataaat aaaaaccagt tttttgctgc cttactgact 1500
gcggtgttaa cctaccacct ggcctgggtc ccaactgtca tgcctgtgga tcaccctccc 1560
atcaaagcct tctcagagaa acgtacctcc cagtcagtga acatgctggc caaaacacat 1620
ccgtataatc ctctttgggc acagctgggt gatctttacg gagccatagg ctctccagtg 1680
agactgactc gcaccgtagt ggtagggaag cagaaggact tagtccagcg aatactttat 1740
gtcctgacct actttctccg ttgctctgag ctacaagaga accagctgac ctggagtggc 1800
aatcatggtg aaggtgacca agttttaaat gggagcaaga tcataacagc cttggagaaa 1860
ggagaggtgg aggagtctga gtatgtggtc attacggtga ggaacgagcc cgctcttgta 1920
ccccccatcc taccaccaac agcagcagag agacacaacc cctggccgac agggtttcct 1980
gagtgcccag agggcactga cagtagagac ctgggtctta aacctgacaa agaagctaac 2040
aggaggccag agcagggttc tgaggcttgc agcgcagggt gcctggggcc agcatcagac 2100
gcttcctgga aacctcagaa tgcattttgt ggggatgaga aaaataaaga ggcaccgcaa 2160
gatggctctt caagacttcc cagctgtgaa gttttggggg caggaatgaa gatggaccag 2220
caagctgtct gtgagctgtt gaaagtggag atgcctacaa gactgccaga ccggtcagtg 2280
gcctggcctt gccctgacag acatctccgg gagaaacctt ccttagaaaa ggtcactttc 2340
cagattggaa gctttgcatc tccagagtct gactttgaaa gccgcatgaa aaaaatggag 2400
gaacgggtga aggcctgtgg cccctccttg gaggccagtg aggctgctga tgtggctcag 2460
gacccgcagg tttctaggag cccttttaaa cctggctttc aggagaatgt ttgctgtcct 2520
cagaatcggc tttcagaggg ggatgaaggc gagtctgaca agggttttgc agaggacaga 2580
ggcagcagaa acgacatggc agcagatatt gctgggcagc tcagccacgc tgctgacttg 2640
ggcacagcct cccacggtgc aggaggaacg ggagggagga ggctggaggc cactagaggt 2700
ttgtatgtga aggctgcgga aggacctgtg ctggagcctg ttgcccccag gtgtgtccag 2760
cggggccctg gcctcgtggc tggtgcgaat atcccctgtg ggatgacaa caagaaggcc 2820
aacttcagga ctgaaggaga cattccccga aatgaaagct cagatagcgc cctgggagac 2880
agtgacgacg aagcctgcgc ttcagccatg ctagatctgg gtcacggtgg tgacaggact 2940
ggagggtcct tggaagtgga gctgcctctg ccaaggtctc agagcatcag cacccagaat 3000
gtaaggaact ttggccgctc acttctggcg ggctactgcc ccacatacat gcctgatctt 3060
gtgcttcatg ggaccggcag tgatgagaag ctgaagcagt gcctggtggc cgaccttgtc 3120
cacacagtcc atcatccagt cctggatgag ccaatagctg aagctgtctg tattatcgca 3180
gacacggata aatggagtgt gcaggtagct acaagtcaga ggaaagtgac ggacaacatg 3240
aaactaggcc aggatgtcct ggtctctagt caggtgtcca gtttgcttca gtccatttta 3300
cagctctata agcttcacct ccctgctgat tttgcatca tgcatcttga agatagacta 3360

cagg agatgt accttaaaag taaaatgcta tctgaatatc tccggggaca cacacgagtc 3420
catgtgaaag aattaggtgt cgtactgggg attgaatcca acgacctgcc tctgttgact 3480
gctattgcca gtactcattc tccttatgtg gctcaaatac tcttataagc taaagctcag 3540
gacagttctt ccttggaaga aaaaaatcaa attctcaact gaaggagaaa ggaataagct 3600
ctctgtgatg tcaaaagcat gagaagagca aacagaaaca gtcattccac ctttttgttt 3660
tgtgtttttg ctgtcaagct gatgcttcat tgaagactta ggtttacttg acataatagc 3720
atttgtgatt gtcgtgaaca ctttaggcca tttgttaccc atgaatcaac aaagaaactg 3780
acctttttgg taggaggaaa cataagcact aaacactaag ctgttgcaac agattgcctt 3840
gtgctgtttg ggcagaataa agacaagtga cttgagcggg gtggtcagca gtgtacataa 3900
tattccagta ggaaactgct tccaagttta agcatgagct ccccaaactg gagaaaacat 3960
attttgctat tctgagacaa caatcagaat acagactttg gattccaggt cacagtttgc 4020
tttttagaca aggtaaagca aagaaagcca cattgtgcca tcttcagctc cagtggcttt 4080
agcagtgact gtttgacata aaacatgtaa gaattgcttg ttgggaagag tgctttaggg 4140
acccactgtt ttcatttctt cttggagttt accttgtttc agatgcagcc atgggtaggt 4200
cagagatgga ttgttggtgc aataaaccca agaatcaatg tagcctctta atcccatcaa 4260
gatgtagttt gtagcagcaa agtgtacagt ctgaaaccgt atgttttatc cttatatttt 4320
agagctttca gcagcctttt taagagaggc cacttaccaa agttatttct ataagctcaa 4380
gagtgtttcg gttggtaagt tcttccagct gaagccactt tttccttata gttaatacaa 4440
atgactattt ttactttaaa aggcacagct gtcctggtgg gaaatgaaac ctgcagcagt 4500
tcaggatgac taatgaaagc aattagcttg aacatttaga aaaaattcat atatgatcta 4560
aatttttata ttatcatttc tgtgccttct aattcctgca tcctgttcaa aacatctttc 4620
cagacattaa cttacacatt gtataaaacc gaccaaaatg atttcctaaa gttcatgcaa 4680
aaaaaaaaaa aaaacaacct aattttctgt taatataaaa gaaacttcag tttactgacc 4740
gtgaaacaga ctatgtactg acatccaggg taaagtaaaa gacttttaaa tattggtcat 4800
taaaggacag gagctaagct agcaaagcaa aacatcttta gcactttgca gatctcaagc 4860
agttaaccag gctctgattc ccttccactg ttttatgaat taattccagt tcttttcatg 4920
tatctttgaa cctaagatta tgaagtaatt tccctattag ggactagaat gacttcagtt 4980
ttttcatttg ataaaaatca gaactgctac ctttcccttt tttaatgatg caaaatgtag 5040
atgagtgcat taaggtttgt aagatcttta tcattttatg tcattcattg aaaattgaaa 5100
tgttcattct ttttaatgtt ttcctatttc cttttgccta gcatttgact ttggtgtttt 5160
aagttctgta gttccatgac atcattgttt gctgttgtgt tacagagaga gaaggaacct 5220
cacctgtggc tcagctcacc ccacatccgt ttctcattac gtgtaaataa actgtcagag 5280
ctgatgttac agcttttaca gtttaaagca ttcccctcgt ctctagttcc ttttttcttg 5340
tttacatgtt ttgggcactt tccctcattc accaccttcc agggtttcat agaaaataac 5400

ttgttacaaa atcagttcaa ttctaatgtg gacatagtgg catgttcata attagaccca 5460

tataggggac actgagcttt aaatcgttga ttctaaactc tatacattaa aaaaattcag 5520

cccaggcccc tcaaagcctg agaaaattta atttgctctt aatttaatgt tccaaaactc 5580

actcttggaa aaatgcctgt tggaaaacta caggtgggtc acatgtgggg gctgtctccg 5640

tgacactcag gattccagtc agaacctaat cctcatatct attgcctaca aaaatagacc 5700

aagaatgttg ctgctctttt ataatccttt aaatatttaa cattcaagtt ttctttgtct 5760

taaattcagc ctcttcctaa aagcaaaaaa aaaaaaaaaa a 5801

<210> 42
<211> 1242
<212> DNA
<213> Homo sapiens

<400> 42
agacactttt aactcacagt aaaagcaaaa gcaacagctc aagcagcctc cttggagaaa 60

acctgaaaat tcaacttgtt caagagaagg tcttgtacgt gcctaagttc tagagcctcc 120

tgacgtgagc atggctgaga gtgaggaccg ctccctgagg atcgttctgg tagggaaaac 180

tggaagtggg aaaagtgcaa cagcgaacac catccttgga gaggaaatct ttgattctag 240

aattgctgcc caagctgtta ccaagaactg tcaaaaagca tcccgggaat ggcaggggag 300

agaccttctt gttgtagaca ctccagggct ctttgacacc aaggagagcc tggacaccac 360

ctgcaaggaa atcagccgct gcatcatctc ctcctgccca gggccccatg ctattgtcct 420

agttctgctg ctgggccgct acacagagga ggagcagaaa accgttgcat tgatcaaggc 480

tgtctttggg aagtcagcca tgaagcacat ggtcatcttg ttcactcgca aagaagagtt 540

ggagggccag agcttccatg acttcatagc agatgcggat gtgggcctaa aaagcatcgt 600

caaggagtgc gggaaccgct gctgtgcctt tagcaacagc aagaaaacca gtaaggcaga 660

gaaggaaagt caagtgcagg agttggtgga gctgatagag aaaatggtgc agtgcaacga 720

aggggcttac ttttctgatg acatatacaa ggacacagag gaaaggctga aacaacggga 780

agaggttttg aggaaaatct acactgacca attaaatgaa gaaattaaac tagtagaaga 840

ggataagcat aaatcagagg aagaaaagga gaaagaaatt aaattactaa aattaaaata 900

tgatgaaaaa ataaaaaata taagggaaga agctgagaga aatatattta aagatgtttt 960

taataggatt tggaagatgc tttcagaaat atggcatagg tttttgtcga aatgtaagtt 1020

ttattcttcc taatttactg tgatttgtta atggatgaat tgtattttgc aaagatagtt 1080

agagaaatac ctccttcccc ttagctttat taaggtatca ttgataaata aaaataaaat 1140

atgtttaatg tatataatgt gatttttaaa tatatatata tatatacaca cattgtgaaa 1200

taatgaaata aaggtaatta acacatctaa aaaaaaaaaa aa 1242

<210> 43
<211> 10759
<212> DNA
<213> Homo sapiens

<400> 43
   attctccgcg gcgcggcggc gagtgctggc tgcggtaccc tcccgctcgc ttgtccgtct 60
   gcgcgcccgc gatgtgacca gccgactggg ggcgggctgg cggctctgcc tgcggcagcg 120
   catcggtttt tctcctggca ggtccctaat tgagccgtga ttcccttgga gccagagaca 180
   cccaccaggg gctcggagcg ccgcgctccg gggctggagg cagctgcagc gctcgggtcc 240
   gcgcggccgg gtgcagtagc ctcagtccca gccgccgcct cgctgagtcg tgtgccctgg 300
   cgaatgcccg gcgcccggca cagctgagcc aaggcgactg gtgcagagcg gcggcgcctg 360
   gtcctgcact tgccgctgct gccgggctgt gccggggcgg cggcgctgcg cgccggagtt 420
   tgcagaggct gcctccgagc gccgtccgcc aggaagactc cctgcctcct gagtcccaaa 480
   aacacgagat ttttctccta tttcagcagt tggccacaac ttcattgctg ggaaaaggcc 540
   aagaaagaag agagaactct caccacaaca tttcaaaggg aatacattgc cagaacttgg 600
   aatactctac tggacaaaca tttcctccaa ggacacagct ctctgcctcc atgtcaccac 660
   ctttgaagga ctgactgatt ccctcggctg gtgccagtgc ctgctcctgc catggggccc 720
   gcggggagcc tgctgggcag cggacagatg cagatcaccc tgtggggaag tctggctgct 780
   gtcgccattt tcttcgtcat caccttcctc atcttcctgt gctctagttg tgacagggaa 840
   aagaagccgc gacagcatag tggggaccat gagaacctga tgaacgtgcc ttcagacaag 900
   gagatgttca gccgttcagt tactagcctg gcaacagatg ctcctgccag cagtgagcag 960
   aatggggcac tcaccaatgg ggacattctt tcagaggaca gtactctgac ctgcatgcag 1020
   cattacgagg aagtccagac atcggcctcg gatctgctgg attcccagga cagcacaggg 1080
   aaaccaaaat gtcatcagag tcgggagctg cccagaatcc ctcccgagag cgcagtggat 1140
   accatgctca cggcgagaag tgtggacggg gaccaggggc tggggatgga agggccctat 1200
   gaagtgctca aggacagctc ctcccaagaa aacatggtgg aggactgctt gtatgaaact 1260
   gtgaaagaga tcaaggaggt ggctgcagct gcacacctgg agaaaggcca cagtggcaag 1320
   gcaaaatcta cttctgcctc gaaagagctc ccagggcccc agactgaagg caaagctgag 1380
   tttgctgaat atgcctcggt ggacagaaac aaaaaatgtc gtcaaagtgt taatgtagag 1440
   agtatccttg gaaattcatg tgatccagaa gaggaggccc caccacctgt ccctgttaag 1500
   cttctggacg agaatgaaaa ccttcaggag aaggaagggg gagaggcgga agagagtgcc 1560
   acagacacga ccagtgaaac taacaagaga tttagctcat tgtcatacaa gtctcgggaa 1620
   gaagacccca ctctcacaga agaagagatc tcagctatgt actcatcagt aaataaacct 1680
   ggacagttag tgaataaatc ggggcagtcg cttacagttc cggagtccac ctacacctcc 1740
   attcaagggg acccacagag gtcaccctcc tcctgtaatg atctctatgc tactgttaaa 1800
   gacttcgaaa aaactccaaa cagcacactt ccaccagcag ggaggcccag cgaggagcca 1860
   gagcctgatt atgaagcgat acagactctc aacagagagg aagaaaaggc caccctgggg 1920
   accaatggcc accacggtct cgtcccaaag gagaacgact acgagagcat aagtgacttg 1980
   cagcaaggca gagatattac caggctctag caacccagaa gacaaccctg ggtagcctgt 2040
   gatcagtgtc tggagacgtt tcttctgtgg aagagaagaa gtgacacaaa cctatacttc 2100
   atatgctgct ttagtcacct gaagatggtt ggagaggccc tgtcgactgt tctcccagtt 2160
   gttcagtttc tgagacagag aggtacggac taggctgcac ctgagtgtgc ccctgcctgc 2220
   cagatggaca ggtacaccca agcacatctc cctgctgcac cctcaccacc cacaaaagat 2280
   cccagctgtc agtggtctca tctcattagt gaggaaagcc aagctgtatg gaaaagctgc 2340
   actcaccaag gaccacaatg cccccggcct aaagtactgc cattcagaaa agcaggtttt 2400
   tcttcctctc tttccttttc tctgtctgct actgacttta aggctttttc ccccttgaaa 2460
   tgtccagatt cctgtggttc atcccaagga aattttcaca caaagcttgg cctttgccct 2520
   caatataggt gttttaggat ggtgacaaac catggctgct gctttctgcc cagctcgcca 2580
   gtcctcccca aagagttgcg catcagcacc tggggatctg gaccctgcgg gtgaagggat 2640
   ggggagggac gtccctggag tctcttctgt ctttgttcct tcttattttg gcattcgata 2700
   tcagcagcct ctccccaaag tacttgaagt cagttttaga tgctttattt tatttttcta 2760
   gtcaaaaacg tgtttccccc agtgtttgaa aactcgtccg aatcttttca gtattttcca 2820
   tgagtattgt ggtacttcta gacttgttta agcccagaac tcattccttc aaaacagaga 2880
   gccttaatct ttatgttggg acacagacca catatttgga cggcagccat gcatccatcg 2940
   ctgaagggct gtggacatga atgtgtattt cccatggtct ccgctgccca caccaacagt 3000
   gtggcatctc ataagttaac tgctacccta aggtaatcta agattaaaat gtaaacattt 3060
   atttttgtta tgtaagttta taagatgttt tatgttcaat gcctaatttc tcaaaagtgc 3120
   cagaaaaaaa tgtatattag ctattttgat tttatgtaca atgatttata ctctcctttt 3180
   gaaaagatac cataaagcac ataagctaga tcactacaag gagctgttat cttttttcta 3240
   atcaagtgtt taaaacactg atggttttta aagactcacc tttttaaatg gtacttggag 3300
   ctcctgattc aaattaccta gaccccctag agaaataaat ggaatataca taaataatca 3360
   ttttcagtgg tttatggtgg gcaatattgc aatatttgaa atggtaaaaa tggaaagaag 3420
   aacaaaatat gatgagaggt ggctgtgaat tataaacctc ataaaagtgt cataattcca 3480
   ttaaggttta attatatttt ttcagaaaac agtgatgaat tctgtagtcc agtgcttgcc 3540
   aatgcaaatt gcctattgga atcttcttcc tatattttac aaacatcagt ggctgaaata 3600
   gctcagagta agagctcagc ctggtttgaa tttaatcatc tctttagatc ttataaggcc 3660
   agcattagga aacttgttca cttttcattt tcaaaggagc ctagttgaag tgctattatg 3720
   agtgtgggct atggaaagac agcttttcct acactgataa agaaaaaaaa tgaggaaatt 3780
   atttcatccc cttgtgacat ctgtgacttt ttggatttaa taatcttgct gtttttcctc 3840
   tttatgacaa agaatataat tgggaggatg aagtgtctta aaaattgtag agaccagctc 3900
   actggaatgt ttttccatcc ctgtattcat ggcttgactt tgtgactgct ctacactgca 3960
   tgtctgacat tgcagagtga gctatgttga ggtaaactgg ttggttgtca ttattttgca 4020
   atcagcctgg tctctcccat gaagatgtcg tgtgcataag cacaatcatc actgattaga 4080
   agatcacagc agaataccct tggattagag agaagttcgt accttgcatt tctctgaatt 4140
   ctagtctctc ataagcactg ctttgctgga tgattttcac tgctttgtgt taatgacttt 4200
   gagcgatctc tcacatgatg gggttcttta gtacatggta acagccatgt catcttacac 4260
   acctagcatt gtgaatgctg tagtgacatc ctttataggc accttacagc tcaaaacttt 4320
   tgtttcattt catgccttac ttatcaaaaa ggcaggaaag taggtatgat ctctaaagta 4380
   aaaaaaaaaa aaaaaaaaaa actttttata gaaagctcat aaataatcat gtcattttgc 4440
   aattttgtta ccaaaatttc ccccaagagt tttcaaatat tagttctgca atgtggctat 4500
   gaaatatgca ctgaaatata ccttttaatt tgagaaccag tggttagaat aagctgtgat 4560
   ataaagtatt ttcagtgtac ttttaaagga actataaggc cctccagcat aaacgctaaa 4620
   agaatagatg gtagcacagg ccatgagggc tgggggagag aagcagagtg aaccttagaa 4680
   agatggctca gctatttgga gcactggata ttttactgaa gttatttact gaggcaccat 4740
   cactgttttg actgtacagt atagtttttc ataaatttca tcacatttac tttgttcaga 4800
   atctgggctt gaatctttga gttggacaaa agcctatggt ttcttttaaa aagtttcatc 4860
   ttgagctaat gctacagttt aaataaaatg tatgaaaggt agttatcaaa aacaatttgt 4920
   atatttaaaa ttctattttg acatccattt tttacagcga attatagcag gttatgtgaa 4980
   atttaaaaat aaattttagg aaattttttt atcttgtaag taagaattat aaatctatct 5040
   cagttaaaag taggatcttt gtttttattc agatattttg aaagttagaa agcattttgt 5100
   atgctacttt ggtatttctt aagtatagtc agcaggaaag ggtaacctgt tttatgcaaa 5160
   atttttttaa tttgtcaaat gtttttagtg tgtctacttt ctgaaatagt ctcaatatcc 5220
   tgtctgtatc ctaggcagat aactacacaa gaacaaaacg ctatatagaa aagaaaatat 5280
   ttgaggaaat cttaaattcc ataagaaagt aattcttgca agaatgttgg ctacatattt 5340
   ttttttctgt cctgaataaa ttacatcatt aggtctgttg aagcttttga agctaccact 5400
   attctttctg ggataaaggc taattactga tttatctgcc ctcaaaatcc cagagttaat 5460
   tctaattgag tactaaatta acagtaagta gttaacacag agaactaaaa tttaaccgca 5520
   ttatatcact gtatatattt ctcatgattt ttgctaatta gagcatttac ttaaccagta 5580
   ataaactaca cacacacaca cacacacaca cacgtatacg tgtgtgtgtg tatatacttg 5640
   gctctgacac aactctggtg cttaattagg atatgttcta ttatgtattt tgaatgttta 5700
   tcctgctagt gtgatgaact tttgttggaa aagcagaatt agaaggaaca gtttttcaat 5760
   cagtttcatt tggtaattac aggaaaacct tgagttgctc taatttaatt tttccaatta 5820
   atacatttag actttaaatt gcatccgtaa ttctcttggc tgaaaaacag tggtatttta 5880
   agacagtttt gttatacatg tgacttactt ttgactcagc ctgagaggag gaaatgtaaa 5940
   gagataagag atttggccca aagcagcaag agactagtaa ccttgagcca ggacgaagcc 6000
   aagacaacgt gactcccaag tagcaggagc agaggtcggt cacctgtggc attctgtttc 6060
   attccctttt aaaattatga gactttatag gcagtgtaaa acatcagcag gcagcgatgt 6120
   gataggatgt gcgaaaaagc tggtctgata ccatggctat aattacagag ctttagttca 6180
   attaggattt tgtaaatgag caaatgacct ttttttccag tgccccttgt aatagttaat 6240
   atgagtccat gcaatcttgt gatgccattc tcctgaaggt gttgatttct ggtacccagc 6300
   cagcttagct ttggctgctg gaagcacagt aggtactgat gattacttcc tggaaatctt 6360
   gacaggctta ttgtactgtg taatggggaa aagttaaagt ataatgtttg gtgttaataa 6420
   atgactgatg tgaaaatagg aacatgtgtc tttaatatca aatatggctt tatttgcagt 6480
   gaaatctaaa ttccttattc tacagtagta ttttcttgcc ctgtgttgta tattttccta 6540
   aatacatttt tcctgacagt ggctttaggc cagttgaaca cacttagact gaaagtgttt 6600
   aacttaaaga gatcagtcca gaaaccatga taaattagaa tctttatctg gaattaccaa 6660
   attaaaattt aaaatcatgg tccagaagag aacaaacatt catggttttt tttatcctac 6720
   tgctcatttt taggtctgtg tttacatcta gtctctacta tttgtgaagt atgtcattat 6780
   ttttttcaag ttcttctttg acttaactga aaaataatat tccaaattct taatgtaaca 6840
   tgaaaagaga aatacaattt ttgcttaaaa gacatttttt aaaaagcgtt caattcagta 6900
   atcatttctg ttaatacagg aagttttttt ggcttgccag ttatatactg tgggtatttt 6960
   ttaaaatgtg ctacccttgg gttgtctcat atcaccttgc gtaatcatgt attacaaagg 7020
   ttgataattg tcatttctca gatgtctgta tgttacattg gcagcagtaa aatgttttaa 7080
   tgttgttacc ttttaaataa ttgtgttgta ttaacatgcc tcatattttc tggggaaact 7140
   tgaaatatat ctaaacaaaa aaggtctgtt ataaatagga attggcattt cattgttaat 7200
   gttttttcct cataaaaata gttacaccaa aagtgacata ttgtctatta catgggccca 7260
   atcagtatta aagtactccc cttactcaaa aatattaaaa aataattcat gacacttagt 7320
   agcattttca ttgattgttt caaaagatct tcataatggt caaattgtcc aattcacaaa 7380
   agagtgagaa agttgttttc agtactggga atttttaaac cttagtttta agaccaaaaa 7440
   tatctcttat tagcttgaac attttgtgat tacttttccc tccccggatc tagtcctttt 7500
   aaggagtaag tctaaagaat gaggccatga agtcactatt tcctcccacc tcagtttgtc 7560
   tcctggtact tagctggcct atcgctttgt gtggcaatac ccctgggtcg cttccccact 7620
   cagccttctg tatctgctgt tcacaggaca tgccattatg tctttccagt acggatcaca 7680
   gtgctgccat atcacagacc ctgccagccg gcacagaacc atgcctcgtc acagctctgc 7740
   ccaactcaca ggctgccaag atgaggccac aggtccacct agggcggcag atgctggtgc 7800
   tatttgtcac gacttttgcc aaaacatgta cactgttgct cacatctttc gtaagaatgg 7860
   ttgacactaa ggggccatgg aaaagcactt ttaaaaaatt atgtggtgga atcaaactca 7920
   cagaggcaga aatgttagtg ccgtgctcta accaagtaag cttagtgttc cccacagtga 7980
   tctgtatctg gcctgagtct cctcaagcag caacccccac tgtccagcat gtggaaggta 8040
   gatccttatg acaacaccag acccatagtt accaggaaag aaaaaggaag ctgagtgttg 8100
   tgaaggggaa gaaatccttt ttataagaag taatcatttt taggccgggt gcagtggctc 8160
   atgcctgtaa tcccagcact ttgggaggct gagacaggtg gatcacttga ggtcaggagt 8220
   ttgagacctg cctggccaac atgatgaaac cctgtctcta ctaaaaatgc aaaaaaaatt 8280
   agccaggcat ggtggcatgt gcctgtagtc ccaactactc aggaggctga ggcaggagaa 8340
   tcacttgaac ctgggaggca gaggttgcaa tgagctgaga tcgcaacact gcactccagc 8400
   ctgggcgaca gagcaagact ccatctcaaa aacaaaaaca aaaaaaaaac ctttttaaag 8460
   taaattgagt gtaaaatgtt ttcaccatga agccattgca ccctctcccc caggaaacag 8520
   tgcctaagga tgatgtcaag ttacagccag tttgctctga tcccccagga cctaagaaaa 8580
   ctgtgagtgc ttcagcatgc aaaggtagag ctaccagata aggagaataa cttggccatt 8640
   tatagcaaac tgcctgactt tggagtgaga aaccaagcac cttctaggtc ctaggatagg 8700
   tgaattggag gtgcagctgc tgaagttccc attttctaca tacttcagga gccagggtgt 8760
   ttcatttggt tttgtaaaac tttgctgtaa gtcatcacag ttaattcttt gaatggacat 8820
   tttagagtgt aacttttttt caaaattagc agaacagata tctctgctca ttctctttat 8880
   acatttagtt ttttaaagtt cccctagtat acctgttagg cactctaaat aggacacagt 8940
   taaagctaaa agaaggacca gttgctccct agagcctaca gttcagaact atcttgttgt 9000
   catgtcctgg tttggtttgg tttgttttta actcaacaag ttggaaacca tgagtttaaa 9060
   gcaaactatt tccttttctt aagcttaaag agaatttttt ttttaatctt tagctcctca 9120
   tttaggtagt gaaggcttcc ctgaaagaca gcagtgccct tctctggaag aagaggctgg 9180
   gaccaaaagc ctgagtctat gggttgagga ttttgagcat tcctttgaca agcttctatg 9240
   tatttttaat ataagactca taaatgaacc tgatgtggtg ttcatgaccc atcccttaag 9300
   catgcttgct ttttttaact ttactttttg agtatagtac ttgtcttgtt tttcctggag 9360
   ccatgctagg atttaattcc tatgtgccac tacccgccca tgtcctggaa gatggccatg 9420
   cctgcagcaa tgctcatgtc ttgaggagaa gcaatgtgaa aagcatcagg aaagctgaac 9480
   cttacgttca agtcagcaaa catttgtgag cattctagta tgcttgcgac cttgacactt 9540
   tttaaaaatt atctttttat ttgcttcgag tatcaaccgt atagttgact ctagtggggc 9600
   gaagggagca gagatgagag caaaattcag acttgctgcc cctgtgtgtc tcagttctca 9660
   tgtctctctg agaaatgtgt tggcccaatt ccctatctgc cactctgagt ctcttgagta 9720
   aatgtgtgta aatgagaaaa attatctcaa agatttaact tattatttag atattttctt 9780
   ctttttaaac tcaggaataa acctggtttg tagataatat aatatggcta ctagagctat 9840
   aattgagtag gtgactagaa gcattttttc aaatatattt cttgtaaaga catctacttt 9900
   gttttaacta ggaaaagaaa ctcctcatat taaaataaac atttccataa gcactttcta 9960
   gaagagtgta gcatccatct accatccatc ccagaatgtt tgtatcttgc tcatagtatt 10020
   tcaaaaattt ttatgggtga aaaaaatgca cttttttatt tattggaaaa gtgtagagtt 10080
   attgaattaa attctcaagt gggacaattt agtgtaaagg gaaacatggg ttttggagac 10140
   aagagcacag ggctgtggtc cctgttccag cattgacttt actatgtgac ctcgagcaaa 10200
   tgatttaact tctctgtgcc tcagtttctc catatacaaa atggggataa tgatactaac 10260
   cattgcctac ctcatagaga tgttatgggg acaaacgaaa taatagagat aagcacgaat 10320
   gctttcaact cttcggaaga aaggtgctat ataaattgaa gttgtgtttt taatgatcca 10380
   attattaatt atgtttaggg tttaaataac aatactgtta gtcatgttaa gaataagttt 10440
   tgttttgatg catatttcca cttctcttct gtggccagat cttgatcatt caaccagtaa 10500
   tggtacctga ggaactgaaa tgggtatttg ttttcgtatg tttctgccag tagtatttca 10560
   attgaatatc cagaaaagac tggagtttaa cttgtaatat cttatagttt acatgtaata 10620
   aaacttattc aagctatata taaaagtatg attacatgta aatagcaggt atgttgtaat 10680
   taaaggcact tatcaaattg tctttgttct tttttaattg taataaaagt cagttttaca 10740
   taaaaaaaaa aaaaaaaaa 10759

<210> 44
<211> 4366
<212> DNA
<213> Homo sapiens

<400> 44
tgcattcttt gccccaaaac tctttccttt ggttgtgcta agaggtgatg cccaaggtgc 60
accacctttc aagaactgga tcatgaacaa ctttatcctc ctggaagaac agctcatcaa 120
gaaatcccaa caaaagagaa gaacttctcc ctcgaacttt aaagtccgct tctttgtgtt 180
aaccaaagcc agcctggcat actttgaaga tcgtcatggg aagaagcgca cgctgaaggg 240
gtccattgag ctctcccgaa tcaaatgtgt tgagattgtg aaaagtgaca tcagcatccc 300
atgccactat aaatacccgt ttcaggtggt gcatgacaac tacctcctat atgtgtttgc 360
tccagatcgt gagagccggc agcgctgggt gctggccctt aaagaagaaa cgaggaataa 420
taacagtttg gtgcctaaat atcatcctaa tttctggatg gatgggaagt ggaggtgctg 480
ttctcagctg gagaagcttg caacaggctg tgcccaatat gatccaacca agaatgcttc 540
aaagaagcct cttcctccta ctcctgaaga caacaggcga ccactttggg aacctgaaga 600
aactgtggtc attgccttat atgactacca aaccaatgat cctcaggaac tcgcactgcg 660
gcgcaacgaa gagtactgcc tgctggacag ttctgagatt cactggtgga gagtccagga 720
caggaatggg catgaaggat atgtaccaag cagttatctg gtggaaaaat ctccaaataa 780
tctggaaacc tatgagtggt acaataagag tatcagccga gacaaagctg aaaaacttct 840
tttggacaca ggcaaagaag gagccttcat ggtaagggat tccaggactg caggaacata 900
caccgtgtct gttttcacca aggctgttgt aagtgagaac aatccctgta taaagcatta 960
tcacatcaag gaaacaaatg acaatcctaa gcgatactat gtggctgaaa agtatgtgtt 1020
cgattccatc cctcttctca tcaactatca ccaacataat ggaggaggcc tggtgactcg 1080
actccggtat ccagtttgtt ttgggaggca gaaagcccca gttacagcag ggctgagata 1140
cgggaaatgg gtgatcgacc cctcagagct cacttttgtg caagagattg gcagtgggca 1200

atttgggttg gtgcatctgg gctactggct caacaaggac aaggtggcta tcaaaaccat 1260
tcgggaaggg gctatgtcag aagaggactt catagaggag gctgaagtaa tgatgaaact 1320
ctctcatccc aaactggtgc agctgtatgg ggtgtgcctg gagcaggccc ccatctgcct 1380
ggtgtttgag ttcatggagc acggctgcct gtcagattat ctacgcaccc agcggggact 1440
ttttgctgca gagaccctgc tgggcatgtg tctggatgtg tgtgagggca tggcctacct 1500
ggaagaggca tgtgtcatcc acagagactt ggctgccaga aattgtttgg tgggagaaaa 1560
ccaagtcatc aaggtgtctg actttgggat gacaaggttc gttctggatg atcagtacac 1620
cagttccaca ggcaccaaat tcccggtgaa gtgggcatcc ccagaggttt tctctttcag 1680
tcgctatagc agcaagtccg atgtgtggtc atttggtgtg ctgatgtggg aagttttcag 1740
tgaaggcaaa atcccgtatg aaaaccgaag caactcagag gtggtggaag acatcagtac 1800
cggatttcgg ttgtacaagc cccggctggc ctccacacac gtctaccaga ttatgaatca 1860
ctgctggaaa gagagaccag aagatcggcc agccttctcc agactgctgc gtcaactggc 1920
tgaaattgca gaatcaggac tttagtagag actgagtacc aggccacggg ctgcagatcc 1980
tgaatggagg aaggatatgt cctcattcca tagagcatta gaagctgcca ccagcccagg 2040
accctccaga ggcagcctgg cctgtggcat cagtccctga gtcaccatgg aagcagcatc 2100
ctgaccacag ctggcagtca agccacagct ggagggtcag ccaccaagct gggagctgag 2160
ccagaacagg agtgatgtct ctgcccttcc tctagcctct tgtcacatgt ggtgcacaaa 2220
cctcaacctg acagctttca gacagcattc ttgcacttct tagcaacaga gagagacatg 2280
agtaagaccc agattgctat ttttattgtt atttttaaca tgaatctaaa gtttatggtt 2340
ccagggactt tttatttgac ccaacaacac agtatcccag gatatggagg caaggggaac 2400
aaagagcatg agtctttttc caagaaaact ggtgagttaa gtaagattag agtgagtgtg 2460
ctctgttgct gtgatgctgt cagccacagc ttcctgccgt agagaatgat agagcagctg 2520
ctcacacagg aggccggata ttctgagaag cagctttatg aggttttaca gagtatgctg 2580
ctacctctct ccttgaaggg agcatggcga gacccattgg atggattggg gtgaacagtt 2640
caggtcccat gcttggagca ttgggtatct gatgtctgca ccagaacaag agaacctctg 2700
acggtggaga accatgtggt gcaagaagag atcttaggtc tcttctttta taccaagctc 2760
atcttttata ccaagctgtg caggtgacta tgcctcctct tctgcacaga atgcttccac 2820
cagcatcctg agaagaaatg attacttctg aaaaacatcc ttttttccag cctctgggaa 2880
tcagcccccc ctctctgcac tatccgatcc tcatcaacag agggcagcat tgtgttggtc 2940
aatgttccct tggcgagcaa ttgaaacttg tttaggccct agggttgagc aattttaagg 3000
ttgagactcc aagtctccta aaattctagg agagaaataa agagtctgtt tttgctcaaa 3060
ccatcaggat ggaaacagtc aggcactgac tggggtgctt ccaagaggca tgagagtgcc 3120
tactctggct tgagcacttc tatatgcaag gtgaatatgt actgagctag gagacttccc 3180
tgcaaatctt ctgttcaccc tgggttcaca tccccatgag gtaatattat tattcccatt 3240

ttacaaataa tgtaactgag gctttaaaaa gccaagacat ctgcccaaag tgatggaact 3300
agaaagtcta gagctggtat tctagcccaa atctgtctga ccgcaataca cagattcttt 3360
attcctattc gacactggct tctactgaaa atgaaacgga ttgcagaggg aataaataca 3420
aagatggaaa gccagtaaag aagtcagtat agaaccacta gcgaatagtg ttgctctggc 3480
acagaccact gtggttgatg gcatggccct ccaacttgga ataggatttt ccttttccta 3540
ttctgtatcc ttaccttggt catgttaatg actttggagt tattcagtta atgacccttt 3600
aattctcaca accaaccagt catgttgctt gaagccattt atagacgagc ttcaaagcaa 3660
ctttaaaaga ttcttctgta gaagtatgag ttcttccttt aattatcatt ccaactttca 3720
gctgtagtct tcttgaacac ttcatgagga gggacattcc ctgatataag agaggatggt 3780
gttgcaattg gctctttcta aatcatgtga cgttttgact ggcttgagat tcagatgcat 3840
aatttttaat tataattatt gtgaagtgga gagcctcaag ataaaactct gtcattcaga 3900
agatgatttt actcagctta tccaaaatta tctctgttta ctttttagaa ttttgtacat 3960
tatcttttgg gatccttaat tagagatgat ttctggaaca ttcagtctag aaagaaaaca 4020
ttggaattga ctgatctctg tggtttggtt tagaaaattc ccctgtgcat ggtattacct 4080
ttttcaagct cagattcatc taatcctcaa ctgtacatgt gtacattctt cacctcctgg 4140
tgccctatcc cgcaaaatgg gcttcctgcc tggtttttct cttctcacat tttttaaatg 4200
gtcccctgtg tttgtagaga actcccttat acagagtttt ggttctagtt ttatttcgta 4260
gattttgcat tttgtacctt ttgagactat gtatttatat ttggatcaga tgcatattta 4320
ttaatgtaca gtcactgcta gtgttcaaaa taaaaatgtt acaaat 4366

<210> 45
<211> 4045
<212> DNA
<213> Homo sapiens

<400> 45
gcagccagag ctcagcaggg ccctggagag atggccacgg tcccagcacc ggggaggact 60
ggagagcgcg cgctgccacc gccccatgtc tcagccaggg cttccttcct cggctccacc 120
ctgtggatgt aatggcggcc cctgctctgt cctggcgtct gcccctcctc atcctcctcc 180
tgcccctggc tacctcttgg gcatctgcag cggtgaatgg cacttcccag ttcacatgct 240
tctacaactc gagagccaac atctcctgtg tctggagcca agatggggct ctgcaggaca 300
cttcctgcca agtccatgcc tggccggaca gacggcggtg gaaccaaacc tgtgagctgc 360
tccccgtgag tcaagcatcc tgggcctgca acctgatcct cggagcccca gattctcaga 420
aactgaccac agttgacatc gtcaccctga gggtgctgtg ccgtgagggg gtgcgatgga 480
gggtgatggc catccaggac ttcaagccct ttgagaacct tcgcctgatg gcccccatct 540
ccctccaagt tgtccacgtg gagacccaca gatgcaacat aagctgggaa atctcccaag 600
cctcccacta ctttgaaaga cacctggagt tcgaggcccg gacgctgtcc ccaggccaca 660

cctgggagga ggcccccctg ctgactctca agcagaagca ggaatggatc tgcctggaga 720
cgctcacccc agacacccag tatgagtttc aggtgcgggt caagcctctg caaggcgagt 780
tcacgacctg gagcccctgg agccagcccc tggccttcag gacaaagcct gcagcccttg 840
ggaaggacac cattccgtgg ctcggccacc tcctcgtggg cctcagcggg gcttttggct 900
tcatcatctt agtgtacttg ctgatcaact gcaggaacac cgggccatgg ctgaagaagg 960
tcctgaagtg taacacccca gacccctcga agttcttttc ccagctgagc tcagagcatg 1020
gaggagacgt ccagaagtgg ctctcttcgc ccttcccctc atcgtccttc agccctggcg 1080
gcctggcacc tgagatctcg ccactagaag tgctggagag ggacaaggtg acgcagctgc 1140
tcctgcagca ggacaaggtg cctgagcccg catccttaag cagcaaccac tcgctgacca 1200
gctgcttcac caaccagggt tacttcttct tccacctccc ggatgccttg gagatagagg 1260
cctgccaggt gtactttact tacgacccct actcagagga agaccctgat gagggtgtgg 1320
ccggggcacc cacagggtct tccccccaac ccctgcagcc tctgtcaggg gaggacgacg 1380
cctactgcac cttcccctcc agggatgacc tgctgctctt ctcccccagt ctcctcggtg 1440
gccccagccc cccaagcact gcccctgggg gcagtggggc cggtgaagag aggatgcccc 1500
cttctttgca agaaagagtc cccagagact gggaccccca gcccctgggg cctcccaccc 1560
caggagtccc agacctggtg gattttcagc caccccctga gctggtgctg cgagaggctg 1620
gggaggaggt ccctgacgct ggccccaggg agggagtcag tttcccctgg tccaggcctc 1680
ctgggcaggg ggagttcagg gcccttaatg ctcgcctgcc cctgaacact gatgcctact 1740
tgtccctcca agaactccag ggtcaggacc caactcactt ggtgtagaca gatggccagg 1800
gtgggaggca ggcagctgcc tgctctgcgc cgagcctcag aaggaccctg ttgagggtcc 1860
tcagtccact gctgaggaca ctcagtgtcc agttgcagct ggacttctcc acccggatgg 1920
cccccaccca gtcctgcaca cttggtccat ccatttccaa acctccactg ctgctcccgg 1980
gtcctgctgc ccgagccagg aactgtgtgt gttgcagggg ggcagtaact ccccaactcc 2040
ctcgttaatc acaggatccc acgaatttag gctcagaagc atcgctcctc tccagccctg 2100
cagctattca ccaatatcag tcctcgcggc tctccagggc tccctgccct gacctcttcc 2160
ctgggttttc tgccccagcc tcctccttcc ctcccctccc cgtccacagg gcagcctgag 2220
cgtgctttcc aaaacccaaa tatggccacg ctccccctcg gttcaaaacc ttgcacaggt 2280
cccactgccc tcagccccac ttctcagcct ggtacttgta cctccggtgt cgtgtgggga 2340
catccccttc tgcaatcctc cctaccgtcc tcctgagcca ctcagagctc cctcacaccc 2400
cctctgttgc acatgctatt ccctggggct gctgtgcgct ccccctcatc taggtgacaa 2460
acttccctga ctcttcaagt gccggttttg cttctcctgg agggaagcac tgcctccctt 2520
aatctgccag aaacttctag cgtcagtgct ggagggagaa gctgtcaggg acccagggcg 2580
cctggagaaa gaggccctgt tactattcct ttgggatctc tgaggcctca gagtgcttgg 2640
ctgctgtatc tttaatgctg gggcccaagt aagggcacag atccccccac aaagtggatg 2700

cctgctgcat cttcccacag tggcttcaca gacccacaag agaagctgat ggggagtaaa 2760
ccctggagtc cgaggcccag gcagcagccc cgcctagtgg tgggccctga tgctgccagg 2820
cctgggacct cccactgccc cctccactgg aggggtctcc tctgcagctc agggactggc 2880
acactggcct ccagaagggc agctccacag ggcagggcct cattattttt cactgcccca 2940
gacacagtgc ccaacacccc gtcgtatacc ctggatgaac gaattaatta cctggcacca 3000
cctcgtctgg gctccctgcg cctgacattc acacagagag gcagagtccc gtgcccatta 3060
ggtctggcat gccccctcct gcaaggggct caacccccta ccccgacccc tccacgtatc 3120
tttcctaggc agatcacgtt gcaatggctc aaacaacatt ccaccccagc aggacagtga 3180
ccccagtccc agctaactct gacctgggag ccctcaggca cctgcactta caggccttgc 3240
tcacagctga ttgggcacct gaccacacgc ccccacaggc tctgaccagc agcctatgag 3300
ggggtttggc accaagctct gtccaatcag gtaggctggg cctgaactag ccaatcagat 3360
caactctgtc ttgggcgttt gaactcaggg agggaggccc ttgggagcag gtgcttgtgg 3420
acaaggctcc acaagcgttg agccttggaa aggtagacaa gcgttgagcc actaagcaga 3480
ggaccttggg ttcccaatac aaaaatacct actgctgaga gggctgctga ccatttggtc 3540
aggattcctg ttgcctttat atccaaaata aactcccctt tcttgaggtt gtctgagtct 3600
tgggtctatg ccttgaaaaa agctgaatta ttggacagtc tcacctcctg ccatagggtc 3660
ctgaatgttt cagaccacaa ggggctccac acctttgctg tgtgttctgg ggcaacctac 3720
taatcctctc tgcaagtcgg tctccttatc cccccaaatg gaaattgtat ttgccttctc 3780
cactttggga ggctcccact tcttgggagg gttacatttt ttaagtctta atcatttgtg 3840
acatatgtat ctatacatcc gtatctttta atgatccgtg tgtaccatct ttgtgattat 3900
ttccttaata tttttctttt aagtcagttc attttcgttg aaatacattt atttaaagaa 3960
aaatctttgt tactctgtaa atgaaaaaac ccattttcgc tataaataaa aggtaactgt 4020
acaaaataag tacaatgcaa caaaa 4045

<210> 46
<211> 3908
<212> DNA
<213> Homo sapiens

<400> 46
   gatttgtgac gcaccctcag aactgcagta atggtccagc tgcttcacag gcaactggta 60
   accacctcat ttggggatgt ttctgccttg ctagcagtgc cagagagaac ttcatcattg 120
   tcacctcatc aaagactact ttttcagaca tctcctgtag ggctagattc agagagcagc 180
   ttctgatatt tggagggtga tctttagaca gtgactgagt atggatcatt tgaacgaggc 240
   aactcagggg aaagaacatt cagaaatgtc taacaatgtg agtgatccga agggtccacc 300
   agccaagatt gcccgcctgg agcagaacgg gagcccgcta ggaagaggaa ggcttgggag 360
   tacaggtgca aaaatgcagg gagtgccttt aaaacactcg ggccatctga tgaaaaccaa 420
   ccttaggaaa ggaaccatgc tgccagtttt ctgtgtggtg gaacattatg aaaacgccat 480
   tgaatatgat tgcaaggagg agcatgcaga atttgtgctg gtgagaaagg atatgctttt 540
   caaccagctg atcgaaatgg cattgctgtc tctaggttat tcacatagct ctgctgccca 600
   ggccaaaggg ctaatccagg ttggaaagtg gaatccagtt ccactgtctt acgtgacaga 660
   tgcccctgat gctacagtag cagatatgct tcaagatgtg tatcatgtgg tcacattgaa 720
   aattcagtta cacagttgcc ccaaactaga agacttgcct cccgaacaat ggtcgcacac 780
   cacagtgagg aatgctctga aggacttact gaaagatatg aatcagagtt cattggccaa 840
   ggagtgcccc ctttcacaga gtatgatttc ttccattgtg aacagtactt actatgcaaa 900
   tgtctcagca gcaaaatgtc aagaatttgg aaggtggtac aaacatttca agaagacaaa 960
   agatatgatg gttgaaatgg atagtctttc tgagctatcc cagcaaggcg ccaatcatgt 1020
   caattttggc cagcaaccag ttccagggaa cacagccgag cagcctccat cccctgcgca 1080
   gctctcccat ggcagccagc cctctgtccg gacacctctt ccaaacctgc accctgggct 1140
   cgtatcaaca cctatcagtc ctcaattggt caaccagcag ctggtgatgg ctcagctgct 1200
   gaaccagcag tatgcagtga atagactttt agcccagcag tccttaaacc aacaatactt 1260
   gaaccaccct ccccctgtca gtagatctat gaataagcct ttggagcaac aggtttcgac 1320
   caacacagag gtgtcttccg aaatctacca gtgggtacgc gatgaactga aacgagcagg 1380
   aatctcccag gcggtatttg cacgtgtggc ttttaacaga actcagggct tgctttcaga 1440
   aatcctccga aaggaagagg accccaagac tgcatcccag tctttgctgg taaaccttcg 1500
   ggctatgcag aatttcttgc agttaccgga agctgaaaga gaccgaatat accaggacga 1560
   aagggaaagg agcttgaatg ctgcctcggc catgggtcct gcccccctca tcagcacacc 1620
   acccagccgt cctccccagg tgaaaacagc tactattgcc actgaaagga atgggaaacc 1680
   agagaacaat accatgaaca ttaatgcttc catttatgat gagattcagc aggaaatgaa 1740
   gcgtgctaaa gtgtctcaag cactgtttgc aaaggttgca gcaaccaaaa gccagggatg 1800
   gttgtgcgag ctgttacgct ggaaagaaga tccttctcca gaaaacagaa ccctgtggga 1860
   gaacctctcc atgatccgaa ggttcctcag tcttcctcag ccagaacgtg atgccattta 1920
   tgaacaggag agcaacgcgg tgcatcacca tggcgacagg ccgccccaca ttatccatgt 1980
   tccagcagag cagattcagc aacagcagca gcaacagcaa cagcagcagc agcagcagca 2040
   ggcaccgccg cctccacagc cacagcagca gccacagaca ggccctcggc tccccccacg 2100
   gcaacccacg gtggcctctc cagcagagtc agatgaggaa aaccgacaga agacccggcc 2160
   acgaacaaaa atttcagtgg aagccttggg aatcctccag agtttcatac aagacgtggg 2220
   cctgtaccct gacgaagagg ccatccagac tctgtctgcc cagctcgacc ttcccaagta 2280
   caccatcatc aagttctttc agaaccagcg gtactatctc aagcaccacg gcaaactgaa 2340
   ggacaattcc ggtttagagg tcgatgtggc agaatataaa gaagaggagc tgctgaagga 2400
   tttggaagag agtgtccaag ataaaaatac taacaccctt ttttcagtga aactagaaga 2460
   agagctgtca gtggaaggaa acacagacat taatactgat ttgaaagact gagataaaag 2520
   tatttgtttc gttcaacagt gccactggta tttactaaca aaatgaaaag tccaccttgt 2580
   cttctctcag aaaacctttg ttgttcattg tttggccaat gaatcttcaa aaacttgcac 2640
   aaacagaaaa gttggaaaag gataatacag actgcactaa atgttttcct ctgttttaca 2700
   aactgcttgg cagccccagg tgaagcatca aggattgttt ggtattaaaa tttgtgttca 2760
   cgggatgcac caaagtgtgt accccgtaag catgaaacca gtgttttttg tttttttttt 2820
   agttcttatt ccggagcctc aaacaagcat tataccttct gtgattatga tttcctctcc 2880
   tataattatt tctgtagcac tccacactga tctttggaaa cttgcccctt atttaaaaaa 2940
   aaaaaagaaa aaaaagagtt tgttactcta ttgtatgtta caaaagaact atagactgtg 3000
   gaatgcagtt taaagatgac atatgccaac aaatgccttg tattatatgg cactgccgta 3060
   attcaaattt gtttttattt tggaaataaa agttcactgt actttttttt cattctcatt 3120
   gttacatgat tttttaaaaa aaggaaaaga aaatgtgaaa cacaatttag tcctcattat 3180
   ttatttgtag atcctgcagc atcatgttgt aattaatttt ttggaagttt ccgttaaatg 3240
   taatattgct tctcttgtta ccatactgat tcttttctat ttataaatgt attttgatgg 3300
   gcagtaaaac aaagtgtctt aaaagtttta aatagagaaa atgtgcttta cacagttgcc 3360
   tataaaaagt gctctatgtt atccaagcaa ttcatactat aagcttcact cttattgttg 3420
   tatgcaattt ttactatcat gcaaataagc ttaggtaaat aaaactaata gatcacctta 3480
   gaaaattatg caattaatgt gaaaataatt gatgtttgca atgtgtcttc ctttggttta 3540
   caatcaattt taaagctaca tctgtataaa atttctgtat aaaggtgtat ttctttttta 3600
   tgagtttatg gctatgaaaa cagctatttt gttacagctg gctgttttta taagtgtatc 3660
   acaattttct ttatgcagaa atgttctgac taggagtggt tattgactgt aactacacaa 3720
   ttaaaattgt ttgtatcgta tgacatggta gggtttgtct gcttatgtga agtaactaaa 3780
   ggagtcaaag gatggccctc tcatttaggt gcatgttaat aacttgttat ttcactgatt 3840
   ttaaaaagag caattgacaa gttacttgaa acactgtaaa tttaaatcac aaacacatgc 3900
   tcattttt 3908

<210> 47
<211> 5423
<212> DNA
<213> Homo sapiens

<400> 47
tttctcgctc gctcccgttc cccggacgcg gcggatgagc cggccccgct ggggaaggct 60
ccgggcggcg gcgggcggcc gggaggaggc tgcgtgctcg gggctggggc tgcgagcggg 120
gtgattttgt attaaaatga ggaggaggaa gaagaggcac ccacagcggc agcggcggcg 180
gcggcggcag cagcagcagg agcagcggcg gagagggctg cagcccgggc ggacgcgcgg 240
agccgagcgg ggcacggcgg cggcagcgac agcggccggg atgagtcaac taataattta 300
atgggggcag agacggcagc gaggggtaga gctagcgagg gagagagcga gagaagcagc 360

cccgtccggg gactcgcgct cacactcacg cacacacaca aacacacaca cacctctccc 420
tgtgccaccc agcaacaccc ggcctcgtca caacaacaac agccgcggcc gccctctatc 480
ctgcccgggg gcccagccga aagccagggc gactctagag gacgctgccc gcccccctct 540
ttcatttcgg gaaactcctg atcagttttg tcggggtttc tgggtttctt ttcccccaaa 600
gtcctagtgc cattgtggtg ctcgttgttt acctcggact ctggacgagt gagagcttgg 660
cgactttttg gggggagggg gcggggagtt tgtcgctgcc taggcggtgg aggtggctgg 720
gggtgccttc tgatcttcct cctcctcccc ctcccccga acctcttctc tcctcacttg 780
ctgggacccc agacgctcac agccccgcgt caatgggcag ggagagggtc cttgcggctg 840
ttgtcagcga gggcagaatc aaaagtggca ttttagtgcc tttccggggc ttttctcgcg 900
accccctgcc ccccaccctc gctgtccccc gctagatgcc ctcgttgggg gtgcgaggct 960
gtggggaaaa gtttaaggtt tgttaatatt agtcgcgatt gttggcgagg ggggtggggg 1020
tgattggaag ggaggcgagg tggccttccc aatgcgcgtt attcggggtt attgaagaat 1080
aatattgcaa gtgacagcca gaagtagact ttctgtcctc acaccgaaga acccgagtga 1140
gcaggaggga gggagagacg cgaagagacc tttttttcctt tttggagacc ttgtccgcag 1200
tgattttttt tttttaaga gaatcctcag tcaccacgtc gtttccccag caccatcaca 1260
gtgtacagct cataacgggt tttgctttgt ttttacgatt tccccccaac gaatcacttg 1320
tcagatcaat tttatcttct tcctcctccc tgcttcccac tctcccctcc tccccatcgc 1380
aaaccctgtt ctctgaggtt agacatttta caaaccccta tatgttggtt ttcgaattgt 1440
gatttttttt ttaaacccct ttctcatggc tactcttcta gacgtttatt tctgcccttc 1500
ccccgcttag gggggcgggg gtaggggaaa ggaaaataat acaatttcag gggaagtcgc 1560
cttcaggtct gctgcttttt tatttttttt tttttaatta aaaaaaaaaa ggacatagaa 1620
aacatcagtc ttgaacttct cttcaagaac ccgggctgca aaggaaatct cctttgtttt 1680
tgttatttat gtgctgtcaa gttttgaagt ggtgatcttt agacagtgac tgagtatgga 1740
tcatttgaac gaggcaactc aggggaaaga acattcagaa atgtctaaca atgtgagtga 1800
tccgaagggt ccaccagcca agattgcccg cctggagcag aacgggagcc cgctaggaag 1860
aggaaggctt gggagtacag gtgcaaaaat gcagggagtg cctttaaaac actcgggcca 1920
tctgatgaaa accaacctta ggaaaggaac catgctgcca gttttctgtg tggtggaaca 1980
ttatgaaaac gccattgaat atgattgcaa ggaggagcat gcagaatttg tgctggtgag 2040
aaaggatatg cttttcaacc agctgatcga aatggcattg ctgtctctag gttattcaca 2100
tagctctgct gcccaggcca aagggctaat ccaggttgga aagtggaatc cagttccact 2160
gtcttacgtg acagatgccc ctgatgctac agtagcagat atgcttcaag atgtgtatca 2220
tgtggtcaca ttgaaaattc agttacacag ttgccccaaa ctagaagact tgcctcccga 2280
acaatggtcg cacaccacag tgaggaatgc tctgaaggac ttactgaaag atatgaatca 2340
gagttcattg gccaaggagt gccccctttc acagagtatg atttcttcca ttgtgaacag 2400

tacttactat gcaaatgtct cagcagcaaa atgtcaagaa tttggaaggt ggtacaaaca 2460
tttcaagaag acaaaagata tgatggttga aatggatagt ctttctgagc tatcccagca 2520
aggcgccaat catgtcaatt ttggccagca accagttcca gggaacacag ccgagcagcc 2580
tccatcccct gcgcagctct cccatggcag ccagccctct gtccggacac ctcttccaaa 2640
cctgcaccct gggctcgtat caacacctat cagtcctcaa ttggtcaacc agcagctggt 2700
gatggctcag ctgctgaacc agcagtatgc agtgaataga cttttagccc agcagtcctt 2760
aaaccaacaa tacttgaacc accctccccc tgtcagtaga tctatgaata agcctttgga 2820
gcaacaggtt tcgaccaaca cagaggtgtc ttccgaaatc taccagtggg tacgcgatga 2880
actgaaacga gcaggaatct cccaggcggt atttgcacgt gtggctttta acagaactca 2940
gggcttgctt tcagaaatcc tccgaaagga agaggacccc aagactgcat cccagtcttt 3000
gctggtaaac cttcgggcta tgcagaattt cttgcagtta ccggaagctg aaagagaccg 3060
aatataccag gacgaaaggg aaaggagctt gaatgctgcc tcggccatgg gtcctgcccc 3120
cctcatcagc acaccaccca gccgtcctcc ccaggtgaaa acagctacta ttgccactga 3180
aaggaatggg aaaccagaga acaataccat gaacattaat gcttccattt atgatgagat 3240
tcagcaggaa atgaagcgtg ctaaagtgtc tcaagcactg tttgcaaagg ttgcagcaac 3300
caaaagccag ggatggttgt gcgagctgtt acgctggaaa gaagatcctt ctccagaaaa 3360
cagaaccctg tgggagaacc tctccatgat ccgaaggttc ctcagtcttc ctcagccaga 3420
acgtgatgcc atttatgaac aggagagcaa cgcggtgcat caccatggcg acaggccgcc 3480
ccacattatc catgttccag cagagcagat tcagcaacag cagcagcaac agcaacagca 3540
gcagcagcag cagcaggcac cgccgcctcc acagccacag cagcagccac agacaggccc 3600
tcggctcccc ccacggcaac ccacggtggc ctctccagca gagtcagatg aggaaaaccg 3660
acagaagacc cggccacgaa caaaaatttc agtggaagcc ttgggaatcc tccagagttt 3720
catacaagac gtgggcctgt accctgacga agaggccatc cagactctgt ctgcccagct 3780
cgaccttccc aagtacacca tcatcaagtt ctttcagaac cagcggtact atctcaagca 3840
ccacggcaaa ctgaaggaca attccggttt agaggtcgat gtggcagaat ataaagaaga 3900
ggagctgctg aaggatttgg aagagagtgt ccaagataaa aatactaaca cccttttttc 3960
agtgaaacta gaagaagagc tgtcagtgga aggaaacaca gacattaata ctgatttgaa 4020
agactgagat aaaagtattt gtttcgttca acagtgccac tggtatttac taacaaaatg 4080
aaaagtccac cttgtcttct ctcagaaaac ctttgttgtt cattgtttgg ccaatgaatc 4140
ttcaaaaact tgcacaaaca gaaaagttgg aaaaggataa tacagactgc actaaatgtt 4200
ttcctctgtt ttacaaactg cttggcagcc ccaggtgaag catcaaggat tgtttggtat 4260
taaaatttgt gttcacggga tgcaccaaag tgtgtacccc gtaagcatga aaccagtgtt 4320
ttttgttttt tttttagttc ttattccgga gcctcaaaca agcattatac cttctgtgat 4380
tatgatttcc tctcctataa ttatttctgt agcactccac actgatcttt ggaaacttgc 4440

cccttattta aaaaaaaaaa agaaaaaaaa gagtttgtta ctctattgta tgttacaaaa 4500
gaactataga ctgtggaatg cagtttaaag atgacatatg ccaacaaatg ccttgtatta 4560
tatggcactg ccgtaattca aatttgtttt tattttggaa ataaaagttc actgtacttt 4620
tttttcattc tcattgttac atgatttttt aaaaaaagga aaagaaaatg tgaaacacaa 4680
tttagtcctc attatttatt tgtagatcct gcagcatcat gttgtaatta attttttgga 4740
agtttccgtt aaatgtaata ttgcttctct tgttaccata ctgattcttt tctatttata 4800
aatgtatttt gatgggcagt aaaacaaagt gtcttaaaag ttttaaatag agaaaatgtg 4860
ctttacacag ttgcctataa aaagtgctct atgttatcca agcaattcat actataagct 4920
tcactcttat tgttgtatgc aatttttact atcatgcaaa taagcttagg taaataaaac 4980
taatagatca ccttagaaaa ttatgcaatt aatgtgaaaa taattgatgt ttgcaatgtg 5040
tcttcctttg gtttacaatc aattttaaag ctacatctgt ataaaatttc tgtataaagg 5100
tgtatttctt ttttatgagt ttatggctat gaaaacagct attttgttac agctggctgt 5160
ttttataagt gtatcacaat tttctttatg cagaaatgtt ctgactagga gtggttattg 5220
actgtaacta cacaattaaa attgtttgta tcgtatgaca tggtagggtt tgtctgctta 5280
tgtgaagtaa ctaaaggagt caaaggatgg ccctctcatt taggtgcatg ttaataactt 5340
gttatttcac tgattttaaa aagagcaatt gacaagttac ttgaaacact gtaaatttaa 5400
atcacaaaca catgctcatt ttt 5423

<210> 48
<211> 3273
<212> DNA
<213> Homo sapiens

<400> 48
   agtccccgcg cagtccccgc gcagtcccag cgccaccggg cagcagcggc gccgtgctcg 60
   ctccagggcg caaccatgtc gccatttctt cggattggct tgtccaactt tgactgcggg 120
   tcctgccagt cttgtcaggg cgaggctgtt aacccttact gtgctgtgct cgtcaaagag 180
   tatgtcgaat cagagaacgg gcagatgtat atccagaaaa agcctaccat gtacccaccc 240
   tgggacagca cttttgatgc ccatatcaac aagggaagag tcatgcagat cattgtgaaa 300
   ggcaaaaacg tggacctcat ctctgaaacc accgtggagc tctactcgct ggctgagagg 360
   tgcaggaaga acaacgggaa gacagaaata tggttagagc tgaaacctca aggccgaatg 420
   ctaatgaatg caagatactt tctggaaatg agtgacacaa aggacatgaa tgaatttgag 480
   acggaaggct tctttgcttt gcatcagcgc cggggtgcca tcaagcaggc aaaggtccac 540
   cacgtcaagt gccacgagtt cactgccacc ttcttcccac agcccacatt ttgctctgtc 600
   tgccacgagt ttgtctgggg cctgaacaaa cagggctacc agtgccgaca atgcaatgca 660
   gcaattcaca agaagtgtat tgataaagtt atagcaaagt gcacaggatc agctatcaat 720
   agccgagaaa ccatgttcca caaggagaga ttcaaaattg acatgccaca cagatttaaa 780
   gtctacaatt acaagagccc gaccttctgt gaacactgtg ggaccctgct gtggggactg 840
   gcacggcaag gactcaagtg tgatgcatgt ggcatgaatg tgcatcatag atgccagaca 900
   aaggtggcca acctttgtgg cataaaccag aagctaatgg ctgaagcgct ggccatgatt 960
   gagagcactc aacaggctcg ctgcttaaga gatactgaac agatcttcag agaaggtccg 1020
   gttgaaattg gtctcccatg ctccatcaaa aatgaagcaa ggccgccatg tttaccgaca 1080
   ccgggaaaaa gagagcctca gggcatttcc tgggagtctc cgttggatga ggtggataaa 1140
   atgtgccatc ttccagaacc tgaactgaac aaagaaagac catctctgca gattaaacta 1200
   aaaattgagg attttatctt gcacaaaatg ttggggaaag gaagttttgg caaggtcttc 1260
   ctggcagaat tcaagaaaac caatcaattt ttcgcaataa aggccttaaa gaaagatgtg 1320
   gtcttgatgg acgatgatgt tgagtgcacg atggtagaga agagagttct ttccttggcc 1380
   tgggagcatc cgtttctgac gcacatgttt tgtacattcc agaccaagga aaacctcttt 1440
   tttgtgatgg agtacctcaa cggaggggac ttaatgtacc acatccaaag ctgccacaag 1500
   ttcgaccttt ccagagcgac gttttatgct gctgaaatca ttcttggtct gcagttcctt 1560
   cattccaaag gaatagtcta cagggacctg aagctagata acatcctgtt agacaaagat 1620
   ggacatatca agatcgcgga ttttggaatg tgcaaggaga acatgttagg agatgccaag 1680
   acgaatacct tctgtgggac acctgactac atcgccccag agatcttgct gggtcagaaa 1740
   tacaaccact ctgtggactg gtggtccttc ggggttctcc tttatgaaat gctgattggt 1800
   cagtcgcctt tccacgggca ggatgaggag gagctcttcc actccatccg catggacaat 1860
   ccctttaccc cacggtggct ggagaaggaa gcaaaggacc ttctggtgaa gctcttcgtg 1920
   cgagaacctg agaagaggct gggcgtgagg ggagacatcc gccagcaccc tttgtttcgg 1980
   gagatcaact gggaggaact tgaacggaag gagattgacc caccgttccg gccgaaagtg 2040
   aaatcaccat ttgactgcag caatttcgac aaagaattct taaacgagaa gccccggctg 2100
   tcatttgccg acagagcact gatcaacagc atggaccaga atatgttcag gaacttttcc 2160
   ttcatgaacc ccgggatgga gcggctgata tcctgaatct tgcccctcca gagacaggaa 2220
   agaatttgcc ttctccctgg gaactggttc aagagacact gcttgggttc ctttttcaac 2280
   ttggaaaaag aaagaaacac tcaacaataa agactgagac ccgttcgccc ccatgtgact 2340
   tttatctgta gcagaaacca agtctacttc actaatgacg atgccgtgtg tctcgtctcc 2400
   tgacatgtct cacagacgct cctgaagtta ggtcattact aaccatagtt atttacttga 2460
   aagatgggtc tccgcacttg gaaaggtttc aagacttgat actgcaataa attatggctc 2520
   ttcacctggg cgccaactgc tgatcaatga aatgcttgtt gaatcagggg caaacggagt 2580
   acagacgtct caagactgaa acggccccat tgcctggtct agtagcggat ctcactcagc 2640
   cgcagacaag taatcactaa cccgttttat tctattccta tctgtggatg tgtaaatggc 2700
   tgggggccca gccctggata ggtttttatg ggaattcttt acaataaaca tagcttgtaa 2760
   cttgagatct acaaatccat tcatcctgat tgggcatgaa atccatggtc aagaggacaa 2820
   gtggaaagtg agagggaagg tttgctagac accttcgctt gttatcttgt caagatagaa 2880
   aagatagtat catttcaccc ttgccagtaa aaacctttcc atccacccat tctcagcaga 2940
   ctccagtatt ggcacagtca ctcactgcca ttctcacact ataacaagaa aagaaatgaa 3000
   gtgcataagt ctcctgggaa aagaacctta accccttctc gtgccatgac tggtgatttc 3060
   atgactcata agcccctccg taggcatcat tcaagatcaa tggcccatgc atgctgtttg 3120
   cagcagtcaa ttgagttgaa ttagaattcc aaccatacat tttaaaggta tttgtgctgt 3180
   gtgtatattt tgataaaatg ttgtgacttc atggcaaaca ggtggatgtg taaaaatgga 3240
   ataaaaaaaa aaaaagagtc aaaaaaaaaa aaa 3273

<210> 49
<211> 1695
<212> DNA
<213> Homo sapiens

<400> 49
gaggcacaga taaagataag ttttactgtc atgctgcttt taacataaca gagcaacatc 60

acctaggaaa aaagtttgta ggaggatttt taatccatat atttgtctta tggctagata 120

aagatttctc tgaaaaaaag aagcatgtca ggaatctctg ggtgcccctt tttcctctgg 180

ggacttctag cattgttggg cttggctttg gttatatcac tgatcttcaa tatttcccac 240

tatgtggaaa agcaacgaca agataaaatg tacagctact ccagtgacca caccagggtt 300

gatgagtatt atattgaaga cacaccaatt tatggtaact tagatgatat gatttcagaa 360

ccaatggatg aaaattgcta tgaacaaatg aaagcccgac cagagaaatc tgtaaataag 420

atgcaggaag ccaccccatc tgcacaggca accaatgaaa cacagatgtg ctacgcctca 480

cttgatcaca gcgttaaggg gaagcgtaga aagcccagga aacagaatac tcatttctca 540

gacaaggatg gagatgagca actacatgca atagatgcca gcgtttctaa gaccacctta 600

gtagacagtt tctccccaga aagccaggca gtagaggaaa acattcatga tgatcccatc 660

agactgtttg gattgatccg tgctaagaga gaacctataa actagctgga ccatgatcta 720

gttcaatgat ttggctccta ttgaagatgg cttctaagaa aacaagatgc acagaggaca 780

cagaaggact tggcagcagg gtgatgacct gatcatttgt tgatgggatg gtggcttacc 840

tcttattcac agcttacact tatgcatgcc aaatgtaagg ccatgaaaat cagtatttca 900

aataacttaa aaaatgcttt actactaaaa tgtaaaaaat taatgtgctc acctcggcag 960

cacatatact aaaaattaat aagacccagc ttgaaaattg agcctgataa caagattaca 1020

aattcacaat acctaatact tagggaaata taaaaattta agcatgaatg cgttctggaa 1080

cacgttagaa gaaaaataaa agccaatgag ttttttttta attctccttt ctcaccaatg 1140

ggcaatagcc cataattgaa ataaatttct gattgaaagg tataggaaac attaaaatgc 1200

attactaaga gaagtaatat aattttctta caaagtattt ttcccaaaga tagctttact 1260

atttcaaaaa ttgtcaattt aatgcatgct ccttacaaca aacaaatatc aaaaagagtt 1320

taggaattct actagccaga gatagtcact tggagaaact ttctatatat ccttctaaat 1380

atttttctgg gcatgcttat gtatgtacat cagttgtttc tttttatttt gaaccaaaaa 1440

tgtggtttct tttgtacaca ttacttaaac tttctttcca gtcaacaata tattgtggat 1500

ttattttcac tgttatattt aactatatat aaatacgcat atattgtaat tttaatgtct 1560

gcttagcacc ccactgataa ccaaatcaca gtttatttaa ataattttaa tgacttttca 1620

aaaacaattt attgatgcaa aaagcaaggt tgagatgaca atgtttcttt caataattaa 1680

aaaatactgc ttcac 1695

<210> 50
<211> 1284
<212> DNA
<213> Homo sapiens

<400> 50
ctgtgattct cactatactg gtcctgagga aagggcttct gtgaactgcg gtttttagtt 60

tttattgtgg ttcttagttc tcatgagacc cctcttgagg atatgtgcct atctggtgcc 120

tctgctctcc actagttgag tgaaaggaag gaggtaattt accaccatgt ttggttcctg 180

tttataagat gttttaagaa agatctgaaa cagattttct gaagaaagca gaagctctct 240

tcccattatg acttcggaaa tcacttatgc tgaagtgagg ttcaaaaatg aattcaagtc 300

ctcaggcatc aacacagcct cttctgcagc ttccaaggag aggactgccc ctcacaaaag 360

taataccgga ttccccaagc tgctttgtgc ctcactgttg atatttttcc tgctattggc 420

aatctcattc tttattgctt ttgtcatttt ctttcaaaaa tattctcagc ttcttgaaaa 480

aaagactaca aaagagctgg ttcatacaac attggagtgt gtgaaaaaaa atatgcccgt 540

ggaagagaca gcctggagct gttgcccaaa gaattggaag tcatttagtt ccaactgcta 600

ctttatttct actgaatcag catcttggca agacagtgag aaggactgtg ctagaatgga 660

ggctcacctg ctggtgataa acactcaaga agagcaggat ttcatcttcc agaatctgca 720

agaagaatct gcttattttg tggggctctc agatccagaa ggtcagcgac attggcaatg 780

ggttgatcag acaccataca atgaaagttc cacattctgg catccacgtg agcccagtga 840

tcccaatgag cgctgcgttg tgctaaattt tcgtaaatca cccaaaagat ggggctggaa 900

tgatgttaat tgtcttggtc ctcaaaggtc agtttgtgag atgatgaaga tccacttatg 960

aactgaacat tctccatgaa caggtggttg gattggtatc tgtcattgta gggatagata 1020

ataagctctt cttattcatg tgtaagggag gtccatagaa tttaggtggt ctgtcaacta 1080

ttctacttat gagagaattg gtctgtacat tgactgattc actttttcat aaagtgagca 1140

tttattgagc attttttcat gtgccagagc ctgtactgga ggcccccatt gtgcacacat 1200

ggagagaaca tgagtctctc ttaattttta tctggttgct aaagaattat ttaccaataa 1260

aattatatga tgtggtgtct caaa 1284

<210> 51
<211> 1185
<212> DNA
<213> Homo sapiens

<400>  51
ctgtgattct cactatactg gtcctgagga aagggcttct gtgaactgcg gtttttagtt      60

tttattgtgg ttcttagttc tcatgagacc cctcttgagg atatgtgcct atctggtgcc     120

tctgctctcc actagttgag tgaaaggaag gaggtaattt accaccatgt ttggttcctg     180

tttataagat gttttaagaa agatctgaaa cagattttct gaagaaagca gaagctctct     240

tcccattatg acttcggaaa tcacttatgc tgaagtgagg ttcaaaaatg aattcaagtc     300

ctcaggcatc aacacagcct cttctgcagc ttccaaggag aggactgccc ctcacaaaag     360

taataccgga ttccccaagc tgctttgtgc ctcactgttg atatttttcc tgctattggc     420

aatctcattc tttattgctt ttgtcaagac agcctggagc tgttgcccaa agaattggaa     480

gtcatttagt tccaactgct actttatttc tactgaatca gcatcttggc aagacagtga     540

gaaggactgt gctagaatgg aggctcacct gctggtgata aacactcaag aagagcagga     600

tttcatcttc cagaatctgc aagaagaatc tgcttatttt gtggggctct cagatccaga     660

aggtcagcga cattggcaat gggttgatca gacaccatac aatgaaagtt ccacattctg     720

gcatccacgt gagcccagtg atcccaatga gcgctgcgtt gtgctaaatt ttcgtaaatc     780

acccaaaaga tggggctgga atgatgttaa ttgtcttggt cctcaaaggt cagtttgtga     840

gatgatgaag atccacttat gaactgaaca ttctccatga acaggtggtt ggattggtat     900

ctgtcattgt agggatagat aataagctct tcttattcat gtgtaaggga ggtccataga     960

atttaggtgg tctgtcaact attctactta tgagagaatt ggtctgtaca ttgactgatt    1020

cactttttca taaagtgagc atttattgag cattttttca tgtgccagag cctgtactgg    1080

aggcccccat tgtgcacaca tggagagaac atgagtctct cttaattttt atctggttgc    1140

taaagaatta tttaccaata aaattatatg atgtggtgtc tcaaa                    1185

<210>  52
<211>  1167
<212>  DNA
<213>  Homo sapiens

<400>  52
ctgtgattct cactatactg gtcctgagga aagggcttct gtgaactgcg gtttttagtt      60

tttattgtgg ttcttagttc tcatgagacc cctcttgagg atatgtgcct atctggtgcc     120

tctgctctcc actagttgag tgaaaggaag gaggtaattt accaccatgt ttggttcctg     180

tttataagat gttttaagaa agatctgaaa cagattttct gaagaaagca gaagctctct     240

tcccattatg acttcggaaa tcacttatgc tgaagtgagg ttcaaaaatg aattcaagtc     300

ctcaggcatc aacacagcct cttctgcagt tttctttcaa aaatattctc agcttcttga     360

aaaaaagact acaaaagagc tggttcatac aacattggag tgtgtgaaaa aaaatatgcc     420

cgtggaagag acagcctgga gctgttgccc aaagaattgg aagtcattta gttccaactg     480

ctactttatt tctactgaat cagcatcttg gcaagacagt gagaaggact gtgctagaat     540

ggaggctcac ctgctggtga taaacactca agaagagcag gatttcatct tccagaatct     600

gcaagaagaa tctgcttatt ttgtggggct ctcagatcca gaaggtcagc gacattggca 660

atgggttgat cagacaccat acaatgaaag ttccacattc tggcatccac gtgagcccag 720

tgatcccaat gagcgctgcg ttgtgctaaa ttttcgtaaa tcacccaaaa gatggggctg 780

gaatgatgtt aattgtcttg gtcctcaaag gtcagtttgt gagatgatga agatccactt 840

atgaactgaa cattctccat gaacaggtgg ttggattggt atctgtcatt gtagggatag 900

ataataagct cttcttattc atgtgtaagg gaggtccata gaatttaggt ggtctgtcaa 960

ctattctact tatgagagaa ttggtctgta cattgactga ttcacttttt cataaagtga 1020

gcatttattg agcatttttt catgtgccag agcctgtact ggaggccccc attgtgcaca 1080

catggagaga acatgagtct ctcttaattt ttatctggtt gctaaagaat tatttaccaa 1140

taaaattata tgatgtggtg tctcaaa 1167

<210> 53
<211> 1068
<212> DNA
<213> Homo sapiens

<400> 53
ctgtgattct cactatactg gtcctgagga aagggcttct gtgaactgcg gtttttagtt 60

tttattgtgg ttcttagttc tcatgagacc cctcttgagg atatgtgcct atctggtgcc 120

tctgctctcc actagttgag tgaaaggaag gaggtaattt accaccatgt ttggttcctg 180

tttataagat gttttaagaa agatctgaaa cagattttct gaagaaagca gaagctctct 240

tcccattatg acttcggaaa tcacttatgc tgaagtgagg ttcaaaaatg aattcaagtc 300

ctcaggcatc aacacagcct cttctgcaga gacagcctgg agctgttgcc caaagaattg 360

gaagtcattt agttccaact gctactttat ttctactgaa tcagcatctt ggcaagacag 420

tgagaaggac tgtgctagaa tggaggctca cctgctggtg ataaacactc aagaagagca 480

ggatttcatc ttccagaatc tgcaagaaga atctgcttat tttgtggggc tctcagatcc 540

agaaggtcag cgacattggc aatgggttga tcagacacca tacaatgaaa gttccacatt 600

ctggcatcca cgtgagccca gtgatcccaa tgagcgctgc gttgtgctaa attttcgtaa 660

atcacccaaa agatggggct ggaatgatgt taattgtctt ggtcctcaaa ggtcagtttg 720

tgagatgatg aagatccact tatgaactga acattctcca tgaacaggtg gttggattgg 780

tatctgtcat tgtagggata gataataagc tcttcttatt catgtgtaag ggaggtccat 840

agaatttagg tggtctgtca actattctac ttatgagaga attggtctgt acattgactg 900

attcactttt tcataaagtg agcatttatt gagcattttt tcatgtgcca gagcctgtac 960

tggaggcccc cattgtgcac acatggagag aacatgagtc tctcttaatt tttatctggt 1020

tgctaaagaa ttatttacca ataaaattat atgatgtggt gtctcaaa 1068

<210> 54
<211> 1310
<212> DNA

<213>  Homo sapiens

<400>  54
aaattgagcc cgcagcctcc cgcttcgctc tctgctcctc ctgttcgaca gtcagccgca      60

tcttcttttg cgtcgccagc cgagccacat cgctcagaca ccatggggaa ggtgaaggtc     120

ggagtcaacg gatttggtcg tattgggcgc ctggtcacca gggctgcttt taactctggt     180

aaagtggata ttgttgccat caatgacccc ttcattgacc tcaactacat ggtttacatg     240

ttccaatatg attccaccca tggcaaattc catggcaccg tcaaggctga gaacgggaag     300

cttgtcatca atggaaatcc catcaccatc ttccaggagc gagatccctc caaaatcaag     360

tggggcgatg ctggcgctga gtacgtcgtg gagtccactg gcgtcttcac caccatggag     420

aaggctgggg ctcatttgca ggggggagcc aaaagggtca tcatctctgc cccctctgct     480

gatgccccca tgttcgtcat gggtgtgaac catgagaagt atgacaacag cctcaagatc     540

atcagcaatg cctcctgcac caccaactgc ttagcacccc tggccaaggt catccatgac     600

aactttggta tcgtggaagg actcatgacc acagtccatg ccatcactgc cacccagaag     660

actgtggatg gcccctccgg gaaactgtgg cgtgatggcc gcggggctct ccagaacatc     720

atccctgcct ctactggcgc tgccaaggct gtgggcaagg tcatccctga gctgaacggg     780

aagctcactg gcatggcctt ccgtgtcccc actgccaacg tgtcagtggt ggacctgacc     840

tgccgtctag aaaaacctgc caaatatgat gacatcaaga aggtggtgaa gcaggcgtcg     900

gagggccccc tcaagggcat cctgggctac actgagcacc aggtggtctc ctctgacttc     960

aacagcgaca cccactcctc cacctttgac gctggggctg gcattgccct caacgaccac    1020

tttgtcaagc tcatttcctg gtatgacaac gaatttggct acagcaacag ggtggtggac    1080

ctcatggccc acatggcctc caaggagtaa gacccctgga ccaccagccc cagcaagagc    1140

acaagaggaa gagagagacc ctcactgctg gggagtccct gccacactca gtcccccacc    1200

acactgaatc tcccctcctc acagttgcca tgtagacccc ttgaagaggg gaggggccta    1260

gggagccgca ccttgtcatg taccatcaat aaagtaccct gtgctcaacc               1310

<210>  55
<211>  1229
<212>  DNA
<213>  Homo sapiens

<400>  55
gtctgacggg cgatggcgca gccaatagac aggagcgcta tccgcggttt ctgattggct      60

actttgttcg cattataaaa ggcacgcgcg ggcgcgaggc ccttctctcg ccaggcgtcc     120

tcgtggaagt gacatcgtct ttaaaccctg cgtggcaatc cctgacgcac cgccgtgatg     180

cccagggaag acagggcgac ctggaagtcc aactacttcc ttaagatcat ccaactattg     240

gatgattatc cgaaatgttt cattgtggga gcagacaatg tgggctccaa gcagatgcag     300

cagatccgca tgtcccttcg cgggaaggct gtggtgctga tgggcaagaa caccatgatg     360

cgcaaggcca tccgagggca cctggaaaac aacccagctc tggagaaact gctgcctcat     420

atccggggga atgtgggctt tgtgttcacc aaggaggacc tcactgagat cagggacatg 480
ttgctggcca ataaggtgcc agctgctgcc cgtgctggtg ccattgcccc atgtgaagtc 540
actgtgccag cccagaacac tggtctcggg cccgagaaga cctccttttt ccaggcttta 600
ggtatcacca ctaaaatctc caggggcacc attgaaatcc tgagtgatgt gcagctgatc 660
aagactggag acaaagtggg agccagcgaa gccacgctgc tgaacatgct caacatctcc 720
cccttctcct ttgggctggt catccagcag gtgttcgaca atggcagcat ctacaaccct 780
gaagtgcttg atatcacaga ggaaactctg cattctcgct tcctggaggg tgtccgcaat 840
gttgccagtg tctgtctgca gattggctac ccaactgttg catcagtacc ccattctatc 900
atcaacgggt acaaacgagt cctggccttg tctgtggaga cggattacac cttcccactt 960
gctgaaaagg tcaaggcctt cttggctgat ccatctgcct ttgtggctgc tgcccctgtg 1020
gctgctgcca ccacagctgc tcctgctgct gctgcagccc cagctaaggt tgaagccaag 1080
gaagagtcgg aggagtcgga cgaggatatg ggatttggtc tctttgacta atcaccaaaa 1140
agcaaccaac ttagccagtt ttatttgcaa aacaaggaaa taaaggctta cttctttaaa 1200
aagtaaaaaa aaaaaaaaaa aaaaaaaaa 1229

<210> 56
<211> 1289
<212> DNA
<213> Homo sapiens

<400> 56
gtctgacggg cgatggcgca gccaatagac aggagcgcta tccgcggttt ctgattggct 60
actttgttcg cattataaaa ggcacgcgcg ggcgcgaggc ccttctctcg ccaggcgtcc 120
tcgtggaagg cccgggaccg cgggatgggt gtcggcgtga ccaggcctga gctccctgtc 180
tctcctcagt gacatcgtct ttaaaccctg cgtggcaatc cctgacgcac cgccgtgatg 240
cccagggaag acagggcgac ctggaagtcc aactacttcc ttaagatcat ccaactattg 300
gatgattatc cgaaatgttt cattgtggga gcagacaatg tgggctccaa gcagatgcag 360
cagatccgca tgtcccttcg cgggaaggct gtggtgctga tgggcaagaa caccatgatg 420
cgcaaggcca tccgagggca cctggaaaac aacccagctc tggagaaact gctgcctcat 480
atccgggga atgtgggctt tgtgttcacc aaggaggacc tcactgagat cagggacatg 540
ttgctggcca ataaggtgcc agctgctgcc cgtgctggtg ccattgcccc atgtgaagtc 600
actgtgccag cccagaacac tggtctcggg cccgagaaga cctccttttt ccaggcttta 660
ggtatcacca ctaaaatctc caggggcacc attgaaatcc tgagtgatgt gcagctgatc 720
aagactggag acaaagtggg agccagcgaa gccacgctgc tgaacatgct caacatctcc 780
cccttctcct ttgggctggt catccagcag gtgttcgaca atggcagcat ctacaaccct 840
gaagtgcttg atatcacaga ggaaactctg cattctcgct tcctggaggg tgtccgcaat 900
gttgccagtg tctgtctgca gattggctac ccaactgttg catcagtacc ccattctatc 960
atcaacgggt acaaacgagt cctggccttg tctgtggaga cggattacac cttcccactt 1020

gctgaaaagg tcaaggcctt cttggctgat ccatctgcct ttgtggctgc tgcccctgtg 1080

gctgctgcca ccacagctgc tcctgctgct gctgcagccc cagctaaggt tgaagccaag 1140

gaagagtcgg aggagtcgga cgaggatatg ggatttggtc tctttgacta atcaccaaaa 1200

agcaaccaac ttagccagtt ttatttgcaa aacaaggaaa taaaggctta cttctttaaa 1260

aagtaaaaaa aaaaaaaaaa aaaaaaaaa 1289

<210> 57
<211> 2439
<212> DNA
<213> Homo sapiens

<400> 57
gagagcagcg gccgggaagg ggcggtgcgg gaggcggggt gtggggcggt agtgtgggcc 60

ctgttcctgc ccgcgcggtg ttccgcattc tgcaagcctc cggagcgcac gtcggcagtc 120

ggctccctcg ttgaccgaat caccgacctc tctccccagc tgtatttcca aaatgtcgct 180

ttctaacaag ctgacgctgg acaagctgga cgttaaaggg aagcgggtcg ttatgagagt 240

cgacttcaat gttcctatga agaacaacca gataacaaac aaccagagga ttaaggctgc 300

tgtcccaagc atcaaattct gcttggacaa tggagccaag tcggtagtcc ttatgagcca 360

cctaggccgg cctgatggtg tgcccatgcc tgacaagtac tccttagagc cagttgctgt 420

agaactcaaa tctctgctgg gcaaggatgt tctgttcttg aaggactgtg taggcccaga 480

agtggagaaa gcctgtgcca acccagctgc tgggtctgtc atcctgctgg agaacctccg 540

ctttcatgtg gaggaagaag ggaagggaaa agatgcttct gggaacaagg ttaaagccga 600

gccagccaaa atagaagctt tccgagcttc actttccaag ctaggggatg tctatgtcaa 660

tgatgctttt ggcactgctc acagagccca cagctccatg gtaggagtca atctgccaca 720

gaaggctggt gggtttttga tgaagaagga gctgaactac tttgcaaagg ccttggagag 780

cccagagcga cccttcctgg ccatcctggg cggagctaaa gttgcagaca agatccagct 840

catcaataat atgctggaca aagtcaatga gatgattatt ggtggtggaa tggcttttac 900

cttccttaag gtgctcaaca acatggagat tggcacttct ctgtttgatg aagagggagc 960

caagattgtc aaagacctaa tgtccaaagc tgagaagaat ggtgtgaaga ttaccttgcc 1020

tgttgacttt gtcactgctg acaagtttga tgagaatgcc aagactggcc aagccactgt 1080

ggcttctggc atacctgctg gctggatggg cttggactgt ggtcctgaaa gcagcaagaa 1140

gtatgctgag gctgtcactc gggctaagca gattgtgtgg aatggtcctg tgggggtatt 1200

tgaatgggaa gcttttgccc ggggaaccaa agctctcatg gatgaggtgg tgaaagccac 1260

ttctaggggc tgcatcacca tcataggtgg tggagacact gccacttgct gtgccaaatg 1320

gaacacggag gataaagtca gccatgtgag cactgggggt ggtgccagtt tggagctcct 1380

ggaaggtaaa gtccttcctg gggtggatgc tctcagcaat atttagtact ttcctgcctt 1440

ttagttcctg tgcacagccc ctaagtcaac ttagcatttt ctgcatctcc acttggcatt 1500

agctaaaacc ttccatgtca agattcagct agtggccaag agatgcagtg ccaggaaccc 1560
ttaaacagtt gcacagcatc tcagctcatc ttcactgcac cctggatttg catacattct 1620
tcaagatccc atttgaattt tttagtgact aaaccattgt gcattctaga gtgcatatat 1680
ttatattttg cctgttaaaa agaaagtgag cagtgttagc ttagttctct tttgatgtag 1740
gttattatga ttagctttgt cactgtttca ctactcagca tggaaacaag atgaaattcc 1800
atttgtaggt agtgagacaa aattgatgat ccattaagta aacaataaaa gtgtccattg 1860
aaaccgtgat tttttttttt ttcctgtcat actttgttag gaagggtgag aatagaatct 1920
tgaggaacgg atcagatgtc tatattgctg aatgcaagaa gtggggcagc agcagtggag 1980
agatgggaca attagataaa tgtccattct ttatcaaggg cctactttat ggcagacatt 2040
gtgctagtgc ttttattcta acttttattt ttatcagtta cacatgatca taatttaaaa 2100
agtcaaggct tataacaaaa aagccccagc ccattcctcc cattcaagat tcccactccc 2160
cagaggtgac cactttcaac tcttgagttt ttcaggtata tacctccatg tttctaagta 2220
atatgcttat attgttcact tctttttttt ttatttttta aagaaatcta tttcatacca 2280
tggaggaagg ctctgttcca catatatttc cacttcttca ttctctcggt atagttttgt 2340
cacaattata gattagatca aaagtctaca taactaatac agctgagcta tgtagtatgc 2400
tatgattaaa tttacttatg taaaaaaaaa aaaaaaaaa 2439

## Description

The present invention relates to a kit consisting of or comprising nucleic acid molecules specific for the expression product of at least two genes selected from the group consisting of (i) to (viii): (i) NRIP1; (ii) SFTPB; (iii) PLEKHA1; (iv) PDE8A; (v) TCF7; (vi) MGC29506; (vii) MSI2; and (viii) one gene selected from the group consisting of MGAT4A, NELL2, BCL11B, CD247, SORL1, KLRB1, IL7R, RUNX2, MAL, RASGRF2, TIAM1, PRKCH, FNIP2, GIMAP7, PAG1, ITK, IL2RB, SATB1, PRKCQ, TRAT1, CLEC4A, wherein each of the nucleic acid molecules has a length of at least 8 nucleotides. The present invention further relates to a kit consisting of or comprising nucleic acid molecules specific for the expression product of at least one reference gene and nucleic acid molecules specific for the expression product of at least two genes selected from the group consisting of (i) to (viii): (i) NRIP1; (ii) SFTPB; (iii) PLEKHA1; (iv) PDE8A; (v) TCF7; (vi) MGC29506; (vii) MSI2; and (viii) one gene selected from the group consisting of MGAT4A, NELL2, BCL11B, CD247, SORL1, KLRB1, IL7R, RUNX2, MAL, RASGRF2, TIAM1, PRKCH, FNIP2, GIMAP7, PAG1, ITK, IL2RB, SATB1, PRKCQ, TRAT1, CLEC4A, wherein each of the nucleic acid molecules has a length of at least 8 nucleotides. The present invention further relates to a method of diagnosing a patient suffering from chronic lymphocytic leukemia as being a poor prognosis or good prognosis patient.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Chronic lymphocytic leukemia (CLL) is one of the most common indolent lymphomas in the European Union and the United States. The clinical course is highly variable, ranging from cases with slow progression with a survival of several decades to rapid progressive and chemotherapy resistant cases with death within one year of diagnosis ¹.

Quite a number of independent prognostic factors have been established to predict the prognosis of CLL patients ² . The two most relevant prognostic factors used for risk prediction are mutational status of the immunoglobulin variable heavy chain (IgH) genes (VH-status) and certain genomic alterations that are usually detected by a panel of 5 fluorescence *in situ* hybridization (FISH) probes.
CLL cases with mutations in the IgH genes have a relatively good prognosis, whereas cases in the unmutated group exhibit a poor prognosis ³. A drawback of the determination of the IgH mutational status is that it is relatively time consuming and expensive.

Patients with deletions of 17p13 or 11q23 on interphase FISH analysis will show a very aggressive disease course. In contrast, an isolated deletion of 13q14 is associated with a more favorable outcome⁴.

Recently, risk-adapted treatment has been shown to be of benefit for patients with CLL. For example allogeneic stem cell transplantation may change overall survival in the high risk group ⁵⁻⁸. Therefore, it is of great clinical importance to identify high risk CLL-patients.

However, the prognostic power of the traditional risk factors (IgH mutational status and interphase FISH analysis) is rather limited. While most patients with mutated IgH genes and a favorable isolated 13q14 deletion will have an indolent and slowly progressive disease course, some will progress rapidly and show an aggressive disease. The inability to correctly predict the clinical course of these patients leads to wrong treatment decisions and may shorten the survival of these patients. It would therefore be desirable to have a more accurate risk classifier for CLL patients.

A variety of prognostic factors and signatures for CLL based on gene expression profiling or microRNA expression levels have been published in the last years. However, all these signatures fell short of surpassing the traditional genetic markers in prognostic power ^{9, 11, 14, 16-19}.

Thus, despite the above described advances in the development of prognostic markers for predicting disease progression in CLL, there remains a need to provide alternative or improved prognostic markers for the reliable identification of poor risk patients.

This need is addressed by the provision of the embodiments characterised in the claims.

Accordingly, the present invention relates to a kit consisting of or comprising nucleic acid molecules specific for the expression product of at least two genes selected from the group consisting of (i) to (viii): (i) NRIP1; (ii) SFTPB; (iii) PLEKHA1; (iv) PDE8A; (v) TCF7; (vi) MGC29506; (vii) MSI2; and (viii) one gene selected from the group consisting of MGAT4A, NELL2, BCL11B, CD247, SORL1, KLRB1, IL7R, RUNX2, MAL, RASGRF2, TIAM1, PRKCH, FNIP2, GIMAP7, PAG1, ITK, IL2RB, SATB1, PRKCQ, TRAT1, CLEC4A, wherein each of the nucleic acid molecules has a length of at least 8 consecutive nucleotides.

The term "kit", as used herein, relates to a combination of nucleic acid molecules. In other words, the kit of the present invention is a set of nucleic acid molecules. In its broadest sense, the term "kit" does not require the presence of any other compounds, vials, containers, manuals and the like. The kit of the present invention requires that more than one molecular species of nucleic acid molecules is present. Thus, nucleic acid molecules specific for the expression product of e.g. at least two genes are contained in the kit, wherein each of the nucleic acid molecules has a length of at least 8 consecutive nucleotides. The term "comprising" in the context of the kit(s) of the invention denotes that further components can be present in the kit. Non-limiting examples of such further components include preservatives, buffers for storage, enzymes etc. If the kit comprises additional nucleic acid molecules that are not specific for expression products of the genes and/or reference genes recited herein, it is preferred that at most 10.000 such additional nucleic acid molecules are comprised in the kit of the invention. More preferably, at most 5.000, such as for example at most 2.000 and more preferably at most 1.000 additional nucleic acid molecules are comprised in the kit of the invention. More preferably, at most 800, such as for example at most 600, more preferable at most 400, such as for example at most 300, at most 200, at most 100 and more preferably at most 80 additional nucleic acid molecules are comprised in the kit of the invention. Even more preferably, at most 50, such as for example at most 40, more preferable at most 30, such as for example at most 20, at most 10, at most 9, at most 8, at most 7, at most 6, at most 5, at most 4, at most 3, at most 2 and yet more preferably at most 1 additional nucleic acid molecule(s) is/are comprised in the kit of the invention. Also preferred is that the kit(s) of the invention do(es) not comprise any nucleic acid molecules coding for or specific for expression products other than the genes and/or reference genes recited herein. More preferably, the kit(s) of the invention do not comprise any nucleic acid molecules other than the genes and/or reference genes referred to in the respective embodiments defining the individual kits. In the latter embodiment, the kit preferably consists of nucleic acid molecules specific for the expression product of at least two genes and at most 28 genes.
The various components of the kit may be present in isolation or combination. For example, the components of the kit may be packaged in one or more containers such as one or more vials.

Accordingly, the present invention also provides a set of nucleic acid molecules, said set comprising or consisting of nucleic acid molecules specific for the expression product of at least two genes selected from the group consisting of (i) to (viii): (i) NRIP1; (ii) SFTPB; (iii) PLEKHA1; (iv) PDE8A; (v) TCF7; (vi) MGC29506; (vii) MSI2; and (viii) one gene selected from the group consisting of MGAT4A, NELL2, BCL11B, CD247, SORL1, KLRB1, IL7R, RUNX2, MAL, RASGRF2, TIAM1, PRKCH, FNIP2, GIMAP7, PAG1, ITK, IL2RB, SATB1, PRKCQ, TRAT1, CLEC4A, wherein each of the nucleic acid molecules has a length of at least 8 consecutive nucleotides.

In accordance with the present invention, for each selected gene at least one specific nucleic acid molecule is provided. Thus, exactly one nucleic acid molecule may be provided. Such single nucleic acid molecules are particularly suitable as hybridisation probes in northern or southern blot assays. In addition, more than one nucleic acid molecule may be provided for each selected gene, such as for example two nucleic acid molecules for use as primers. Furthermore, several different nucleic acid molecules specific for one selected gene may be provided, such as for example at least 2, at least 3, such as at least 4, at least 5, such as at least 10, more preferably at least 20 and most preferably at least 30 different nucleic acid molecules.

"Nucleic acid molecules", in accordance with the present invention, include DNA, such as for example cDNA and RNA. It is understood that the term "RNA" as used herein comprises all forms of RNA including mRNA. Preferably, embodiments reciting "RNA" are directed to mRNA. In a preferred embodiment the nucleic acid molecule(s) of the invention is/are DNA.

Further included are nucleic acid mimicking molecules known in the art such as synthetic or semisynthetic derivatives of DNA or RNA and mixed polymers, both sense and antisense strands. They may contain additional non-natural or derivatised nucleotide bases, as will be readily appreciated by those skilled in the art. Such nucleic acid mimicking molecules or nucleic acid derivatives according to the invention include peptide nucleic acid, phosphorothioate nucleic acid, phosphoramidate nucleic acid, 2'-O-methoxyethyl ribonucleic acid, morpholino nucleic acid, hexitol nucleic acid (HNA) and locked nucleic acid (LNA) (see, for example, Braasch and Corey, Chemistry & Biology 8, 1-7 (2001)). LNA is an RNA derivative in which the ribose ring is constrained by a methylene linkage between the 2'-oxygen and the 4'-carbon.

For the purposes of the present invention, a peptide nucleic acid (PNA) is a polyamide type of DNA analogue with an amide backbone in place of the sugar-phosphate backbone of DNA. As a consequence, certain components of DNA, such as phosphorus, phosphorus oxides, or deoxyribose derivatives, are not present in PNAs. As disclosed by Nielsen et al., Science 254:1497 (1991); and Egholm et al., Nature 365:666 (1993), PNAs bind specifically and tightly to complementary DNA strands and are not degraded by nucleases. Furthermore, they are stable under acidic conditions and resistant to proteases (Demidov et al. (1994), Biochem. Pharmacol., 48, 1310-1313). Their electrostatically neutral backbone increases the binding strength to complementary DNA as compared to the stability of the corresponding DNA-DNA duplex (Wittung et al. (1994), Nature 368, 561-563; Ray and Norden (2000), Faseb J., 14, 1041-1060). In fact, PNA binds more strongly to DNA than DNA itself does. This is probably because there is no electrostatic repulsion between the two strands, and also the polyamide backbone is more flexible. Because of this, PNA/DNA duplexes bind under a wider range of stringency conditions than DNA/DNA duplexes, making it easier to perform multiplex hybridization. Smaller probes can be used than with DNA due to the strong binding. In addition, it is more likely that single base mismatches can be determined with PNA/DNA hybridization because a single mismatch in a PNA/DNA 15-mer lowers the melting point (Tₘ) by 8°-20° C, vs. 4°-16° C for the DNA/DNA 15-mer duplex. Thereby discrimination between perfect matches and mismatches is improved. For its uncharged nature, PNA also permits the hybridisation of DNA samples at low salt or no-salt conditions, since no inter-strand repulsion as between two negatively charged DNA strands needs to be counteracted. As a consequence, the target DNA has fewer secondary structures under hybridisation conditions and is more accessible to probe molecules.

The nucleic acid molecules of the invention may be of natural as well as of (semi) synthetic origin. Thus, the nucleic acid molecules may, for example, be nucleic acid molecules that have been synthesized according to conventional protocols of organic chemistry. The nucleic acid molecules of the invention may, for example, be used as probes in Northern or Southern Blot analysis of RNA or DNA preparations, respectively, or can be used as oligonucleotide primers in PCR analysis dependent on their respective size. The person skilled in the art is familiar with the preparation and the use of said probes and primers (see, e.g., Sambrook and Russel "Molecular Cloning, A Laboratory Manual", Cold Spring Harbor Laboratory, N.Y. (2001)).
In this regard, it is important to note that the nucleic acid molecules of the invention may be detectably labelled. Detectable labels include radioactive labels such as ³H, or ³²P or fluorescent labels. Labelling of nucleic acids is well understood in the art and described, for example, in Sambrook and Russel "Molecular Cloning, A Laboratory Manual", Cold Spring Harbor Laboratory, N.Y. (2001).

The term "nucleic acid molecules specific for the expression product of', in accordance with the present invention, refers to a nucleic acid molecule which hybridises under stringent hybridisation conditions to the expression product of one of the genes referred to above. Preferably, the nucleic acid molecules hybridise to a detectably greater degree to the expression product of one of the genes referred to above than to other, unrelated sequences (e.g., at least 2-fold over background) or to any other of the above referenced genes. Preferably, the nucleic acid molecule hybridises only to sequences that have at least 90% sequence identity, more preferably 95%, such as 98% and more preferred 100% (highly stringent hybridisation conditions) sequence identity with the gene for which the expression level is to be determined. The term "expression product", in accordance with the present invention, relates to the transcribed product of the respective gene. The term includes, for example, mRNA naturally obtained in the cell upon expression of a gene, including pre-mRNA, as well as to cDNA obtained from such mRNA. Thus, the nucleic acid molecule of the invention comprises nucleic acid molecules hybridising to the mRNA or the sense strand of a corresponding cDNA as well as nucleic acid molecules hybridising to the anti-sense strand of such a corresponding cDNA. Nucleic acid molecules which hybridise under stringent hybridisation conditions to the expression product of one of the genes referred to above include nucleic acid molecules that exhibit at least 90% identity with these genes. Preferably, the identity is between 90 and 100% such as at least 94%, more preferred at least 96%, more preferred 98% and most preferred the identity is 100%. Preferably, said identity is over at least 8, such as at least 9, more preferably over at least 10 consecutive nucleotides. More preferably, said identity is over at least 20, such as at least 50 and more preferably at least 100 consecutive nucleotides. Most preferably, said identity is over the entire length of the molecule.

Such highly stringent conditions for hybridization are well known to the person skilled in the art. For example, highly stringent conditions for hybridization comprise, e.g. an overnight incubation at 65°C in 4x SSC (600 mM NaCl, 60 mM sodium citrate) followed by washing at 65°C in 0.1 x SSC for one hour. Alternatively, highly stringent hybridization conditions can comprise: an overnight incubation at 42°C in a solution comprising 50% formamide, 5x SSC (750 mM NaCl, 75 mM sodium citrate), 50 mM sodium phosphate (pH 7.6), 5x Denhardt's solution, 10% dextran sulphate, and 20 µg/ml denatured, sheared salmon sperm DNA, followed by washing in e.g. 0.1-0.5x SSC at about 55-65°C for about 5 to 20 min. Changes in the stringency of hybridization are primarily accomplished through the manipulation of formamide concentration (higher percentages of formamide result in higher stringency), salt conditions, or temperature. It is of note that variations in the above conditions may be accomplished through the inclusion and/or substitution of alternate blocking reagents. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility. Such modifications can generally be effected by the skilled person without further ado. A hybridization complex may be formed in solution (e.g., Cot or Rot analysis) or between one nucleic acid sequence present in solution and another nucleic acid sequence immobilized on a solid support (e.g., membranes, filters, chips, pins or glass slides to which, e.g., cells have been fixed).

It is further preferred that the nucleic acid molecules of the invention each have a length of at least 8 nucleotides, such as at least 9, at least 10, at least 11, at least 12 nucleotides, more preferred at least 13, such as at least 14, at least 15 nucleotides, at least 16, such as at least 17 nucleotides, more preferred at least 18, such as at least 19, at least 20, such as at least 21 nucleotides, and most preferred at least 25 nucleotides such as at least 30 nucleotides. Nucleic acid molecules of the present invention to be used as probes preferably have a length of at least 100, more preferably at least 200, and most preferably at least 500 nucleotides.

In accordance with the present invention, the term "at least two genes" relates to two or more genes. Preferably, said term relates to at least three genes, such as at least four genes, more preferably at least five genes, even more preferably at least six genes, such as at least seven genes and most preferably at least 8 genes. The term further encompasses exactly two or exactly three or exactly four or exactly five or exactly six or exactly seven or exactly eight genes.

In accordance with this embodiment of the present invention, the genes have to be selected from one of groups (i) to (viii). The selection of more than one gene from group (viii) is only envisaged in case that more than two genes are selected. Otherwise, only one of the genes listed in group (viii) is to be selected. In other words, at least one gene has to be selected from groups (i) to (vii).

NRIP1 refers to "Nuclear receptor interacting protein 1" and is a nuclear protein that specifically interacts with the hormone-dependent activation domain AF2 of nuclear receptors. NRIP1, which is also known as RIP140, modulates transcriptional activity of the estrogen receptor. Human NRIP1 is represented by the NCBI reference NM_003489.3 and is shown in SEQ ID NO: 1.

SFTPB refers to "surfactant protein B", an amphipathic surfactant protein essential for lung function and homeostasis after birth. Human SFTPB is represented by the NCBI references NM_198843.1 and NM_000542.2 and is shown in SEQ ID NOs: 2 and 3.

PLEKHA1 refers to "pleckstrin homology domain containing, family A (phosphoinositide binding specific) member 1", which binds specifically to phosphatidylinositol-3,4-diphosphate (Ptdlns3,4P2), but not to other phosphoinositides. PLEKHA1 is considered to possibly recruit other proteins to the plasma membrane. Human PLEKHA1 is represented by the NCBI references NM_001001974.1 and NM_021622.3 and is shown in SEQ ID NOs: 4 and 5.

PDE8A refers to "phosphodiesterase 8A", which regulates the intracellular levels of cAMP and cGMP as it selectively catalyses the hydrolysis of 3' cyclic phosphate bonds in adenosine and/or guanine 3',5' cyclic monophosphate. Human PDE8A is represented by the NCBI references NM_002605.2 and NM_173454.1 and is shown in SEQ ID NOs: 6 and 7.

TCF7 refers to "transcription factor 7 (T-cell specific, HMG-box)", which is a transcriptional activator involved in T-cell lymphocyte differentiation and is necessary for the survival of CD4(+), CD8(+) immature thymocytes. Human TCF7 is represented by the NCBI references NM_003202.3, NM_201634.2, NM_201632.2, NM_201633.2, NM_213648.2, NM_001134851.1 and NM_001134852.1 and is shown in SEQ ID NOs: 8 to 14.

MGC29506 refers to "plasma cell-induced endoplasmatic reticulum protein 1" and seems to play an important role in the secretion of IgM molecules in plasma cells ^{20,21}. Human MGC29506 is represented by the NCBI reference NM_016459.3 and is shown in SEQ ID NO: 15.

MSI2 refers to "musashi homolog 2 (Drosophila)", which is an RNA binding protein that regulates the expression of target mRNAs at the translation level. MSI2 is believed to potentially play a role in the proliferation and maintenance of stem cells in the central nervous system. Human MSI2 is represented by the NCBI references NM_138962.2 and NM_170721.1 and is shown in SEQ ID NOs: 16 and 17.

MGAT4A refers to "mannosyl (alpha-1,3-)-glycoprotein beta-1,4-N-acetylglucosaminyltransferase, isozyme A ". MGAT4A is a key glycosyltransferase that regulates the formation of tri- and multiantennary branching structures in the Golgi apparatus. MGAT4A, in addition to the related isoenzyme B, catalyzes the transfer of N-acetylglucosamine (GlcNAc) from UDP-GlcNAc in a beta-1,4 linkage to the Man-alpha-1,3-Man-beta-1,4-GlcNAc arm of R-Man-alpha-1,6(GlcNAc-beta-1,2-Man-alpha-1,3)Man-beta-1,4-GlcNAc-beta-1,4-GlcNAc-beta-1-Asn. The protein may play a role in regulating the availability of serum glycoproteins, oncogenesis, and differentiation. Human MGAT4A is represented by the NCBI reference NM_012214.2 and is shown in SEQ ID NO: 18.

NELL2 refers to "NEL-like 2 (chicken)", which is a glycoprotein containing several von Willebrand factor C domains and epidermal growth factor (EGF)-like domains. The encoded protein acts as a homotrimer and is found in the cytoplasm. Several variants encoding a few different isoforms exist, and at least one isoform appears to be a secreted protein. Studies in mouse suggest that this protein plays a role in neural cell growth and differentiation as well as in oncogenesis. Human NELL2 is represented by the NCBI references NM_001145110.1, NM_001145107.1, NM_001145108.1, NM_006159.2 and NM_001145109.1 and is shown in SEQ ID NOs: 19 to 23.

BCL11 B refers to "B-cell CLUlymphoma 11 B (zinc finger protein)", a C2H2-type zinc finger protein that is closely related to BCL11A, a gene whose translocation may be associated with B-cell malignancies. The specific function of this gene has not yet been determined. Human BCL11 B is represented by the NCBI references NM_022898.1 and NM_138576.2 and is shown in SEQ ID NOs: 24 and 25.

CD247 refers to "CD247 molecule", which is the T-cell receptor zeta, which together with T-cell receptor alpha/beta and gamma/delta heterodimers, and with CD3-gamma, -delta and -epsilon, forms the T-cell receptor-CD3 complex. The zeta chain plays an important role in coupling antigen recognition to several intracellular signal-transduction pathways. Low expression of the antigen results in impaired immune response. Human CD247 is represented by the NCBl references NM_198053.2 and NM_000734.3 and is shown in SEQ ID NOs: 26 and 27.

SORL1 refers to "sortilin-related receptor, L(DLR class) A repeats-containing". SORL1 belongs to the families of vacuolar protein sorting 10 (VPS10) domain-containing receptor proteins, of low density lipoprotein receptor (LDLR) proteins, and of fibronectin type III repeats proteins. In addition to VPS10, LDLR and fibronectin type 3 domains, this protein also includes an epidermal growth factor precursorlike module, a single transmembrane segment and a cytoplasmic tail with features similar to endocytosis- and sorting-competent receptors. Members of the VPS10 domain-containing receptor family are large with many exons but the CDS lengths are usually less than 3700 nt; this gene is an exception to the pattern with a CDS length greater than 6600 nt. Very large introns typically separate the exons encoding the VPS10 domain; the remaining exons are separated by much smaller-sized introns. The encoded protein is mainly intracellular and localizes in the paranuclear compartment. It is synthesized as a preproprotein, and when the propeptide is still attached, no binding occurs to the VPS10 domain. This gene is strongly expressed in the central nervous system. Human SORL1 is represented by the NCBI reference NM_003105.4 and is shown in SEQ ID NO: 28.

KLRB1 refers to "killer cell lectin-like receptor subfamily B, member". KLRB1 is classified as a type II membrane protein because it has an external C terminus protein. It contains an extracellular domain with several motifs characteristic of C-type lectins, a transmembrane domain, and a cytoplasmic domain. KLRB1 plays an inhibitory role on natural killer (NK) cells cytotoxicity. Activation results in specific acid sphingomyelinase/SMPD1 stimulation with subsequent marked elevation of intracellular ceramide. Activation also leads to AKT1/PKB and RPS6KA1/RSK1 kinases stimulation as well as markedly enhanced T-cell proliferation induced by anti-CD3. It acts as a lectin that binds to the terminal carbohydrate Gal-alpha(1,3)Gal epitope as well as to the N-acetyllactosamine epitope. It also binds to CLEC2D/LLT1 as a ligand and inhibits NK cell-mediated cytotoxicity as well as interferon-gamma secretion in target cells. Human KLRB1 is represented by the NCBI reference NM_002258.2 and is shown in SEQ ID NO: 29.

IL7R refers to the "interleukin 7 receptor". The function of this receptor requires the interleukin 2 receptor, gamma chain (IL2RG), which is a common gamma chain shared by the receptors of various cytokines, including interleukine 2, 4, 7, 9, and 15. IL7R has been shown to play a critical role in the V(D)J recombination during lymphocyte development. IL7R is also found to control the accessibility of the TCR gamma locus by STAT5 and histone acetylation. Knockout studies in mice suggested that blocking apoptosis is an essential function of this protein during differentiation and activation of T lymphocytes. The functional defects in this protein may be associated with the pathogenesis of the severe combined immunodeficiency (SCID) Human IL7R is represented by the NCBI reference NM_002185.2 and is shown in SEQ ID NO: 30.

RUNX2 refers to "runt-related transcription factor 2", which is a nuclear protein with a Runt DNA-binding domain that is a member of the RUNX family of transcription factors. RUNX2 is essential for osteoblastic differentiation and skeletal morphogenesis and acts as a scaffold for nucleic acids and regulatory factors involved in skeletal gene expression. RUNX2 can bind DNA both as a monomer or, with more affinity, as a subunit of a heterodimeric complex. Mutations in this gene have been associated with the bone development disorder cleidocranial dysplasia (CCD). Human RUNX2 is represented by the NCBI references NM_001024630.2, N_001015051.2 and NM_004348.3 and is shown in SEQ ID NOs: 31 to 33.

MAL refers to "mal, T-cell differentiation protein ", and is a highly hydrophobic integral membrane protein belonging to the MAL family of proteolipids. The protein has been localized to the endoplasmic reticulum of T-cells and is a candidate linker protein in T-cell signal transduction. In addition, this proteolipid is localized in compact myelin of cells in the nervous system and has been implicated in myelin biogenesis and/or function. MAL plays a role in the formation, stabilization and maintenance of glycosphingolipid-enriched membrane microdomains. Human MAL is represented by the NCBI references NM_002371.2, NM_022438.1, NM_022439.1 and NM_022440.1 and is shown in SEQ ID NOs: 34 to 37.

RASGRF2 refers to the "Ras protein-specific guanine nucleotide-releasing factor 2". RASGRF2 functions as a calcium-regulated nucleotide exchange factor activating both Ras and RAC1 through the exchange of bound GDP for GTP. RASGRF2 preferentially activates HRAS in vivo compared to RRAS based on their different types of prenylation. It functions in synaptic plasticity by contributing to the induction of long term potentiation. Human RASGRF2 is represented by the NCBI reference N_006909.1 and is shown in SEQ ID NO: 38.

TIAM1 refers to "T-cell lymphoma invasion and metastasis 1". TIAM1 modulates the activity of RHO-like proteins and connects extracellular signals to cytoskeletal activities. It acts as a GDP-dissociation stimulator protein that stimulates the GDP-GTP exchange activity of RHO-like GTPases and activates them. It also activates RAC1, CDC42, and to a lesser extent RHOA. Human TIAM1 is represented by the NCBI reference NM_003253.2 and is shown in SEQ ID NO: 39.

PRKCH refers to "protein kinase C, eta", which belongs to the PKC family. It is a calcium-independent and phospholipid-dependent protein kinase. It is predominantly expressed in epithelial tissues and has been shown to reside specifically in the cell nucleus. PRKCH can regulate keratinocyte differentiation by activating the MAP kinase MAPK13 (p38delta)-activated protein kinase cascade that targets CCAAT/enhancer-binding protein alpha (CEBPA). It is also found to mediate the transcription activation of the transglutaminase 1 (TGM1) gene. Human PRKCH is represented by the NCBI reference NM_006255.3 and is shown in SEQ ID NO: 40.

FNIP2 refers to "folliculin interacting protein 2", which is believed to be involved in energy and/or nutrient sensing through the AMPK and mTOR signaling pathways. Human FNIP2 is represented by the NCBI reference NM_020840.1 and is shown in SEQ ID NO: 41.

GIMAP7 refers to "GTPase, IMAP family member 7", a protein belonging to the GTP-binding superfamily and to the immuno-associated nucleotide (IAN) subfamily of nucleotide-binding proteins. In humans, the IAN subfamily genes are located in a cluster at 7q36.1. Human GIMAP7 is represented by the NCBI reference NM_153236.3 and is shown in SEQ ID NO: 42.

PAG1 refers to "phosphoprotein associated with glycosphingolipid microdomains 1", a type III transmembrane adaptor protein that binds to the tyrosine kinase csk protein. PAG1 negatively regulates TCR (T-cell antigen receptor)-mediated signaling in T-cells and FCER1 (high affinity immunoglobulin epsilon receptor)-mediated signaling in mast cells. It also promotes CSK activation and recruitment to lipid rafts, which results in LCK inhibition and inhibits immunological synapse formation by preventing dynamic arrangement of lipid raft proteins. PAG1 may be involved in cell adhesion signaling. Human PAG1 is represented by the NCBI reference NM_018440.3 and is shown in SEQ ID NO: 43.

ITK refers to "IL2-inducible T-cell kinase", an intracellular tyrosine kinase expressed in T-cells. The protein contains both SH2 and SH3 domains which are often found in intracellular kinases. ITK is thought to play a role in T-cell proliferation and differentiation. Human ITK is represented by the NCBI reference NM_005546.3 and is shown in SEQ ID NO: 44.

IL2RB refers to "interleukin 2 receptor, beta", which is involved in T cell-mediated immune responses. The interleukin 2 receptor is present in 3 forms with respect to ability to bind interleukin 2. The low affinity form is a monomer of the alpha subunit and is not involved in signal transduction. The intermediate affinity form consists of an alpha/beta subunit heterodimer, while the high affinity form consists of an alpha/beta/gamma subunit heterotrimer. Both the intermediate and high affinity forms of the receptor are involved in receptor-mediated endocytosis and transduction of mitogenic signals from interleukin 2. Human IL2RB is represented by the NCBI reference NM_000878.2 and is shown in SEQ ID NO: 45.

SATB1 refers to "SATB homeobox 1", which is assumed to be a crucial silencing factor contributing to the initiation of X inactivation mediated by Xist RNA that occurs during embryogenesis and in lymphoma. It binds to DNA at special AT-rich sequences, the consensus SATB1-binding sequence (CSBS), at nuclear matrix- or scaffold-associated regions. SATB1 is thought to recognize the sugar-phosphate structure of double-stranded DNA. It further is a transcriptional repressor controlling nuclear and viral gene expression in a phosphorylated and acetylated status-dependent manner, by binding to matrix attachment regions (MARs) of DNA and inducing a local chromatin-loop remodeling. SATB1 acts as a docking site for several chromatin remodeling enzymes (e.g. PML at the MHC-I locus) and also by recruiting corepressors (HDACs) or coactivators (HATs) directly to promoters and enhancers. It modulates genes that are essential in the maturation of the immune T-cell CD8SP from thymocytes. It is required for the switching of fetal globin species, and beta- and gamma-globin genes regulation during erythroid differentiation and plays a role in chromatin organization and nuclear architecture during apoptosis. SATB1 interacts with the unique region (UR) of cytomegalovirus (CMV). Alu-like motifs and SATB1-binding sites provide a unique chromatin context which seems preferentially targeted by the HIV-1 integration machinery. Moreover, HIV-1 Tat may overcome SATB1-mediated repression of IL2 and IL2RA (interleukin) in T-cells by binding to the same domain as HDAC1. It delineates specific epigenetic modifications at target gene loci, directly up-regulating metastasis-associated genes while down-regulating tumor-suppressor genes. It reprograms chromatin organization and the transcription profiles of breast tumors to promote growth and metastasis. Human SATB1 is represented by the NCBI references NM_001131010.1 and NM_002971.3 and is shown in SEQ ID NOs: 46 and 47.

PRKCQ refers to "protein kinase C, theta", which is is one of the PKC family members. It is a calcium-independent and phospholipid-dependent protein kinase. This kinase is important for T-cell activation. It is required for the activation of the transcription factors NF-kappaB and AP-1, and may link the T cell receptor (TCR) signaling complex to the activation of the transcription factors. It is further required for interleukin-2 (IL2) production. Human PRKCQ is represented by the NCBI reference NM_006257.2 and is shown in SEQ ID NO: 48.

TRAT1 refers to "T cell receptor associated transmembrane adaptor 1", which stabilizes the TCR (T-cell antigen receptor)/CD3 complex at the surface of T-cells. Human TRAT1 is represented by the NCBI reference NM_016388.2 and is shown in SEQ ID NO: 49.

CLEC4A refers to "C-type lectin domain family 4, member A", a member of the C-type lectin/C-type lectin-like domain (CTL/CTLD) superfamily. Members of this family share a common protein fold and have diverse functions, such as cell adhesion, cell-cell signalling, glycoprotein turnover, and roles in inflammation and immune response. The encoded type 2 transmembrane protein may play a role in inflammatory and immune response. Multiple transcript variants encoding distinct isoforms have been identified for this gene, which is closely linked to other CTL/CTLD superfamily members on chromosome 12p13 in the natural killer gene complex region. Human CLEC4A is represented by the NCBI references NM_016184.3, NM_194450.2, NM_194447.2 and NM_194448.2 and is shown in SEQ ID NOs: 50 to 53.

In accordance with the present invention, it was surprisingly found that a prognostic score for patients with CLL could be based on the expression levels of a specific set of genes as defined above. This set of genes was derived from the gene expression profiles of 151 microarray profiles (from 149 patients) with CLL (training set), representing one of the largest data sets available for CLL patient, with a median follow up of 4.11 years. This set of genes was further validated in a second group of 147 CLL patients using quantitative real time PCR.

A prognostic score based on the expression levels of said limited set of genes achieved a higher prognostic accuracy than any other previously used combination of prognostic markers in CLL and will, therefore, greatly facilitate risk adapted therapy of CLL patients. In other words, based on a simple and cost-effective analysis of the expression levels of genes selected from this limited set of genes, it is now possible to derive an assessment of the risk of an individual CLL patient for being a poor prognosis patient, i.e. showing a rapid and aggressive disease progression or being a good prognosis patient, i.e. showing an indolent and slowly progressive disease course. Knowledge of the disease prognosis subsequently allows risk-adapted treatment, which has been shown to be of benefit for patients with CLL. For example, based on a diagnosis as poor prognosis patient, allogeneic stem cell transplantation may by offered to the patient, which has been shown to change overall survival in this high risk group ⁵⁻⁸. Therefore, the present invention allows the clinically important identification of high risk CLL-patients.

In particular, a prognostic score based on eight genes was able to add information to risk prediction in several subgroups defined by the established genetic markers as, for example in poor prognosis CLL patients who had unmutated IgVH genes or 11q or 17p deletions. Also in CLL subgroups with favourable prognosis, e.g. mutated IgVH genes and absence of 11q or 17p deletions, the prognostic score in accordance with the invention was able to predict patients with a shorter time-to-treatment (TTT, i.e. the time from study entry until start of first or next chemotherapy treatment or death). This is also the case in the Binet A subgroup. In multivariate analysis for overall survival (OS) and TTT the score had smaller p-values than the traditional genetic markers. The analysis of the prediction performance using prediction error curves yielded superior performances for the models containing the prognostic score compared to the models based solely on FISH and VH-status (see Example 9).

Importantly, a prognostic score for the clinical course of CLL can now be easily derived by standard techniques, such as for example real time PCR, in a routine diagnostic setting thus providing a less labour intensive, fast and cost effective alternative to established methods, such as traditional CLL FISH panel analysis and determining the mutational status of the IgH locus. Risk prediction using this score has the potential to increase availability and clinical use of risk stratified therapy in CLL. To the inventors best knowledge, no prognostic set of markers based on the specific advantageous combination of genes presented herein is presently available in the art.

In addition, the findings in accordance with the present invention relating to genes associated with a poor or good prognosis for CLL patients now also link several molecular pathways for the first time with progression in CLL and possibly also with the development of this disease. The pathogenesis of CLL is still poorly understood. Without wishing to be bound by theory, it is speculated that some of the genes identified herein play an important role in the pathogenesis of CLL, especially those genes which have been found altered in other malignancies or which belong to genetic networks involved in malignant transformation.

The present invention further relates to a method of identifying a compound suitable as a lead compound and/or as a medicament for the treatment and/or prevention of CLL, the method comprising the steps of (a) contacting a test compound with (i) a protein encoded by a gene referred to above; or (ii) with a cell comprising a protein encoded by a gene referred to above and (b) determining whether said test compound, upon contacting in step (a) alters the activity of said protein, wherein said alteration indicates a compound suitable as a lead compound and/or as a medicament for the treatment and/or prevention of CLL. Preferably, said alteration is an inhibition or activation of protein activity. Preferably, the protein is selected from the group consisting of MSI2, PLEKHA1, MGC29506, MGAT4A, SFTPB.

In a preferred embodiment, the kit consists of or comprises nucleic acid molecules specific for the expression product of (a) at least four genes selected from the group consisting of (i) to (viii): (i) NRIP1; (ii) SFTPB; (iii) PLEKHA1; (iv) PDE8A; (v) TCF7; (vi) MGC29506; (vii) MS12; and (viii) one gene selected from the group consisting of MGAT4A, NELL2, BCL11B, CD247, SORL1, KLRB1, IL7R, RUNX2, MAL, RASGRF2, TIAM1, PRKCH, FNIP2, GIMAP7, PAG1, ITK, IL2RB, SATB1, PRKCQ, TRAT1, CLEC4A; or (b) at least two genes selected from the group consisting of (i) to (v): (i) SFTPB; (ii) PLEKHA1; (iii) MGC29506; (iv) MSI2; and (v) one gene selected from the group consisting of MGAT4A, NELL2, BCL11B, CD247, SORL1, KLRB1, IL7R, RUNX2, MAL, RASGRF2, TIAM1, PRKCH, FNIP2, GIMAP7, PAG1, ITK, IL2RB, SATB1, PRKCQ, TRAT1, CLEC4A.

Accordingly, the present invention also provides a set of nucleic acid molecules, said set comprising or consisting of nucleic acid molecules specific for the expression product of (a) at least four genes selected from the group consisting of (i) to (viii): (i) NRIP1; (ii) SFTPB; (iii) PLEKHA1; (iv) PDE8A; (v) TCF7; (vi) MGC29506; (vii) MSI2; and (viii) one gene selected from the group consisting of MGAT4A, NELL2, BCL11B, CD247, SORL1, KLRB1, IL7R, RUNX2, MAL, RASGRF2, TIAM1, PRKCH, FNIP2, GIMAP7, PAG1, ITK, IL2RB, SATB1, PRKCQ, TRAT1, CLEC4A; or (b) at least two genes selected from the group consisting of (i) to (v): (i) SFTPB; (ii) PLEKHA1; (iii) MGC29506; (iv) MSI2; and (v) one gene selected from the group consisting of MGAT4A, NELL2, BCL11B, CD247, SORL1, KLRB1, IL7R, RUNX2, MAL, RASGRF2, TIAM1, PRKCH, FNIP2, GIMAP7, PAG1, ITK, IL2RB, SATB1, PRKCQ, TRAT1, CLEC4A.

In accordance with this preferred embodiment of the present invention, the selection of more than one gene from group (viii) is only envisaged in case that more than four genes are selected in (a) or in case that more than two genes are selected in (b). Otherwise, at most one of the genes listed in group (a)(viii) or in (b)(v) is to be selected. In other words, at least three genes have to be selected in (a) from groups (i) to (vii) or at least one gene has to be selected in (b) from groups (i) to (iv).

In this preferred embodiment, the kit preferably consists of or comprises nucleic acid molecules specific for the expression product of at most 28 genes.

The present invention also relates to a kit consisting of or comprising (a) nucleic acid molecules specific for the expression product of at least one reference gene; and (b) nucleic acid molecules specific for the expression product of (b) at least two genes selected from the group consisting of (i) to (viii): (i) NRIP1; (ii) SFTPB; (iii) PLEKHA1; (iv) PDE8A; (v) TCF7; (vi) MGC29506; (vii) MSI2; and (viii) one gene selected from the group consisting of MGAT4A, NELL2, BCL11B, CD247, SORL1, KLRB1, IL7R, RUNX2, MAL, RASGRF2, TIAM1, PRKCH, FNIP2, GIMAP7, PAG1, ITK, IL2RB, SATB1, PRKCQ, TRAT1, CLEC4A, wherein each of the nucleic acid molecules has a length of at least 8 nucleotides.

Accordingly, the present invention also provides a set of nucleic acid molecules, said set comprising or consisting of (a) nucleic acid molecules specific for the expression product of at least one reference gene; and (b) nucleic acid molecules specific for the expression product of (b) at least two genes selected from the group consisting of (i) to (viii): (i) NRIP1; (ii) SFTPB; (iii) PLEKHA1; (iv) PDE8A; (v) TCF7; (vi) MGC29506; (vii) MSI2; and (viii) one gene selected from the group consisting of MGAT4A, NELL2, BCL11B, CD247, SORL1, KLRB1, IL7R, RUNX2, MAL, RASGRF2, TIAM1, PRKCH, FNIP2, GIMAP7, PAG1, ITK, IL2RB, SATB1, PRKCQ, TRAT1, CLEC4A, wherein each of the nucleic acid molecules has a length of at least 8 nucleotides.

The term "reference gene" as used herein refers to a gene that is expressed in the cells contained in the sample with substantially constant expression levels. In other words, the expression level of a reference gene should not differ (apart from deviations caused by the limits of accuracy of established detection methods) between samples of different origin. Often, housekeeping genes are used as reference genes. Examples of reference genes include, without being limiting, GAPDH, RPLPO, PGK1, HSP90AB1, cyclophilin, actin and many more. Further examples are detailed for example in Eisenberg *et al.* 2003 and Velculescu *et al.* 1999 ^{36, 37}. Preferably, the at least one reference gene is selected from the group consisting of GAPDH, RPLPO and PGK1.

GAPDH refers to "glyceraldehyde-3-phosphate dehydrogenase", catalyses an important energy-yielding step in carbohydrate metabolism, the reversible oxidative phosphorylation of glyceraldehyde-3-phosphate in the presence of inorganic phosphate and nicotinamide adenine dinucleotide (NAD). The enzyme exists as a tetramer of identical chains. Human GAPDH is represented by the NCBI reference NM_002046.3 and is shown in SEQ ID NO: 54.

RPLPO refers to "ribosomal protein, large, P0", a ribosomal protein that is a component of the 60S subunit. The protein, which is the functional equivalent of the E. coli L10 ribosomal protein, belongs to the L10P family of ribosomal proteins. It is a neutral phosphoprotein with a C-terminal end that is nearly identical to the C-terminal ends of the acidic ribosomal phosphoproteins P1 and P2. The P0 protein can interact with P1 and P2 to form a pentameric complex consisting of P1 and P2 dimers, and a P0 monomer. The protein is located in the cytoplasm. Human RPLPO is represented by the NCBI references NM_001002.3 and NM_053275.3 and is shown in SEQ ID NOs: 55 and 56.

PGK1 refers to "phosphoglycerate kinase 1". A protein encoded by this gene is a glycolytic enzyme that catalyses the conversion of 1,3-diphosphoglycerate to 3-phosphoglycerate. The encoded protein may also act as a cofactor for polymerase alpha. This gene lies on the X-chromosome, while a related pseudogene also has been found on the X-chromosome and another on chromosome 19. Human PGK1 is represented by the NCBI references NM_000291.3 and is shown in SEQ ID NO: 57.

Such reference genes may also be determined by statistical tools known in the art for a given data set. Methods of selecting appropriate reference genes include, without being limiting programs based on the excel platform (Huggett et al. Genes and Immunity (2005), 1-6) such as for example gnorm, which allows the most appropriate reference gene to be chosen by using the geometric mean of the expression of the candidate cDNA. This software is freely available (http://www.genomebiology.com/2002/3/7/research/0034/) and has been described in Vandesompele et al. Genome Biol 2002; 3: RESEARCH0034. BestKeeper also selects the least variable gene using the geometric mean but uses raw data instead of data converted to copy number. This programme has been described in Pfaffl et al. Biotechnol Lett 2004; 26: 509-515 and is available at http://www.genequantification.de/BestKeeper-1.zip. A third programme, Norm-Finder, which is also freely available on request, not only measures the variation but also ranks the potential reference genes by how much they differ between study groups, that is, the extent by which they are effected by the experimental conditions (Andersen et al. Cancer Res 2004; 64: 5245-5250).

In accordance with this embodiment of the present invention, the selection of more than one gene from group (viii) is only envisaged in case that more than two genes are selected. Otherwise, only one of the genes listed in group (viii) is to be selected. In other words, at least one gene has to be selected from groups (i) to (vii).

In this embodiment, the kit consists of or comprises (a) nucleic acid molecules specific for the expression product of at least one reference gene, such as for example at least two, more preferably at least three and most preferably at least 5 reference genes. The kit preferably consists of or comprises nucleic acid molecules specific for the expression product of less than 100 reference genes, such as for example less than 75, more preferably less than 50, such as for example less than 25 and most preferably less than 15 reference genes. Preferably, the kit consists of or comprises nucleic acid molecules specific for the expression product of at most 28 genes as defined in (b).

In accordance with this embodiment of the invention, it is preferred that the at least one reference gene is selected from the group consisting of GAPDH, RPLPO and PGK1. More preferably, the kit contains or comprises nucleic acid molecules specific for the expression product of the reference genes GAPDH, RPLPO and PGK1.

The present invention also relates to a kit consisting of or comprising (a) nucleic acid molecules specific for the expression product of at least one reference gene; and (b) nucleic acid molecules specific for the expression product of (ba) at least four genes selected from the group consisting of (i) to (viii): (i) NRIP1; (ii) SFTPB; (iii) PLEKHA1; (iv) PDE8A; (v) TCF7; (vi) MGC29506; (vii) MSI2; and (viii) one gene selected from the group consisting of MGAT4A, NELL2, BCL11B, CD247, SORL1, KLRB1, IL7R, RUNX2, MAL, RASGRF2, TIAM1, PRKCH, FNIP2, GIMAP7, PAG1, ITK, IL2RB, SATB1, PRKCQ, TRAT1, CLEC4A; or (bb) at least two genes selected from the group consisting of (i) to (v): (i) SFTPB; (ii) PLEKHA1; (iii) MGC29506; (iv) MSI2; and (v) one gene selected from the group consisting of MGAT4A, NELL2, BCL11B, CD247, SORL1, KLRB1, IL7R, RUNX2, MAL, RASGRF2, TIAM1, PRKCH, FNIP2, GIMAP7, PAG1, ITK, IL2RB, SATB1, PRKCQ, TRAT1, CLEC4A, wherein each of the nucleic acid molecules has a length of at least 8 nucleotides. Accordingly, the invention provides a set of nucleic acid molecules specific for these genes recited.

Accordingly, the present invention also provides a set of nucleic acid molecules, said set comprising or consisting of (a) nucleic acid molecules specific for the expression product of at least one reference gene; and (b) nucleic acid molecules specific for the expression product of (ba) at least four genes selected from the group consisting of (i) to (viii): (i) NRIP1; (ii) SFTPB; (iii) PLEKHA1; (iv) PDE8A; (v) TCF7; (vi) MGC29506; (vii) MSI2; and (viii) one gene selected from the group consisting of MGAT4A, NELL2, BCL11B, CD247, SORL1, KLRB1, IL7R, RUNX2, MAL, RASGRF2, TIAM1, PRKCH, FNIP2, GIMAP7, PAG1, ITK, IL2RB, SATB1, PRKCQ, TRAT1, CLEC4A; or (bb) at least two genes selected from the group consisting of (i) to (v): (i) SFTPB; (ii) PLEKHA1; (iii) MGC29506; (iv) MSI2; and (v) one gene selected from the group consisting of MGAT4A, NELL2, BCL11B, CD247, SORL1, KLRB1, IL7R, RUNX2, MAL, RASGRF2, TIAM1, PRKCH, FNIP2, GIMAP7, PAG1, ITK, IL2RB, SATB1, PRKCQ, TRAT1, CLEC4A, wherein each of the nucleic acid molecules has a length of at least 8 nucleotides.

In accordance with this embodiment of the present invention, the selection of more than one gene from group (viii) is only envisaged in case that more than four genes are selected in (ba) or in case that more than two genes are selected in (bb). Otherwise, only one of the genes listed in group (viii) is to be selected. In other words, at least three genes have to be selected in (ba) from groups (i) to (vii) or at least one gene has to be selected in (bb) from groups (i) to (iv).

In this embodiment, the kit consists of or comprises (a) nucleic acid molecules specific for the expression product of at least one reference gene, such as for example at least two, more preferably at least three and most preferably at least 5 reference genes. The kit preferably consists of nucleic acid molecules specific for the expression product of less than 100 reference genes, such as for example less than 75, more preferably less than 50, such as for example less than 25 and most preferably less than 15 reference genes. In addition, the kit consists of (b) nucleic acid molecules specific for the expression product of at most 28 genes.

In accordance with this embodiment of the invention, it is preferred that the at least one reference gene is selected from the group consisting of GAPDH, RPLP0 and PGK1. More preferably, the kit contains or comprises nucleic acid molecules specific for the expression product of the reference genes GAPDH, RPLPO and PGK1.

In a more preferred embodiment, the nucleic acid molecules that are specific for the expression product of genes other than reference genes are specific for the expression product of at least eight genes.

Accordingly, in this embodiment the nucleic acid molecules are specific for the expression product of at least the genes NRIP1, SFTPB, PLEKHA1, PDE8A, TCF7, MGC29506, MSI2 and at least one gene selected from the group consisting of MGAT4A, NELL2, BCL11B, CD247, SORL1, KLRB1, IL7R, RUNX2, MAL, RASGRF2, TIAM1, PRKCH, FNIP2, GIMAP7, PAG1, ITK, IL2RB, SATB1, PRKCQ, TRAT1, CLEC4A.

Most preferred, the at least eight genes are NRIP1, SFTPB, PLEKHA1, PDE8A, TCF7, MGC29506, MSI2 and MGAT4A.

The present invention further relates to a diagnostic composition consisting of or comprising the kit of the invention.

Thus, in accordance with the present invention, also a diagnostic composition is provided consisting of or comprising a set of nucleic acid molecules of the invention.

The term "diagnostic composition" relates to compositions for diagnosing a disease. Preferably, the diagnostic composition is for diagnosing disease progression in individual patients suffering from chronic lymphocytic leukemia. In other words, the diagnosis amounts to determining the predisposition of an individual patient for being a poor prognosis or good prognosis patient. The diagnostic composition of the invention consists of any one of the kits of the previous embodiments. The term "comprising" in the context of the diagnostic composition of the invention denotes that further components can be present in the diagnostic composition. Non-limiting examples of such further components include preservatives, buffers for storage, enzymes such as reverse transcriptase, thermostable polymerases etc. If the diagnostic composition comprises additional nucleic acid molecules that are not specific for expression products of the genes and/or reference genes recited herein, it is preferred that at most 10.000 such additional nucleic acid molecules are comprised in the diagnostic composition of the invention. More preferably, at most 5.000, such as for example at most 2.000 and more preferably at most 1.000 additional nucleic acid molecules are comprised in the diagnostic composition of the invention. More preferably, at most 800, such as for example at most 600, more preferable at most 400, such as for example at most 300, at most 200, at most 100 and more preferably at most 80 additional nucleic acid molecules are comprised in the diagnostic composition of the invention. Even more preferably, at most 50, such as for example at most 40, more preferable at most 30, such as for example at most 20, at most 10, at most 9, at most 8, at most 7, at most 6, at most 5, at most 4, at most 3, at most 2 and yet more preferably at most 1 additional nucleic acid molecule(s) is/are comprised in the diagnostic composition of the invention. Also preferred is that the diagnostic composition does not encompass nucleic acid molecules coding for or specific for expression products other than the genes and/or reference genes referred to in the respective embodiments defining the kits of the invention. Preferably, the diagnostic composition of the invention does not comprise any nucleic acid molecules other than the genes and/or reference genes referred to in the respective embodiments defining the individual kits. The diagnostic composition of the invention may be used to test for the expression levels of the specific genes recited herein using methods well known in the art. Such methods are described, e. g. in Sambrook and Russel "Molecular Cloning, A Laboratory Manual", Cold Spring Harbor Laboratory, N.Y. (2001).

In accordance with the present invention, the term "chronic lymphocytic leukemia" (also referred to herein as CLL) relates to a type of leukemia, i.e. it is an abnormal and malignant neoplastic proliferation of leukocytes, in particular of B cell lymphocytes. Guidelines for the diagnosis of CLL are defined and published by the National Cancer Institute-sponsored Working Group (NCI-WG) and the International Workshop on Chronic Lymphocytic Leukemia (IWCLL) ^{22, 23}. Affected cells accumulate in the bone marrow and blood, where they outgrow healthy blood cells and cause a variety of symptoms that finally limit life quality and expectancy.

A "poor prognosis patient" in accordance with the present invention is a patient at increased risk of showing a rapid and aggressive disease progression. A rapid and aggressive disease progression is characterized by a reduced survival time compared to the average survival time at the corresponding disease stage. Whereas the median overall survival time from first diagnosis is generally around 8 to 10 years, poor prognosis patients show a reduced survival time of less than 5 years, such as for example less than 4 years, more preferably less than 3 years, such as for example less than 2 years and more preferably less than one year from first diagnosis. A short time to treatment and/or a short time to next treatment interval is a marker of poor prognosis. Poor prognosis may further be accompanied by rapid development and progression of disease related symptoms including, without being limiting, advanced disease stage, lymph node enlargement, anemia or thrombocytopenia and symptoms like night sweat, weight loss or fever. Furthermore, the group of poor prognosis patients also encompasses patients relapsing early after initial treatment or being resistant to standard treatment.

A "good prognosis patient" in accordance with the present invention is a patient having a reduced risk for showing a rapid and aggressive disease progression, instead showing an indolent and slowly progressive disease course. An indolent and slowly progressive disease course is characterized by an increased survival time compared to the average survival time at the corresponding disease stage. An increased survival time preferably relates to more than 12 years, such as for example more than 15 years and most preferably more than 20 years. A prolonged time to treatment and/or a prolonged time to next treatment interval is a marker of good prognosis.

The present invention further relates to the kit of any of the previous embodiments for use in the diagnosis of disease progression in a patient suffering from chronic lymphocytic leukemia. The term "disease progression", as used herein, includes future progression of the disease, i.e. the possibility of good and poor prognosis as defined above.

Accordingly, the present invention also provides a set of nucleic acid molecules, said set comprising or consisting of nucleic acid molecules specific for the genes as recited herein and being for use in the diagnosis of disease progression in a patient suffering from chronic lymphocytic leukemia.

Preferably, the diagnosis of disease progression is based on the expression levels of at least two genes selected from the group consisting of (i) to (viii): (i) NRIP1; (ii) SFTPB; (iii) PLEKHA1; (iv) PDE8A; (v) TCF7; (vi) MGC29506; (vii) MSI2; and (viii) one gene selected from the group consisting of MGAT4A, NELL2, BCL11B, CD247, SORL1, KLRB1, IL7R, RUNX2, MAL, RASGRF2, TIAM1, PRKCH, FNIP2, GIMAP7, PAG1, ITK, IL2RB, SATB1, PRKCQ, TRAT1, CLEC4A. In an alternative preferred embodiment, the diagnosis of disease progression is based on the expression levels of (a) at least four genes selected from the group consisting of (i) to (viii): (i) NRIP1; (ii) SFTPB; (iii) PLEKHA1; (iv) PDE8A; (v) TCF7; (vi) MGC29506; (vii) MSI2; and (viii) one gene selected from the group consisting of MGAT4A, NELL2, BCL11B, CD247, SORL1, KLRB1, IL7R, RUNX2, MAL, RASGRF2, TIAM1, PRKCH, FNIP2, GIMAP7, PAG1, ITK, IL2RB, SATB1, PRKCQ, TRAT1, CLEC4A; or (b) at least two genes selected from the group consisting of (i) to (v): (i) SFTPB; (ii) PLEKHA1; (iii) MGC29506; (iv) MSI2; and (v) one gene selected from the group consisting of MGAT4A, NELL2, BCL11B, CD247, SORL1, KLRB1, IL7R, RUNX2, MAL, RASGRF2, TIAM1, PRKCH, FNIP2, GIMAP7, PAG1, ITK, IL2RB, SATB1, PRKCQ, TRAT1, CLEC4A. A diagnostic result may be obtained from these expression levels as described in detail below in accordance with the method of the present invention.

In a preferred embodiment of any of the kits of the present invention, the nucleic acid molecules are provided on a solid support.

A "solid support" according to the invention provides a surface for the attachment of the nucleic acid molecules. Said surface according to the invention may be any surface. The surface may be a coating applied to the support or carrier, or the surface of the support or carrier itself may be used. Support or carrier materials commonly used in the art and comprising glass, plastic, gold and silicon are envisaged for the purpose of the present invention. Coatings according to the invention, if present, include poly-L-lysine- and amino-silane-coatings as well as epoxy- and aldehyde-activated surfaces. Non-limiting examples of solid phase supports are beads, chips and membranes.

In a more preferred embodiment, the solid support is a DNA chip. Methods for the production of DNA chips as well as the use of DNA chip technology are well known in the art and described e.g. in Lohara et al., Nucleic Acids Res. 30 (2002) e87; Flavell et al., Nucleic Acids Res. 31 (2003) e115; Gunderson et al., Nature Genetics 37 (2005) 549-554. Such a DNA chip is easy to manufacture due to the small amount of genes present thereon in accordance with the present invention. Thus, they are more cost-and time-effective to prepare and use than DNA chips presently available in the art.

The present invention further relates to a method of diagnosing a patient suffering from chronic lymphocytic leukemia as being a poor prognosis or good prognosis patient comprising (a) determining in a sample obtained from the patient the expression level of at least two genes selected from the group consisting of (i) to (viii): (i) NRIP1; (ii) SFTPB; (iii) PLEKHA1; (iv) PDE8A; (v) TCF7; (vi) MGC29506; (vii) MSI2; and (viii) one gene selected from the group consisting of MGAT4A, NELL2, BCL11B, CD247, SORL1, KLRB1, IL7R, RUNX2, MAL, RASGRF2, TIAM1, PRKCH, FNIP2, GIMAP7, PAG1, ITK, IL2RB, SATB1, PRKCQ, TRAT1, CLEC4A; (b) normalising the expression levels determined in (a); (c) multiplying each normalised expression level obtained in (b) with a gene-specific weight as shown in Table 1; and (d) adding up the individual values obtained for each gene in (c) to derive a prognostic score; wherein a prognostic score below a reference score, said reference score being characteristic of normal disease progression, is indicative of a poor prognosis and a prognostic score significantly above the prognostic score is indicative of a good prognosis.

The "sample obtained form the patient" can be selected from blood, serum, plasma, lymph nodes, bone marrow, faeces, fetal tissue, saliva, urine, mucosal tissue, mucus, vaginal tissue, skin, hair, hair follicle or other human tissue. Preferably, the sample is selected from blood, lymph nodes, bone marrow.

The term "expression level", as used herein, refers to a quantitative value of expression of a particular gene in a sample of interest. The expression level of a gene corresponds to the number of copies of the expressed gene, either on a nucleic acid level (e.g. mRNA) or on the protein level. Thus, the determination of the expression level of a particular gene can, for example, be carried out on the nucleic acid level or on the level of the respective protein encoded by said gene.

Methods for determining expression levels on the nucleic acid level include, but are not limited to, Northern blotting, PCR, RT-PCR or real RT-PCR. PCR is well known in the art and is employed to make large numbers of copies of a target sequence. This is done on an automated cycler device, which can heat and cool containers with the reaction mixture in a very short time. The PCR, generally, consists of many repetitions of a cycle which consists of: (a) a denaturing step, which melts both strands of a DNA molecule and terminates all previous enzymatic reactions; (b) an annealing step, which is aimed at allowing the primers to anneal specifically to the melted strands of the DNA molecule; and (c) an extension step, which elongates the annealed primers by using the information provided by the template strand. Generally, PCR can be performed, for example, in a 50 µl reaction mixture containing 5 µl of 10 x PCR buffer with 1.5 mM MgCl₂, 200 µM of each deoxynucleoside triphosphate, 0.5 µl of each primer (10 µM), about 10 to 100ng of template DNA and 1 to 2.5 units of Taq Polymerase. The primers for the amplification may be labeled or be unlabeled. DNA amplification can be performed, e.g., with a model 2400 thermal cycler (Applied Biosystems, Foster City, CA): 2 min at 94°C, followed by 30 to 40 cycles consisting of annealing (e. g. 30 s at 50°C), extension (e. g. 1 min at 72°C, depending on the length of DNA template and the enzyme used), denaturing (e. g. 10 s at 94°C) and a final annealing step at 55°C for 1 min as well as a final extension step at 72°C for 5 min. Suitable polymerases for use with a DNA template include, for example, E. coli DNA polymerase I or its Klenow fragment, T4 DNA polymerase, Tth polymerase, Taq polymerase, a heat-stable DNA polymerase isolated from Thermus aquaticus Vent, Amplitaq, Pfu and KOD, some of which may exhibit proof-reading function and/or different temperature optima. However, the person skilled in the art knows how to optimize PCR conditions for the amplification of specific nucleic acid molecules with primers of different length and/or composition or to scale down or increase the volume of the reaction mix. The "reverse transcriptase polymerase chain reaction" (RT-PCR) is used when the nucleic acid to be amplified consists of RNA. The term "reverse transcriptase" refers to an enzyme that catalyzes the polymerization of deoxyribonucleoside triphosphates to form primer extension products that are complementary to a ribonucleic acid template. The enzyme initiates synthesis at the 3'-end of the primer and proceeds toward the 5'-end of the template until synthesis terminates. Examples of suitable polymerizing agents that convert the RNA target sequence into a complementary, copy-DNA (cDNA) sequence are avian myeloblastosis virus reverse transcriptase and Thermus thermophilus DNA polymerase, a thermostable DNA polymerase with reverse transcriptase activity marketed by Perkin Elmer. Typically, the genomic RNA/cDNA duplex template is heat denatured during the first denaturation step after the initial reverse transcription step leaving the DNA strand available as an amplification template. High-temperature RT provides greater primer specificity and improved efficiency. U.S. patent application Serial No. 07/746, 121, filed Aug. 15, 1991, describes a "homogeneous RT-PCR" in which the same primers and polymerase suffice for both the reverse transcription and the PCR amplification steps, and the reaction conditions are optimized so that both reactions occur without a change of reagents. Thermus thermophilus DNA polymerase, a thermostable DNA polymerase that can function as a reverse transcriptase, can be used for all primer extension steps, regardless of template. Both processes can be done without having to open the tube to change or add reagents; only the temperature profile is adjusted between the first cycle (RNA template) and the rest of the amplification cycles (DNA template). The RT Reaction can be performed, for example, in a 20µl reaction mix containing: 4 µl of 5x AMV-RT buffer, 2 µl of Oligo dT (100 µg/ml), 2µl of 10 mM dNTPs, 1µl total RNA, 10 Units of AMV reverse transcriptase, and H₂O to 20µl final volume. The reaction may be, for example, performed by using the following conditions: The reaction is held at 70 C° for 15 minutes to allow for reverse transcription. The reaction temperature is then raised to 95 C° for 1 minute to denature the RNA-cDNA duplex. Next, the reaction temperature undergoes two cycles of 95°C for 15 seconds and 60 C° for 20 seconds followed by 38 cycles of 90 C° for 15 seconds and 60 C° for 20 seconds. Finally, the reaction temperature is held at 60 C° for 4 minutes for the final extension step, cooled to 15 C°, and held at that temperature until further processing of the amplified sample. Any of the above mentioned reaction conditions may be scaled up according to the needs of the particular case.

The resulting products are loaded onto an agarose gel and band intensities are compared after staining the nucleic acid molecules with an intercalating dye such as ethidiumbromide or SybrGreen.

Real-time PCR employs a specific probe, in the art also referred to as TaqMan probe, which has a reporter dye covalently attached at the 5' end and a quencher at the 3' end. After the TaqMan probe has been hybridized in the annealing step of the PCR reaction to the complementary site of the polynucleotide being amplified, the 5' fluorophore is cleaved by the 5' nuclease activity of Taq polymerase in the extension phase of the PCR reaction. This enhances the fluorescence of the 5' donor, which was formerly quenched due to the close proximity to the 3' acceptor in the TaqMan probe sequence. Thereby, the process of amplification can be monitored directly and in real time, which permits a significantly more precise determination of expression levels than conventional end-point PCR. Also of use in Real time RT-PCR experiments is a DNA intercalating dye such as SybrGreen for monitoring the de novo synthesis of double stranded DNA molecules.

Methods for the determination of expression levels of a protein on the amino acid level include but are not limited to Western blotting or polyacrylamide gel electrophoresis in conjunction with protein staining techniques such as Coomassie Brilliant blue or silver-staining. The total protein is loaded onto a polyacrylamide gel and electrophoresed. Afterwards, the separated proteins are transferred onto a membrane, e.g. a polyvinyldifluoride (PVDF) membrane, by applying an electrical current. The proteins on the membrane are exposed to an antibody specifically recognizing the protein of interest. After washing, a second antibody specifically recognizing the first antibody and carrying a readout system such as a fluorescent dye is applied. The amount of the protein of interest is determined by comparing the fluorescence intensity of the protein derived from a sample of the patient of interest with the fluorescence intensity of the protein derived from a control sample. Also of use in protein quantification is the Agilent Bioanalyzer technique.

In accordance with the present invention, the term "normalising the expression levels" relates to a correction of the measured value. This correction is usually carried out in order to control for bias introduced during the process of sample collection and analysis, which can for example arise due to variations based on different laboratories and/or different machines used, due to differences between extraction protocols that may co-purify inhibitors, and due to different reverse transcription and PCR efficiencies. Importantly, normalisation enables a direct comparison of values obtained from individual patients.

Several strategies for normalisation in case of quantitative PCR measurement are known in the art and include, without being limiting, normalising against the expression level(s) of (an) internal reference gene(s), which is determined in the same sample, normalisation against sample size, normalisation against total RNA amount or genomic DNA amount or normalisation against an artificially introduced molecule of known amount. Normalisation is preferably achieved by dividing the expression values from the gene to be investigated by the expression values of a reference gene. This is particularly preferred if the expression values are given in a linear scale. If the expression values are expressed in a logarithmic scale (e.g. as CT values) normalisation is achieved by subtracting the expression value of the reference gene from the expression value of the gene of interest.

In case of microarray analysis normalisation techniques including, without being limiting, RMA, GCRMA, MAS5, dChip and VSN and others could be used to process raw data to achieve comparability. Details of these methods can be found in Stafford (2008) "Methods in microarray Normalisation" (ISBN-13: 978-1420052787) and Quakenbush Nat. Gene. 2002)³⁵.

In accordance with the present invention, the term "reference score" relates to a score that represents the average score obtained for patients that are neither poor prognosis nor good prognosis patients. Said reference score may be obtained from an individual CLL patient with normal disease progression (i.e. neither falling into the category of poor prognosis nor good prognosis patient). Alternatively, a mean, i.e. average, score can be determined based on a CLL patient cohort comprising patients with poor prognosis, good prognosis as well as normal disease progression without any bias towards one of these groups. Such un-biased patient cohorts will be available to hospitals and can be analysed to derive the reference score. Preferably, a prognostic score significantly below the reference score is indicative of a poor prognosis and a prognostic score significantly above the reference score is indicative of a good prognosis.

In accordance with this preferred embodiment of the present invention, the selection of more than one gene from group (viii) is only envisaged in case that more than two genes are selected. Otherwise, only one of the genes listed in group (viii) is to be selected. In other words, at least one gene has to be selected from groups (i) to (vii).

In a preferred embodiment of the method of the invention, the normalisation in (b) is against the expression level of at least one reference gene.

In a more preferred embodiment, multiple reference genes are used for normalisation.

If multiple reference genes are employed, it is preferred that the normalisation is against the geometric mean of the expression levels of these multiple reference genes.

It is well known in the art how to determine the geometric mean of a set of values, such as expression levels. In a set of n values, the numbers are multiplied and then the nth root of the resulting product is taken. As an example, the geometric mean of two numbers is the square root of their product while the geometric mean of three numbers is the cube root of their product etc.

In a more preferred embodiment, the at least one reference gene is selected from the group consisting of GAPDH, RPLP0 and PGK1.

In a further more preferred embodiment, the normalisation in (b) is against the geometric mean of the expression levels of the three reference genes GAPDH, RPLPO and PGK1.

The normalised expression levels obtained in (b) are then each multiplied with a gene-specific weight as shown in Table 1.

Each weight represents the individual importance of the corresponding gene in the prognostic model. The number and composition of the selected combination of genes determine the level of the specific weight factor due to their individual influence and importance in predicting prognosis of the selected combination. The prognostic score is therefore obtained as a weighted sum of these gene expressions adjusted to their individual importance. The weights are calculated with the first principal component of the supervised principal component analysis following the procedure proposed by Bair and Tibshirani ("superPC method") ³⁸.

The weights for each possible combination of genes to be determined by the method of the invention are shown in table 1. In the left column, the combinations of genes are shown. The second column indicates the weights, in the order of the genes as represented in column 1. These weights are applicable independently of which reference gene(s) is (are) used for the normalisation of expression levels. However the score limits are dependent on normalisation and are here given normalised against the geometric mean of the expression levels of the three reference genes GAPDH, RPLP0 and PGK1. For genes of group (viii) other than MGAT4A the weight as shown for MGAT4A is applicable, as all the genes of group (viii) are correlated.

**Table 1: Gene-specific weights as applicable for particular genes when measured in the combinations indicated in the left column.**

| **Genes** | **Weights^{(a)}** | **Lower score limit^{(b)}** | **Upper score limit^{(b)}** |
|---|---|---|---|
| NRIP1, TCF7 | -0.76, -0.24 | -8.396 | -5.465 |
| NRIP1, PDE8A | -0.717, -0.283 | -8.312 | -5.618 |
| NRIP1, MGC29506 | -0.75,0.25 | -6.385 | -3.233 |
| NRIP1, MGAT4A | -0.611, -0.389 | -9.16 | -5.828 |
| NRIP1, PLEKHA1 | -0.739, -0.261 | -8.411 | -5.594 |
| NRIP1, MSI2 | -0.814, 0.186 | -7.476 | -4.503 |
| NRIP1, SFTPB | -0.526, 0.474 | -2.691 | 1.275 |
| TCF7, PDE8A | -0.425, -0.575 | -5.333 | -3.451 |
| TCF7, MGC29506 | -0.473, 0.527 | -0.876 | 1.423 |
| TCF7, MGAT4A | -0.333, -0.667 | -7.528 | -4.403 |
| TCF7, PLEKHA1 | -0.363, -0.637 | -5.481 | -3.189 |
| TCF7, MSI2 | -0.509, 0.491 | -2.106 | -0.344 |
| TCF7, SFTPB | -0.183, 0.817 | 2.71 | 8.045 |
| PDE8A, MGC29506 | -0.551, 0.449 | -1.399 | 0.412 |
| PDE8A, MGAT4A | -0.325, -0.675 | -7.213 | -4.712 |
| PDE8A, PLEKHA1 | -0.45, -0.55 | -5.419 | -3.44 |
| PDE8A, MSI2 | -0.617, 0.383 | -2.762 | -1.362 |
| PDE8A, SFTPB | -0.247, 0.753 | 2.164 | 6.677 |
| MGC29506, MGAT4A | 0.303, -0.697 | -4.833 | -1.724 |
| MGC29506, PLEKHA1 | 0.377, -0.623 | -2.13 | -0.128 |
| MGC29506, MSI2 | 0.542, 0.458 | 2.241 | 4.424 |
| MGC29506, SFTPB | 0.146, 0.854 | 4.447 | 9.714 |
| MGAT4A, PLEKHA1 | -0.594, -0.406 | -6.998 | -4.513 |
| MGAT4A, MSI2 | -0.707, 0.293 | -5.613 | -2.598 |
| MGAT4A, SFTPB | -0.349, 0.651 | 0.477 | 4.838 |
| PLEKHA1, MSI2 | -0.668, 0.332 | -3.127 | -1.532 |
| PLEKHA1, SFTPB | -0.261, 0.739 | 2.134 | 6.697 |
| MSI2, SFTPB | 0.217, 0.783 | 3.909 | 8.9 |
| NRIP1, TCF7, PDE8A | -0.568, -0.196, -0.237 | -7.619 | -5.194 |
| NRIP1, TCF7, MGC29506 | -0.594, -0.198, 0.208 | -5.975 | -3.217 |
| NRIP1, TCF7, MGAT4A | -0.485, -0.189, -0.326 | -8.455 | -5.454 |
| NRIP1, TCF7, PLEKHA1 | -0.578, -0.202, -0.22 | -7.692 | -5.187 |
| NRIP1, TCF7, MSI2 | -0.632, -0.21, 0.158 | -6.859 | -4.222 |
| NRIP1, TCF7, SFTPB | -0.449, -0.163, 0.388 | -3.217 | 0.365 |
| NRIP1, PDE8A, MGC29506 | -0.56, -0.236, 0.204 | -5.909 | -3.359 |
| NRIP1, PDE8A, MGAT4A | -0.491, -0.2, -0.309 | -8.506 | -5.458 |
| NRIP1, PDE8A, PLEKHA1 | -0.539, -0.24, -0.22 | -7.592 | -5.152 |
| NRIP1, PDE8A, MSI2 | -0.605, -0.245, 0.15 | -6.893 | -4.389 |
| NRIP1, PDE8A, SFTPB | -0.429, -0.198, 0.372 | -3.382 | 0.167 |
| NRIP1, MGC29506, MGAT4A | -0.497, 0.182, -0.32 | -6.907 | -3.814 |
| NRIP1, MGC29506, PLEKHA1 | -0.575, 0.208, -0.217 | -5.937 | -3.376 |
| NRIP1, MGC29506, MSI2 | -0.626, 0.218, 0.156 | -5.237 | -2.316 |
| NRIP1, MGC29506, SFTPB | -0.458, 0.157, 0.385 | -1.886 | 1.729 |
| NRIP1, MGAT4A, PLEKHA1 | -0.473, -0.324, -0.203 | -8.292 | -5.478 |
| NRIP1, MGAT4A, MSI2 | -0.511, -0.339, 0.15 | -7.652 | -4.538 |
| NRIP1, MGAT4A, SFTPB | -0.406, -0.267, 0.327 | -4.458 | -0.77 |
| NRIP1, PLEKHA1, MSI2 | -0.611, -0.229, 0.16 | -6.877 | -4.418 |
| NRIP1, PLEKHA1, SFTPB | -0.428, -0.186, 0.386 | -3.04 | 0.36 |
| NRIP1, MSI2, SFTPB | -0.442, 0.15, 0.408 | -1.965 | 1.571 |
| TCF7, PDE8A, MGC29506 | -0.291, -0.39, 0.319 | -2.488 | -0.606 |
| TCF7, PDE8A, MGAT4A | -0.267, -0.252, -0.481 | -6.681 | -4.286 |
| TCF7, PDE8A, PLEKHA1 | -0.256, -0.345, -0.399 | -5.314 | -3.426 |
| TCF7, PDE8A, MSI2 | -0.324, -0.409, 0.267 | -3.495 | -1.962 |
| TCF7, PDE8A, SFTPB | -0.168, -0.224, 0.608 | 0.891 | 4.763 |
| TCF7, MGC29506, MGAT4A | -0.265, 0.23, -0.504 | -5.05 | -2.222 |
| TCF7, MGC29506, PLEKHA1 | -0.278, 0.288, -0.434 | -2.872 | -0.836 |
| TCF7, MGC29506, MSI2 | -0.334, 0.359, 0.307 | -0.257 | 1.695 |
| TCF7, MGC29506, SFTPB | -0.173, 0.143, 0.684 | 2.773 | 7.331 |
| TCF7, MGAT4A, PLEKHA1 | -0.24, -0.454, -0.306 | -6.632 | -4.154 |
| TCF7, MGAT4A, MSI2 | -0.264, -0.516, 0.22 | -5.644 | -2.911 |
| TCF7, MGAT4A, SFTPB | -0.182, -0.318, 0.5 | -0.932 | 2.799 |
| TCF7, PLEKHA1, MSI2 | -0.287, -0.459, 0.255 | -3.717 | -1.938 |
| TCF7, PLEKHA1, SFTPB | -0.166, -0.236, 0.598 | 0.938 | 4.791 |
| TCF7, MSI2, SFTPB | -0.161, 0.193, 0.646 | 2.39 | 6.789 |
| PDE8A, MGC29506, MGAT4A | -0.27, 0.252, -0.478 | -4.528 | -2.237 |
| PDE8A, MGC29506, PLEKHA1 | -0.345, 0.267, -0.388 | -3.031 | -1.221 |
| PDE8A, MGC29506, MSI2 | -0.403, 0.342, 0.255 | -0.627 | 0.931 |
| PDE8A, MGC29506, SFTPB | -0.231, 0.148, 0.621 | 2.145 | 6.236 |
| PDE8A, MGAT4A, PLEKHA1 | -0.25, -0.424, -0.325 | -6.495 | -4.416 |
| PDE8A, MGAT4A, MSI2 | -0.256, -0.514, 0.23 | -5.292 | -3.077 |
| PDE8A, MGAT4A, SFTPB | -0.201, -0.292, 0.508 | -0.802 | 2.845 |
| PDE8A, PLEKHA1, MSI2 | -0.354, -0.428, 0.218 | -3.831 | -2.264 |
| PDE8A, PLEKHA1, SFTPB | -0.221, -0.234, 0.545 | 0.269 | 3.916 |
| PDE8A, MSI2, SFTPB | -0.21, 0.182, 0.608 | 2.047 | 5.917 |
| MGC29506, MGAT4A, PLEKHA1 | 0.219, -0.459, -0.321 | -4.64 | -2.388 |
| MGC29506, MGAT4A, MSI2 | 0.241, -0.526, 0.232 | -3.359 | -0.538 |
| MGC29506, MGAT4A, SFTPB | 0.148, -0.318, 0.533 | 0.701 | 4.568 |
| MGC29506, PLEKHA1, MSI2 | 0.295, -0.452, 0.253 | -1.099 | 0.676 |
| MGC29506, PLEKHA1, SFTPB | 0.142, -0.241, 0.617 | 2.195 | 6.254 |
| MGC29506, MSI2, SFTPB | 0.135, 0.198, 0.667 | 3.79 | 8.254 |
| MGAT4A, PLEKHA1, MSI2 | -0.471, -0.321, 0.208 | -5.267 | -3.064 |
| MGAT4A, PLEKHA1, SFTPB | -0.301, -0.229, 0.47 | -1.188 | 2.39 |
| MGAT4A, MSI2, SFTPB | -0.301, 0.185, 0.514 | 0.487 | 4.184 |
| PLEKHA1, MSI2, SFTPB | -0.224, 0.183, 0.592 | 1.899 | 5.83 |
| NRIP1, TCF7, PDE8A, MGC29506 | -0.458, -0.165, -0.202, 0.174 | -5.725 | -3.293 |
| NRIP1, TCF7, PDE8A, MGAT4A | -0.401, -0.163, -0.171, -0.266 | -7.695 | -5.217 |
| NRIP1, TCF7, PDE8A, PLEKHA1 | -0.44, -0.166, -0.205, -0.189 | -7.156 | -4.946 |
| NRIP1, TCF7, PDE8A, MSI2 | -0.487, -0.175, -0.208, 0.13 | -6.483 | -4.186 |
| NRIP1, TCF7, PDE8A, SFTPB | -0.371, -0.142, -0.176, 0.31 | -3.639 | -0.486 |
| NRIP1, TCF7, MGC29506, MGAT4A | -0.407, -0.163, 0.155, -0.275 | -6.629 | -3.704 |
| NRIP1, TCF7, MGC29506, PLEKHA1 | -0.466, -0.17, 0.176, -0.188 | -5.787 | -3.388 |
| NRIP1, TCF7, MGC29506, MSI2 | -0.505, -0.176, 0.184, 0.135 | -5.065 | -2.388 |
| NRIP1, TCF7, MGC29506, SFTPB | -0.394, -0.146, 0.14, 0.319 | -2.454 | 0.99 |
| NRIP1, TCF7, MGAT4A, PLEKHA1 | -0.388, -0.162, -0.277, -0.174 | -7.868 | -5.095 |
| NRIP1, TCF7, MGAT4A, MSI2 | -0.415, -0.168, -0.288, 0.129 | -7.23 | -4.333 |
| NRIP1, TCF7, MGAT4A, SFTPB | -0.347, -0.143, -0.238, 0.271 | -4.778 | -1.454 |
| NRIP1, TCF7, PLEKHA1, MSI2 | -0.488, -0.178, -0.196, 0.137 | -6.491 | -4.142 |
| NRIP1, TCF7, PLEKHA1, SFTPB | -0.37, -0.144, -0.166, 0.32 | -3.399 | -0.335 |
| NRIP1, TCF7, MSI2, SFTPB | -0.383, -0.145, 0.133, 0.339 | -2.501 | 0.641 |
| NRIP1, PDE8A, MGC29506, MGAT4A | -0.407, -0.175, 0.158, -0.26 | -6.414 | -3.792 |
| NRIP1, PDE8A, MGC29506, PLEKHA1 | -0.437, -0.205, 0.172, -0.186 | -5.672 | -3.446 |
| NRIP1, PDE8A, MGC29506, MSI2 | -0.482, -0.208, 0.182, 0.128 | -4.897 | -2.502 |
| NRIP1, PDE8A, MGC29506, SFTPB | -0.376, -0.179, 0.139, 0.306 | -2.547 | 0.756 |
| NRIP1, PDE8A, MGAT4A, PLEKHA1 | -0.386, -0.175, -0.261, -0.178 | -7.645 | -5.271 |
| NRIP1, PDE8A, MGAT4A, MSI2 | -0.422, -0.176, -0.276, 0.127 | -7.206 | -4.5 |
| NRIP1, PDE8A, MGAT4A, SFTPB | -0.345, -0.157, -0.224, 0.273 | -4.759 | -1.352 |
| NRIP1, PDE8A, PLEKHA1, MSI2 | -0.463, -0.211, -0.197, 0.129 | -6.373 | -4.196 |
| NRIP1, PDE8A, PLEKHA1, SFTPB | -0.353, -0.177, -0.165, 0.306 | -3.555 | -0.496 |
| NRIP1, PDE8A, MSI2, SFTPB | -0.37, -0.175, 0.126, 0.329 | -2.743 | 0.52 |
| NRIP1, MGC29506, MGAT4A, PLEKHA1 | -0.397, 0.155, -0.273, -0.175 | -6.537 | -3.785 |
| NRIP1, MGC29506, MGAT4A, MSI2 | -0.425, 0.161, -0.284, 0.13 | -5.73 | -2.906 |
| NRIP1, MGC29506, MGAT4A, SFTPB | -0.358, 0.132, -0.237, 0.273 | -3.643 | -0.162 |
| NRIP1, MGC29506, PLEKHA1, MSI2 | -0.487, 0.183, -0.194, 0.136 | -4.908 | -2.503 |
| NRIP1, MGC29506, PLEKHA1, SFTPB | -0.377, 0.139, -0.167, 0.318 | -2.389 | 0.809 |
| NRIP1, MGC29506, MSI2, SFTPB | -0.39, 0.139, 0.134, 0.337 | -1.379 | 1.928 |
| NRIP1, MGAT4A, PLEKHA1, MSI2 | -0.404, -0.286, -0.181, 0.129 | -7.083 | -4.38 |
| NRIP1, MGAT4A, PLEKHA1, SFTPB | -0.335, -0.234, -0.159, 0.272 | -4.534 | -1.35 |
| NRIP1, MGAT4A, MSI2, SFTPB | -0.348, -0.237, 0.127, 0.288 | -3.746 | -0.252 |
| NRIP1, PLEKHA1, MSI2, SFTPB | -0.366, -0.165, 0.131, 0.337 | -2.502 | 0.616 |
| TCF7, PDE8A, MGC29506, MGAT4A | -0.215, -0.217, 0.197, -0.371 | -4.645 | -2.482 |
| TCF7, PDE8A, MGC29506, PLEKHA1 | -0.206, -0.276, 0.216, -0.301 | -3.434 | -1.564 |
| TCF7, PDE8A, MGC29506, MSI2 | -0.239, -0.305, 0.257, 0.199 | -1.72 | -0.075 |
| TCF7, PDE8A, MGC29506, SFTPB | -0.156, -0.208, 0.14, 0.495 | 0.987 | 4.516 |
| TCF7, PDE8A, MGAT4A, PLEKHA1 | -0.198, -0.206, -0.342, -0.254 | -6.251 | -4.146 |
| TCF7, PDE8A, MGAT4A, MSI2 | -0.221, -0.209, -0.393, 0.178 | -5.289 | -3.087 |
| TCF7, PDE8A, MGAT4A, SFTPB | -0.162, -0.177, -0.265, 0.396 | -1.803 | 1.257 |
| TCF7, PDE8A, PLEKHA1, MSI2 | -0.217, -0.28, -0.324, 0.179 | -4.119 | -2.436 |
| TCF7, PDE8A, PLEKHA1,SFTPB | -0.149, -0.199, -0.209, 0.443 | -0.699 | 2.336 |
| TCF7, PDE8A, MSI2, SFTPB | -0.149, -0.192, 0.161, 0.498 | 0.871 | 4.296 |
| TCF7, MGC29506, MGAT4A, PLEKHA1 | -0.201, 0.179, -0.367, -0.252 | -4.823 | -2.542 |
| TCF7, MGC29506, MGAT4A, MSI2 | -0.218, 0.193, -0.407, 0.182 | -3.743 | -1.181 |
| TCF7, MGC29506, MGAT4A, SFTPB | -0.167, 0.136, -0.287, 0.41 | -0.71 | 2.788 |
| TCF7, MGC29506, PLEKHA1, MSI2 | -0.229, 0.234, -0.336, 0.201 | -1.94 | -0.164 |
| TCF7, MGC29506, PLEKHA1, SFTPB | -0.155, 0.135, -0.216, 0.494 | 0.977 | 4.519 |
| TCF7, MGC29506, MSI2, SFTPB | -0.153,0.131,0.175,0.541 | 2.447 | 6.254 |
| TCF7, MGAT4A, PLEKHA1, MSI2 | -0.202, -0.377, -0.253, 0.168 | -5.306 | -3.117 |
| TCF7, MGAT4A, PLEKHA1, SFTPB | -0.157, -0.271, -0.2, 0.372 | -2.106 | 1.038 |
| TCF7, MGAT4A, MSI2, SFTPB | -0.158, -0.275, 0.16, 0.408 | -0.8 | 2.57 |
| TCF7, PLEKHA1, MSI2, SFTPB | -0.147, -0.204, 0.163, 0.486 | 0.802 | 4.131 |
| PDE8A, MGC29506, MGAT4A, PLEKHA1 | -0.215, 0.186, -0.337, -0.262 | -4.544 | -2.489 |
| PDE8A, MGC29506, MGAT4A, MSI2 | -0.22, 0.208, -0.389, 0.183 | -3.485 | -1.23 |
| PDE8A, MGC29506, MGAT4A, SFTPB | -0.185, 0.14, -0.263, 0.412 | -0.434 | 2.763 |
| PDE8A, MGC29506, PLEKHA1, MSI2 | -0.282, 0.225, -0.317, 0.176 | -2.12 | -0.517 |
| PDE8A, MGC29506, PLEKHA1, SFTPB | -0.204, 0.136, -0.212, 0.447 | 0.482 | 3.776 |
| PDE8A, MGC29506, MSI2, SFTPB | -0.197, 0.135, 0.164, 0.505 | 1.985 | 5.616 |
| PDE8A, MGAT4A, PLEKHA1, MSI2 | -0.209, -0.356, -0.269, 0.166 | -5.081 | -3.238 |
| PDE8A, MGAT4A, PLEKHA1, SFTPB | -0.176, -0.25, -0.203, 0.371 | -1.952 | 0.965 |
| PDE8A, MGAT4A, MSI2, SFTPB | -0.171, -0.256, 0.157, 0.416 | -0.547 | 2.626 |
| PDE8A, PLEKHA1, MSI2, SFTPB | -0.19, -0.204, 0.152, 0.454 | 0.359 | 3.561 |
| MGC29506, MGAT4A, PLEKHA1, MSI2 | 0.185, -0.378, -0.263, 0.174 | -3.59 | -1.454 |
| MGC29506, MGAT4A, PLEKHA1, SFTPB | 0.133, -0.272, -0.207, 0.388 | -0.966 | 2.39 |
| MGC29506, MGAT4A, MSI2, SFTPB | 0.132, -0.275, 0.166, 0.427 | 0.617 | 3.975 |
| MGC29506, PLEKHA1, MSI2, SFTPB | 0.129, -0.208, 0.166, 0.497 | 1.95 | 5.492 |
| MGAT4A, PLEKHA1, MSI2, SFTPB | -0.262, -0.196, 0.153, 0.388 | -1.008 | 2.233 |
| NRIP1, TCF7, PDE8A, MGC29506, MGAT4A | -0.341, -0.142, -0.152, 0.137, -0.228 | -6.199 | -3.769 |
| NRIP1, TCF7, PDE8A, MGC29506, PLEKHA1 | -0.367, -0.143, -0.178, 0.149, -0.163 | -5.541 | -3.4 |
| NRIP1, TCF7, PDE8A, MGC29506, MSI2 | -0.4, -0.15, -0.18, 0.157, 0.113 | -4.866 | -2.582 |
| NRIP1, TCF7, PDE8A, MGC29506, SFTPB | -0.328, -0.128, -0.16, 0.125, 0.259 | -2.909 | 0.18 |
| NRIP1, TCF7, PDE8A, MGAT4A, PLEKHA1 | -0.325, -0.14, -0.152, -0.228, -0.155 | -7.323 | -4.912 |
| NRIP1, TCF7, PDE8A, MGAT4A, MSI2 | -0.351, -0.146, -0.153, -0.24, 0.111 | -6.75 | -4.312 |
| NRIP1, TCF7, PDE8A, MGAT4A, SFTPB | -0.299, -0.127, -0.14, -0.202, 0.231 | -4.79 | -1.83 |
| NRIP1, TCF7, PDE8A, PLEKHA1, MSI2 | -0.383, -0.15, -0.182, -0.171, 0.114 | -6.181 | -4.086 |
| NRIP1, TCF7, PDE8A, PLEKHA1, SFTPB | -0.309, -0.126, -0.158, -0.148, 0.259 | -3.762 | -1.051 |
| NRIP1, TCF7, PDE8A, MSI2, SFTPB | -0.325, -0.128, -0.157, 0.113, 0.278 | -3.073 | -0.114 |
| NRIP1, TCF7, MGC29506, MGAT4A, PLEKHA1 | -0.333, -0.142, 0.134, -0.238, -0.153 | -6.397 | -3.718 |
| NRIP1, TCF7, MGC29506, MGAT4A, MSI2 | -0.354, -0.146, 0.139, -0.247, 0.113 | -5.818 | -2.994 |
| NRIP1, TCF7, MGC29506, MGAT4A, SFTPB | -0.31, -0.13, 0.118, -0.213, 0.231 | -3.925 | -0.715 |
| NRIP1, TCF7, MGC29506, PLEKHA1, MSI2 | -0.402, -0.153, 0.157, -0.169, 0.119 | -5.039 | -2.552 |
| NRIP1, TCF7, MGC29506, PLEKHA1, SFTPB | -0.328, -0.13, 0.125, -0.149, 0.267 | -2.743 | 0.194 |
| NRIP1, TCF7, MGC29506, MSI2, SFTPB | -0.341, -0.131, 0.126, 0.119, 0.283 | -2.022 | 1.189 |
| NRIP1, TCF7, MGAT4A, PLEKHA1, MSI2 | -0.338, -0.145, -0.248, -0.157, 0.112 | -6.844 | -4.246 |
| NRIP1, TCF7, MGAT4A, PLEKHA1, SFTPB | -0.292, -0.127, -0.21, -0.141, 0.23 | -4.732 | -1.79 |
| NRIP1, TCF7, MGAT4A, MSI2, SFTPB | -0.303, -0.129, -0.214, 0.112, 0.243 | -4.116 | -0.913 |
| NRIP1, TCF7, PLEKHA1, MSI2, SFTPB | -0.321, -0.129, -0.149, 0.117, 0.284 | -2.902 | 0.008 |
| NRIP1, PDE8A, MGC29506, MGAT4A, PLEKHA1 | -0.33, -0.155, 0.136, -0.224, -0.156 | -6.123 | -3.81 |
| NRIP1, PDE8A, MGC29506, MGAT4A, MSI2 | -0.356, -0.156, 0.143, -0.235, 0.111 | -5.599 | -3.083 |
| NRIP1, PDE8A, MGC29506, MGAT4A, SFTPB | -0.306, -0.144, 0.119, -0.2, 0.231 | -3.882 | -0.745 |
| NRIP1, PDE8A, MGC29506, PLEKHA1, MSI2 | -0.382, -0.182, 0.155, -0.169, 0.112 | -4.809 | -2.72 |
| NRIP1, PDE8A, MGC29506, PLEKHA1, SFTPB | -0.313, -0.16, 0.123, -0.148, 0.256 | -2.851 | 0.124 |
| NRIP1, PDE8A, MGC29506, MSI2, SFTPB | -0.328, -0.159, 0.125, 0.113, 0.274 | -2.05 | 0.977 |
| NRIP1, PDE8A, MGAT4A, PLEKHA1, MSI2 | -0.338, -0.156, -0.235, -0.161, 0.11 | -6.59 | -4.364 |
| NRIP1, PDE8A, MGAT4A, PLEKHA1, SFTPB | -0.288, -0.142, -0.198, -0.143, 0.23 | -4.539 | -1.749 |
| NRIP1, PDE8A, MGAT4A, MSI2, SFTPB | -0.302, -0.14, -0.203, 0.11, 0.245 | -3.959 | -0.958 |
| NRIP1, PDE8A, PLEKHA1, MSI2, SFTPB | -0.309, -0.157, -0.149, 0.111, 0.274 | -3.107 | -0.181 |
| NRIP1, MGC29506, MGAT4A, PLEKHA1, MSI2 | -0.345, 0.139, -0.244, -0.158, 0.114 | -5.7 | -3.052 |
| NRIP1, MGC29506, MGAT4A, PLEKHA1, SFTPB | -0.299, 0.117, -0.209, -0.143, 0.231 | -3.68 | -0.741 |
| NRIP1, MGC29506, MGAT4A, MSI2, SFTPB | -0.311, 0.118, -0.212, 0.114, 0.245 | -3.102 | 0.157 |
| NRIP1, MGC29506, PLEKHA1, MSI2, SFTPB | -0.326, 0.125, -0.15, 0.118, 0.282 | -1.926 | 1.112 |
| NRIP1, MGAT4A, PLEKHA1, MSI2, SFTPB | -0.293, -0.21, -0.142, 0.112, 0.243 | -3.956 | -0.84 |
| TCF7, PDE8A, MGC29506, MGAT4A, PLEKHA1 | -0.168, -0.181, 0.156, -0.283, -0.213 | -4.72 | -2.63 |
| TCF7, PDE8A, MGC29506, MGAT4A, MSI2 | -0.183, -0.184, 0.169, -0.315, 0.149 | -3.753 | -1.691 |
| TCF7, PDE8A, MGC29506, MGAT4A, SFTPB | -0.147, -0.163, 0.127, -0.238, 0.325 | -1.468 | 1.528 |
| TCF7, PDE8A, MGC29506, PLEKHA1, MSI2 | -0.18, -0.232, 0.186, -0.254, 0.148 | -2.651 | -1.001 |
| TCF7, PDE8A, MGC29506, PLEKHA1, SFTPB | -0.137, -0.183, 0.126, -0.189, 0.364 | -0.37 | 2.372 |
| TCF7, PDE8A, MGC29506, MSI2, SFTPB | -0.139, -0.179, 0.127, 0.144, 0.41 | 1.005 | 4.081 |
| TCF7, PDE8A, MGAT4A, PLEKHA1, MSI2 | -0.172, -0.177, -0.296, -0.217, 0.138 | -5.124 | -3.251 |
| TCF7, PDE8A, MGAT4A, PLEKHA1, SFTPB | -0.139, -0.157, -0.226, -0.178, 0.301 | -2.711 | 0.026 |
| TCF7, PDE8A, MGAT4A, MSI2, SFTPB | -0.142, -0.153, -0.234, 0.136, 0.335 | -1.451 | 1.318 |
| TCF7, PDE8A, PLEKHA1, MSI2, SFTPB | -0.133, -0.173, -0.183, 0.135, 0.375 | -0.409 | 2.308 |
| TCF7, MGC29506, MGAT4A, PLEKHA1, MSI2 | -0.173, 0.156, -0.313, -0.214, 0.144 | -3.956 | -1.798 |
| TCF7, MGC29506, MGAT4A, PLEKHA1, SFTPB | -0.144, 0.121, -0.245, -0.181, 0.31 | -1.758 | 1.285 |
| TCF7, MGC29506, MGAT4A, MSI2, SFTPB | -0.145, 0.121, -0.249, 0.143, 0.341 | -0.539 | 2.698 |
| TCF7, MGC29506, PLEKHA1, MSI2, SFTPB | -0.138, 0.122, -0.187, 0.147, 0.406 | 0.959 | 3.876 |
| TCF7, MGAT4A, PLEKHA1, MSI2, SFTPB | -0.138, -0.238, -0.174, 0.134, 0.316 | -1.763 | 1.204 |
| PDE8A, MGC29506, MGAT4A, PLEKHA1, MSI2 | -0.184, 0.162, -0.291, -0.222, 0.141 | -3.708 | -1.825 |
| PDE8A, MGC29506, MGAT4A, PLEKHA1, SFTPB | -0.162, 0.123, -0.225, -0.182, 0.308 | -1.588 | 1.255 |
| PDE8A, MGC29506, MGAT4A, MSI2, SFTPB | -0.159, 0.125, -0.232, 0.14, 0.344 | -0.264 | 2.571 |
| PDE8A, MGC29506, PLEKHA1, MSI2, SFTPB | -0.177, 0.123, -0.186, 0.137, 0.377 | 0.651 | 3.567 |
| PDE8A, MGAT4A, PLEKHA1, MSI2, SFTPB | -0.153, -0.222, -0.177, 0.131, 0.317 | -1.63 | 1.14 |
| MGC29506, MGAT4A, PLEKHA1, MSI2, SFTPB | 0.119, -0.238, -0.179, 0.138, 0.326 | -0.694 | 2.431 |
| NRIP1, TCF7, PDE8A, MGC29506, MGAT4A, PLEKHA1 | -0.283, -0.124, -0.137, 0.12, -0.199, -0.137 | -5.955 | -3.757 |
| NRIP1, TCF7, PDE8A, MGC29506, MGAT4A, MSI2 | -0.303, -0.129, -0.138, 0.125, -0.208, 0.098 | -5.469 | -3.126 |
| NRIP1, TCF7, PDE8A, MGC29506, MGAT4A, SFTPB | -0.268, -0.115, -0.129, 0.107, -0.182, 0.198 | -3.972 | -1.141 |
| NRIP1, TCF7, PDE8A, MGC29506, PLEKHA1, MSI2 | -0.325, -0.131, -0.16, 0.135, -0.149, 0.1 | -4.85 | -2.794 |
| NRIP1, TCF7, PDE8A, MGC29506, PLEKHA1, SFTPB | -0.276, -0.114, -0.144, 0.112, -0.134, 0.22 | -3.002 | -0.434 |
| NRIP1, TCF7, PDE8A, MGC29506, MSI2, SFTPB | -0.29, -0.117, -0.143, 0.114, 0.102, 0.235 | -2.459 | 0.392 |
| NRIP1, TCF7, PDE8A, MGAT4A, PLEKHA1, MSI2 | -0.289, -0.128, -0.137, -0.208, -0.141, 0.098 | -6.402 | -4.175 |
| NRIP1, TCF7, PDE8A, MGAT4A, PLEKHA1, SFTPB | -0.254, -0.113, -0.127, -0.18, -0.128, 0.198 | -4.682 | -2.101 |
| NRIP1, TCF7, PDE8A, MGAT4A, MSI2, SFTPB | -0.265, -0.116, -0.126, -0.184, 0.098, 0.21 | -4.167 | -1.378 |
| NRIP1, TCF7, PDE8A, PLEKHA1, MSI2, SFTPB | -0.274, -0.115, -0.142, -0.135, 0.1, 0.235 | -3.338 | -0.639 |
| NRIP1, TCF7, MGC29506, MGAT4A, PLEKHA1, MSI2 | -0.294, -0.128, 0.122, -0.216, -0.139, 0.1 | -5.594 | -3.042 |
| NRIP1, TCF7, MGC29506, MGAT4A, PLEKHA1, SFTPB | -0.263, -0.116, 0.105, -0.189, -0.129, 0.198 | -3.98 | -1.11 |
| NRIP1, TCF7, MGC29506, MGAT4A, MSI2, SFTPB | -0.273, -0.117, 0.107, -0.193, 0.102, 0.209 | -3.382 | -0.296 |
| NRIP1, TCF7, MGC29506, PLEKHA1, MSI2, SFTPB | -0.288, -0.118, 0.113, -0.135, 0.106, 0.24 | -2.29 | 0.39 |
| NRIP1, TCF7, MGAT4A, PLEKHA1, MSI2, SFTPB | -0.258, -0.115, -0.191, -0.128, 0.1, 0.209 | -4.098 | -1.292 |
| NRIP1, PDE8A, MGC29506, MGAT4A, PLEKHA1, MSI2 | -0.293, -0.14, 0.124, -0.204, -0.142, 0.098 | -5.351 | -3.184 |
| NRIP1, PDE8A, MGC29506, MGAT4A, PLEKHA1, SFTPB | -0.259, -0.13, 0.106, -0.178, -0.13,0.198 | -3.755 | -1.108 |
| NRIP1, PDE8A, MGC29506, MGAT4A, MSI2, SFTPB | -0.271, -0.129, 0.108, -0.182, 0.099, 0.21 | -3.236 | -0.387 |
| NRIP1, PDE8A, MGC29506, PLEKHA1, MSI2, SFTPB | -0.277, -0.144, 0.112, -0.135, 0.1, 0.232 | -2.408 | 0.327 |
| NRIP1, PDE8A, MGAT4A, PLEKHA1, MSI2, SFTPB | -0.256, -0.128, -0.18, -0.13, 0.097, 0.209 | -3.981 | -1.303 |
| NRIP1, MGC29506, MGAT4A, PLEKHA1, MSI2, SFTPB | -0.264, 0.106, -0.189, -0.13, 0.101,0.21 | -3.158 | -0.319 |
| TCF7, PDE8A, MGC29506, MGAT4A, PLEKHA1, MSI2 | -0.148, -0.158, 0.138, -0.25, -0.186,0.12 | -3.992 | -2.087 |
| TCF7, PDE8A, MGC29506, MGAT4A, PLEKHA1,SFTPB | -0.127, -0.145, 0.111, -0.204, -0.16, 0.253 | -2.197 | 0.446 |
| TCF7, PDE8A, MGC29506, MGAT4A, MSI2, SFTPB | -0.13, -0.143, 0.114, -0.211, 0.122,0.28 | -1.14 | 1.592 |
| TCF7, PDE8A, MGC29506, PLEKHA1, MSI2, SFTPB | -0.124, -0.16, 0.115, -0.167, 0.122, 0.313 | -0.136 | 2.371 |
| TCF7, PDE8A, MGAT4A, PLEKHA1, MSI2, SFTPB | -0.124, -0.138, -0.203, -0.157, 0.115, 0.263 | -2.27 | 0.214 |
| TCF7, MGC29506, MGAT4A, PLEKHA1, MSI2, SFTPB | -0.127, 0.109, -0.216, -0.158, 0.121, 0.268 | -1.362 | 1.315 |
| PDE8A, MGC29506, MGAT4A, PLEKHA1, MSI2, SFTPB | -0.142, 0.111, -0.201, -0.16, 0.118, 0.268 | -1.255 | 1.334 |
| NRIP1, TCF7, PDE8A, MGC29506, MGAT4A, PLEKHA1, MSI2 | -0.254, -0.114, -0.125, 0.11, -0.183, -0.126, 0.088 | -5.322 | -3.173 |
| NRIP1, TCF7, PDE8A, MGC29506, MGAT4A, PLEKHA1, SFTPB | -0.23, -0.103, -0.118, 0.096, -0.163, -0.117, 0.172 | -3.955 | -1.455 |
| NRIP1, TCF7, PDE8A, MGC29506, MGAT4A, MSI2, SFTPB | -0.24, -0.106, -0.117, 0.098, -0.167, 0.089, 0.182 | -3.423 | -0.795 |
| NRIP1, TCF7, PDE8A, MGC29506, PLEKHA1, MSI2, SFTPB | -0.247, -0.105, -0.13, 0.102, -0.123, 0.091, 0.201 | -2.616 | -0.164 |
| NRIP1, TCF7, PDE8A, MGAT4A, PLEKHA1, MSI2, SFTPB | -0.228, -0.104, -0.116, -0.165, -0.118, 0.088, 0.182 | -4.09 | -1.656 |
| NRIP1, TCF7, MGC29506, MGAT4A, PLEKHA1, MSI2, SFTPB | -0.235, -0.106, 0.096, -0.173, -0.117, 0.091, 0.182 | -3.469 | -0.769 |
| NRIP1, PDE8A, MGC29506, MGAT4A, PLEKHA1, MSI2, SFTPB | -0.232, -0.118, 0.097, -0.164, -0.119, 0.089, 0.182 | -3.358 | -0.796 |
| TCF7, PDE8A, MGC29506, MGAT4A, PLEKHA1, MSI2, SFTPB | -0.114, -0.128, 0.101, -0.184, -0.143, 0.104, 0.225 | -1.866 | 0.647 |

| | | | |
|---|---|---|---|
| ^{(a)} The weights are given in the order of the genes they refer to as shown in column 1, i.e. the first value refers to the first listed gene, the second value to the second gene etc. ^{(b)} These lower and upper score limits refer to values determined by PCR and using the reference genes GAPDH, RPLPO and PGK1 for normalisation, as described in the examples. | | | |

When a method different from quantitative PCR is employed or if reference genes other than GAPDH, RPLPO and PGK1 are used, some corrections are necessary to translate the obtained values into values directly comparable with the values given in table 1. Such methods are well known to the skilled person. Non-limiting examples of such adaptations are given below.

When determining the normalised expression levels according to the method of the invention using the reference genes GAPDH, RPLPO and PGK1 together but employing a method other than PCR, for example by direct sequencing of the transcriptome of cells, the expression levels of the genes and the reference genes have to be transformed analogue to the measurement of copy numbers by quantitative PCR (e.g. into a logarithmic scale (log2)) and the weights (see Table 1) can be used without adjustment.

If the skilled person used different housekeeping genes for normalisation of quantitative PCR, the following step to reach comparability is necessary: For at least 10, more preferably at least 75 and most preferably at least 100 patients, the expression levels of the housekeeping genes GAPDH, RPLPO and PGK1 have to be analysed simultaneously with (a) more preferred housekeeping gene(s) to calculate a constant factor that can be employed subsequently to reach comparability with the weight factors given in Table 1. This constant factor can be determined by subtracting the geometric mean of expression level of the reference genes GAPDH, RPLPO and PGK1 from the mean of the expression level(s) of the new reference gene(s).

In case of microarray analysis, a skilled person has to multiply the expression values of each gene with a correction factor which has to be determined empirically to account for different dynamics of the microarray analysis in comparison to quantitative PCR. To do so, the skilled person can for example analyse the expression levels of the measured genes and the microarray gene expressions simultaneously in at least 10, more preferably at least 75 and most preferably at least 100 patients to calculate a correcting factor that accounts for the differences in technique of quantitative PCR and microarray gene expression analysis.

As used herein, the term "adding up the individual values obtained for each gene in (c) to derive a prognostic score" relates to deriving the sum of all of the normalised and weight-corrected values. This sum represents the prognostic score.

In accordance with the method of the invention, a prognostic score significantly below the reference score is indicative of a poor prognosis and a prognostic score significantly above the reference score is indicative of a good prognosis.

A prognostic score is significantly below or above the reference score if it differs from the reference score to a clinically relevant extent. For example, a threshold below or above which the prognostic score differs significantly from the mean prognostic score may be defined, such as for example the 30th and 70th percentile, preferably the 20th and 80th percentile, more preferably the 10th and 90th percentile and most preferably the 5th and 95th percentile. A percentile as used herein is the value of a variable below which a certain percent of observations fall. For example, the 30th percentile is the value (or score) below which 30 percent of the observations may be found. In other words, when using the 30th and 70th percentile, the prognostic score differs significantly from the reference score when it corresponds to a value that falls below a threshold determined by the values below and above which 30 percent of the observations obtained in a reference group are found. Using this approach, the reference score represents a mean or average score representative of normal disease progression while the e.g. 30th percentile represents the limits above and below which the difference becomes a significant difference. Alternatively, the prognostic score is considered to be significantly below or above the reference score when it differs for example by at least 2σ, i.e. two-times the standard deviation of the reference score, i.e. for repeated determination of the reference score by relying upon different patients with normal disease progression

In order to determine whether the prognostic score differs significantly from the reference score, the skilled person may for example refer to the values as represented in table 1 above. In order to derive a diagnosis based on these values, the determination of the expression levels of the at least two genes in step (a) is carried out by PCR amplification and the normalisation in step (b) is carried out against the geometric mean of the expression levels of the three reference genes GAPDH, RPLPO and PGK1. The lower score limit represented in column 3 corresponds to the threshold below which the prognostic score falls significantly below the reference score. The upper score limit represented in column 4, on the other hand, corresponds to the threshold above which the prognostic score is significantly above the reference score. Thus, a prognostic score below the lower score limit shown in table 1 is indicative of a poor prognosis and a prognostic score above the upper score limit sown in table 1 is indicative of a good prognosis.

Alternatively, the skilled person may initially determine in a sufficiently large patient group, such as for example at least 10, more preferably at least 75 and most preferably at least 100 patients, the expression levels of the genes NRIP1, SFTPB, PLEKHA1, PDE8A, TCF7, MGC29506, MSI2, MGAT4A, NELL2, BCL11B, CD247, SORL1, KLRB1, IL7R, RUNX2, MAL, RASGRF2, TIAM1, PRKCH, FNIP2, GIMAP7, PAG1, ITK, IL2RB, SATB1, PRKCQ, TRAT1 and/or CLEC4A. Preferably, the patient group is a representative group of CLL-patients predicted by conventional methods to be poor prognosis or good prognosis patients. The expression data obtained from this group may then be correlated with disease progression, as detailed in the examples below.
For example, after measurement of at least 10, more preferably at least 75 and most preferably at least 100 patients in a heterogeneous prognostic group and determination of the above mentioned gene expression values, correlation of the follow up data with the gene expression values using the first principal component analysis (following the procedure proposed by Bair and Tibshirani ("superPC method") as mentioned above) results in the derivations of specific weights. The calculated scores (sum of the expression results multiplied with weights) should be sorted due to their magnitude. The cut offs should be selected due to clinical relevance. For example the magnitudes could be split into the 20th and 80th percentile, whereas a lower score is indicative for poor prognosis and a higher score is indicative for good prognosis.

In a preferred embodiment of the method of the invention, the method comprises (a) determining in a sample obtained from the patient the expression level of (ai) at least four genes selected from the group consisting of (i) to (viii): (i) NRIP1; (ii) SFTPB; (iii) PLEKHA1; (iv) PDE8A; (v) TCF7; (vi) MGC29506; (vii) MSI2; and (viii) one gene selected from the group consisting of MGAT4A, NELL2, BCL11B, CD247, SORL1, KLRB1, IL7R, RUNX2, MAL, RASGRF2, TIAM1, PRKCH, FNIP2, GIMAP7, PAG1, ITK, IL2RB, SATB1, PRKCQ, TRAT1, CLEC4A; or (aii) at least two genes selected from the group consisting of (i) to (v): (i) SFTPB; (ii) PLEKHA1; (iii) MGC29506; (iv) MSI2; and (v) one gene selected from the group consisting of MGAT4A, NELL2, BCL11B, CD247, SORL1, KLRB1, IL7R, RUNX2, MAL, RASGRF2, TIAM1, PRKCH, FNIP2, GIMAP7, PAG1, ITK, IL2RB, SATB1, PRKCQ, TRAT1, CLEC4A; (b) normalising the expression levels determined in (a); (c) multiplying each normalised expression level obtained in (b) with a gene-specific weight as shown in Table 1; and (d) adding up the individual values obtained for each gene in (c) to derive a prognostic score; wherein a prognostic score below a reference score, said reference score being characteristic of normal disease progression, is indicative of a poor prognosis and a prognostic score significantly above the mean prognostic score is indicative of a good prognosis.

In accordance with this preferred embodiment of the present invention, the selection of more than one gene from group (viii) is only envisaged in case that more than four genes are selected in (ai) or in case that more than two genes are selected in (aii). Otherwise, at most one of the genes listed in group (ai)(viii) or group (aii)(v) is to be selected. In other words, at least one gene has to be selected from groups (i) to (vii) in (ai) or at least one gene has to be selected from groups (i) to (iv) in (aii).

In a further preferred embodiment, the method of the invention is applied in combination with methods known in the art, such as the above described methods of determining VH and/or FISH. Most preferably, the method of the invention is employed in combination with both VH and FISH determination.

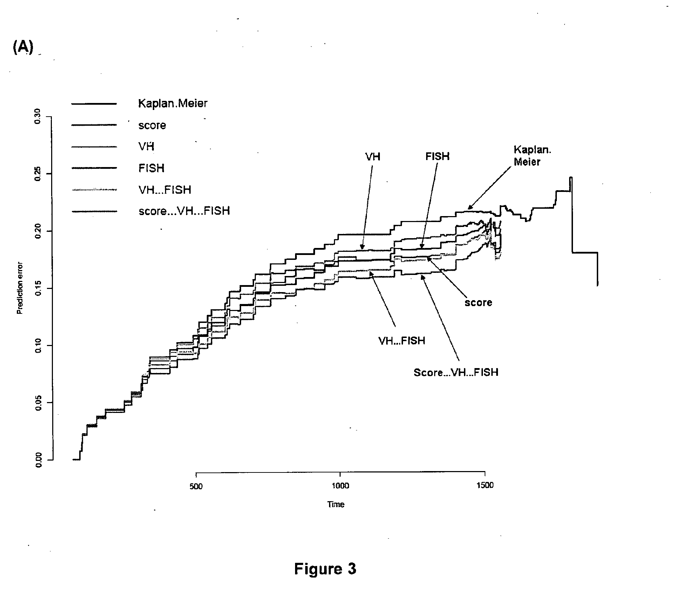

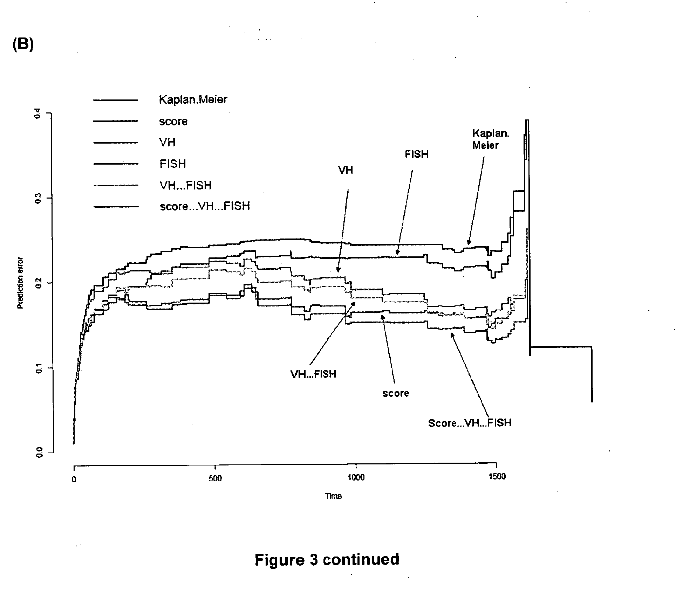

VH and FISH determination are well known in the art and have been described, for example, in Dohner H, Stilgenbauer S, Benner A, et al. Genomic aberrations and survival in chronic lymphocytic leukemia. N Engl J Med 2000;343:1910-6 and Hamblin TJ, Davis Z, Gardiner A, Oscier DG, Stevenson FK. Unmutated Ig V(H) genes are associated with a more aggressive form of chronic lymphocytic leukemia. Blood 1999;94:1848-54.
As is shown in Figure 3B with regard to time-to-treatment (TTT), the method of the present invention showed an improved prognostic quality as compared to established methods such as VH or FISH. Furthermore, an improvement as was also seen as compared to the combined use of VH and FISH. Also with regard to overall survival (OS) the method of the present invention showed an improved prediction quality as compared to established methods such as either VH or FISH (Figure 3A). For both TTT and OS, a further improvement in prediction quality could be observed when all three methods were used in combination (Figure 3).

In a preferred embodiment of the method of the invention, the expression level of at least eight genes is determined in step (a).

In a more preferred embodiment of the method of the invention, the at least eight genes are NRIP1, SFTPB, PLEKHA1, PDE8A, TCF7, MGC29506, MSI2 and MGAT4A.

In a preferred embodiment of the method of the invention, cDNA is prepared from total RNA extracted from said sample prior to the step of determining expression levels.

Total RNA extraction as well as cDNA preparation methods are well known in the art and have been described herein above.

In another preferred embodiment of the method of the invention, the expression levels are determined by microarray analysis, PCR, RT-PCR, real time RT-PCR or sequencing analysis.

The determination of expression levels by microarray analysis is well known in the art and has been described, for example by Botwell 1999 and others³⁹⁻⁴¹.

Methods for the determination of expression levels by PCR methods, including for example nested PCR, RT-PCR, PCR extension assays and real time RT-PCT are also well known in the art and have been described above. Further details of such methods can be found in art, see, for example, Newton et al., Nucleic Acids Res. 17 (1989) 2503-2516; Agrawal (Ed.), "Protocols for Oligonucleotides and Analogs: Synthesis and Properties (Methods in Molecular Biology, 20)", Humana Press, 1993; Haque et al., Diagn. Mol. Pathol. 7 (1998) 248-252; Innis et al. (Ed.), "PCR Applications: Protocols for Functional Genomics", Academic Press, 1999; Chen and Janes (Ed.), "PCR Cloning Protocols: From Molecular Cloning to Genetic", 2nd edition, Humana Press, 2002.

Methods of determining expression levels by sequencing analysis are known in the art. These methods rely on the sequencing of each individual nucleic acid molecule present in the sample and the subsequent determination of the amount of each nucleic acid molecule (i.e. copy numbers) present in the sample. Such methods have been described, for example whole transcriptome sequencing using next generation sequencing technologies⁴².

Unless otherwise defined, all technical and scientific terms used herein have the same meaning as commonly understood by one of ordinary skill in the art to which this invention belongs. In case of conflict, the patent specification, including definitions, will prevail.

The present invention further relates to the use of nucleic acid molecules specific for the expression product of at least two genes selected from the group consisting of (i) to (viii): (i) NRIP1; (ii) SFTPB; (iii) PLEKHA1; (iv) PDE8A; (v) TCF7; (vi) MGC29506; (vii) MSI2, and (viii) one gene selected from the group consisting of MGAT4A, NELL2, BCL11B, CD247, SORL1, KLRB1, IL7R, RUNX2, MAL, RASGRF2, TIAM1, PRKCH, FNIP2, GIMAP7, PAG1, ITK, IL2RB, SATB1, PRKCQ, TRAT1, CLEC4A wherein each of the nucleic acid molecules has a length of at least 8 nucleotides, in the diagnosis of disease progression in a patient suffering from chronic lymphocytic leukemia, wherein said diagnosis is not practised on the human or animal body.

The present invention further relates to the use of nucleic acid molecules specific for the expression product of (a) at least four genes selected from the group consisting of (i) to (viii): (i) NRIP1; (ii) SFTPB; (iii) PLEKHA1; (iv) PDE8A; (v) TCF7; (vi) MGC29506; (vii) MSI2; and (viii) one gene selected from the group consisting of MGAT4A, NELL2, BCL11B, CD247, SORL1, KLRB1, IL7R, RUNX2, MAL, RASGRF2, TIAM1, PRKCH, FNIP2, GIMAP7, PAG1, ITK, IL2RB, SATB1, PRKCQ, TRAT1, CLEC4A; or (b) at least two genes selected from the group consisting of (i) to (v): (i) SFTPB; (ii) PLEKHA1; (iii) MGC29506; (iv) MS12; and (v) one gene selected from the group consisting of MGAT4A, NELL2, BCL11B, CD247, SORL1, KLRB1, IL7R, RUNX2, MAL, RASGRF2, TIAM1, PRKCH, FNIP2, GIMAP7, PAG1, ITK, IL2RB, SATB1, PRKCQ, TRAT1, CLEC4A wherein each of the nucleic acid molecules has a length of at least 8 nucleotides, in the diagnosis of disease progression in a patient suffering from chronic lymphocytic leukemia, wherein said diagnosis is not practised on the human or animal body.

The definitions as well as the preferred embodiments provided herein above with regard to other embodiments such as e.g. the kit of the invention, the set of the invention, the diagnostic composition of the invention, the method of the invention etc. apply mutatis mutandis also to the embodiments relating to the use of nucleic acid molecules in the diagnosis of disease progression in a patient suffering from chronic lymphocytic leukemia as outlined above. For example, preferred embodiments of the kit have their counterparts in preferred embodiments of the above defined use.

The figures show:

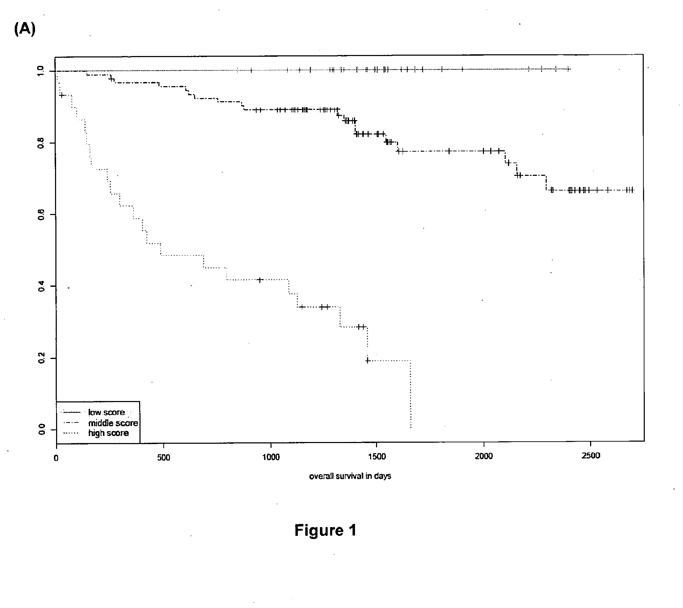

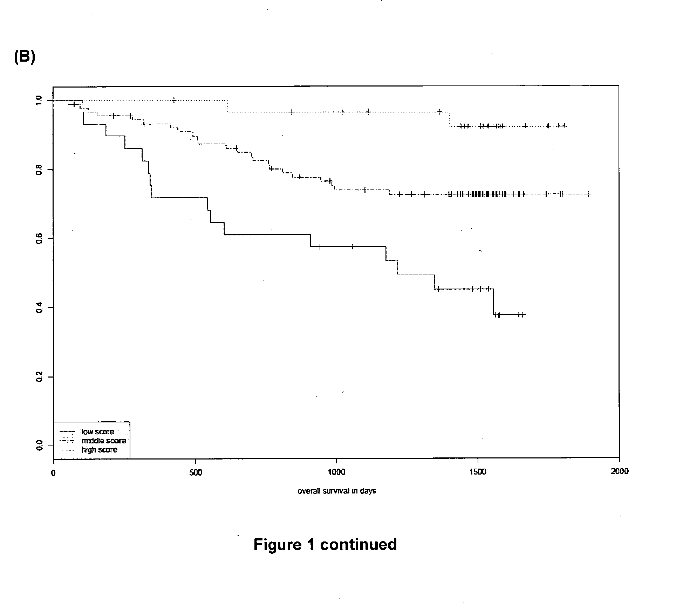

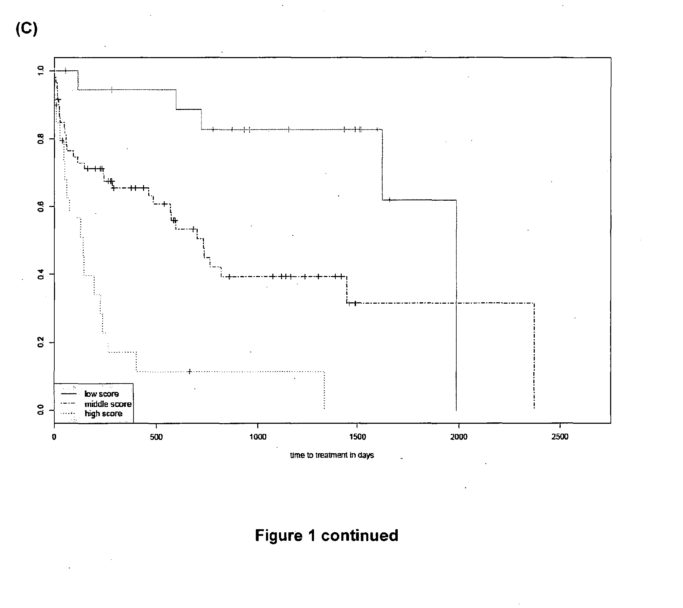

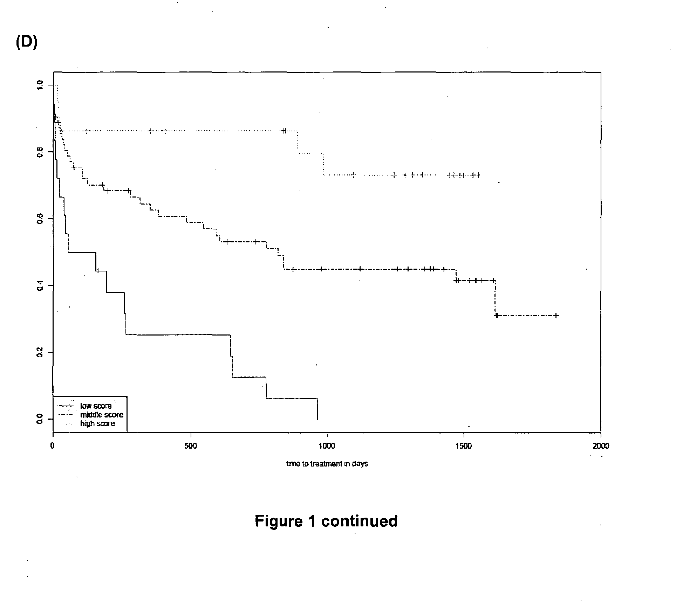

**Figure 1****: OS and TTT in the initial and validation set.** Prediction of OS (A) and TTT (C) in the microarray set and validation set (LDA group) OS (B) and TTT (D); for display purposes of the linear score a cut off of 20 % of the highest and lowest scores was chosen (Chi-square distribution: A: <0,001; B: 6.41 × 10⁻⁵; C: 3.14 × 10⁻⁷; D: 1.28 × 10⁻⁶; Median OS and TTT: microarray set NR/NR/16 months for OS and 66/24/5 months for TTT; Validation set NR/NR/40 months for OS and NR/27/4 months for TTT).

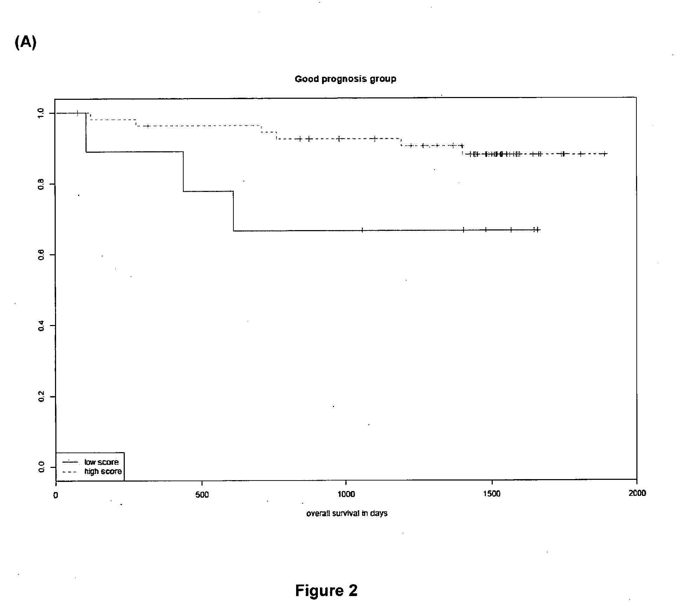

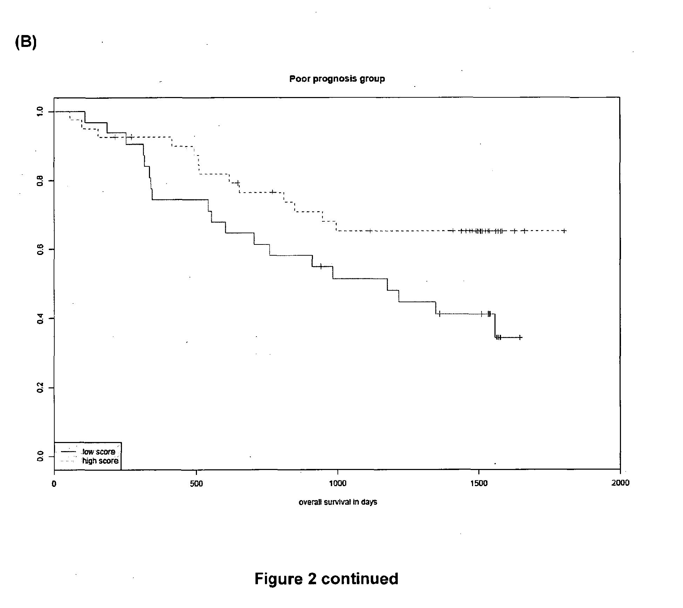

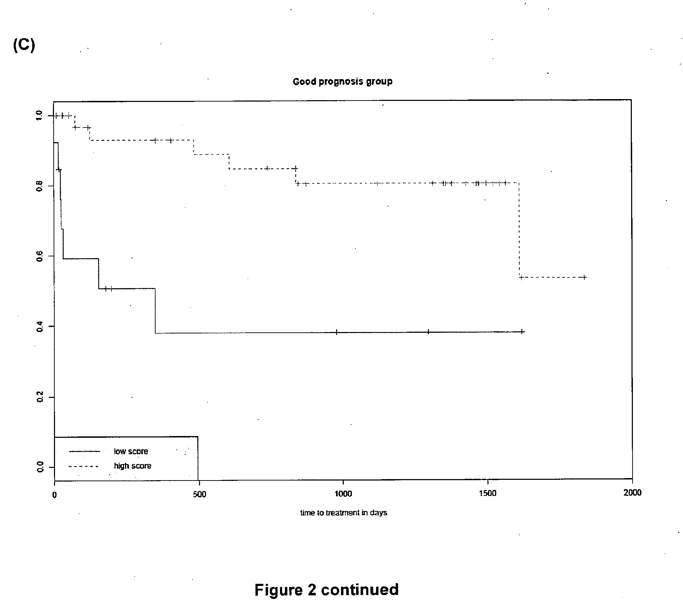

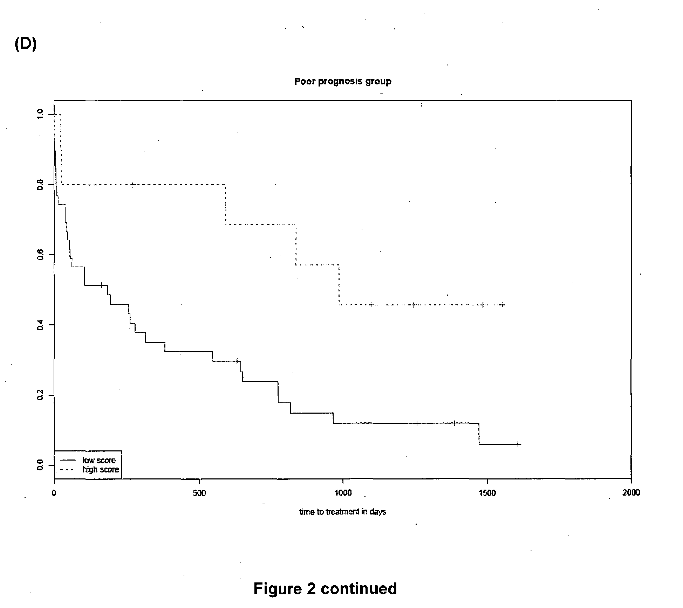

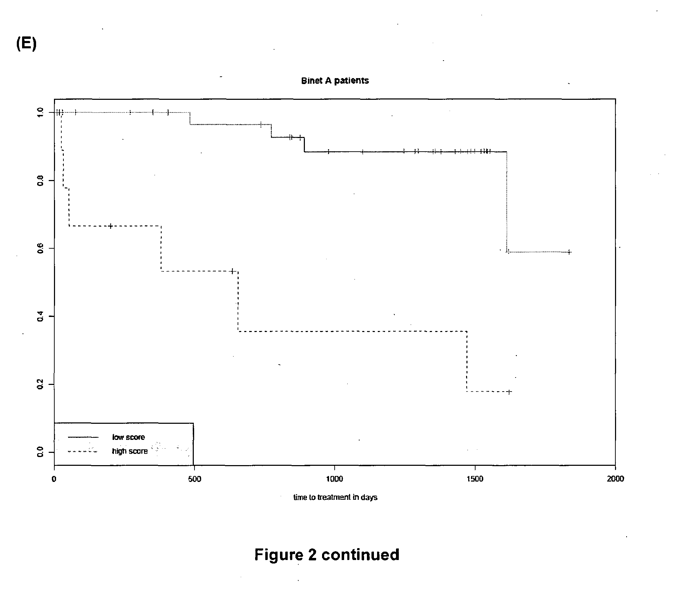

**Figure 2****: OS and TTT in subgroups of the validation set.** Prediction of OS (A, B) and TTT (C, D, E) in the good prognosis subgroup (mutated IgH genes, no 17p13 or 11q23 deletions; A, C), poor prognosis subgroup (unmutated IgH genes or 17p13 or 11q23 deletion; B, D) and Binet A patient group (E) in the validation set. A dichotomized cut off of the same size as the number of events in the subgroups was used (A: 9/64, 14%; B: 32/72, 44.4%; C: 13/46, 28.3%; D: 39/49, 80%, E: 10/44, 23%). Chi-square distribution: A: 0.0514; B: 0.0429; C: 0.0021; D: 0.0105; E: 6.72 × 10⁻⁵
**Figure 3****: Assessment of the additional prognostic value.** Additional prognostic value displayed by prediction error curves for OS (A) and TTT (B). Score: Eight gene score; VH/FISH: Combined prediction model of VH-Status and FISH markers. ScoreNH/FISH: Combined prediction model of VH-Status, FISH and the eight gene score. A lower curve is indicative for additional predictive value. The prediction quality of the eight gene score is slightly inferior to a combined model of FISH and VH-status in the prediction of OS but improves prediction quality further when added (A). In prediction of TTT the model of the eight gene score is superior to the traditional molecular markers. Prediction quality is only slightly improved by the addition of VH and FISH (B).

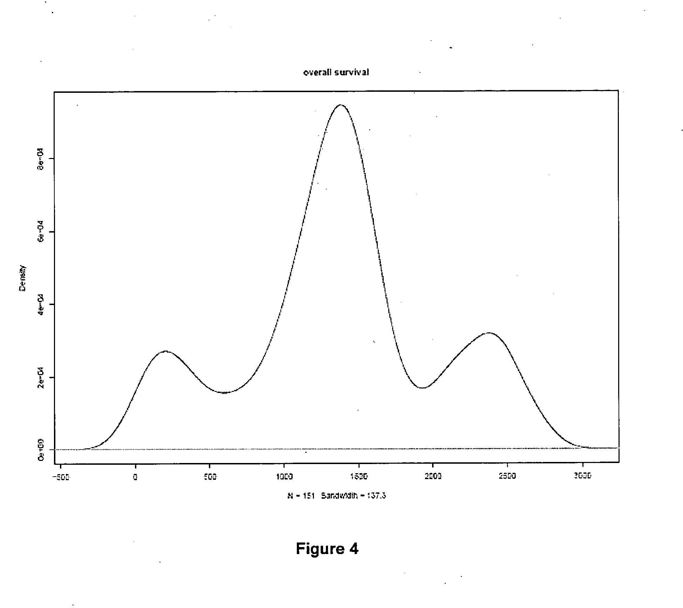

**Figure 4****: Distribution of OS.** Distribution of the overall survival rates in the initial set. Three groups could be identified, leading to the used cut off of 20 % of the highest and lowest scores in the validation set.

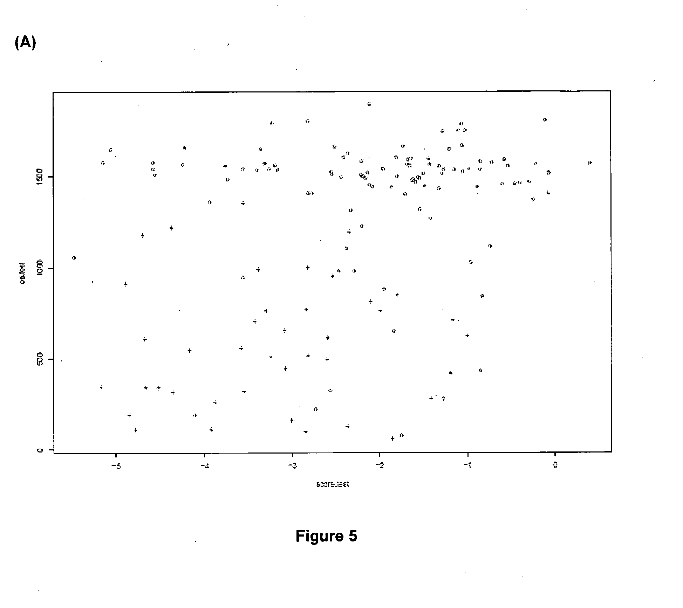

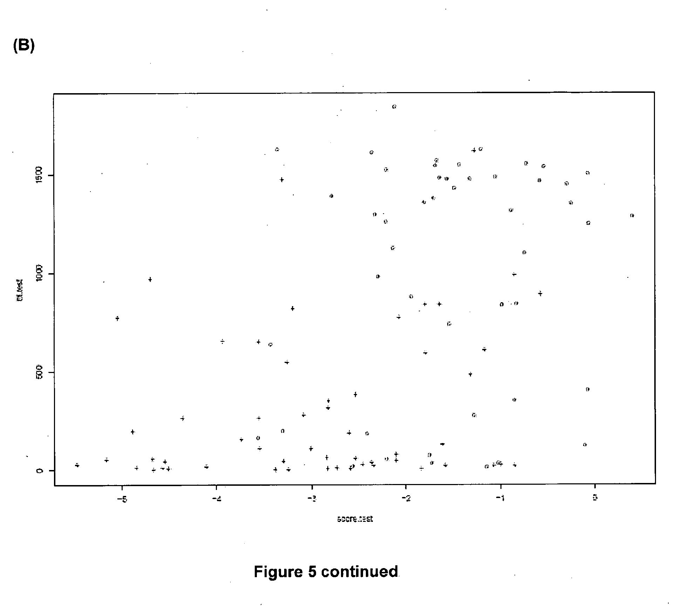

**Figure 5****: Distribution of OS and TTT.** (A) Distribution of OS (days: y-axis and score value: x-axis; cross: death; dot: censorship) in the validation set. A higher score is associated with prolonged survival. (B) Distribution of TTT (days: y-axis and score value: x-axis; cross: death; dot: censorship) in the validation set. A higher score is associated with prolonged treatment free survival.

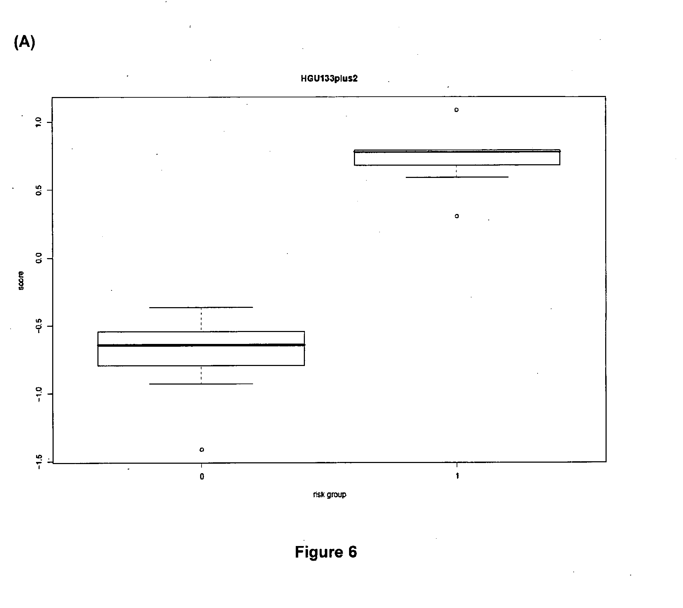

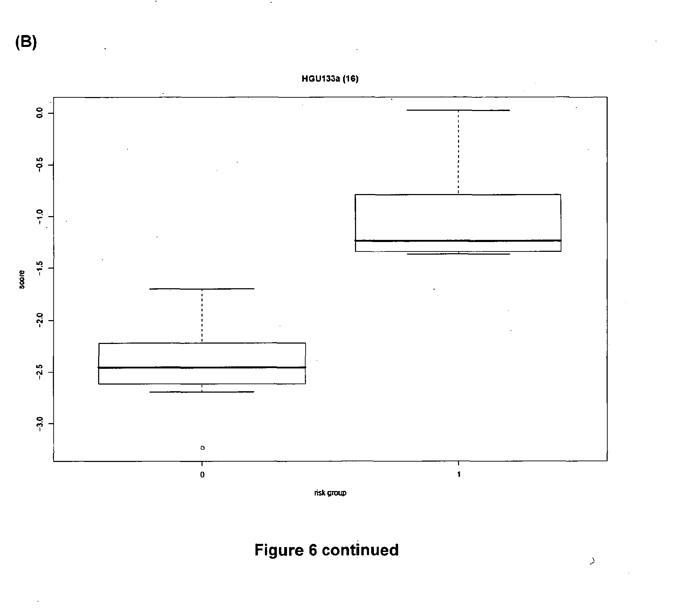

**Figure 6****: Validation of the 8 gene score on public available microarray datasets.** (A) Stamatopoulos *et al.* used 14 Affymetrix HG-U133 plus 2.0 microarrays to build a classifier that discriminate between ZAP70 positive and negative patients. All signature genes are represented on this array. The gene expression signature was able to discriminate highly significant between these groups (P-value: 0.0005828) (21). (B) Hüttmann et al. used 16 Affymetrix HG-U133 A microarrays to discriminate between ZAP70 and CD38 positive and ZAP70 and CD38 negative samples (22). On the HG-U133 A platform only the following signature genes are represented: NRIP1, TCF7, PDE8A, MGC29506 and SFTPB (5 of 8). A signature using only these 5 genes was able to discriminate highly significant both groups without modification of the calculated weights (P-value: 0.0001554).

The examples illustrate the invention:

### Example 1: Materials and Methods

### Patients:

In the current study 310 peripheral blood samples (routine diagnostic) from patients with CLL were sent to the Laboratory for Leukemia Diagnostics, Department of Medicine III, Munich, Germany between 2001 and 2007. Samples were collected from patients at different disease stages. Most samples were collected at the time of first diagnosis from untreated patients, but also after relapse or in chronic phase. The diagnostic work-up included standard cytomorphology, multiparameter immunophenotyping, fluorescence in situ hybridization (FISH) (6q21, 11q22.3, 12p11.1-q11, 13q14.3, 17p13.1 and IgH break-apart probe) and IgVH-analysis. FISH-probes were obtained from Abbott Laboratories (Abbott Park, USA) and Kreatech Diagnostics (Amsterdam, NL). Diagnosis of CLL was defined according to the revised National Cancer Institute-Sponsored Working Group Guidelines for Chronic Lymphocytic Leukemia from 1996 ²³. Patients with an IgH-CCND1 rearrangement (t(11;14)(q21;q32)) were not included in this study. Follow-up data was obtained from the *Tumorregister München (Tumorzentrum München)* or the clinical database (*Department of Medicine III, University Hospital Grosshadern, Ludwig-Maximilians-University, Munich*)*.* 163 patients were analyzed by microarray gene expression profiling. The data from 12 patients were excluded from the study due to poor microarray quality or because no follow-up data were available. Two patients were analysed twice at different disease stages (151 arrays from 149 patients). A further validation cohort consisted of 147 additional patients. Distribution of genomic aberrations, VH-status, mean white-cell, platelet and hemoglobin count, Binet stage, pretreatments, treatment schedules, TTT and OS were comparable in both groups and are specified in Table 2. Patient data were anonymised before analysis. The study design adhered to the tenets of the Declaration of Helsinki and was approved by an institutional review board.

### Sample preparation

Samples were prepared after Ficoll gradient centrifugation. No B-cell enrichment or cell sorting was performed.

### Microarray analysis:

Patient samples were processed for microarray analysis according to the manufacturer's protocol (Affymetrix, Santa Clara, CA, USA). Details regarding sample preparation, hybridization, and image acquisition have been described elsewhere^{10, 43-45}, The quality control consisted of visual inspection of the array image for artefacts, assessment of RNA degradation plots, and inspection of rank-vs.-residual plots after normalization. Gene expression profiling was performed on HG-U133 A&B and Affymetrix HG-U133 plus 2.0 chips. Initially, 46 HG-U133 A&B chip pairs and 117 HG-U133 plus 2.0 microarrays were evaluated. The data from 12 patients were excluded as described in *patients.* The 44 HG-U133 A, B chips and the 107 HG-U133 plus 2.0 chips were normalized separately by the Robust Multichip Average (RMA) method as described by Irizarry et al 2003 ⁴⁵. Only the 44754 probe sets present on A, B chips and the 2.0 chips were included in the analysis.
Some probe sets on the A, B chips tend to have lower mean signal levels and higher standard deviations than the corresponding probe sets on the plus 2.0 chips ¹⁰. To eliminate this batch effect resulting from the different chip designs a second normalization was performed using an empirical Bayesian method ⁴⁶

### Real time quantitative PCR analysis

The real time quantitative PCR analysis was performed on an Applied Biosystems 7900HT fast real-time PCR system with pre-designed gene expression assays purchased from Applied Biosystems (Applied Biosystems, Foster City, CA, USA). The samples were assayed in triplicate and fold expression was calculated using the ΔΔCT method after normalisation using the geometric mean of the three housekeeping genes (GAPDH, PGK1 and RPLP0) ⁴⁷. Note that ACT values are inversely correlated to gene expression levels. In samples with undetectable ΔCT values (negative samples), the ΔCT value was calculated by subtracting the CT value of the geometric mean of the three housekeeping genes from the maximum number of cycles used in the assay (40 cycles).

### Statistical Analysis

Overall survival (OS) was defined as time from study entry until death from any cause. Patients alive without an event were censored at the time of their last follow-up. Time to Treatment (TTT) was defined as time from study entry until start of first or next chemotherapy treatment or death from any cause. Patients without an event were censored at the time of their last follow-up.
Fisher's exact test was used to compare categorical clinical variables between the test and the validation group. For the continuous variables the Wilcoxon-Mann-Whitney-Test was used. The reverse Kaplan-Meier method was used to estimate the median follow up.
The prognostic score was derived in a two step approach based on the gene expression profiles of the 151 microarrays as follows. First, 5000 bootstrap data sets were randomly drawn with replacement from the original training data set in order to mimick perturbations of the data set and obtain a more robust signature ⁴⁸. For each of these 5000 data sets the genes that were significant in univariate Cox-regression were retained, following the procedure proposed by Bair and Tibshirani ("superPC method") ³⁸. A subset of genes that were consistently selected over the 5000 data sets was chosen. Second, in an approach to further shrink the resulting signature, 24 patient samples from which we also had microarray data were correlated with quantitative PCR and genes selected due to correlating expression levels, inter-signature correlation and performance. The prognostic score was obtained as a weighted sum of these gene expressions. The approach is further described in the result section.

The prognostic score of the present invention was first evaluated with a univariate Cox proportional hazards model. To adjust for important clinical variables, a multivariate Cox model with VH-status, FISH, age (< 65 years vs. ≥65 years) ⁴⁹ and the prognostic score as covariates was fitted to the validation data set. The predictive value of the prognostic score was subsequently assessed based on the prediction error curve using 10-fold cross-validation as implemented in the R package 'pec' ⁵⁰.Based on the validation data set, prediction error curves were obtained both for models with and without the prognostic score in order to assess the additional predictive value of the score.
To further examine the predictive value of the prognostic score based on the validation data set, a dichotomization and a trichotomization were performed. The patients were divided into two groups based on their prognostic score: a high risk group of the same size as the number of events and a low risk group including the rest of the patients. The score was also trichotomized into the lowest 20% group, the middle 60% group and the highest 20% group. This cut off was selected due to the distribution of the overall survival rates in the training set (Figure 4). Kaplan-Meier curves were used to visualize the survival in the different score groups and P-values were calculated based on the log-rank test.
All statistical analyses were performed using the R 2.7.2 software package and routines from the biostatistics software repository Bioconductor. A P-value <0.05 was considered significant.

### Example 2: Patient Characteristics

The patient groups consisted of the training set of 151 microarray data sets and the validation set with 147 patients. The patient characteristics are listed in Table 2. Patient characteristics were similar between both groups. There were no significant differences in the numbers of pre-treated patients and unfavourable cytogenetic markers between training and validation set.

### Example 3: Development of a prognostic score in the training set

The gene expression profiles of 151 microarrays and their follow-up data were used to develop a prognostic score as described in the Methods section. A subset of 17 probes sets that were selected most frequently based on the 5000 bootstrap data sets was identified: these 17 probe sets, which represent 15 genes, were selected in more than 4250 of the 5000 bootstrap samples.

### Example 4: Correlating microarray derived expression levels with quantitative real time PCR measurements in the training set

To reduce the number of probe sets in the score, the expression levels of these 15 genes were correlated with each other. The expression levels of 6 out of the 15 genes were highly correlated while the remaining 9 genes did not show strong correlation with any of the other genes. It was thus possible to group the 15 genes into 10 clusters (9 clusters with 1 gene each, and one cluster with 6 genes). Quantitative PCR assays were designed for all 15 genes and their expression was measured in 24 patients from which microarray data were available. Genes with low correlation between the quantitative PCR measurements and the microarray probe sets were excluded. Additionally, only genes that were expressed at measurable levels in the majority of samples were used for further validation. At the end of this selection process eight genes (7 from the 9 clusters with only 1 gene and 1 from the clusters with the 6 genes) were identified which performed well on the TaqMan low density Array (LDA) platform (Table 3). In addition, genes correlated with MGAT4A were identified, that are also suitable for use in the gene score. Note that the survival data of the validation data set were not used during the selection process, thus enabling an unbiased evaluation of the score's accuracy.

### Example 5: Performance of the preferred 8 gene score in the training set and in additional independent microarray data sets

The expression levels of the preferred eight genes (Table 3) were used to calculate a prognostic score using the superPC method based on the training data ³⁸. Based on this eight gene prognostic score the 151 microarray datasets of the training set were divided into 3 groups using cut-offs at the 20%- and 80%-percentiles of the score, resulting in a good risk group, an intermediate group and a poor risk group. Figure 1 demonstrates that the prognostic score was highly predictive both for OS and TTT with median OS in the poor risk group of 16 months (good and intermediate risk not reached) and median TTT of 66 months in the good, 24 months in the intermediate and 5 months in the poor risk group (Figure 1A, C). In univariate Cox-regression analysis p-values of 1.8 × 10⁻¹³ for OS and of 2.9 × 10⁻¹⁰ for TTT were achieved.

Additionally using publicly available small, independent microarray data sets from CLL patients it could be shown that the prognostic score performed surprisingly well in the classification of predefined risk groups (Figure 6).

### Example 6: Validation of the 8 gene based prognostic score in an independent patient cohort using a different technical platform

Quantitative real time PCR using the Taqman LDA platform for the preferred eight genes of the prognostic score was performed with cDNA samples from 147 independent CLL patients. For each patient from the validation data set, the prognostic score was calculated using the weights derived from the training data set. The score influenced OS and TTT significantly in the validation cohort (P-values in the univariate Cox-model: 2.1 ×10⁻⁶ OS, 5.1 × 10⁻⁹ TTT). The OS and TTT of the 20% patients with the highest and the 20% patients with the lowest prognostic scores in the validation set are shown in Figure 1B, D. The median OS in the validation set was 40 months in the poor prognosis group (good and intermediate risk not reached). The median TTT was not reached in the good prognosis group and was 27 months in the intermediate and 4 months in the poor prognosis group.

### Example 7: Analysis of subgroups in the validation set

The performance of the prognostic score was evaluated in patient subgroups classified by known risk factors into good and poor prognostic groups. The good prognosis group consisted of patients with mutated IgH genes and no 17p13 or 11q23 deletions in FISH analysis. The poor prognosis group consisted of patients with unmutated IgH genes or with a 17p13 or an 11 q23 deletion.

The univariate Cox-regression analysis for OS showed a p-value of 0.13 in the good prognosis group and a p-value of 0.00094 in the poor prognosis group. The non- significant p-value in the good prognosis group is most probably due to the low death rate of 14% in this group. The Kaplan-Meier curves obtained with the dichotomized score are shown in Figure 2A and B (OS p= 0.0514 for the good prognosis group and p=0.0429 for the poor prognosis group).

The univariate Cox-regression analysis of the score for time to treatment showed a p-value of 0.0021 in the good prognostic group and a p-value of 2 × 10⁻⁴ in the poor prognosis group. The Kaplan-Meier curves are shown in Figure 2C and D (TTT good prognosis group p=0.0021 and p=0.0105 for the poor prognosis group). 50 patients from the validation data set were defined as Binet A at time of study entry. Only four of these patients died so that an analysis of overall survival was impossible. However, the analysis of time to treatment showed highly significant prediction of TTT in the Binet A subgroup with a p-value of 0.0282 in univariate Cox-regression. The Kaplan-Meier curves are shown in Figure 2E (p=6.7 × 10⁻⁵). Analyses of Binet stage B and C patients are not shown because these subgroups were too small.

In conclusion the score is able to provide predictive information in clinically relevant subgroups in addition to established risk factors (namely FISH and VH-status) in particular for the time to treatment interval in early stage patients.

### Example 8: Multivariate analysis for overall survival and time to treatment in the validation set

In the validation set, multivariate Cox-regression models were fitted to TTT and OS using the prognostic score, VH status, FISH status and age as covariates. The prognostic score showed high significance in both models (Table 4). In the analysis of OS, age and the score of the present invention were highly significant and had much smaller p-values than the other covariates. The 17p13 deletion was significant for OS only. For TTT the prognostic score was the only significant covariate.

### Example 9: Assessment of the additional prognostic value of the eight gene prognostic score

The additional prognostic value of the score was assessed using the prediction error curve methodology in 10-fold cross-validation. For OS the prognostic score performed slightly worse than the combined model of the established markers FISH and VH-status. This could be due to the lower rate of events in the OS group compared to the TTT group. The score was able to improve the prognostic performance when added to FISH and VH-status (Figure 3A, C). For TTT the prognostic score was superior to the combined FISH and VH-status model. Addition of FISH and VH-status increased the prediction performance of the prognostic score only slightly (Figure 3B, D).

In conclusion the eight gene expression score is able to improve prognostic accuracy for OS and TTT. The score is able to replace FISH and VH-status if used as single prognostic factors. A combined model of FISH and VH-status is further improved by the gene expression score.

In addition, the findings in accordance with the present invention relating to genes associated with a poor or good prognosis for CLL patients now also link several molecular pathways for the first time with progression in CLL and possibly also with the development of this disease.

The pathogenesis of CLL is still poorly understood. Without wishing to be bound by theory, it is speculated that some of the genes identified herein play an important role in the pathogenesis of CLL, especially those genes which have been found altered in other malignancies or which belong to genetic networks involved in malignant transformation.

The Wnt-signalling pathway is important in stem cell maintenance and is also activated in CLL ²⁴. As part of the prognostic gene set of the present invention, low expression levels of TCF7 (T cell specific, HMG box), a downstream target of the Wnt-signalling pathway, have been found to be associated with poor overall survival of CLL patients. Similar results have recently been reported by Kienle and coworkers ¹⁹. These data strengthen the case for an important role of the Wnt-signalling pathway in CLL.

Also as part of the prognostic gene set of the present invention, low transcript levels of the phosphodiesterase 8A *(PDE8A)* were associated with poor prognosis in CLL patients. A similar association was reported by Stamatopoulos and co-worker, who showed that PDE8A was part of a gene signature that distinguishes between ZAP70 positive and ZAP70 negative CLL samples ¹⁸. PDE8A might be connected to the NFκ-B signalling pathway, which is frequently activated in lymphoid malignancies, transmitting signals from the B cell receptor ^{27,28}

The gene encoding for the nuclear receptor interacting protein 1 *(NRIP1)* was expressed at low levels in poor prognosis cases as part of the prognostic gene set of the present invention. The prognostic significance of *NRIP1* expression levels in CLL has also been reported by van't Veer and co-workers ²⁹. NRIP1 is involved in the regulation of the estrogen receptor activity and of retinoid acid receptor signalling ³⁰. All-trans retinoic acid (RA) has the ability to induce the expression of *NRIP1* suggesting the interesting possibility that RA treatment might be able to induce *NRIP1* expression in bad prognosis CLL and thereby be of benefit for the patients.

One of the genes in the prognostic gene set of the present invention that has not been implicated in CLL prognosis before is the Musashi homologue 2 gene (*MS*/*2*)*.* In the present study it was shown that as part of the prognostic gene set of the invention, high expression levels of *MS*/*2* in CLL are associated with poor survival. *MS*/*2* encodes for an RNA-binding translational regulator, that may play a role in cell renewal and differentiation of stem cells ³¹. Very interestingly, *MS*/*2* was found to be the target of chromosomal translocations in rare cases of myeloid leukemia ³², ³³. An *MSI2*/*HOXA9* fusion gene was reported in a CML case with a t(7;17)(p15;q23) translocation ³³ and 9 of 13 t(3;17)(q26;q22) translocations had breakpoints in the *MS*/*2* locus on 17q22 ³².

The remaining four genes of the eight-gene prognostic gene set of the invention *(PLEKHA1, MGC29506, MGAT4A, SFTPB)* have not been described in the context of CLL. However, they might lead to new pathways and gene regulatory networks important for CLL progression. MCG29506, which was recently described as the plasma cell induced endoplasmatic reticulum protein pERp1, seems to play an important role in the secretion of IgM molecules in plasma cells ^{20,21}. Deregulated expression of MGAT4A (mannosyl (alpha-1,3-)-glycoprotein beta-1,4-N-acetylglucosaminyltransferase, isozyme A) has been reported in pancreatic and gastric cancers ³⁴. Neither *PLEKHA1* (pleckstrin homology domain containing, family A (phosphoinositide binding specific) member 1) nor *SFTPB* (surfactant protein B) have an obvious connection to B cell function or malignant disease.

**Table 2: Patient characteristics**

| **Variable** | **Microarray set¹** | **Validation set** | **P value** |
|---|---|---|---|
| No. of patients | 149² | 147 | |
| No. of analyzed samples | 151 | 147 | |
| Mean age (yr) | 60.7 (30-84) | 61.6 (33-85) | 0.65 |
| Male sex (%) | 58.9 | 66 | 0.23 |
| Deceased (%) | 27.2 | 27.9 | 0.9 |
| Pre-treated patients (%) | 21.2 | 29.6 | 0.17 |
| Treated at progression (%) | 54.5 | 53.4 | 1 |
| Median follow up | 4.11 | 4.17 | 0.34 |
| FISH (%) | | | |
| Del 17p13 | 8.6 | 4.8 | 0.25 |
| Del 11q22-q23 | 11.9 | 8.8 | 0.45 |
| Trisomy 12q13 | 13.2 | 11 | 0.6 |
| ,,Normal" FISH | 22 | 29 | 0.24 |
| Del 13q14 (single) | 38 | 44 | 0.41 |
| VH-mutated (%) | 51.1 | 51.5 | 1 |
| Disease stage at enrolment | | | |
| Binet (%) | | | |
| A | 56.9 | 52 | 0.57 |
| B | 22.5 | 24 | 0.87 |
| C | 20.6 | 24 | 0.61 |
| Mean white-cell count (× 10^{- 3}/mm³) | 62178 | 54029 | 0.07 |
| Mean hemoglobin (g/dl) | 13.3 | 13.4 | 0.8 |
| Mean Platelet count (x 10^{- 3}/mm³) | 170669 | 169651 | 0.81 |

| | | | |
|---|---|---|---|
| ¹Note that 12 patients were excluded due to poor array quality or missing follow up data ²Note that 2 patients were analyzed twice at different disease stages and counted due to disease evolution as different samples; data recapitulate therefore 149 patients with 151 microarrays | | | |

**Table 3: Regulation of the preferred set of eight genes**

| **Gene** | | **Location** | **Probe set** | **Primer** | **Weight** | **Regulation in poor prognosis** |
|---|---|---|---|---|---|---|
| **NRIP1** | Nuclear receptor interacting protein 1 | 21q11.2 | 202600_s_ 202599_s_ at | Hs00942766_s1 at | -0,20837454 | down |
| **SFTPB** | Surfactant protein | 2p11.2 B | 37004_at | Hs01090658_gl | 0,15991109 | up |
| **MGAT4A** | Mannosyl (alpha- 1,3-)-glycoprotein beta-1,4-N-acetylglucosaminy Itransferase, isozyme A | 2q12 | 226039_at | Hs00923405_m1 | -0,15093638 | down |
| **PLEKHA 1** | Pleckstrin homology domain containing, family A (phosphoinositide binding specific) member 1 | 10q26.13 | 226247_at | Hs00608662_m1 | -0,10804431 | down |
| **PDE8A** | Phospho- diesterase | 15q25.3 | 212522_at 8A | Hs00400174_m1 | -0,10773687 | down |
| **TCF7** | Transcription factor 7 (T-cell specific, HMG-box) | 5q31.1 | 205255_x_ | Hs00175273_m1 at | -0,09553483 | down |
| **MGC295 06** | Proapoptotic caspase adapter protein precursor | 5q31.2 | 221286_s_ at | Hs00414907_m1 | 0,08887669 | up |
| **MSI2** | Musashi homolog 2 (Drosophila) | 17q22 | 243010_at | Hs00292670_m1 | 0,0805853 | up |

**Table 4: Multivariate analysis in the validation set**

| | ***Overall survival*** | | ***Time to treatment*** | |
|---|---|---|---|---|
| ***Variable*** | ***HR (95% Cl)*** | ***P-Value*** | ***HR (95% CI)*** | ***P-Value*** |
| *Score*¹ | 0.51 (0.37 - 0.71) | 5.2 × 10⁻⁵ | 0.60 (0.46 - 0.79) | 0.0002 |
| *VH unmutated* | 1.64 (0.73 - 3.67) | 0.232 | 1.92 (0.90 - 4.06) | 0.09 |
| *Del. 11q* | 1.35 (0.55 - 3.30) | 0.511 | 1.30 (0.55 - 3.11) | 0.55 |
| *Del. 17p* | 6.75 (2.05 - 22.14) | 0.002 | 2.95 (1.06 - 8.25) | 0.039 |
| *Trisomy 12* | 1.45 (0.39 - 5.33) | 0.576 | 0.87 (0.25 - 2.99) | 0.82 |
| *"Normal" FISH* | 1.00 (0.44 - 2.28) | 0.996 | 1.15 (0.58 - 2.29) | 0.694 |
| *Age ≥ 65* | 6.03 (2.84 - 12.78) | 2.8 × 10⁻⁶ | 1.75 (0.97 - 3.18) | 0.064 |

| | | | | |
|---|---|---|---|---|
| ¹The hazard ratio (HR) is given for a change of the score equal to 1.HR: Hazard ratio; Cl: Confidence interval. Multivariate Cox-regression models with covariates eight gene score, VH-status, FISH aberrations and age (< 65 or ≥ 65) for the prediction of OS and TTT. In case of the eight gene expression score the hazard ratio is given for a change in the score of 1, starting with the lowest scores (worst prognosis). The gene expression score shows highly significant prediction of OS in the multivariate model. Age and 17p-Deletion are also significant predictors of OS. In the multivariate model for the prediction of TTT the gene expression score is dominating all other risk factors. | | | | |

### References:

1. Chiorazzi N, Rai KR, Ferrarini M. Chronic lymphocytic leukemia. N Engl J Med 2005;352:804-15.
2. Hallek M. Prognostic factors in chronic lymphocytic leukemia. Ann Oncol 2008;19 Suppl 4:iv51-3.
3. Hamblin TJ, Davis Z, Gardiner A, Oscier DG, Stevenson FK. Unmutated Ig V(H) genes are associated with a more aggressive form of chronic lymphocytic leukemia. Blood 1999;94:1848-54.
4. Dohner H, Stilgenbauer S, Benner A, et al. Genomic aberrations and survival in chronic lymphocytic leukemia. N Engl J Med 2000;343:1910-6.
5. Gine E, Moreno C, Esteve J, Montserrat E. The role of stem-cell transplantation in chronic lymphocytic leukemia risk-adapted therapy. Best Pract Res Clin Haematol 2007;20:529-43.
6. Gribben JG. Stem cell transplantation in chronic lymphocytic leukemia. Biol Blood Marrow Transplant 2008; 15:53-8.
7. Gribben JG. Stem-cell transplantation in chronic lymphocytic leukaemia. Best Pract Res Clin Haematol 2007;20:513-27.
8. Delgado J, Milligan DW, Dreger P. Allogeneic hematopoietic cell transplantation for chronic lymphocytic leukemia: ready for prime time? Blood 2009;114:2581-8.
9. Friedman DR, Weinberg JB, Barry WT, et al. A genomic approach to improve prognosis and predict therapeutic response in chronic lymphocytic leukemia. Clin Cancer Res 2009;15:6947-55.
10. Metzeler KH, Hummel M, Bloomfield CD, et al. An 86-probe-set gene-expression signature predicts survival in cytogenetically normal acute myeloid leukemia. Blood 2008;112:4193-201.
11. Calin GA, Ferracin M, Cimmino A, et al. A MicroRNA signature associated with prognosis and progression in chronic lymphocytic leukemia. N Engl J Med 2005;353:1793-801.
12. Bullinger L, Dohner K, Bair E, et al. Use of gene-expression profiling to identify prognostic subclasses in adult acute myeloid leukemia. N Engl J Med 2004;350:1605-16.
13. Bhojwani D, Kang H, Menezes RX, et al. Gene expression signatures predictive of early response and outcome in high-risk childhood acute lymphoblastic leukemia: A Children's Oncology Group Study [corrected]. J Clin Oncol 2008;26:4376-84.
14. Rodriguez A, Villuendas R, Yanez L, et al. Molecular heterogeneity in chronic lymphocytic leukemia is dependent on BCR signaling: clinical correlation. Leukemia 2007;21:1984-91.
15. Codony C, Crespo M, Abrisqueta P, Montserrat E, Bosch F. Gene expression profiling in chronic lymphocytic leukaemia. Best Pract Res Clin Haematol 2009;22:211-22.
16. Falt S, Merup M, Gahrton G, Lambert B, Wennborg A. Identification of progression markers in B-CLL by gene expression profiling. Exp Hematol 2005;33:883-93.
17. Huttmann A, Klein-Hitpass L, Thomale J, et al. Gene expression signatures separate B-cell chronic lymphocytic leukaemia prognostic subgroups defined by ZAP-70 and CD38 expression status. Leukemia 2006;20:1774-82.
18. Stamatopoulos B, Haibe-Kains B, Equeter C, et al. Gene expression profiling reveals differences in microenvironment interaction between patients with chronic lymphocytic leukemia expressing high versus low ZAP70 mRNA. Haematologica 2009;94:790-9.
19. Kienle D, Benner A, Laufle C, et al. Gene expression markers for genetic risk groups and survival in cronic lymphocytic leukemia. Haematologica 2009.
20. Shimizu Y, Meunier L, Hendershot LM. pERp1 is significantly up-regulated during plasma cell differentiation and contributes to the oxidative folding of immunoglobulin. Proc Natl Acad Sci U S A 2009;106:17013-8.
21. van Anken E, Pena F, Hafkemeijer N, et al. Efficient IgM assembly and secretion require the plasma cell induced endoplasmic reticulum protein pERp1. Proc Natl Acad Sci U S A 2009;106:17019-24.
22. Hallek M, Cheson BD, Catovsky D, et al. Guidelines for the diagnosis and treatment of chronic lymphocytic leukemia: a report from the International Workshop on Chronic Lymphocytic Leukemia updating the National Cancer Institute-Working Group 1996 guidelines. Blood 2008;111:5446-56.
23. Cheson BD, Bennett JM, Grever M, et al. National Cancer Institute-sponsored Working Group guidelines for chronic lymphocytic leukemia: revised guidelines for diagnosis and treatment. Blood 1996;87:4990-7.
24. Lu D, Zhao Y, Tawatao R, et al. Activation of the Wnt signaling pathway in chronic lymphocytic leukemia. Proc Natl Acad Sci U S A 2004; 101:3118-23.
25. Zhou Q, Chipperfield H, Melton DA, Wong WH. A gene regulatory network in mouse embryonic stem cells. Proc Natl Acad Sci U S A 2007;104:16438-43.
26. Sato N, Meijer L, Skaltsounis L, Greengard P, Brivanlou AH. Maintenance of pluripotency in human and mouse embryonic stem cells through activation of Wnt signaling by a pharmacological GSK-3-specific inhibitor. Nat Med 2004;10:55-63.
27. Ghia P, Chiorazzi N, Stamatopoulos K. Microenvironmental influences in chronic lymphocytic leukaemia: the role of antigen stimulation. J Intern Med 2008;264:549-62.
28. Wu P, Wang P. Per-Arnt-Sim domain-dependent association of cAMP-phosphodiesterase 8A1 with IkappaB proteins. Proc Natl Acad Sci U S A 2004;101:17634-9.
29. van't Veer MB, Brooijmans AM, Langerak AW, et al. The predictive value of lipoprotein lipase for survival in chronic lymphocytic leukemia. Haematologica 2006;91:56-63.
30. White KA, Yore MM, Deng D, Spinella MJ. Limiting effects of RIP140 in estrogen signaling: potential mediation of anti-estrogenic effects of retinoic acid. J Biol Chem 2005;280:7829-35.
31. Siddall NA, McLaughlin EA, Marriner NL, Hime GR. The RNA-binding protein Musashi is required intrinsically to maintain stem cell identity. Proc Natl Acad Sci U S A 2006;103:8402-7.
32. De Weer A, Speleman F, Cauwelier B, et al. EVI1 overexpression in t(3;17) positive myeloid malignancies results from juxtaposition of EVI1 to the MSI2 locus at 17q22. Haematologica 2008;93:1903-7.
33. Barbouti A, Hoglund M, Johansson B, et al. A novel gene, MSI2, encoding a putative RNA-binding protein is recurrently rearranged at disease progression of chronic myeloid leukemia and forms a fusion gene with HOXA9 as a result of the cryptic t(7;17)(p15;q23). Cancer Res 2003;63:1202-6.
34. Ide Y, Miyoshi E, Nakagawa T, et al. Aberrant expression of N-acetylglucosaminyltransferase-IVa and IVb (GnT-IVa and b) in pancreatic cancer. Biochem Biophys Res Commun 2006;341:478-82.
35. Quackenbush J. Microarray data normalization and transformation. Nat Genet 2002;32 Suppl:496-501.
36. Eisenberg E, Levanon EY. Human housekeeping genes are compact. Trends Genet 2003;19:362-5.
37. Velculescu VE, Madden SL, Zhang L, et al. Analysis of human transcriptomes. Nat Genet 1999;23:387-8.
38. Bair E, Tibshirani R. Semi-supervised methods to predict patient survival from gene expression data. PLoS Biol 2004;2:E108.
39. Quackenbush J. Microarray analysis and tumor classification. N Engl J Med 2006;354:2463-72.
40. Fathallah-Shaykh HM. Microarrays: applications and pitfalls. Arch Neurol 2005;62:1669-72.
41. Bowtell DD. Options available--from start to finish--for obtaining expression data by microarray. Nat Genet 1999;21:25-32.
42. Mortazavi A, Williams BA, McCue K, Schaeffer L, Wold B. Mapping and quantifying mammalian transcriptomes by RNA-Seq. Nat Methods 2008;5:621-8.
43. Haferlach T, Kohlmann A, Schnittger S, et al. Global approach to the diagnosis of leukemia using gene expression profiling. Blood 2005; 106:1189-98.
44. Schoch C, Kohlmann A, Schnittger S, et al. Acute myeloid leukemias with reciprocal rearrangements can be distinguished by specific gene expression profiles. Proc Natl Acad Sci U S A 2002;99:10008-13.
45. Irizarry RA, Hobbs B, Collin F, et al. Exploration, normalization, and summaries of high density oligonucleotide array probe level data. Biostatistics 2003;4:249-64.
46. Johnson WE, Li C, Rabinovic A. Adjusting batch effects in microarray expression data using empirical Bayes methods. Biostatistics 2007;8:118-27.
47. Vandesompele J, De Preter K, Pattyn F, et al. Accurate normalization of real-time quantitative RT-PCR data by geometric averaging of multiple internal control genes. Genome Biol 2002;3:RESEARCH0034.
48. Boulesteix AL, Slawski M. Stability and aggregation of ranked gene lists. Brief Bioinform 2009;10:556-68.
49. Bergmann MA, Eichhorst BF, Busch R, et al. Prospective Evaluation of Prognostic Parameters in Early Stage Chronic Lymphocytic Leukemia (CLL): Results of the CLL1-Protocol of the German CLL Study Group (GCLLSG). ASH Annual Meeting Abstracts 2007;110:625-.
50. Gerds TA, Schumacher M. Efron-type measures of prediction error for survival analysis. Biometrics 2007;63:1283-7.

## Claims

1. A kit consisting of or comprising nucleic acid molecules specific for the expression product of
(a) at least four genes selected from the group consisting of (i) to (viii):
(i) NRIP1;
(ii) SFTPB;
(iii) PLEKHA1;
(iv) PDE8A;
(v) TCF7;
(vi) MGC29506;
(vii) MSI2; and
(viii) one gene selected from the group consisting of MGAT4A, NELL2, BCL11B, CD247, SORL1, KLRB1, IL7R, RUNX2, MAL, RASGRF2, TIAM1, PRKCH, FNIP2, GIMAP7, PAG1, ITK, IL2RB, SATB1, PRKCQ, TRAT1, CLEC4A; or
(b) at least two genes selected from the group consisting of (i) to (v):
(i) SFTPB;
(ii) PLEKHA1;
(iii) MGC29506;
(iv) MSI2; and
(v) one gene selected from the group consisting of MGAT4A, NELL2, BCL11 B, CD247, SORL1, KLRB1, IL7R, RUNX2, MAL, RASGRF2, TIAM1, PRKCH, FNIP2, GIMAP7, PAG1, ITK, IL2RB, SATB1, PRKCQ, TRAT1, CLEC4A;
wherein each of the nucleic acid molecules has a length of at least 8 nucleotides.

2. A kit consisting of or comprising
(a) nucleic acid molecules specific for the expression product of at least one reference gene; and
(b) nucleic acid molecules specific for the expression product of
(ba) at least four genes selected from the group consisting of (i) to (viii):
(i) NRIP1;
(ii) SFTPB;
(iii) PLEKHA1;
(iv) PDE8A;
(v) TCF7;
(vi) MGC29506;
(vii) MSI2; and
(viii) one gene selected from the group consisting of MGAT4A, NELL2, BCL11B, CD247, SORL1, KLRB1, IL7R, RUNX2, MAL, RASGRF2, TIAM1, PRKCH, FNIP2, GIMAP7, PAG1, ITK, IL2RB, SATB1, PRKCQ, TRAT1, CLEC4A; or
(bb) at least two genes selected from the group consisting of (i) to (v):
(i) SFTPB;
(ii) PLEKHA1;
(iii) MGC29506;
(iv) MSI2; and
(v) one gene selected from the group consisting of MGAT4A, NELL2, BCL11B, CD247, SORL1, KLRB1, IL7R, RUNX2, MAL, RASGRF2, TIAM1, PRKCH, FNIP2, GIMAP7, PAG1, ITK, IL2RB, SATB1, PRKCQ, TRAT1, CLEC4A
wherein each of the nucleic acid molecules has a length of at least 8 nucleotides.

3. The kit according to claim 1 or 2, wherein the nucleic acid molecules in claim 1(a) and/or (b) and/or claim 2(b) are specific for the expression product of at least eight genes.

4. The kit according to claim 3, wherein the at least eight genes are NRIP1, SFTPB, PLEKHA1, PDE8A, TCF7, MGC29506, MSI2 and MGAT4A.

5. A diagnostic composition consisting of or comprising the kit according to any one of claims 1 to 4.

6. The kit according to any one of claims 1 to 4 for use in the diagnosis of disease progression in a patient suffering from chronic lymphocytic leukemia.

7. The kit according to any one of claims 1 to 4 or 6, wherein the gene-specific nucleic acid molecules are provided on a solid support.

8. A method of diagnosing a patient suffering from chronic lymphocytic leukemia as being a poor prognosis or good prognosis patient comprising
(a) determining in a sample obtained from the patient the expression level of
(ai) at least four genes selected from the group consisting of (i) to (viii):
(i) NRIP1;
(ii) SFTPB;
(iii) PLEKHA1;
(iv) PDE8A;
(v) TCF7;
(vi) MGC29506;
(vii) MSI2; and
(viii) one gene selected from the group consisting of MGAT4A, NELL2, BCL11B, CD247, SORL1, KLRB1, IL7R, RUNX2, MAL, RASGRF2, TIAM1, PRKCH, FNIP2, GIMAP7, PAG1, ITK, IL2RB, SATB1, PRKCQ, TRAT1, CLEC4A; or
(aii) at least two genes selected from the group consisting of (i) to (v):
(i) SFTPB;
(ii) PLEKHA1;
(iii) MGC29506;
(iv) MSI2; and
(v) one gene selected from the group consisting of MGAT4A, NELL2, BCL11B, CD247, SORL1, KLRB1, IL7R, RUNX2, MAL, RASGRF2, TIAM1, PRKCH, FNIP2, GIMAP7, PAG1, ITK, IL2RB, SATB1, PRKCQ, TRAT1, CLEC4A;
(b) normalising the expression levels determined in (a);
(c) multiplying each normalised expression level obtained in (b) with a gene-specific weight as shown in Table 1; and
(d) adding up the individual values obtained for each gene in (c) to derive a prognostic score; wherein a prognostic score below a reference score, said reference score being characteristic of normal disease progression, is indicative of a poor prognosis and a prognostic score significantly above the prognostic score is indicative of a good prognosis.

9. The method of claim 8, wherein in step (a) the expression level of at least eight genes is determined.

10. The method of claim 9, wherein the at least eight genes are NRIP1, SFTPB, PLEKHA1, PDE8A, TCF7, MGC29506, MSI2 and MGAT4A.

11. The method of any one of claims 8 to 10, wherein the normalisation in (b) is against the expression level of at least one reference gene.

12. The method of any one of claims 8 to 11, wherein cDNA is prepared from total RNA extracted from said sample prior to the step of determining expression levels.

13. The method of any one of claims 8 to 12, wherein the expression levels are determined by microarray analysis, PCR, RT-PCR, real time RT-PCR or sequencing analysis.

14. The method of any one of claims 8 to 13, wherein the sample is selected from blood, serum, plasma, lymph nodes, bone marrow or other human tissue.

15. Use of nucleic acid molecules specific for the expression product of
(a) at least four genes selected from the group consisting of (i) to (viii):
(i) NRIP1;
(ii) SFTPB;
(iii) PLEKHA1;
(iv) PDE8A;
(v) TCF7;
(vi) MGC29506;
(vii) MSI2; and
(viii) one gene selected from the group consisting of MGAT4A, NELL2, BCL11 B, CD247, SORL1, KLRB1, IL7R, RUNX2, MAL, RASGRF2, TIAM1, PRKCH, FNIP2, GIMAP7, PAG1, ITK, IL2RB, SATB1, PRKCQ, TRAT1, CLEC4A; or
(b) at least two genes selected from the group consisting of (i) to (v):
(i) SFTPB;
(ii) PLEKHA1;
(iii) MGC29506;
(iv) MSI2; and
(v) one gene selected from the group consisting of MGAT4A, NELL2, BCL11B, CD247, SORL1, KLRB1, IL7R, RUNX2, MAL, RASGRF2, TIAM1, PRKCH, FNIP2, GIMAP7, PAG1, ITK, IL2RB, SATB1, PRKCQ, TRAT1, CLEC4A
wherein each of the nucleic acid molecules has a length of at least 8 nucleotides,
in the diagnosis of disease progression in a patient suffering from chronic lymphocytic leukemia, wherein said diagnosis is not practised on the human or animal body.
